# FASCICULE DE BREVET EUROPEEN

(11) **EP 2 976 317 B1**
(45) Date de publication et mention de la délivrance du brevet: **24.03.2021**
(21) Numéro de dépôt: 14711815.2
(22) Date de dépôt: 21.02.2014
(51) Int. Cl.: C07C 21/18, C01B 7/19, C09K 5/04

(54) **COMPOSITION COMPRENANT HF ET 2,3,3,3-TETRAFLUOROPROPENE**
ZUSAMMENSETZUNG MIT HF UND 2,3,3,3-TETRAFLUORPROPEN
COMPOSITION COMPRISING HF AND 2,3,3,3-TETRAFLUOROPROPENE

(30) Priorité: 20.03.2013 FR 1352482
(43) Date de publication de la demande: 27.01.2016
(73) Titulaire: Arkema France, 92700 Colombes (FR)
(72) Inventeur: BONNET, Philippe, F-69007 Lyon (FR); COLLIER, Bertrand, F-69230 Saint-genis-laval (FR); DEUR-BERT, Dominique, F-69390 Charly (FR); WENDLINGER, Laurent, F-69510 Soucieu En Jarrest (FR)
(74) Mandataire: Arkema Patent
(86) Numéro de dépôt international: PCT/FR2014/050357
(87) Numéro de publication internationale: WO 2014/147310

(56) Documents cités:
- WO-A1-2008/002500
- WO-A1-2010/059493
- WO-A2-2007/053736

## Description

La présente invention concerne des compositions azéotropiques ou quasi-azéotropiques comprenant du 2,3,3,3-tetrafluoropropène et du fluorure d'hydrogène. Ces compositions peuvent provenir de la production du 2,3,3,3-tetrafluoropropène et sont généralement utiles dans des procédés de recyclage de fluorure d'hydrogène.

La fabrication du 2,3,3,3-tetrafluoropropène s'accompagnant d'une multitude de sous-produits, ayant un point d'ébullition proche du HFO-1234yf, conduit à des étapes de purification assez complexes et coûteuses. La difficulté rencontrée au cours de la purification du HFO-1234yf implique généralement une perte conséquente en produit recherché. De plus, ces sous-produits peuvent former des compositions azéotropiques avec le 2,3,3,3-tetrafluoropropène, rendant la séparation par distillation simple, très difficile, voir impossible.

Les azéotropes binaires HF-HFO-1234yf (WO2007/053736), HF-HFO-1234zeE (US2007/0100173), HF-HFO-1234zeZ (WO2008/002500), HF-HCFO-1233xf (WO2008/054781, US2009/0224207), HF-HFO-1243zf (WO2009/105517), HF-HFCFO-1233zdZ (WO2012/075283) et HF-HFC-245cb (WO2008/054781) ne sont pas homogènes mais hétérogènes et sont donc tous des hétéro-azéotropes. Le document D2 décrit une distillation azéotropique dans laquelle le distillat est une composition azéotropique de HF et un composé organique.

La présente invention a pour objet une composition hétéro-azéotropique ou quasi-hétéro-azéotropique comprenant du fluorure d'hydrogène, du 2,3,3,3-tetrafluoropropene, et un ou plusieurs composé(s) (hydro)halogénocarboné(s) comprenant entre 1 et 3 atome(s) de carbone choisi(s) parmi le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le 1,1,1,3,3-pentafluoropropane, le 2-chloro,1,1,1,2-tetrafluoropropane, le trifluoropropyne, le Z-1,1,1,2,3-pentafluoropropène et le 1,1,1,3,3-pentafluoropropène.

Un mélange hétéro-azéotropique ou quasi-hétéro-azéotropique est un mélange azéotropique ou quasi-azéotropique dont le liquide condensé forme deux solutions non miscibles qui peuvent être facilement séparées, par exemple par décantation. Cette propriété est un avantage considérable pour la récupération d'HF.

L'expression « quasi-azéotropique » ou « quasi-hétéro-azéotropique » a un sens large et est destinée à inclure les compositions qui sont strictement azéotropique ou strictement hétéro-azéotropique et celles qui se comportent comme un mélange azéotropique ou hétéro-azéotropique.

Un mélange est azéotropique lorsque que la pression au point de rosée est égale à celle au point de formation de bulle, ce qui signifie que la composition de vapeur est égale à celle du liquide condensée.

Un mélange est considéré comme quasi-azéotropique lorsque que la pression au point de rosée est substantiellement égale à celle au point de formation de bulle, ce qui signifie que la composition de vapeur est substantiellement égale à celle du liquide condensée.

Une autre manière de caractériser un mélange comme quasi-azéotropique lorsque la différence de pression entre la pression au point de rosée et la pression au point de formation de bulles est faible, préférentiellement inférieure ou égale à 5 %, sur la base de la pression au point de formation de bulles.

Les compositions selon l'invention concernent notamment les composés suivants dont les acronymes représentent :
- HF : fluorure d'hydrogène
- HCC-40: chlorométhane, ou CH₃Cl
- HCFC-115: chloropentafluoroéthane, ou C₂F₅Cl
- HCFC-124: chlorotétrafluoroéthane, ou C₂HF₄Cl
- HFC-125: pentafluoroéthane, ou C₂HF₅
- HCFC-133a: 1-chloro-2,2,2-trifluoroéthane, ou C₂H₂F₃Cl
- HFC-134a: 1,1,1,2-tétrafluoroéthane, ou C₂H₂F₄
- HCFC-142b: 1-chloro-1,1-difluoroéthane, ou C₂H₃F₂Cl
- HFC-143a : 1,1,1-trifluoroéthane, ou C₂H₃F₃
- HFC-152a : 1,1-difluoroéthane, ou C₂H₄F₂
- HFO-1132 : 1,2-difluoroéthylène, ou C₂H₂F₂
- HFO-1141 : fluoroéthylène, ou C₂H₃F
- HFO-1234yf : 2,3,3,3-tetrafluoropropene ou CH₂=CF-CF₃
- HFC-245cb : 1,1,1,2,2-pentafluoropropane ou CF₃-CF₂-CH₃
- HFO-1234zeE : E-1,3,3,3-tetrafluoropropene ou E-CF₃-CH=CHF
- HFO-1234zeZ : Z-1,3,3,3-tetrafluoropropene ou Z-CF₃-CH=CHF
- HFO-1243zf : 3,3,3-trifluoropropene ou CF₃-CH=CH₂
- HCFO-1233xf : 3,3,3-trifluoro-2-chloropropene ou CF₃-CCl=CH₂
- HCFO-1233zdE : E-3,3,3-trifluoro-1-chloropropene ou E-CF₃-CH=CHCl
- HCFO-1233zdZ : Z-3,3,3-trifluoro-1-chloropropene ou Z-CF₃-CH=CHCl
- HFO-1225yeZ : Z-1,1,1,2,3-pentafluoropropene ou Z-CHF=CF-CF₃
- HFO-1225yeE : E-1,1,1,2,3-pentafluoropropene ou E-CHF=CF-CF₃
- HFO-1225zc : 1,1,3,3,3-pentafluoropropene ou CF₂=CH-CF₃
- HFO-1225yc : 1,1,2,3,3-pentafluoropropene ou CF₂=CF-CF₂
- HCFC-1214 : dichlorotetrafluoropropène, ou C₃F₄Cl₂
- HCFO-1215 : chloropentafluoropropène, ou C₃F₅Cl
- HFO-1216 : hexafluoropropène, ou C₃F₆
- HCFO-1223 : dichlorotrifluoropropène, ou C₃HF₃Cl2
- HCFO-1224 : chlorotetrafluoropropène, ou C₃HF₄Cl
- HCFO-1232 : dichlorodifluoropropène, ou C₃H₂F₂Cl₂
- HCFO-1233xc: 1,1,3-trifluoro-2-chloropropene ou CH₂F-CCl=CF₂
- HCFO-1233xe : 1,3,3-trifluoro-2-chloropropene ou CHF₂-CCl=CHF
- HCFO-1233yb : 1,2,3-trifluoro-1-chloropropene ou CH₂F-CF=CFCl
- HCFO-1233yc: 1,1,2-trifluoro-3-chloropropene ou CH₂Cl-CF=CF₂
- HCFO-1233yd : 2,3,3-trifluoro-1-chloropropene ou CHF₂-CF=CHCl
- HCFO-1233ye : 1,2,3-trifluoro-3-chloropropene ou CHClF-CF=CHF
- HCFO-1233yf : 2,3,3-trifluoro-3-chloropropene ou CClF₂-CF=CH₂
- HCFO-1233zb : 1,3,3-trifluoro-1-chloropropene ou CHF₂-CH=CFCl
- HCFO-1233zc: 1,1,3-trifluoro-3-chloropropene ou CHClF-CH=CF₂
- HCFO-1233ze : 1,3,3-trifluoro-3-chloropropene ou CClF₂-CH=CHF
- HFO-1234yc : 1,1,2,3-tetrafluoropropene ou CF₂=CF-CH₂F
- HFO-1234ye : 1,2,3,3-tetrafluoropropene ou CHF=CF-CHF₂
- HFO-1234zc: 1,1,3,3-tetrafluoropropene ou CF₂=CH-CHF₂
- HCFO-1242 : chlorodifluoropropène, ou C₃H₃F₂Cl
- HFO-1243yc : 1,1,2-trifluoropropene ou CH₃-CF=CF₂
- HFO-1243ye : 1,2,3-trifluoropropene ou CH₂F-CF=CHF
- HFO-1243yf : 2,3,3-trifluoropropene ou CHF₂-CF=CH₂
- HFO-1243zc : 1,1,3-trifluoropropene ou CH₂F-CH=CF₂
- HFO-1243ze : 1,3,3-trifluoropropene ou CHF₂-CH=CHF
- HCFO-1251 : chlorofluoropropène, ou C₃H₄FCl
- HFO-1252 : difluoropropène, ou C₃H₄F₂
- HFO-216 : hexafluoropropene, ou C₃F₆Cl₂
- HCFO-217 : chloroheptafluoropropane, ou C₃F₇Cl
- HFC-218 : octafluoropropane, ou C₃F₈
- HCFC-225 : dichloropentafluoropropane, ou C₃HF₅Cl₂
- HCFC-226 : chlorohexafluoropropane, ou C₃HF₆Cl
- HFC-227: heptafluoropropane, ou C₃HF₇
- HCFC-234 : dichlorotétrafluoropropane, ou C₃H₂F₄Cl₂
- HCFC-235 : chloropentafluoropropane, ou C₃H₂F₅Cl
- HFC-236 : hexafluoropropane, ou C₃H₂F₆
- HCFC-243 : dichlorotrifluoropropane, ou C₃H₃F₃Cl₂
- HCFC-244 : chlorotétrafluoropropane, ou C₃H₃F₄Cl
- HCFC-244bb : 2-chloro, 1,1,1,2-tetrafluoropropane ou CF₃-CFCl-CH₃
- HFC-245fa : 1,1,1,3,3-pentafluoropropane ou CF₃-CH₂-CHF₂
- HFC-245ea : 1,1,2,3,3-pentafluoropropane ou CHF₂-CHF-CHF₂
- HFC-245eb : 1,1,1,2,3-pentafluoropropane ou CF₃-CHF-CH₂F
- HFC-245ca : 1,1,2,2,3-pentafluoropropane ou CHF₂-CF₂-CH₂F
- HCFC-253 : chlorotrifluoropropane, ou C₃H₄F₃Cl
- HFC-254 : tétrafluoropropane, ou C₃H₄F₄
- HCFC-262 : Chlorodifluoropropane, ou C₃H₅F₂Cl
- HFC-263 : trifluoropropane, ou C₃H₅F₃
- Trifluoropropyne : CF₃-C≡CH

La composition selon l'invention peut éventuellement être un mélange d'un ou de plusieurs azéotropes et/ou hétéro-azéotropes de systèmes ternaires, quaternaires, pentanaires, système à six composés, système à sept composés, système à huit composés ou supérieur.

Le composé 1,3,3,3-tetrafluoropropene comprend soit le composé E-1,3,3,3-tetrafluoropropene soit le composé Z-1,3,3,3-tetrafluoropropene soit le mélange des composés E-1,3,3,3-tetrafluoropropene et Z-1,3,3,3-tetrafluoropropene.

Le composé 1,1,1,2,3-pentafluoropropene comprend soit le composé E-1,1,1,2,3-pentafluoropropene soit le composé Z-1,1,1,2,3-pentafluoropropene soit le mélange des composés E-1,1,1,2,3-pentafluoropropene et Z-1,1,1,2,3-pentafluoropropene.

Le(s) composé(s) à 1 et/ou 2 atome(s) de carbone peut/peuvent être notamment choisi(s) parmi le chlorométhane, le chloropentafluoroéthane, le 1-chloro-1,2,2,2-tétrafluoroéthane, le 1-chloro-1,1,2,2-tétrafluoroéthane, le pentafluoroéthane, le 1-chloro-1,2,2-trifluoroéthane, le 1-chloro-2,2,2-trifluoroéthane, le 1,1,2,2-tétrafluoroéthane, le 1,1,1,2-tétrafluoroéthane, le 1-chloro-1,2-difluoroéthane, le 1-chloro-1,1-difluoroéthane, le 1,1,2-trifluoroéthane, le 1,1,1-trifluoroéthane, le 1,1,2-trifluoroéthane, le 1,1-difluoroéthane, le 1,2-difluoroéthylène et le fluoroéthylène.

Le(s) composé(s) ayant 3 atomes de carbone peut/peuvent être notamment choisi(s) parmi le 1,2-dichloro-1,1,2,3,3,3-hexafluoropropane, le 1,3-dichloro-1,1,2,2,3,3-hexafluoropropane, le 1,1-dichloro-1,2,2,3,3,3-hexafluoropropane, le 2,2-dichloro-1,1,1,3,3,3-hexafluoropropane, le 1-chloro-1,1,2,2,3,3,3-heptafluoropropane, le 2-chloro-1,1,1,2,3,3,3-heptafluoropropane, octafluoropropane, le dichloropentafluoropropane, le 2,2-dichloro-1,1,1,3,3-pentafluoropropane, le 2,3-dichloro-1,1,1,2,3-pentafluoropropane, le 1,2-dichloro-1,1,2,3,3-pentafluoropropane, le 3,3-dichloro-1,1,1,2,2-pentafluoropropane, le 1,3-dichloro-1,1,2,2,3-pentafluoropropane, le 1,1-dichloro-1,2,2,3,3-pentafluoropropane, le 1,2-dichloro-1,1,3,3,3-pentafluoropropane, le 1,3-dichloro-1,1,2,3,3-pentafluoropropane, le 1,1-dichloro-1,2,3,3,3-pentafluoropropane, chlorohexafluoropropane, le 2-chloro-1,1,1,2,3,3-hexafluoropropane, le 3-chloro-1,1,1,2,2,3-hexafluoropropane, le 1-chloro-1,1,2,2,3,3-hexafluoropropane, le 2-chloro-1,1,1,3,3,3-hexafluoropropane, le 1-chloro-1,1,2,3,3,3-hexafluoropropane, 1,1,2,2,3,3,3-heptafluoropropane, le 1,1,1,2,3,3,3-Heptafluoropropane, dichlorotétrafluoropropane, le 2,2-dichloro-1,1,3,3-tétrafluoropropane, le 2,2-dichloro-1,1,1,3-tétrafluoropropane, le 1,2-dichloro-1,2,3,3-tétrafluoropropane, le 2,3-dichloro-1,1,1,2-tétrafluoropropane, le 1,2-dichloro-1,1,2,3-tétrafluoropropane, le 1,3-dichloro-1,2,2,3-tétrafluoropropane, le 1,1-dichloro-2,2,3,3-tétrafluoropropane, le 1,3-dichloro-1,1,2,2-tétrafluoropropane, le 1,1-dichloro-1,2,2,3-tétrafluoropropane, le 2,3-dichloro-1,1,1,3-tétrafluoropropane, le 1,3-dichloro-1,1,3,3-tétrafluoropropane, le 1,3-dichloro-1,1,3,3-tétrafluoropropane, le 1,1-dichloro-1,3,3,3-tétrafluoropropane, le 1,1-dichloro-2,3,3,3-tétrafluoropropane, le 1,3-dichloro-1,1,2,3-tétrafluoropropane, le 1,1-dichloro-1,2,3,3-tétrafluoropropane le chloropentafluoropropane, le 1-chloro-1,2,2,3,3-pentafluoropropane, le 3-chloro-1,1,1,2,3-pentafluoropropane, le 1-chloro-1,1,2,2,3-pentafluoropropane, le 2-chloro-1,1,1,3,3-pentafluoropropane, le 1-chloro-1,1,3,3,3-pentafluoropropane, le 1-chloro-1,1,2,3,3-pentafluoropropane, le 3-chloro-1,1,1,2,2-pentafluoropropane, le 2-chloro-1,1,2,3,3-pentafluoropropane, le 2-chloro-1,1,1,2,3-pentafluoropropane,le 1,1,1,2,2,3-hexafluoropropane, le 1,1,1,2,3,3-hexafluoropropane, le 1,1,1,3,3,3-hexafluoropropane, le 1,1,2,2,3,3-hexafluoropropane, le dichlorotrifluoropropane, le 1,1-dichloro-3,3,3-trifluoropropane, le 1,3-dichloro-1,1,3-trifluoropropane, le 1,1-dichloro-1,3,3-trifluoropropane, le 1,3-dichloro-1,2,3-trifluoropropane, le 1,1-dichloro-2,3,3-trifluoropropane, le 1,3-dichloro-1,1,2-trifluoropropane, le 1,1-dichloro-1,2,3-trifluoropropane, le 1,2-dichloro-1,3,3-trifluoropropane, le 2,3-dichloro-1,1,1-trifluoropropane, le 1,2-dichloro-1,1,3-trifluoropropane, le 1,3-dichloro-1,2,2-trifluoropropane, le 1,1-dichloro-2,2,3-trifluoropropane, le 1,1-dichloro-1,2,2-trifluoropropane, le 2,3-dichloro-1,1,2-trifluoropropane, le 1,2-dichloro-1,2,3-trifluoropropane, le 1,2-dichloro-1,1,2-trifluoropropane, le 2,2-dichloro-1,1,3-trifluoropropane, le 2,2-dichloro-3,3,3-trifluoropropane, le chlorotétrafluoropropane, le 2-chloro-1,2,3,3-tétrafluoropropane, le 2-chloro-1,1,1,2-tétrafluoropropane, le 3-chloro-1,1,2,2-tétrafluoropropane, le 1-chloro-1,2,2,3-tétrafluoropropane, le 1-chloro-1,1,2,2-tétrafluoropropane, le 2-chloro-1,1,3,3-tétrafluoropropane, le 2-chloro-1,1,1,3-tétrafluoropropane, le 3-chloro-1,1,2,3-tétrafluoropropane, le 3-chloro-1,1,1,2-tétrafluoropropane, le 1-chloro-1,1,2,3-tétrafluoropropane, le 3-chloro-1,1,1,3-tétrafluoropropane, le 1-chloro-1,1,3,3-tétrafluoropropane, le pentafluoropropane, le 1,1,2,2,3-pentafluoropropane, le 1,1,2,3,3-pentafluoropropane, le 1,1,1,2,3-pentafluoropropane, le 1,1,1,3,3-pentafluoropropane, le chlorotrifluoropropane, le 2-chloro-1,2,3-trifluoropropane, le 2-chloro-1,1,2-trifluoropropane, le 1-chloro-2,2,3-trifluoropropane, le 1-chloro-1,2,2-trifluoropropane, le 3-chloro-1,1,2-trifluoropropane, le 1-chloro-1,2,3-trifluoropropane, le 1-chloro-1,1,2-trifluoropropane, le 3-chloro-1,3,3-trifluoropropane, le 3-chloro-1,1,1-trifluoropropane, le 1-chloro-1,1,3-trifluoropropane, le 2-chloro-1,1,3-trifluoropropane, le 2-chloro-1,1,1-trifluoropropane, le 1,1,2,2-tétrafluoropropane, le 1,1,1,3-tétrafluoropropane, le 1,1,2,3-tétrafluoropropane, le 1,1,1,2-tétrafluoropropane, le 1,2,2,3-tétrafluoropropane, le 1,1,3,3-tétrafluoropropane,le chlorodifluoropropane, le 1-chloro-2,2-difluoropropane, le 3-chloro-1,1-difluoropropane, le 1-chloro-1,3-difluoropropane, le 1-chloro-1,1-difluoropropane, le 1-chloro-2,3-difluoropropane, le 1-chloro-1,2-difluoropropane, le 2-chloro-1,3-difluoropropane, le 2-chloro-1,1-difluoropropane, le 2-chloro-1,2-difluoropropane, le trifluoropropane, le 1,1,1-trifluoropropane, le 1,1,3-trifluoropropane, le 1,2,3-trifluoropropane, le 1,1,2-trifluoropropane, le 1,2,2-trifluoropropane, le dichlorotetrafluoropropène le 1,2-dichloro-1,3,3,3-tetrafluoropropène, le 1,1-dichloro-2,3,3,3tetrafluoropropène, le 1,3-dichloro-1,2,3,3tetrafluoropropène, le 2,3-dichloro-1,1,3,3-tetrafluoropropène, le 3,3-dichloro-1,1,2,3tetrafluoropropène, le chloropentafluoropropène, le 1-chloropentafluoropropène, le 2-chloropentafluoropropène, le 3-chloropentafluoropropène, le hexafluoropropène, dichlorotrifluoropropène, le 1,1-dichloro-3,3,3-trifluoropropène, le 1,2-dichloro-3,3,3-trifluoropropène, le 2,3-dichloro-1,3,3-trifluoropropène, le 1,3-dichloro-2,3,3-trifluoropropène, le 1,2-dichloro-1,3,3-trifluoropropène, le 2,3-dichloro-1,1,3-trifluoropropène, le 1,1-dichloro-2,3,3-trifluoropropène, le 1,3-dichloro-1,2,3-trifluoropropène, le 3,3-dichloro-1,1,2-trifluoropropène, le 3,3-dichloro-1,2,3-trifluoropropène, le 1,3-dichloro-1,3,3-trifluoropropène, le 3,3-dichloro-1,1,3-trifluoropropène,le 1-chloro-2,3,3,3-tetrafluoropropène, le 1-chloro-1,3,3,3-tetrafluoropropène, le 2-chloro-1,3,3,3-tetrafluoropropène, le 3-chloro-1,2,3,3-tetrafluoropropène, le 3-chloro-1,1,3,3-tetrafluoropropène, le 2-chloro-1,1,3,3-tetrafluoropropène, le 1-chloro-1,2,3,3-tetrafluoropropène, le 3-chloro-1,1,2,3-tetrafluoropropène, le 1,2,3,3,3-pentafluoropropène, le 1,1,3,3,3-pentafluoropropène, le 1,1,2,3,3-pentafluoropropène, le dichlorodifluoropropène, le 2,3-dichloro-3,3-difluoropropène, le 1,2-dichloro-1,3-difluoropropène, le 2,3-dichloro-1,1-difluoropropène, le 1,2-dichloro-3,3-difluoropropène, le 2,3-dichloro-1,3-difluoropropène, le 1,1-dichloro-2,3-difluoropropène, le 1,3-dichloro-1,2-difluoropropène, le 1,3-dichloro-2,3-difluoropropène, le 3,3-dichloro-1,2-difluoropropène, le 3,3-dichloro-2,3-difluoropropène, le 1,1-dichloro-3,3-difluoropropène, le 1,3-dichloro-1,3-difluoropropène, le 3,3-dichloro-1,1-difluoropropène, le 1,3-dichloro-3,3-difluoropropène, le 3,3-dichloro-1,3-difluoropropène, le chlorotrifluoropropene, le 2-chloro-1,1,3-trifluoropropène, le 2-chloro-1,3,3-trifluoropropène, le 1-chloro-1,2,3-trifluoropropène, le 3-chloro-1,1,2-trifluoropropène, le 1-chloro-2,3,3-trifluoropropène, le 3-chloro-1,2,3-trifluoropropène, le 3-chloro-2,3,3-trifluoropropène, le 1-chloro-1,3,3-trifluoropropène, le 3-chloro-1,1,3-trifluoropropène, le 3-chloro-1,3,3-trifluoropropène, le 1,1,2,3-tetrafluoropropene le 1,2,3,3-tetrafluoropropene, le 1,1,3,3-tetrafluoropropene, le chlorodifluoropropene, le 3-chloro-3,3-difluoropropène, le 3-chloro-1,3-difluoropropène, le 2-chloro-1,1-difluoropropène, le 2-chloro-1,3-difluoropropène, le 2-chloro-3,3-difluoropropène, le 1-chloro-1,2-difluoropropène, le 1-chloro-2,3-difluoropropène, le 3-chloro-1,2-difluoropropène, le 3-chloro-2,3-difluoropropène, le 1-chloro-1,3-difluoropropène, le 3-chloro-1,1-difluoropropène, le 1-chloro-3,3-difluoropropène, le trifluoropropene, le 1,1,2-trifluoropropene, le 1,2,3-trifluoropropene, le 2,3,3-trifluoropropene, le 1,1,3-trifluoropropene, le 1,3,3-trifluoropropene, le chlorofluoropropène, le 1-chloro-3-fluoropropène, le 1-chloro-1-fluoropropène, le 1-chloro-2-fluoropropène, le 2-chloro-1-fluoropropène, le 2-chloro-3-fluoropropène, le 3-chloro-2-fluoropropène, le 3-chloro-1-fluoropropène, le 3-chloro-3-fluoropropène, le difluoropropène, le 1,2-difluoropropène, le 2,3-difluoropropène, le 1,1-difluoropropène, le 1,3-difluoropropène, le 3,3-difluoropropène, le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le Z-3,3,3-trifluoro-1-chloropropene trifluoropropyne.

La présente invention a également pour objet une composition azéotropique ou quasi-azéotropique comprenant du fluorure d'hydrogène, du 2,3,3,3-tetrafluoropropene, et un ou plusieurs composé(s) choisi(s) parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

La présente invention a en outre pour objet une composition azéotropique ou quasi- azéotropique comprenant du fluorure d'hydrogène, du 2,3,3,3-tetrafluoropropene, et un ou plusieurs composé(s) choisi(s) parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene.

La présente invention a pour objet une composition azéotropique ou quasi-azéotropique comprenant du fluorure d'hydrogène, 2,3,3,3-tetrafluoropropene , et un ou plusieurs composé(s) choisi(s) parmi le E-1,3,3,3-tetrafluoropropene, le Z-1,3,3,3-tetrafluoropropene, le 3,3,3-trifluoropropene, le 3,3,3-trifluoro-2-chloropropene, le E-3,3,3- trifluoro-1-chloropropene.

Selon un mode de réalisation de l'invention, la composition comprend du fluorure d'hydrogène, du 2,3,3,3-tetrafluoropropene, du 1,1,1,2,2-pentafluoropropane et un ou plusieurs composé(s) choisi(s) parmi le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Selon un autre mode de réalisation de l'invention, la composition comprend du fluorure d'hydrogène, du 2,3,3,3-tetrafluoropropene, du E-1,3,3,3-tetrafluoropropene et éventuellement un ou plusieurs composé(s) choisi(s) parmi le Z-1,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro,1,1,1,2-tetrafluoropropane.

Selon un autre mode de réalisation de l'invention, la composition comprend du fluorure d'hydrogène, du 2,3,3,3-tetrafluoropropene, du 2-chloro,1,1,1,2-tetrafluoropropane et éventuellement un ou plusieurs composé(s) choisi(s) parmi le Z-1,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène et le 1,1,1,2,3-pentafluoropropène.

Dans un mode de mise œuvre, la composition comprend du fluorure d'hydrogène, du 2,3,3,3-tetrafluoropropene, du Z-1,3,3,3-tetrafluoropropene et éventuellement un ou plusieurs composé(s) choisi(s) parmi le 3,3,3-trifluoropropene, le 3,3,3-trifluoro-2-chloropropene, le E-3,3,3-trifluoro-1-chloropropene. le trifluoropropyne, le 1,1,1,3,3-pentafluoropropane, le 1,1,1,3,3-pentafluoropropène, et le 1,1,1,2,3-pentafluoropropène.

Dans un autre mode de mise œuvre, la composition comprend du fluorure d'hydrogène, du 2,3,3,3-tetrafluoropropene, du 3,3,3-trifluoropropene et éventuellement un ou plusieurs composé(s) choisi(s) parmi le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, et le 1,1,1,2,3-pentafluoropropène.

Selon un mode de réalisation de l'invention, la composition comprend du fluorure d'hydrogène, du 2,3,3,3-tetrafluoropropene, du 3,3,3-trifluoro-2-chloropropene et éventuellement un ou plusieurs composé(s) choisi(s) parmi le E-3,3,3-trifluoro-1-chloropropene, le trifluoropropyne, le 1,1,1,3,3-pentafluoropropane, le 1,1,1,3,3-pentafluoropropène et le 1,1,1,2,3-pentafluoropropène.

Selon un autre mode de réalisation de l'invention, la composition comprend du fluorure d'hydrogène, du 2,3,3,3-tetrafluoropropene et du E-3,3,3-trifluoro-1-chloropropene et éventuellement un ou plusieurs composé(s) choisi(s) parmi le trifluoropropyne, le 1,1,1,3,3-pentafluoropropane, le 1,1,1,3,3-pentafluoropropène et le 1,1,1,2,3-pentafluoropropène.

Selon un autre mode de réalisation de l'invention, la composition comprend du fluorure d'hydrogène, du 2,3,3,3-tetrafluoropropene et du trifluoropropyne et éventuellement un ou plusieurs composé(s) choisi(s) parmi le 1,1,1,3,3-pentafluoropropane, le 1,1,1,3,3-pentafluoropropène et le 1,1,1,2,3-pentafluoropropène.

Selon un autre mode de réalisation de l'invention, la composition comprend du fluorure d'hydrogène, du 2,3,3,3-tetrafluoropropene et du 1,1,1,3,3-pentafluoropropane et éventuellement un ou plusieurs composé(s) choisi(s) parmi le 1,1,1,3,3-pentafluoropropène et le 1,1,1,2,3-pentafluoropropène.

Selon un autre mode de réalisation de l'invention, la composition comprend du fluorure d'hydrogène, du 2,3,3,3-tetrafluoropropene, du 1,1,1,3,3-pentafluoropropène et éventuellement et le 1,1,1,2,3-pentafluoropropène.

Selon un autre mode de réalisation de l'invention, la composition comprend du fluorure d'hydrogène, du 2,3,3,3-tetrafluoropropene et du 1,1,1,2,3-pentafluoropropène.

Quelque soit le mode de mise en œuvre, la composition comprend de préférence de 1 à 85 %, et avantageusement de 5 à 80 % en poids de fluorure d'hydrogène et de 99 à 15 %, et avantageusement de 20 à 95% en poids de la somme des composés organiques, plus particulièrement la composition comprend de 1 à 85 % en poids de fluorure d'hydrogène et de 99 à 15 % en poids de la somme des composés organiques (HFO-1234yf et les composés (hydro)halogénocarbonés).

Quelque soit le mode de réalisation, le point d'ébullition de la composition selon l'invention est compris entre -20 °C et 80 °C, et à une pression comprise entre 0,1 et 44 bars absolue, préférentiellement compris entre 0 °C et 40 °C et préférentiellement à une pression comprise entre 0,7 et 18 bars absolue, avantageusement entre 0,9 et 12,5 bars absolue.

Lorsque le composé HFC-245cb ou HCFO-1232xf est présent, on préfère des compositions quaternaires et supérieures.

La demanderesse a découvert que les compositions selon l'invention présentent des propriétés intéressantes en particulier pour le recyclage de l'HF à l'étape réactionnelle. Ainsi, la phase condensée de ces compositions, éventuellement lorsqu'elles sont soumises à une étape de distillation et/ou une étape de séparation liquide/liquide, telle que par décantation, forment deux phases liquides non miscibles.

A titre d'exemple, pour les composés ternaires contenant du fluorure d'hydrogène, du 2,3,3,3-tetrafluoropropene et un composé choisi parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, l'apparition d'un hétéro-azéotrope caractérisé par deux phases liquides, l'une riche en HF et l'autre appauvrie en HF dépend de la quantité d'HF dans la composition. Ces plages de décantation en fonction de la teneur en HF dans les compositions ont été caractérisées pour au moins des isothermes à 0 °C, 25 °C et 40 °C.

De même, les plages de décantation pour les composés ternaires contenant du fluorure d'hydrogène, du 2,3,3,3-tetrafluoropropene et un composé choisi parmi le trifluoropropyne, le 1,1,1,3,3-pentafluoropropane, le 1,1,1,3,3-pentafluoropropène, le Z-1,1,1,2,3-pentafluoropropène et le 2-chloro,1,1,1,2-tetrafluoropropane sont caractérisées par une phase appauvrie en HF et une phase enrichie en HF pour au moins des isothermes à 0 °C, 25 °C et 40 °C.

La demanderesse a observé le même phénomène pour des compositions de fluorure d'hydrogène, 2,3,3,3-tetrafluoropropene comprenant plusieurs composés choisis parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Une composition azéotropique ou quasi-azéotropique préférée selon l'invention comprend de 1 à 85 % en poids de fluorure d'hydrogène, et de 99 à 15 % en poids de la somme de HFO-1234yf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de 1 à 85% en poids de fluorure d'hydrogène, et de 99 à 15% en poids de la somme de HFO-1234yf, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80% en poids de fluorure d'hydrogène, et de 95 à 20% en poids de la somme de HFO-1234yf, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFC-245cb et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFC-245cb et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233xf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233xf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HFO-1225yeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HFO-1225zc, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HFC-245fa, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de HCFC-244bb, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1225yeZ et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1225yeZ et de trifluoropropyne, le point d'ébullition de cette composition préférée est compris entre 0 et 40 °C à une pression comprise entre 0,9 et 18.0 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFC-244bb et de trifluoropropyne, le point d'ébullition de cette composition préférée est compris entre 0 et 40 °C à une pression comprise entre 0.7 et 18.0 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFC-244bb et de trifluoropropyne, le point d'ébullition de cette composition préférée est compris entre 0 et 40 °C à une pression comprise 0.7 et 18.0 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFC-244bb et de HFC-245fa, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFC-244bb et de HFC-245fa, le point d'ébullition de cette composition préférée est compris entre 0 et 40 °C à une pression comprise entre 0.7 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1233xf et de HCFC-244bb, le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1243zf et de HCFO-1233zdE, le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HCFO-1233xf et de HFC-245cb le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HCFO-1233xf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeE, de HCFO-1233xf et de HFC-245cb le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeE, de HCFO-1233xf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HCFO-1233xf et de HFC-245cb le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HCFO-1233xf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1243zf, de HCFO-1233xf et de HFC-245cb le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1243zf, de HCFO-1233xf et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ, de HCFO-1233xf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ, de HCFO-1233xf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1243zf, de HCFO-1233xf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1243zf, de HCFO-1233xf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HCFO-1233xf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HCFO-1233xf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1243zf, de HCFO-1233xf et de HFO-1234zeZ le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1243zf, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HCFO-1233xf et de HFO-1234zeZ le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HCFO-1233xf et de HFO-1243zf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HFO-1234zeZ et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HFO-1234zeZ et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HFO-1243zf et de HFO-1234zeZ le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HFO-1243zf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1243zf, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1243zf, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb et de HCFO-1233zdE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb et de HFO-1243zf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HFC-245cb et de HFO-1243zf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233xf, de HFC-245cb et de HCFC-244bb le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233xf, de HFC-245cb et de HCFC-244bb, le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeE, de HFO-1234zeZ, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeE, de HFO-1234zeZ, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeE, de HCFO-1233zdE, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeE, de HCFO-1233zdE, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeE, de HFO-1243zf, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeE, de HFO-1243zf, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HFO-1234zeZ, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HFO-1234zeZ, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1243zf, de HFO-1234zeZ, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1243zf, de HFO-1234zeZ, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1243zf, de HCFO-1233zdE, de HFC-245cb et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1243zf, de HCFO-1233zdE, de HFC-245cb et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE, de HFO-1234zeZ, de HCFO-1233zdE et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE, de HFO-1234zeZ, de HCFO-1233zdE et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE, de HFO-1234zeZ, de HFO-1243zf et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE, de HFO-1234zeZ, de HFO-1243zf et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeE, de HCFO-1233zdE, de HFO-1243zf et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeE, de HCFO-1233zdE, de HFO-1243zf et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ, de HCFO-1233zdE, de HFO-1243zf et de HCFO-1233xf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ, de HCFO-1233zdE, de HFO-1243zf et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HCFO-1233zdE, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HCFO-1233zdE, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFO-1243zf, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFO-1243zf, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HFO-1243zf, de HFC-245cb et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HFO-1243zf, de HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HFO-1243zf, de HFC-245cb et de HFO-1234zeZ le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HFO-1243zf, de HFC-245cb et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HCFO-1233zdE, de HFO-1243zf, de HFO-1234zeZ et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HCFO-1233zdE, de HFO-1243zf, de HFO-1234zeZ et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HCFO-1233xf, de HFC-245cb, de HFO-1234zeZ et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HCFO-1233xf, de HFC-245cb, de HFO-1234zeZ et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HCFO-1233xf, de HFC-245cb, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HCFO-1233xf, de HFC-245cb, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HCFO-1233zdE, de HCFO-1233xf, de HFC-245cb, de HFO-1243zf et de HFO-1234zeZ le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HCFO-1233zdE, de HCFO-1233xf, de HFC-245cb, de HFO-1243zf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HCFO-1233xf, de HFC-245cb, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HCFO-1233xf, de HFC-245cb, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFO-1234zeZ, de HCFO-1233xf, de HCFO-1233zdE, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFO-1234zeZ, de HCFO-1233xf, de HCFO-1233zdE, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234yf, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234yf, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1243zf et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HCFC-244bb et de HFO-1234zeE le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HCFC-244bb et de HFO-1234zeE, le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de trifluoropropyne et de HFO-1234zeE le point d'ébullition de cette composition préférée est compris entre 0 et 40 °C à une pression comprise entre 0,9 et 18 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de trifluoropropyne et de HFO-1234zeE, le point d'ébullition de cette composition préférée est compris entre 0 et 40 °C à une pression comprise entre 0,9 et 18 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFC-245fa et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFC-245fa et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225yeZ et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225yeZ et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225zc et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225zc et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225zc, de trifluoropropyne, de HCFC-244bb, de HFC-245fa, de HFO-1225yeZ, de HFO-1243zf et de HFO-1234zeE, le point d'ébullition de cette composition préférée est compris entre 0 et 40 °C à une pression comprise entre 0,7 et 18,0 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225zc, de trifluoropropyne, de HCFC-244bb, de HFC-245fa, de HFO-1225yeZ, de HFO-1243zf et de HFO-1234zeE, le point d'ébullition de cette composition préférée est compris entre 0 et 40 °C à une pression comprise entre 0,7 et 18,0 bars absolue.

Une composition azéotropique ou quasi-azéotropique selon l'invention comprend de 1 à 80 % en poids de fluorure d'hydrogène, et de 99 à 20 % en poids de trifluoropropyne le point d'ébullition de cette composition est compris entre 0 et 40 °C à une pression comprise entre 6 et 18 bars absolue.

Une composition azéotropique ou quasi-azéotropique selon l'invention comprend de préférence de 5 à 75 % en poids de fluorure d'hydrogène, et de 95 à 25 % en poids de trifluoropropyne, le point d'ébullition de cette composition est compris entre 0 et 40 °C à une pression comprise entre 6 et 18 bars absolue.

Une composition azéotropique ou quasi-azéotropique selon l'invention comprend de 1 à 90 % en poids de fluorure d'hydrogène, et de 99 à 10 % en poids de 2-chloro,1,1,1,2-tetrafluoropropane le point d'ébullition de cette composition est compris entre 0 et 40 °C à une pression comprise entre 0,7 et 3 bars absolue.

Une composition azéotropique ou quasi-azéotropique selon l'invention comprend de préférence de 5 à 85 % en poids de fluorure d'hydrogène, et de 95 à 15 % en poids de 2-chloro,1,1,1,2-tetrafluoropropane, le point d'ébullition de cette composition est compris entre 0 et 40 °C à une pression comprise entre 0,7 et 3 bars absolue.

Une composition azéotropique ou quasi-azéotropique selon l'invention comprend de 1 à 90 % en poids de fluorure d'hydrogène, et de 99 à 10 % en poids de 1,1,1,3,3-pentafluoropropane le point d'ébullition de cette composition est compris entre 0 et 40 °C à une pression comprise entre 1 et 4,4 bars absolue.

Une composition azéotropique ou quasi-azéotropique selon l'invention comprend de préférence de 5 à 85 % en poids de fluorure d'hydrogène, et de 95 à 15 % en poids de 1,1,1,3,3-pentafluoropropane, le point d'ébullition de cette composition est compris entre 0 et 40 °C à une pression comprise entre 1 et 4,4 bars absolue

Les caractéristiques de pression des mélanges des exemples 1 à 7 ont été calculées pour une isotherme à 25 °C.

Les exemples 8 à 14 représentent les plages de température d'ébullition et de pression des mélanges correspondants à ceux des exemples 1 à 7.

Les exemples 15 à 21 représentent les plages de décantation des mélanges des exemples 1 à 7 en fonction du pourcentage massique d'HF caractérisées pour des isothermes à 0 °C, 25 °C et 40 °C.

L'exemple 22 représente les caractéristiques de pression des mélanges binaires HF-trifluoropropyne, HF-HCFC-244bb et HF-HFC-245fa, calculées pour une isotherme à 25 °C.

L'exemple 23 représente les plages de température d'ébullition et de pression des mélanges correspondants à ceux de l'exemple 22.

L'exemple 24 représente les plages de décantation des mélanges de l'exemple 22 en fonction du pourcentage massique d'HF caractérisées pour des isothermes à 0 °C, 25 °C et 40 °C.

Les plages de décantations des exemples 15 à 21 et 24 sont calculées pour des mélanges de composés organiques ayant des teneurs equimassiques. A titre d'exemple, pour un mélange ternaire, un mélange à 50 % en poids de chacun des deux composés organiques est considéré ; pour un mélange pentanaire, un mélange à 25% en poids de chacun des quatre composés organiques est considéré, la fraction massique d'HF variant de 0 à 1. Ces calculs sont réalisés à l'équilibre liquide-vapeur, dans des conditions azéotropiques.

### Exemple 1 : Mélanges ternaires

**HF - HFO-1234yf - HFC-245cb (comparatif)**

| Organics | | Organics | | Organics | |
|---|---|---|---|---|---|
| 0,95 F1234yf | | 0,05 F1234yf | | 0,05 F1234yf | |
| +0,05 F245cb | | +0,95 F245cb | | +0,95 F245cb | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 6,7 | 0 | 5,8 | 0 | 4,8 |
| 0,05 | 77 | 0,05 | 6.9 | 0,05 | 5.9 |
| 0,1 | 7,7 | 0,1 | 6,9 | 0,1 | 5,9 |
| 0,15 | 7,7 | 0,15 | 6,9 | 0,15 | 5,9 |
| 0,2 | 7,7 | 0,2 | 6,9 | 0,2 | 5,9 |
| 0,25 | 7,7 | 0,25 | 6,9 | 0,25 | 5,9 |
| 0,3 | 7,7 | 0,3 | 6,9 | 0,3 | 5,9 |
| 0,35 | 7,7 | 0,35 | 6,9 | 0,35 | 5,9 |
| 0,4 | 7,7 | 0,4 | 6,9 | 0,4 | 5,9 |
| 0,45 | 7,7 | 0,45 | 6,9 | 0,45 | 5,9 |
| 0,5 | 7,7 | 0,5 | 6,8 | 0,5 | 5,9 |
| 0,55 | 7,7 | 0,55 | 6,8 | 0,55 | 5,9 |
| 0,6 | 7,7 | 0,6 | 6,8 | 0,6 | 5,9 |
| 0,65 | 7,7 | 0,65 | 6,8 | 0,65 | 5,9 |
| 0,7 | 7,6 | 0,7 | 6,8 | 0,7 | 5,9 |
| 0,75 | 7,5 | 0,75 | 6,8 | 0,75 | 5,9 |
| 0,8 | 7,2 | 0,8 | 6,8 | 0,8 | 5,9 |
| 0,85 | 6,6 | 0,85 | 6,3 | 0,85 | 5,9 |
| 0,9 | 5,6 | 0,9 | 5,3 | 0,9 | 5,1 |
| 0,95 | 3,9 | 0,95 | 3,7 | 0,95 | 3,6 |
| 1 | 1,2 | 1 | 1,2 | 1 | 1,2 |

**HF - HFO-1234yf - HFO-1234zeE**

| Organics 0,95 F1234zeE +0,05 F1234yf | | Organics 0,5 F1234zeE +0,5 F1234yf | | Organics 0,05 F1234zeE +0,95 F1234yf | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 5,0 | 0 | 5,9 | 0 | 6,7 |
| 0,05 | 5,9 | 0,05 | 6,8 | 0,05 | 7,7 |
| 0,1 | 5,9 | 0,1 | 6,8 | 0,1 | 7,7 |
| 0,15 | 5,9 | 0,15 | 6,8 | 0,15 | 7,7 |
| 0.2 | 5,9 | 0,2 | 6,8 | 0,2 | 7,7 |
| 0,25 | 5,9 | 0,25 | 6,8 | 0,25 | 7,6 |
| 0,3 | 5,9 | 0,3 | 6,8 | 0,3 | 7,7 |
| 0,35 | 5,9 | 0,35 | 6,8 | 0,35 | 7,7 |
| 0,4 | 5,9 | 0,4 | 6,8 | 0,4 | 7,7 |
| 0,45 | 5,9 | 0,45 | 6,8 | 0,45 | 7,7 |
| 0,5 | 5,9 | 0,5 | 6,8 | 0,5 | 7,7 |
| 0,55 | 5,8 | 0,55 | 6,8 | 0,55 | 7,7 |
| 0,6 | 5,8 | 0,6 | 6,8 | 0,6 | 7,7 |
| 0,65 | 5,7 | 0,65 | 6,7 | 0,65 | 7,7 |
| 0,7 | 5,5 | 0,7 | 6,6 | 0,7 | 7,6 |
| 0,75 | 5,3 | 0,75 | 6,4 | 0,75 | 7,5 |
| 0,8 | 4,9 | 0,8 | 6,1 | 0,8 | 7,1 |
| 0,85 | 4,4 | 0,85 | 5,5 | 0,85 | 6,5 |
| 0,9 | 3,7 | 0,9 | 4,6 | 0,9 | 5,5 |
| 0,95 | 2,6 | 0,95 | 3,2 | 0,95 | 3,8 |
| 1 | 1,2 | 1 | 1,2 | 1 | 1,2 |

**HF - HFO-1234yf- HFO-1243zf**

| Organics | | Organics | | Organics | |
|---|---|---|---|---|---|
| 0,95 F1243zf | | 0,5 F1243zf | | 0,05 F1243zf | |
| +0,05 F1234yf | | +0,5 F1234yf | | +0,95 F1234yf | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 5,9 | 0 | 6,3 | 0 | 6,7 |
| 0,05 | 6,8 | 0,05 | 7,2 | 0,05 | 7,7 |
| 0,1 | 6,9 | 0,1 | 7,3 | 0,1 | 7,7 |
| 0,15 | 6,9 | 0,15 | 7,3 | 0,15 | 7,7 |
| 0,2 | 6,8 | 0,2 | 7,2 | 0,2 | 7,7 |
| 0,25 | 6,8 | 0,25 | 7,2 | 0,25 | 7,7 |
| 0,3 | 6,8 | 0,3 | 7,2 | 0,3 | 7,7 |
| 0,35 | 6,8 | 0,35 | 7,2 | 0,35 | 7,7 |
| 0,4 | 6,8 | 0,4 | 7,2 | 0,4 | 7,7 |
| 0,45 | 6,8 | 0,45 | 7,2 | 0,45 | 7,7 |
| 0,5 | 6,8 | 0,5 | 7,2 | 0,5 | 7,7 |
| 0,55 | 6,8 | 0,55 | 7,2 | 0,55 | 7,7 |
| 0,6 | 6,8 | 0,6 | 7,2 | 0,6 | 7,7 |
| 0,65 | 6,7 | 0,65 | 7,2 | 0,65 | 7,7 |
| 0,7 | 6,6 | 0,7 | 7,1 | 0,7 | 7,7 |
| 0,75 | 6,5 | 0,75 | 7,0 | 0,75 | 7,5 |
| 0,8 | 6,2 | 0,8 | 6,7 | 0,8 | 7,2 |
| 0,85 | 5,7 | 0,85 | 6,1 | 0,85 | 6,6 |
| 0,9 | 4,8 | 0,9 | 5,2 | 0,9 | 5,6 |
| 0,95 | 3,4 | 0,95 | 3,6 | 0,95 | 3,8 |
| 1 | 1,2 | 1 | 1,2 | 1 | 1,2 |

**HF - HFO-1234yf - HFO-1234zeZ**

| Organics | | Organics | | Organics | |
|---|---|---|---|---|---|
| 0,95 F1234zeZ | | 0,5 F1234zeZ | | 0,05 F1234zeZ | |
| +0,05 F1234yf | | +0,5 F1234yf | | +0,95 F1234yf | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 2,1 | 0 | 4,3 | 0 | 6,5 |
| 0,05 | 3,2 | 0,05 | 5,4 | 0,05 | 7,5 |
| 0,1 | 3,2 | 0,1 | 5,4 | 0,1 | 7,5 |
| 0,15 | 3,2 | 0,15 | 5,4 | 0,15 | 7,5 |
| 0,2 | 3,2 | 0,2 | 5,4 | 0,2 | 7,5 |
| 0,25 | 3,2 | 0,25 | 5,4 | 0,25 | 7,5 |
| 0,3 | 3,2 | 0,3 | 5,4 | 0,3 | 7,5 |
| 0,35 | 3,2 | 0,35 | 5,4 | 0,35 | 7,5 |
| 0,4 | 3,2 | 0,4 | 5,4 | 0,4 | 7,5 |
| 0,45 | 3,2 | 0,45 | 5,4 | 0,45 | 7,5 |
| 0,5 | 3,2 | 0,5 | 5,4 | 0,5 | 7,5 |
| 0,55 | 3,2 | 0,55 | 5,4 | 0,55 | 7,5 |
| 0,6 | 3,2 | 0,6 | 5,4 | 0,6 | 7,5 |
| 0,65 | 3,2 | 0,65 | 5,4 | 0,65 | 7,5 |
| 0,7 | 3,2 | 0,7 | 5,4 | 0,7 | 7,5 |
| 0,75 | 3,2 | 0,75 | 5,3 | 0,75 | 7,3 |
| 0,8 | 3,0 | 0,8 | 5,0 | 0,8 | 7,0 |
| 0,85 | 2,8 | 0,85 | 4,6 | 0,85 | 6,5 |
| 0,9 | 2,5 | 0,9 | 3,9 | 0,9 | 5,4 |
| 0,95 | 1,9 | 0,95 | 2,8 | 0,95 | 3,8 |
| 1 | 1,2 | 1 | 1,2 | 1 | 1,2 |

**HF - HFO-1234yf - HCFO-1233xf**

| Organics | | Organics | | Organics | |
|---|---|---|---|---|---|
| 0,95 F1233xf | | 0,5 F1233xf | | 0,05 F1233xf | |
| +0,05 F1234yf | | +0,5 F1234yf | | +0,95 F1234yf | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 1,9 | 0 | 4,3 | 0 | 6,6 |
| 0,05 | 3,0 | 0,05 | 5,4 | 0,05 | 7,5 |
| 0,1 | 3,0 | 0,1 | 5,4 | 0,1 | 7,5 |
| 0,15 | 3,0 | 0,15 | 5,4 | 0,15 | 7,5 |
| 0,2 | 3,0 | 0,2 | 5,4 | 0,2 | 7,5 |
| 0,25 | 3,0 | 0,25 | 5,4 | 0,25 | 7,5 |
| 0,3 | 3,0 | 0,3 | 5,4 | 0,3 | 7,5 |
| 0,35 | 3,0 | 0,35 | 5,4 | 0,35 | 7,5 |
| 0,4 | 3,0 | 0,4 | 5,4 | 0,4 | 7,5 |
| 0,45 | 3,0 | 0,45 | 5,4 | 0,45 | 7,5 |
| 0,5 | 3,0 | 0,5 | 5,4 | 0,5 | 7,5 |
| 0,55 | 3,0 | 0,55 | 5,4 | 0,55 | 7,5 |
| 0,6 | 3,0 | 0,6 | 5,4 | 0,6 | 7,5 |
| 0,65 | 2,9 | 0,65 | 5,4 | 0,65 | 7,5 |
| 0,7 | 2,9 | 0,7 | 5,4 | 0,7 | 7,5 |
| 0,75 | 2,9 | 0,75 | 5,3 | 0,75 | 7.4 |
| 0,8 | 2,8 | 0,8 | 5,0 | 0,8 | 7,0 |
| 0,85 | 2,6 | 0,85 | 4,6 | 0,85 | 6,5 |
| 0,9 | 2,3 | 0,9 | 3,9 | 0,9 | 5,4 |
| 0,95 | 1,8 | 0,95 | 2,8 | 0,95 | 3,8 |
| 1 | 1,2 | 1 | 1,2 | 1 | 1,2 |

**HF - HFO-1234yf - HCFO-1233zdE**

| Organics | | Organics | | Organics | |
|---|---|---|---|---|---|
| 0,95 F1233zdE | | 0,5 F1233zdE | | 0,05 F1233zdE | |
| +0.05 F1234vf | | +0,5 F1234vf | | +0,95 F1234vf | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 1,6 | 0 | 4,2 | 0 | 6,5 |
| 0,05 | 2,7 | 0,05 | 5,3 | 0,05 | 7,5 |
| 0,1 | 2,7 | 0,1 | 5,3 | 0,1 | 7,5 |
| 0,15 | 2,7 | 0,15 | 5,3 | 0,15 | 7,5 |
| 0,2 | 2,7 | 0,2 | 5,3 | 0,2 | 7,5 |
| 0,25 | 2,7 | 0,25 | 5,3 | 0,25 | 7,5 |
| 0,3 | 2,7 | 0,3 | 5,3 | 0,3 | 7,5 |
| 0,35 | 2,7 | 0,35 | 5,3 | 0,35 | 7,5 |
| 0,4 | 2,7 | 0,4 | 5,3 | 0,4 | 7,5 |
| 0,45 | 2,7 | 0,45 | 5,3 | 0,45 | 7,5 |
| 0,5 | 2,7 | 0,5 | 5,3 | 0,5 | 7,5 |
| 0,55 | 2,7 | 0,55 | 5,3 | 0,55 | 7,5 |
| 0,6 | 2,7 | 0,6 | 5,3 | 0,6 | 7,5 |
| 0,65 | 2,7 | 0,65 | 5,3 | 0,65 | 7,5 |
| 0,7 | 2,7 | 0,7 | 5,3 | 0,7 | 7,5 |
| 0,75 | 2,7 | 0,75 | 5,2 | 0,75 | 7,4 |
| 0,8 | 2,6 | 0,8 | 4,9 | 0,8 | 7,0 |
| 0,85 | 2,4 | 0,85 | 4,5 | 0,85 | 6,5 |
| 0,9 | 2,1 | 0,9 | 3,8 | 0,9 | 5,4 |
| 0,95 | 1,7 | 0,95 | 2,8 | 0,95 | 3,8 |
| 1 | 1,2 | 1 | 1,2 | 1 | 1,2 |

**HF - HFO-1234yf - HCFC-244bb**

| **Organics** | | **Organics** | | **Organics 0,05** | |
|---|---|---|---|---|---|
| **0,5 F1234yf** | | **0,95 F1234yf** | | **F1234yf** | |
| **+ 0,5 F244bb** | | **+ 0,05 F244bb** | | **+ 0,95 F244bb** | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 4,1 | 0 | 6,5 | 0 | 1,0 |
| 0,05 | 5,2 | 0,05 | 7,5 | 0,05 | 2,2 |
| 0,1 | 5,2 | 0,1 | 7,5 | 0,1 | 2,2 |
| 0,15 | 5,2 | 0,15 | 7,5 | 0,15 | 2,2 |
| 0,2 | 5,2 | 0,2 | 7,5 | 0,2 | 2,2 |
| 0,25 | 5,1 | 0,25 | 7,5 | 0,25 | 2,2 |
| 0,3 | 5,1 | 0,3 | 7,5 | 0,3 | 2,2 |
| 0,35 | 5,1 | 0,35 | 7,5 | 0,35 | 2,2 |
| 0,4 | 5,1 | 0,4 | 7,5 | 0,4 | 2,2 |
| 0,45 | 5,1 | 0,45 | 7,5 | 0,45 | 2,2 |
| 0,5 | 5,1 | 0,5 | 7,5 | 0,5 | 2,1 |
| 0,55 | 50 | 0,55 | 7,5 | 0,55 | 2,1 |
| 0,6 | 5,0 | 0,6 | 7,5 | 0,6 | 2,1 |
| 0,65 | 4,9 | 0,65 | 7,5 | 0,65 | 2,1 |
| 0,7 | 4,9 | 0,7 | 7,5 | 0,7 | 2,1 |
| 0,75 | 4,8 | 0,75 | 7,3 | 0,75 | 2,1 |
| 0,8 | 4,6 | 0,8 | 7,0 | 0,8 | 2,1 |
| 0,85 | 4,3 | 0,85 | 6,4 | 0,85 | 2,0 |
| 0,9 | 3,7 | 0,9 | 5,4 | 0,9 | 1,9 |
| 0,95 | 2,7 | 0,95 | 3,8 | 0,95 | 1,6 |
| 1 | 1,2 | 1 | 1,2 | 1 | 1,2 |

**HF - HFO-1234yf - HFO-1225yeZ**

| **Organics** | | **Organics** | | **Organics** | |
|---|---|---|---|---|---|
| **0,5 F1234yf** | | **0,95 F1234yf** | | **0,05 F1234yf** | |
| **+ 0,5 F1225yeZ** | | **+ 0,05 F1225yeZ** | | **+ 0,95 F1225yeZ** | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 6,0 | 0 | 6,7 | 0 | 5,3 |
| 0,05 | 7,0 | 0,05 | 7,7 | 0,05 | 6,3 |
| 0,1 | 7,1 | 0,1 | 7,7 | 0,1 | 6,3 |
| 0,15 | 7,0 | 0,15 | 7,7 | 0,15 | 6,3 |
| 0,2 | 7,0 | 0,2 | 7,7 | 0,2 | 6,3 |
| 0,25 | 7,0 | 0,25 | 7,7 | 0,25 | 6,3 |
| 0,3 | 7,0 | 0,3 | 7,7 | 0,3 | 6,3 |
| 0,35 | 7,0 | 0,35 | 7,7 | 0,35 | 6,3 |
| 0,4 | 7,0 | 0,4 | 7,7 | 0,4 | 6,3 |
| 0,45 | 7,0 | 0,45 | 7,7 | 0,45 | 6,3 |
| 0,5 | 7,0 | 0,5 | 7,7 | 0,5 | 6,3 |
| 0,55 | 7,1 | 0,55 | 7,7 | 0,55 | 6,3 |
| 0,6 | 7,1 | 0,6 | 7,7 | 0,6 | 6,3 |
| 0,65 | 7,1 | 0,65 | 7,7 | 0,65 | 6,3 |
| 0,7 | 7,0 | 0,7 | 7,7 | 0,7 | 6,2 |
| 0,75 | 6,8 | 0,75 | 7,5 | 0,75 | 6,0 |
| 0,8 | 6,5 | 0,8 | 7,2 | 0,8 | 5,7 |
| 0,85 | 5,9 | 0,85 | 6,6 | 0,85 | 5,2 |
| 0,9 | 4,9 | 0,9 | 5,5 | 0,9 | 4,3 |
| 0,95 | 3,4 | 0,95 | 3,8 | 0,95 | 3,0 |
| 1 | 1,2 | 1 | 1,2 | 1 | 1,2 |

**HF - HFO-1234yf - Trifluoropropyne**

| **Organics** | | **Organics** | | **Organics** | |
|---|---|---|---|---|---|
| **0,5 F1234yf** | | **0,95 F1234yf** | | **0,05 F1234yf** | |
| **+ 0,5 TFP** | | **+ 0,05 TFP** | | **+ 0,95 TFP** | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 9,4 | 0 | 7,1 | 0 | 11,4 |
| 0,05 | 10,2 | 0,05 | 8,0 | 0,05 | 12,2 |
| 0,1 | 10,2 | 0,1 | 8,0 | 0,1 | 12,1 |
| 0,15 | 10,1 | 0,15 | 8,0 | 0,15 | 12,0 |
| 0,2 | 10,1 | 0,2 | 8,0 | 0,2 | 11,9 |
| 0,25 | 10,0 | 0,25 | 8,0 | 0,25 | 11,8 |
| 0,3 | 9,9 | 0,3 | 8,0 | 0,3 | 11,7 |
| 0,35 | 9,9 | 0,35 | 8,0 | 0,35 | 11,6 |
| 0,4 | 9,9 | 0,4 | 8,0 | 0,4 | 11,6 |
| 0,45 | 9,9 | 0,45 | 8,0 | 0,45 | 11,6 |
| 0,5 | 9,9 | 0,5 | 8,0 | 0,5 | 11,6 |
| 0,55 | 9,9 | 0,55 | 8,0 | 0,55 | 11,6 |
| 0,6 | 9,9 | 0,6 | 8,0 | 0,6 | 11,6 |
| 0,65 | 9,9 | 0,65 | 8,0 | 0,65 | 11,6 |
| 0,7 | 9,9 | 0,7 | 8,0 | 0,7 | 11,6 |
| 0,75 | 9,9 | 0,75 | 7,8 | 0,75 | 11,6 |
| 0,8 | 9,5 | 0,8 | 7,5 | 0,8 | 11,4 |
| 0,85 | 8,8 | 0,85 | 6,9 | 0,85 | 10,6 |
| 0,9 | 7,4 | 0,9 | 5,8 | 0,9 | 9,0 |
| 0,95 | 5,0 | 0,95 | 4,0 | 0,95 | 6,0 |
| 1 | 1,2 | 1 | 1,2 | 1 | 1,2 |

**HF - HFO-1234yf - HFC-245fa**

| **Organics** | | **Organics** | | **Organics** | |
|---|---|---|---|---|---|
| **0,5 F1234yf** | | **0,95 F1234yf** | | **0,05 F1234yf** | |
| **+ 0.5 F245fa** | | **+ 0.05 F245fa** | | **+ 0.95 F245fa** | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 4,3 | 0 | 6,6 | 0 | 1,8 |
| 0,05 | 5,4 | 0,05 | 7,5 | 0,05 | 2,9 |
| 0,1 | 5,4 | 0,1 | 7,5 | 0,1 | 2,9 |
| 0,15 | 5,4 | 0,15 | 7,5 | 0,15 | 2,9 |
| 0,2 | 5,4 | 0,2 | 7,5 | 0,2 | 2,9 |
| 0,25 | 5,4 | 0,25 | 7,5 | 0,25 | 2,9 |
| 0,3 | 5,4 | 0,3 | 7,5 | 0,3 | 2,9 |
| 0,35 | 5,4 | 0,35 | 7,5 | 0,35 | 2,9 |
| 0,4 | 5,4 | 0,4 | 7,5 | 0,4 | 2,9 |
| 0,45 | 5,4 | 0,45 | 7,5 | 0,45 | 2,9 |
| 0,5 | 5,4 | 0,5 | 7,5 | 0,5 | 2,9 |
| 0,55 | 5,4 | 0,55 | 7,5 | 0,55 | 2,9 |
| 0,6 | 5,4 | 0,6 | 7,5 | 0,6 | 2,9 |
| 0,65 | 5,4 | 0,65 | 7,5 | 0,65 | 2,9 |
| 0,7 | 5,4 | 0,7 | 7,5 | 0,7 | 2,9 |
| 0,75 | 5,3 | 0,75 | 7,4 | 0,75 | 2,9 |
| 0,8 | 5,0 | 0,8 | 7,0 | 0,8 | 2,8 |
| 0,85 | 4,6 | 0,85 | 6,5 | 0,85 | 2,6 |
| 0,9 | 3,9 | 0,9 | 5,4 | 0,9 | 2,3 |
| 0,95 | 2,8 | 0,95 | 3,8 | 0,95 | 1,8 |
| 1 | 1,2 | 1 | 1,2 | 1 | 1,2 |

**HF - HFO-1234yf - HFO-1225zc**

| **Organics** | | **Organics** | | **Organics** | |
|---|---|---|---|---|---|
| **0,5 F1234yf** | | **0,95 F1234yf** | | **0,05 F1234yf** | |
| **+ 0,5 F1225zc** | | **+ 0,05 F1225zc** | | **+ 0,95 F1225zc** | |
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 6,1 | 0 | 6,7 | 0 | 5,4 |
| 0,05 | 7,1 | 0,05 | 7,7 | 0,05 | 6,4 |
| 0,1 | 7,1 | 0,1 | 7,7 | 0,1 | 6,4 |
| 0,15 | 7,1 | 0,15 | 7,7 | 0,15 | 6,4 |
| 0,2 | 7,1 | 0,2 | 7,7 | 0,2 | 6,4 |
| 0,25 | 7,1 | 0,25 | 7,7 | 0,25 | 6,4 |
| 0,3 | 7,1 | 0,3 | 7,7 | 0,3 | 6,4 |
| 0,35 | 7,1 | 0,35 | 7,7 | 0,35 | 6,4 |
| 0,4 | 7,1 | 0,4 | 7,7 | 0,4 | 6,4 |
| 0,45 | 7,1 | 0,45 | 7,7 | 0,45 | 6,4 |
| 0,5 | 7,1 | 0,5 | 7,7 | 0,5 | 6,3 |
| 0,55 | 7,1 | 0,55 | 7,7 | 0,55 | 6,3 |
| 0,6 | 7,1 | 0,6 | 7,7 | 0,6 | 6,2 |
| 0,65 | 7,0 | 0,65 | 7,7 | 0,65 | 6,1 |
| 0,7 | 6,9 | 0,7 | 7,7 | 0,7 | 6,0 |
| 0,75 | 6,7 | 0,75 | 7,5 | 0,75 | 5,7 |
| 0,8 | 6,3 | 0,8 | 7,2 | 0,8 | 5,3 |
| 0,85 | 5,7 | 0,85 | 6,6 | 0,85 | 4,7 |
| 0,9 | 4,8 | 0,9 | 5,5 | 0,9 | 3,9 |
| 0,95 | 3,3 | 0,95 | 3,8 | 0,95 | 2,8 |
| 1 | 1,2 | 1 | 1,2 | 1 | 1,2 |

### Exemple 2 : Mélanges quaternaires

**HF - HCFO-1233xf - HFO-1234yf - HFC-245cb**

| Organics 0,9 F1233xf + 0,05 F1234yf +0,05 F245cb | | Organics 0,4 F1233xf + 0,3 F1234yf +0,3 F245cb | | Organics 0,05 F1233xf + 0,9 F1234yf +0,05 F245cb | | Organics 0,05 F1233xf + 0,05 F1234yf +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 2,0 | 0 | 4,2 | 0 | 6,5 | 0 | 4,6 |
| 0,05 | 3,1 | 0,05 | 5,3 | 0,05 | 7,4 | 0,05 | 5,8 |
| 0,1 | 3,1 | 0,1 | 5,3 | 0,1 | 7,4 | 0,1 | 5,8 |
| 0,15 | 3,1 | 0,15 | 5,3 | 0,15 | 7,4 | 0,15 | 5,8 |
| 0,2 | 3,1 | 0,2 | 5,3 | 0,2 | 7,4 | 0,2 | 5,8 |
| 0,25 | 3,1 | 0,25 | 5,3 | 0,25 | 7,4 | 0,25 | 5,8 |
| 0,3 | 3,1 | 0,3 | 5,3 | 0,3 | 7,4 | 0,3 | 5,8 |
| 0,35 | 3,1 | 0,35 | 5,3 | 0,35 | 7,4 | 0,35 | 5,8 |
| 0,4 | 3,1 | 0,4 | 5,3 | 0,4 | 7,4 | 0,4 | 5,8 |
| 0,45 | 3,1 | 0,45 | 5,3 | 0,45 | 7,4 | 0,45 | 5,8 |
| 0,5 | 3,1 | 0,5 | 5,3 | 0,5 | 7,4 | 0,5 | 5,8 |
| 0,55 | 3,1 | 0,55 | 5,3 | 0,55 | 7,4 | 0,55 | 5,8 |
| 0,6 | 3,1 | 0,6 | 5,3 | 0,6 | 7,4 | 0,6 | 5,8 |
| 0,65 | 3,1 | 0,65 | 5,3 | 0,65 | 7,4 | 0,65 | 5,8 |
| 0,7 | 3,1 | 0,7 | 5,3 | 0,7 | 7,4 | 0,7 | 5,8 |
| 0,75 | 3,1 | 0,75 | 5,4 | 0,75 | 7,3 | 0,75 | 5,8 |
| 0,8 | 3,0 | 0,8 | 5,2 | 0,8 | 7,0 | 0,8 | 5,8 |
| 0,85 | 2,8 | 0,85 | 4,8 | 0,85 | 6,4 | 0,85 | 5,7 |
| 0,9 | 2,4 | 0,9 | 4,1 | 0,9 | 5,4 | 0,9 | 4,9 |
| 0,95 | 1,9 | 0,95 | 2,9 | 0,95 | 3,7 | 0,95 | 3,5 |
| 1 | 1,2 | 1 | 1,2 | 1 | 1,2 | 1 | 1,2 |

**HF - HFO-1234yf - HCFO-1233zdE - HFC-245cb**

| Organics 0,9 F1234yf + 0,05 F1233zdE +0,05 F245cb | | Organics 0,4 F1234yf + 0,3 F1233zdE +0,3 F245cb | | Organics 0,05 F1234yf + 0,9 F1233zdE +0,05 F245cb | | Organics 0,05 F1234yf + 0,05 F1233zdE +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,6 | 0 | 1,8 | 0 | 4,6 |
| 0,05 | 7,4 | 0,05 | 5,7 | 0,05 | 2,9 | 0,05 | 5,8 |
| 0,1 | 7,4 | 0,1 | 5,7 | 0,1 | 2,9 | 0,1 | 5,8 |
| 0,15 | 7,4 | 0,15 | 5,7 | 0,15 | 2,9 | 0,15 | 5,8 |
| 0,2 | 7,4 | 0,2 | 5,7 | 0,2 | 2,9 | 0,2 | 5,8 |
| 0,25 | 7,4 | 0,25 | 5,7 | 0,25 | 2,9 | 0,25 | 5,8 |
| 0,3 | 7,4 | 0,3 | 5,7 | 0,3 | 2,9 | 0,3 | 5,8 |
| 0,35 | 7,4 | 0,35 | 5,7 | 0,35 | 2,9 | 0,35 | 5,8 |
| 0,4 | 7,4 | 0,4 | 5,7 | 0,4 | 2,9 | 0,4 | 5,8 |
| 0,45 | 7,4 | 0,45 | 5,7 | 0,45 | 2,9 | 0,45 | 5,8 |
| 0,5 | 7,4 | 0,5 | 5,7 | 0,5 | 2,9 | 0,5 | 5,8 |
| 0,55 | 7,4 | 0,55 | 5,7 | 0,55 | 2,9 | 0,55 | 5,8 |
| 0,6 | 7,4 | 0,6 | 5,7 | 0,6 | 2,9 | 0,6 | 5,8 |
| 0,65 | 7,4 | 0,65 | 5,8 | 0,65 | 2,9 | 0,65 | 5,8 |
| 0,7 | 7,4 | 0,7 | 5,8 | 0,7 | 2,9 | 0,7 | 5,8 |
| 0,75 | 7,3 | 0,75 | 5,8 | 0,75 | 2,9 | 0,75 | 5,8 |
| 0,8 | 7,0 | 0,8 | 5,6 | 0,8 | 2,8 | 0,8 | 5,8 |
| 0,85 | 6,4 | 0,85 | 5,1 | 0,85 | 2,6 | 0,85 | 5,7 |
| 0,9 | 5,4 | 0,9 | 4,4 | 0,9 | 2,3 | 0,9 | 4,9 |
| 0,95 | 3,7 | 0,95 | 3,1 | 0,95 | 1,8 | 0,95 | 3,5 |
| 1 | 1,2 | 1 | 1,2 | 1 | 1,2 | 1 | 1,2 |

**HF - HFO-1234yf - HFO-1234zeE - HFC-245cb**

| Organics 0,9 F1234yf + 0,05 F1234zeE +0,05 F245cb | | Organics 0,4 F1234yf + 0,3 F1234zeE +0,3 F245cb | | Organics 0,05 F1234yf + 0,9 F1234zeE +0,05 F245cb | | Organics 0,05 F1234yf + 0,05 F1234zeE +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,6 | 0 | 5,6 | 0 | 5,0 | 0 | 4,8 |
| 0,05 | 7,6 | 0,05 | 6,7 | 0,05 | 5,9 | 0,05 | 6,0 |
| 0,1 | 7,6 | 0,1 | 6,7 | 0,1 | 6,0 | 0,1 | 6,0 |
| 0,15 | 7,6 | 0,15 | 6,7 | 0,15 | 6,0 | 0,15 | 6,0 |
| 0,2 | 7,6 | 0,2 | 6,7 | 0,2 | 6,0 | 0,2 | 6,0 |
| 0,25 | 7,6 | 0,25 | 6,7 | 0,25 | 6,0 | 0,25 | 6,0 |
| 0,3 | 7,6 | 0,3 | 6,7 | 0,3 | 6,0 | 0,3 | 6,0 |
| 0,35 | 7,6 | 0,35 | 6,7 | 0,35 | 6,0 | 0,35 | 6,0 |
| 0,4 | 7,6 | 0,4 | 6,7 | 0,4 | 6,0 | 0,4 | 6,0 |
| 0,45 | 7,6 | 0,45 | 6,7 | 0,45 | 5,9 | 0,45 | 6,0 |
| 0,5 | 7,6 | 0,5 | 6,7 | 0,5 | 5,9 | 0,5 | 6,0 |
| 0,55 | 7,6 | 0,55 | 6,7 | 0,55 | 5,9 | 0,55 | 5,9 |
| 0,6 | 7,6 | 0,6 | 6,7 | 0,6 | 5,8 | 0,6 | 5,9 |
| 0,65 | 7,6 | 0,65 | 6,7 | 0,65 | 5,7 | 0,65 | 5,9 |
| 0,7 | 7,6 | 0,7 | 6,7 | 0,7 | 5,6 | 0,7 | 5,9 |
| 0,75 | 7,4 | 0,75 | 6,6 | 0,75 | 5,3 | 0,75 | 5,9 |
| 0,8 | 7,1 | 0,8 | 6,3 | 0,8 | 5,0 | 0,8 | 5,9 |
| 0,85 | 6,5 | 0,85 | 5,7 | 0,85 | 4,5 | 0,85 | 5,8 |
| 0,9 | 5,5 | 0,9 | 4,8 | 0,9 | 3,8 | 0,9 | 5,0 |
| 0,95 | 3,8 | 0,95 | 3,4 | 0,95 | 2,7 | 0,95 | 3,5 |
| 1 | 1,2 | 1 | 1,2 | 1 | 1,2 | 1 | 1,2 |

**HF - HFO-1234yf - HFO-1234zeZ - HFC-245cb**

| Organics 0,9 F1234yf + 0,05 F1234zeZ +0,05 F245cb | | Organics 0,4 F1234yf + 0,3 F1234zeZ +0,3 F245cb | | Organics 0,05 F1234yf + 0,9 F1234zeZ +0,05 F245cb | | Organics 0,05 F1234yf + 0,05 F1234zeZ +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,6 | 0 | 2,2 | 0 | 4,6 |
| 0,05 | 7,4 | 0,05 | 5,7 | 0,05 | 3,3 | 0,05 | 5,8 |
| 0,1 | 7,4 | 0,1 | 5,7 | 0,1 | 3,3 | 0,1 | 5,8 |
| 0,15 | 7,4 | 0,15 | 5,7 | 0,15 | 3,3 | 0,15 | 5,8 |
| 0,2 | 7,4 | 0,2 | 5,7 | 0,2 | 3,3 | 0,2 | 5,8 |
| 0,25 | 7,4 | 0,25 | 5,7 | 0,25 | 3,3 | 0,25 | 5,8 |
| 0,3 | 7,4 | 0,3 | 5,7 | 0,3 | 3,3 | 0,3 | 5,8 |
| 0,35 | 7,4 | 0,35 | 5,7 | 0,35 | 3,3 | 0,35 | 5,8 |
| 0,4 | 7,4 | 0,4 | 5,8 | 0,4 | 3,3 | 0,4 | 5,8 |
| 0,45 | 7,4 | 0,45 | 5,8 | 0,45 | 3,3 | 0,45 | 5,8 |
| 0,5 | 7,4 | 0,5 | 5,8 | 0,5 | 3,3 | 0,5 | 5,8 |
| 0,55 | 7,4 | 0,55 | 5,8 | 0,55 | 3,3 | 0,55 | 5,8 |
| 0,6 | 7,4 | 0,6 | 5,8 | 0,6 | 3,3 | 0,6 | 5,8 |
| 0,65 | 7,4 | 0,65 | 5,8 | 0,65 | 3,3 | 0,65 | 5,8 |
| 0,7 | 7,4 | 0,7 | 5,8 | 0,7 | 3,3 | 0,7 | 5,8 |
| 0,75 | 7,3 | 0,75 | 5,8 | 0,75 | 3,3 | 0,75 | 5,8 |
| 0,8 | 7,0 | 0,8 | 5,6 | 0,8 | 3,2 | 0,8 | 5,8 |
| 0,85 | 6,4 | 0,85 | 5,2 | 0,85 | 3,0 | 0,85 | 5,7 |
| 0,9 | 5,4 | 0,9 | 4,4 | 0,9 | 2,6 | 0,9 | 4,9 |
| 0,95 | 3,8 | 0,95 | 3,2 | 0,95 | 2,0 | 0,95 | 3,5 |
| 1 | 1,2 | 1 | 1,2 | 1 | 1,2 | 1 | 1,2 |

**HF - HFO-1234yf - HFO-1243zf-HFC-245cb**

| Organics 0,9 F1234yf + 0,05 F1243zf +0,05 F245cb | | Organics 0,4 F1234yf + 0,3 F1243zf +0,3 F245cb | | Organics 0,05 F1234yf + 0,9 F1243zf +0,05 F245cb | | Organics 0,05 F1234yf + 0,05 F1243zf +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,7 | 0 | 5,9 | 0 | 5,8 | 0 | 4,9 |
| 0,05 | 7,6 | 0,05 | 6,9 | 0,05 | 6,8 | 0,05 | 6,0 |
| 0,1 | 7,6 | 0,1 | 7,0 | 0,1 | 6,8 | 0,1 | 6,0 |
| 0,15 | 7,6 | 0,15 | 6,9 | 0,15 | 6,8 | 0,15 | 6,0 |
| 0,2 | 7,6 | 0,2 | 6,9 | 0,2 | 6,8 | 0,2 | 6,0 |
| 0,25 | 7,6 | 0,25 | 6,9 | 0,25 | 6,8 | 0,25 | 6,0 |
| 0,3 | 7,6 | 0,3 | 6,9 | 0,3 | 6,8 | 0,3 | 6,0 |
| 0,35 | 7,6 | 0,35 | 6,9 | 0,35 | 6,8 | 0,35 | 6,0 |
| 0,4 | 7,6 | 0,4 | 6,9 | 0,4 | 6,8 | 0,4 | 6,0 |
| 0,45 | 7,6 | 0,45 | 6,9 | 0,45 | 6,8 | 0,45 | 6,0 |
| 0,5 | 7,6 | 0,5 | 6,9 | 0,5 | 6,8 | 0,5 | 6,0 |
| 0,55 | 7,6 | 0,55 | 6,9 | 0,55 | 6,8 | 0,55 | 6,0 |
| 0,6 | 7,6 | 0,6 | 6,9 | 0,6 | 6,8 | 0,6 | 6,0 |
| 0,65 | 7,6 | 0,65 | 6,9 | 0,65 | 6,7 | 0,65 | 6,0 |
| 0,7 | 7,6 | 0,7 | 6,9 | 0,7 | 6,6 | 0,7 | 6,0 |
| 0,75 | 7,5 | 0,75 | 6,9 | 0,75 | 6,5 | 0,75 | 6,0 |
| 0,8 | 7,2 | 0,8 | 6,6 | 0,8 | 6,2 | 0,8 | 6,0 |
| 0,85 | 6,6 | 0,85 | 6_{'}1 | 0,85 | 5,7 | 0,85 | 5,9 |
| 0,9 | 5,5 | 0,9 | 5,2 | 0,9 | 4,8 | 0,9 | 5_{'}1 |
| 0,95 | 3,8 | 0,95 | 3,6 | 0,95 | 3,4 | 0,95 | 3,6 |
| 1 | 1,2 | 1 | 1,2 | 1 | 1,2 | 1 | 1,2 |

**HF - HCFO-1233xf - HFO-1234yf - HCFO-1233zdE**

| Organics 0,9 F1233xf + 0,05 F1234yf +0,05 F1233zdE | | Organics 0,05 F1233xf + 0,9 F1234yf +0,05 F1233zdE | | Organics 0,05 F1233xf + 0,05 F1234yf +0,9 F1233zdE | | Organics 0,4 F1233xf + 0,3 F1234yf +0,3 F1233zdE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,8 | 0 | 6,3 | 0 | 1,7 | 0 | 3,2 |
| 0,05 | 3,0 | 0,05 | 7,3 | 0,05 | 2,8 | 0,05 | 4,3 |
| 0,1 | 3,0 | 0,1 | 7,3 | 0,1 | 2,8 | 0,1 | 4,3 |
| 0,15 | 3,0 | 0,15 | 7,3 | 0,15 | 2,8 | 0,15 | 4,3 |
| 0,2 | 2,9 | 0,2 | 7,3 | 0,2 | 2,8 | 0,2 | 4,3 |
| 0,25 | 2,9 | 0,25 | 7,3 | 0,25 | 2,8 | 0,25 | 4,3 |
| 0,3 | 2,9 | 0,3 | 7,3 | 0,3 | 2,8 | 0,3 | 4,3 |
| 0,35 | 2,9 | 0,35 | 7,3 | 0,35 | 2,8 | 0,35 | 4,3 |
| 0,4 | 2,9 | 0,4 | 7,3 | 0,4 | 2,8 | 0,4 | 4,3 |
| 0,45 | 2,9 | 0,45 | 7,3 | 0,45 | 2,7 | 0,45 | 4,3 |
| 0,5 | 2,9 | 0,5 | 7,3 | 0,5 | 2,7 | 0,5 | 4,3 |
| 0,55 | 2,9 | 0,55 | 7,3 | 0,55 | 2,7 | 0,55 | 4,3 |
| 0,6 | 2,9 | 0,6 | 7,3 | 0,6 | 2,7 | 0,6 | 4,3 |
| 0,65 | 2,9 | 0,65 | 7,3 | 0,65 | 2,7 | 0,65 | 4,3 |
| 0,7 | 2,9 | 0,7 | 7,3 | 0,7 | 2,7 | 0,7 | 4,3 |
| 0,75 | 2,9 | 0,75 | 7,1 | 0,75 | 2,7 | 0,75 | 4,2 |
| 0,8 | 2,8 | 0,8 | 6,8 | 0,8 | 2,6 | 0,8 | 4,0 |
| 0,85 | 2,6 | 0,85 | 6,2 | 0,85 | 2,4 | 0,85 | 3,7 |
| 0,9 | 2,3 | 0,9 | 5,3 | 0,9 | 2,1 | 0,9 | 3,1 |
| 0,95 | 1,8 | 0,95 | 3,7 | 0,95 | 1,7 | 0,95 | 2,3 |
| 1 | 1,2 | 1 | 1,2 | 1 | 1,2 | 1 | 1,2 |

**HF - HCFO-1233xf - HFO-1234yf - HFO-1234zeE**

| Organics 0,9 F1233xf + 0,05 F1234yf +0,05 F1234zeE | | Organics 0,05 F1233xf + 0,9 F1234yf +0,05 F1234zeE | | Organics 0,05 F1233xf + 0,05 F1234yf +0,9 F1234zeE | | Organics 0,4 F1233xf + 0,3 F1234yf +0,3 F1234zeE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 2,0 | 0 | 6,5 | 0 | 4,8 | 0 | 4,2 |
| 0,05 | 3,1 | 0,05 | 7,4 | 0,05 | 5,8 | 0,05 | 5,3 |
| 0,1 | 3,1 | 0,1 | 7,4 | 0,1 | 5,8 | 0,1 | 5,3 |
| 0,15 | 3,1 | 0,15 | 7,4 | 0,15 | 5,8 | 0,15 | 5,3 |
| 0,2 | 3,1 | 0,2 | 7,4 | 0,2 | 5,8 | 0,2 | 5,3 |
| 0,25 | 3,1 | 0,25 | 7,4 | 0,25 | 5,8 | 0,25 | 5,3 |
| 0,3 | 3,1 | 0,3 | 7,4 | 0,3 | 5,8 | 0,3 | 5,3 |
| 0,35 | 3,1 | 0,35 | 7,4 | 0,35 | 5,8 | 0,35 | 5,3 |
| 0,4 | 3,1 | 0,4 | 7,4 | 0,4 | 5,8 | 0,4 | 5,3 |
| 0,45 | 3,1 | 0,45 | 7,4 | 0,45 | 5,8 | 0,45 | 5,3 |
| 0,5 | 3,1 | 0,5 | 7,4 | 0,5 | 5,8 | 0,5 | 5,3 |
| 0,55 | 3,1 | 0,55 | 7,4 | 0,55 | 5,7 | 0,55 | 5,3 |
| 0,6 | 3,1 | 0,6 | 7,4 | 0,6 | 5,6 | 0,6 | 5,3 |
| 0,65 | 3,1 | 0,65 | 7,5 | 0,65 | 5,5 | 0,65 | 5,3 |
| 0,7 | 3,1 | 0,7 | 7,4 | 0,7 | 5,4 | 0,7 | 5,2 |
| 0,75 | 3,1 | 0,75 | 7,2 | 0,75 | 5,2 | 0,75 | 5,0 |
| 0,8 | 2,9 | 0,8 | 6,9 | 0,8 | 4,8 | 0,8 | 4,7 |
| 0,85 | 2,7 | 0,85 | 6,3 | 0,85 | 4,3 | 0,85 | 4,3 |
| 0,9 | 2,3 | 0,9 | 5,3 | 0,9 | 3,6 | 0,9 | 3,6 |
| 0,95 | 1,9 | 0,95 | 3,7 | 0,95 | 2,6 | 0,95 | 2,6 |
| 1 | 1,2 | 1 | 1,2 | 1 | 1,2 | 1 | 1,2 |

**HF - HCFO-1233xf - HFO-1234yf - HFO-1234zeZ**

| Organics 0,9 F1233xf + 0,05 F1234yf +0,05 F1234zeZ | | Organics 0,05 F1233xf +0,9 F1234yf +0,05 F1234zeZ | | Organics 0,05 F1233xf + 0,05 F1234yf +0,9 F1234zeZ | | Organics 0,4 F1233xf + 0,3 F1234yf +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,9 | 0 | 6,3 | 0 | 2,1 | 0 | 3,3 |
| 0,05 | 3,0 | 0,05 | 7,3 | 0,05 | 3,2 | 0,05 | 4,4 |
| 0,1 | 3,0 | 0,1 | 7,3 | 0,1 | 3,2 | 0,1 | 4,4 |
| 0,15 | 3,0 | 0,15 | 7,3 | 0,15 | 3,2 | 0,15 | 4,4 |
| 0,2 | 3,0 | 0,2 | 7,3 | 0,2 | 3,2 | 0,2 | 4,4 |
| 0,25 | 3,0 | 0,25 | 7,3 | 0,25 | 3,2 | 0,25 | 4,4 |
| 0,3 | 3,0 | 0,3 | 7,3 | 0,3 | 3,2 | 0,3 | 4,4 |
| 0,35 | 3,0 | 0,35 | 7,3 | 0,35 | 3,2 | 0,35 | 4,4 |
| 0,4 | 3,0 | 0,4 | 7,3 | 0,4 | 3,2 | 0,4 | 4,4 |
| 0,45 | 3,0 | 0,45 | 7,3 | 0,45 | 3,2 | 0,45 | 4,4 |
| 0,5 | 3,0 | 0,5 | 7,3 | 0,5 | 3,2 | 0,5 | 4,4 |
| 0,55 | 3,0 | 0,55 | 7,3 | 0,55 | 3,2 | 0,55 | 4,4 |
| 0,6 | 3,0 | 0,6 | 7,3 | 0,6 | 3,2 | 0,6 | 4,4 |
| 0,65 | 3,0 | 0,65 | 7,3 | 0,65 | 3,2 | 0,65 | 4,4 |
| 0,7 | 3,0 | 0,7 | 7,3 | 0,7 | 3,2 | 0,7 | 4,4 |
| 0,75 | 2,9 | 0,75 | 7,1 | 0,75 | 3,1 | 0,75 | 4,3 |
| 0,8 | 2,8 | 0,8 | 6,8 | 0,8 | 3,0 | 0,8 | 4,1 |
| 0,85 | 2,6 | 0,85 | 6,3 | 0,85 | 2,8 | 0,85 | 3,7 |
| 0,9 | 2,3 | 0,9 | 5,3 | 0,9 | 2,4 | 0,9 | 3,2 |
| 0,95 | 1,8 | 0,95 | 3,7 | 0,95 | 1,9 | 0,95 | 2,4 |
| 1 | 1,2 | 1 | 1,2 | 1 | 1,2 | 1 | 1,2 |

**HF - HCFO-1233xf - HFO-1234yf - HFO-1243zf**

| Organics 0,9 F1233xf + 0,05 F1234yf +0,05 F1243zf | | Organics 0,05 F1233xf + 0,9 F1234yf +0,05 F1243zf | | Organics 0,05 F1233xf + 0,05 F1234yf +0,9 F1243zf | | Organics 0,4 F1233xf + 0,3 F1234yf +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 2,1 | 0 | 6,5 | 0 | 5,8 | 0 | 4,6 |
| 0,05 | 3,2 | 0,05 | 7,5 | 0,05 | 6,8 | 0,05 | 5,6 |
| 0,1 | 3,2 | 0,1 | 7,5 | 0,1 | 6,8 | 0,1 | 5,7 |
| 0,15 | 3,2 | 0,15 | 7,5 | 0,15 | 6,8 | 0,15 | 5,6 |
| 0,2 | 3,2 | 0,2 | 7,5 | 0,2 | 6,8 | 0,2 | 5,6 |
| 0,25 | 3,2 | 0,25 | 7,5 | 0,25 | 6,8 | 0,25 | 5,6 |
| 0,3 | 3,2 | 0,3 | 7,5 | 0,3 | 6,8 | 0,3 | 5,6 |
| 0,35 | 3,2 | 0,35 | 7,5 | 0,35 | 6,8 | 0,35 | 5,6 |
| 0,4 | 3,2 | 0,4 | 7,5 | 0,4 | 6,8 | 0,4 | 5,6 |
| 0,45 | 3,2 | 0,45 | 7,5 | 0,45 | 6,8 | 0,45 | 5,6 |
| 0,5 | 3,2 | 0,5 | 7,5 | 0,5 | 6,8 | 0,5 | 5,6 |
| 0,55 | 3,2 | 0,55 | 7,5 | 0,55 | 6,7 | 0,55 | 5,6 |
| 0,6 | 3,2 | 0,6 | 7,5 | 0,6 | 6,7 | 0,6 | 5,6 |
| 0,65 | 3,2 | 0,65 | 7,5 | 0,65 | 6,7 | 0,65 | 5,6 |
| 0,7 | 3,2 | 0,7 | 7,4 | 0,7 | 6,6 | 0,7 | 5,6 |
| 0,75 | 3,2 | 0,75 | 7,3 | 0,75 | 6,4 | 0,75 | 5,4 |
| 0,8 | 3,0 | 0,8 | 7,0 | 0,8 | 6,1 | 0,8 | 5,1 |
| 0,85 | 2,8 | 0,85 | 6,4 | 0,85 | 5,6 | 0,85 | 4,7 |
| 0,9 | 2,4 | 0,9 | 5,4 | 0,9 | 4,8 | 0,9 | 4,0 |
| 0,95 | 1,9 | 0,95 | 3,7 | 0,95 | 3,4 | 0,95 | 2,9 |
| 1 | 1,2 | 1 | 1,2 | 1 | 1,2 | 1 | 1,2 |

**HF - HFO-1234yf - HFO-1234zeE - HFO-1234zeZ**

| Organics 0,9 F1234yf + 0,05 F1234zeE +0,05 F1234zeZ | | Organics 0,05 F1234yf + 0,9 F1234zeE +0,05 F1234zeZ | | Organics 0,05 F1234yf + 0,05 F1234zeE +0,9 F1234zeZ | | Organics 0,4 F1234yf + 0,3 F1234zeE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,8 | 0 | 2,2 | 0 | 4,7 |
| 0,05 | 7,4 | 0,05 | 5,8 | 0,05 | 3,3 | 0,05 | 5,7 |
| 0,1 | 7,4 | 0,1 | 5,8 | 0,1 | 3,3 | 0,1 | 5,7 |
| 0,15 | 7,4 | 0,15 | 5,8 | 0,15 | 3,3 | 0,15 | 5,7 |
| 0,2 | 7,4 | 0,2 | 5,8 | 0,2 | 3,3 | 0,2 | 5,7 |
| 0,25 | 7,4 | 0,25 | 5,8 | 0,25 | 3,3 | 0,25 | 5,8 |
| 0,3 | 7,4 | 0,3 | 5,8 | 0,3 | 3,3 | 0,3 | 5,8 |
| 0,35 | 7,4 | 0,35 | 5,8 | 0,35 | 3,3 | 0,35 | 5,8 |
| 0,4 | 7,4 | 0,4 | 5,8 | 0,4 | 3,3 | 0,4 | 5,8 |
| 0,45 | 7,4 | 0,45 | 5,8 | 0,45 | 3,3 | 0,45 | 5,8 |
| 0,5 | 7,4 | 0,5 | 5,7 | 0,5 | 3,3 | 0,5 | 5,8 |
| 0,55 | 7,4 | 0,55 | 5,7 | 0,55 | 3,3 | 0,55 | 5,8 |
| 0,6 | 7,4 | 0,6 | 5,6 | 0,6 | 3,3 | 0,6 | 5,8 |
| 0,65 | 7,4 | 0,65 | 5,5 | 0,65 | 3,3 | 0,65 | 5,7 |
| 0,7 | 7,4 | 0,7 | 5,4 | 0,7 | 3,3 | 0,7 | 5,6 |
| 0,75 | 7.2 | 0,75 | 5,2 | 0,75 | 3,3 | 0,75 | 5,5 |
| 0,8 | 6,9 | 0,8 | 4,8 | 0,8 | 3,1 | 0,8 | 5,2 |
| 0,85 | 6,3 | 0,85 | 4,3 | 0,85 | 2,9 | 0,85 | 4,7 |
| 0,9 | 5,3 | 0,9 | 3,6 | 0,9 | 2,5 | 0,9 | 4,0 |
| 0,95 | 3,7 | 0,95 | 2,6 | 0,95 | 2,0 | 0,95 | 2,9 |
| 1 | 1,2 | 1 | 1,2 | 1 | 1,2 | 1 | 1,2 |

**HF - HFO-1234yf - HFO-1234zeE - HCFO-1233zdE**

| Organics 0,9 F1234yf + 0,05 F1234zeE +0,05 F1233zdE | | Organics 0,05 F1234yf + 0,9 F1234zeE +0,05 F1233zdE | | Organics 0,05 F1234yf + 0,05 F1234zeE +0,9 F1233zdE | | Organics 0,4 F1234yf + 0,3 F1234zeE +0,3 F1233zdE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,8 | 0 | 1,8 | 0 | 4,7 |
| 0,05 | 7,4 | 0,05 | 5,8 | 0,05 | 2,9 | 0,05 | 5,7 |
| 0,1 | 7,4 | 0,1 | 5,8 | 0,1 | 2,9 | 0,1 | 5,7 |
| 0,15 | 7,4 | 0,15 | 5,8 | 0,15 | 2,9 | 0,15 | 5,7 |
| 0,2 | 7,4 | 0,2 | 5,8 | 0,2 | 2,9 | 0,2 | 5,7 |
| 0,25 | 7,4 | 0,25 | 5,8 | 0,25 | 2,9 | 0,25 | 5,7 |
| 0,3 | 7,4 | 0,3 | 5,8 | 0,3 | 2,9 | 0,3 | 5,7 |
| 0,35 | 7,4 | 0,35 | 5,8 | 0,35 | 2,9 | 0,35 | 5,7 |
| 0,4 | 7,4 | 0,4 | 5,8 | 0,4 | 2,9 | 0,4 | 5,7 |
| 0,45 | 7,4 | 0,45 | 5,8 | 0,45 | 2,9 | 0,45 | 5,7 |
| 0,5 | 7,4 | 0,5 | 5,7 | 0,5 | 2,9 | 0,5 | 5,7 |
| 0,55 | 7,4 | 0,55 | 5,7 | 0,55 | 2,9 | 0,55 | 5,7 |
| 0,6 | 7,4 | 0,6 | 5,6 | 0,6 | 2,9 | 0,6 | 5,7 |
| 0,65 | 7,4 | 0,65 | 5.5 | 0,65 | 2,9 | 0,65 | 5,7 |
| 0,7 | 7,4 | 0,7 | 5,4 | 0,7 | 2,9 | 0,7 | 5,6 |
| 0,75 | 7,2 | 0,75 | 5,1 | 0,75 | 2,9 | 0,75 | 5,4 |
| 0,8 | 6,9 | 0,8 | 4,8 | 0,8 | 2,7 | 0,8 | 5,1 |
| 0,85 | 6,3 | 0,85 | 4,3 | 0,85 | 2,5 | 0,85 | 4,7 |
| 0,9 | 5,3 | 0,9 | 3,6 | 0,9 | 2,2 | 0,9 | 3,9 |
| 0,95 | 3,7 | 0,95 | 2,6 | 0,95 | 1,8 | 0,95 | 2,8 |
| 1 | 1,2 | 1 | 1,2 | 1 | 1,2 | 1 | 1,2 |

**HF - HFO-1234yf - HFO-1234zeE - HFO-1243zf**

| Organics 0,9 F1234yf + 0,05 F1234zeE +0,05 F1243zf | | Organics 0,05 F1234yf + 0,9 F1234zeE+0,05 F1243zf | | Organics 0,05 F1234yf + 0,05 F1234zeE +0,9 F1243zf | | Organics 0,4 F1234yf + 0,3 F1234zeE +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,7 | 0 | 5,0 | 0 | 5,8 | 0 | 5,9 |
| 0,05 | 7,6 | 0,05 | 6,0 | 0,05 | 6,8 | 0,05 | 6,9 |
| 0,1 | 7,6 | 0,1 | 6,0 | 0,1 | 6,8 | 0,1 | 6,9 |
| 0,15 | 7,6 | 0,15 | 6,0 | 0,15 | 6,8 | 0,15 | 6,9 |
| 0,2 | 7,6 | 0,2 | 6,0 | 0,2 | 6,8 | 0,2 | 6,9 |
| 0,25 | 7,6 | 0,25 | 6,0 | 0,25 | 6,8 | 0,25 | 6,9 |
| 0,3 | 7,6 | 0,3 | 6,0 | 0,3 | 6,8 | 0,3 | 6,9 |
| 0,35 | 7,6 | 0,35 | 6,0 | 0,35 | 6,8 | 0,35 | 6,9 |
| 0,4 | 7,6 | 0,4 | 6,0 | 0,4 | 6,8 | 0,4 | 6,9 |
| 0,45 | 7,6 | 0,45 | 6,0 | 0,45 | 6,8 | 0,45 | 6,9 |
| 0,5 | 7,6 | 0,5 | 6,0 | 0,5 | 6,8 | 0,5 | 6,9 |
| 0,55 | 7,6 | 0,55 | 5,9 | 0,55 | 6,8 | 0,55 | 6,9 |
| 0,6 | 7,6 | 0,6 | 5,8 | 0,6 | 6,7 | 0,6 | 6,9 |
| 0,65 | 7,6 | 0,65 | 5,7 | 0,65 | 6,7 | 0,65 | 6,8 |
| 0,7 | 7,6 | 0,7 | 5,6 | 0,7 | 6,6 | 0,7 | 6,7 |
| 0,75 | 7,4 | 0,75 | 5,4 | 0,75 | 6,4 | 0,75 | 6,5 |
| 0,8 | 7,1 | 0,8 | 5,0 | 0,8 | 6,1 | 0,8 | 6,2 |
| 0,85 | 6,5 | 0,85 | 4,5 | 0,85 | 5,6 | 0,85 | 5,7 |
| 0,9 | 5,5 | 0,9 | 3,8 | 0,9 | 4,8 | 0,9 | 4,8 |
| 0,95 | 3,8 | 0,95 | 2,7 | 0,95 | 3,4 | 0,95 | 3,3 |
| 1 | 1,2 | 1 | 1,2 | 1 | 1,2 | 1 | 1,2 |

**HF - HFO-1234yf - HFO-1234zeZ - HCFO-1233zdE**

| Organics 0,9 F1234yf + 0,05 F1234zeZ +0,05 F1233zdE | | Organics 0,05 F1234yf + 0,9 F1234zeZ+0,05 F1233zdE | | Organics 0,05 F1234yf + 0,05 F1234zeZ+0,9 F1233zdE | | Organics 0,4 F1234yf + 0,3 F1234zeZ +0,3 F1233zdE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,3 | 0 | 2,0 | 0 | 1,7 | 0 | 3,8 |
| 0,05 | 7,3 | 0,05 | 3,2 | 0,05 | 2,8 | 0,05 | 4,8 |
| 0,1 | 7,3 | 0,1 | 3,2 | 0,1 | 2,8 | 0,1 | 4,8 |
| 0,15 | 7,3 | 0,15 | 3,2 | 0,15 | 2,8 | 0,15 | 4,8 |
| 0,2 | 7,3 | 0,2 | 3,2 | 0,2 | 2,8 | 0,2 | 4,8 |
| 0,25 | 7,3 | 0,25 | 3,2 | 0,25 | 2,8 | 0,25 | 4,8 |
| 0,3 | 7,3 | 0,3 | 3,2 | 0,3 | 2,8 | 0,3 | 4,8 |
| 0,35 | 7,3 | 0,35 | 3,1 | 0,35 | 2,8 | 0,35 | 4,8 |
| 0,4 | 7,3 | 0,4 | 3,1 | 0,4 | 2,8 | 0,4 | 4,8 |
| 0,45 | 7,3 | 0,45 | 3,1 | 0,45 | 2,8 | 0,45 | 4,8 |
| 0,5 | 7,3 | 0,5 | 3,1 | 0,5 | 2,8 | 0,5 | 4,8 |
| 0,55 | 7,3 | 0,55 | 3,1 | 0,55 | 2,8 | 0,55 | 4,8 |
| 0,6 | 7,3 | 0,6 | 3,1 | 0,6 | 2,8 | 0,6 | 4,8 |
| 0,65 | 7,3 | 0,65 | 3,1 | 0,65 | 2,8 | 0,65 | 4,8 |
| 0,7 | 7,3 | 0,7 | 3,1 | 0,7 | 2,7 | 0,7 | 4,8 |
| 0,75 | 7.1 | 0,75 | 3,1 | 0,75 | 2,7 | 0,75 | 4,7 |
| 0,8 | 6,8 | 0,8 | 3,0 | 0,8 | 2,6 | 0,8 | 4,5 |
| 0,85 | 62 | 0,85 | 2.8 | 0,85 | 2,4 | 0,85 | 4,1 |
| 0,9 | 5,3 | 0,9 | 2,4 | 0,9 | 2,2 | 0,9 | 3,5 |
| 0,95 | 3,7 | 0,95 | 1,9 | 0,95 | 1,7 | 0,95 | 2,6 |
| 1 | 1,2 | 1 | 1,2 | 1 | 1,2 | 1 | 1,2 |

**HF- HFO-1234yf - HFO-1234zeZ - HFO-1243zf**

| Organics 0,9 F1234yf + 0,05 F1234zeZ+0,05 F1243zf | | Organics 0,05 F1234yf + 0,9 F1234zeZ +0,05 F1243zf | | Organics 0,05 F1234yf + 0,05 F1234zeZ +0,9 F1243zf | | Organics 0,4 F1234yf + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,5 | 0 | 2,3 | 0 | 5,7 | 0 | 5,0 |
| 0,05 | 7,5 | 0,05 | 3,4 | 0,05 | 6,7 | 0,05 | 6,0 |
| 0,1 | 7,5 | 0,1 | 3,4 | 0,1 | 6,7 | 0,1 | 6,1 |
| 0,15 | 7,5 | 0,15 | 3,4 | 0,15 | 6,7 | 0,15 | 6,1 |
| 0,2 | 7,4 | 0,2 | 3,4 | 0,2 | 6,7 | 0,2 | 6,1 |
| 0,25 | 7,4 | 0,25 | 3,4 | 0,25 | 6,7 | 0,25 | 6,1 |
| 0,3 | 7,4 | 0,3 | 3,4 | 0,3 | 6,7 | 0,3 | 6,1 |
| 0,35 | 7,5 | 0,35 | 3,4 | 0,35 | 6,7 | 0,35 | 6,1 |
| 0,4 | 7,5 | 0,4 | 3,4 | 0,4 | 6,7 | 0,4 | 6,1 |
| 0,45 | 7,5 | 0,45 | 3,4 | 0,45 | 6,7 | 0,45 | 6,1 |
| 0,5 | 7,5 | 0,5 | 3,4 | 0,5 | 6,7 | 0,5 | 6,1 |
| 0,55 | 7,5 | 0,55 | 3,4 | 0,55 | 6,6 | 0,55 | 6,1 |
| 0,6 | 7,5 | 0,6 | 3,4 | 0,6 | 6,6 | 0,6 | 6,1 |
| 0,65 | 7,5 | 0,65 | 3,4 | 0,65 | 6,6 | 0,65 | 6,1 |
| 0,7 | 7,4 | 0,7 | 3,4 | 0,7 | 6,5 | 0,7 | 6,0 |
| 0,75 | 7,3 | 0,75 | 3,3 | 0,75 | 6,3 | 0,75 | 5,9 |
| 0,8 | 7,0 | 0,8 | 3,2 | 0,8 | 6,0 | 0,8 | 5,6 |
| 0,85 | 6,4 | 0,85 | 3,0 | 0,85 | 5,5 | 0,85 | 5,1 |
| 0,9 | 5,4 | 0,9 | 2,6 | 0,9 | 4,7 | 0,9 | 4,4 |
| 0,95 | 3,7 | 0,95 | 2,0 | 0,95 | 3,3 | 0,95 | 3,1 |
| 1 | 1,2 | 1 | 1,2 | 1 | 1,2 | 1 | 1,2 |

**HF - HFO-1234yf - HCFO-1233zdE - HFO-1243zf**

| Organics 0,9 F1234yf + 0,05 F1233zdE +0,05 F1243zf | | Organics 0,05 F1234yf + 0,9 F1233zdE +0,05 F1243zf | | Organics 0,05 F1234yf + 0,05 F1233zdE +0,9 F1243zf | | Organics 0,4 F1234yf + 0,3 F1233zdE +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,5 | 0 | 1,9 | 0 | 5,7 | 0 | 5,0 |
| 0,05 | 7,5 | 0,05 | 3,0 | 0,05 | 6,7 | 0,05 | 6,0 |
| 0,1 | 7,5 | 0,1 | 3,0 | 0,1 | 6,7 | 0,1 | 6,0 |
| 0,15 | 7,5 | 0,15 | 3,0 | 0,15 | 6,7 | 0,15 | 6,0 |
| 0,2 | 7,4 | 0,2 | 3,0 | 0,2 | 6,7 | 0,2 | 6,0 |
| 0,25 | 7,4 | 0,25 | 3,0 | 0,25 | 6,7 | 0,25 | 6,0 |
| 0,3 | 7,5 | 0,3 | 3,0 | 0,3 | 6,7 | 0,3 | 6,0 |
| 0,35 | 7,5 | 0,35 | 3,0 | 0,35 | 6,7 | 0,35 | 6,0 |
| 0,4 | 7,5 | 0,4 | 3,0 | 0,4 | 6,7 | 0,4 | 6,0 |
| 0,45 | 7,5 | 0,45 | 3,0 | 0,45 | 6,7 | 0,45 | 6,0 |
| 0,5 | 7,5 | 0,5 | 3,0 | 0,5 | 6,6 | 0,5 | 6,0 |
| 0,55 | 7,5 | 0,55 | 3,0 | 0,55 | 6,6 | 0,55 | 6,0 |
| 0,6 | 7,5 | 0,6 | 3,0 | 0,6 | 6,6 | 0,6 | 6,0 |
| 0,65 | 7,5 | 0,65 | 3,0 | 0,65 | 6,6 | 0,65 | 6,0 |
| 0,7 | 7,4 | 0,7 | 3,0 | 0,7 | 6,5 | 0,7 | 6,0 |
| 0,75 | 7,3 | 0,75 | 3,0 | 0,75 | 6,3 | 0,75 | 5,8 |
| 0,8 | 7,0 | 0,8 | 2,8 | 0,8 | 6,0 | 0,8 | 5,5 |
| 0,85 | 6,4 | 0,85 | 2,6 | 0,85 | 5,5 | 0,85 | 5,1 |
| 0,9 | 5,4 | 0,9 | 2,3 | 0,9 | 4,7 | 0,9 | 4,3 |
| 0,95 | 3,7 | 0,95 | 1,8 | 0,95 | 3,3 | 0,95 | 3,1 |
| 1 | 1,2 | 1 | 1,2 | 1 | 1,2 | 1 | 1,2 |

**HF - HFO-1234yf - HFC-245cb - Trifluoropropyne**

| **Organics 0,34 F1234yf + 0,33 F245cb + 0,33 TFP** | | **Organics 0,99 F1234yf + 0,005 F245cb + 0,005 TFP** | | **Organics 0,005 F1234yf + 0,99 F245cb + 0,005 TFP** | | **Organics 0,005 F1234yf + 0,005 F245cb + 0,99 TFP** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 8,0 | 0 | 6,8 | 0 | 4,7 | 0 | 11,6 |
| 0,05 | 8,9 | 0,05 | 7,8 | 0,05 | 5,9 | 0,05 | 12,4 |
| 0,1 | 8,9 | 0,1 | 7,8 | 0,1 | 5,9 | 0,1 | 12,3 |
| 0,15 | 8,8 | 0,15 | 7,8 | 0,15 | 5,9 | 0,15 | 12,2 |
| 02 | 8,8 | 0,2 | 7,8 | 0,2 | 5,9 | 0,2 | 12,1 |
| 0,25 | 8,7 | 0,25 | 7,8 | 0,25 | 5,9 | 0,25 | 12,0 |
| 0,3 | 8,8 | 0,3 | 7,7 | 0,3 | 5,9 | 0,3 | 11,9 |
| 0,35 | 8,8 | 0,35 | 7,8 | 0,35 | 5,9 | 0,35 | 11,8 |
| 0,4 | 8,8 | 0,4 | 7,8 | 0,4 | 5,9 | 0,4 | 11,7 |
| 0,45 | 8,8 | 0,45 | 7,8 | 0,45 | 5,9 | 0,45 | 11,8 |
| 0,5 | 8,7 | 0,5 | 7,8 | 0,5 | 5,9 | 0,5 | 11,8 |
| 0,55 | 8,7 | 0,55 | 7,8 | 0,55 | 5,9 | 0,55 | 11,8 |
| 0,6 | 8,7 | 0,6 | 7,8 | 0,6 | 5,9 | 0,6 | 11,8 |
| 0,65 | 8,7 | 0,65 | 7.8 | 0,65 | 5,9 | 0,65 | 11,8 |
| 0,7 | 8,7 | 0,7 | 7,7 | 0,7 | 5,9 | 0,7 | 11,8 |
| 0,75 | 8,7 | 0,75 | 7,6 | 0,75 | 5,9 | 0,75 | 11,8 |
| 0,8 | 8,5 | 0,8 | 7,3 | 0,8 | 5,9 | 0,8 | 11,6 |
| 0,85 | 7,9 | 0,85 | 6,7 | 0,85 | 5,9 | 0,85 | 10,8 |
| 0,9 | 6,6 | 0,9 | 5,6 | 0,9 | 5,1 | 0,9 | 9,1 |
| 0,95 | 4,5 | 0,95 | 3,9 | 0,95 | 3,6 | 0,95 | 6,1 |
| 1 | 1,2 | 1 | 1,2 | 1 | 1,2 | 1 | 1,2 |

**HF - HFO-1234yf- HFC-245cb - HCFC-244bb**

| **Organics 0,34 F1234yf + 0,33 F245cb + 0.33 F244bb** | | **Organics 0,99 F1234yf + 0,005 F245cb + 0,005 F244bb** | | **Organics 0,005 F1234yf + 0,99 F245cb + 0,005 F244bb** | | **Organics 0,005 F1234yf + 0,005 F245cb + 0,99 F244bb** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRES | HF | PRES | HF | PRES |
| | bar | | bar | | bar | | bar |
| 0 | 4,3 | 0 | 6,8 | 0 | 4,6 | 0 | 0,7 |
| 0,05 | 5,4 | 0,05 | 7,7 | 0,05 | 5,8 | 0,05 | 1,8 |
| 0,1 | 5,4 | 0,1 | 7,7 | 0,1 | 5,8 | 0,1 | 1,8 |
| 0,15 | 5,4 | 0,15 | 7,7 | 0,15 | 5,8 | 0,15 | 1,8 |
| 0,2 | 5,4 | 0,2 | 7,7 | 0,2 | 5,8 | 0,2 | 1,8 |
| 0,25 | 5,4 | 0,25 | 7,7 | 0,25 | 5,8 | 0,25 | 1,8 |
| 0,3 | 5,4 | 0,3 | 7,7 | 0,3 | 5,8 | 0,3 | 1,8 |
| 0,35 | 5,4 | 0,35 | 7,7 | 0,35 | 5,8 | 0,35 | 1,8 |
| 0,4 | 5,4 | 0,4 | 7,7 | 0,4 | 5,8 | 0,4 | 1,8 |
| 0,45 | 5,3 | 0,45 | 7,7 | 0,45 | 5,8 | 0,45 | 1,8 |
| 0,5 | 5,3 | 0,5 | 7,7 | 0,5 | 5,8 | 0,5 | 1,8 |
| 0,55 | 5,3 | 0,55 | 7,7 | 0,55 | 5,8 | 0,55 | 1,8 |
| 0,6 | 5,3 | 0,6 | 7,7 | 0,6 | 5,8 | 0,6 | 1,8 |
| 0,65 | 5,3 | 0,65 | 7,7 | 0,65 | 5,8 | 0,65 | 1,8 |
| 0,7 | 5,2 | 0,7 | 7,7 | 0,7 | 5,8 | 0,7 | 1,8 |
| 0,75 | 5,2 | 0,75 | 7,5 | 0,75 | 5,8 | 0,75 | 1,8 |
| 0,8 | 5,1 | 0,8 | 7,2 | 0,8 | 5,8 | 0,8 | 1,8 |
| 0,85 | 4,8 | 0,85 | 6,6 | 0,85 | 5,8 | 0,85 | 1,8 |
| 0,9 | 4,2 | 0,9 | 5,6 | 0,9 | 5,0 | 0,9 | 1,8 |
| 0,95 | 3,0 | 0,95 | 3,9 | 0,95 | 3,6 | 0,95 | 1,5 |
| 1 | 1,2 | 1 | 1,2 | 1 | 1,2 | 1 | 1,2 |

**HF- HFO-1234yf - HFC-245cb - HFC-245fa**

| **Organics 0,34 F1234yf + 0,33 F245cb + 0,33 F245fa** | | **Organics 0,99 F1234yf + 0,005 F245cb + 0,005 F245fa** | | **Organics 0,005 F1234yf + 0,99 F245cb + 0, 005 F245fa** | | **Organics 0,005 F1234yf + 0,005 F245cb + 0,99 F245fa** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,4 | 0 | 6,8 | 0 | 4,6 | 0 | 1,6 |
| 0,05 | 5,6 | 0,05 | 7,7 | 0,05 | 5,8 | 0,05 | 2,7 |
| 0,1 | 5,6 | 0,1 | 7,7 | 0,1 | 5,8 | 0,1 | 2,7 |
| 0,15 | 5,6 | 0,15 | 7,7 | 0,15 | 5,8 | 0,15 | 2,7 |
| 0,2 | 5,6 | 0,2 | 7,7 | 0,2 | 5,8 | 0,2 | 2,7 |
| 0,25 | 5,6 | 0,25 | 7,7 | 0,25 | 5,8 | 0,25 | 2,7 |
| 0,3 | 5,6 | 0,3 | 7,7 | 0,3 | 5,8 | 0,3 | 2,7 |
| 0,35 | 5,6 | 0,35 | 7,7 | 0,35 | 5,8 | 0,35 | 2,7 |
| 0,4 | 5,6 | 0,4 | 7,7 | 0,4 | 5,8 | 0,4 | 2,7 |
| 0,45 | 5,6 | 0,45 | 7,7 | 0,45 | 5,8 | 0,45 | 2,7 |
| 0,5 | 5,6 | 0,5 | 7,7 | 0,5 | 5,8 | 0,5 | 2,7 |
| 0,55 | 5,6 | 0,55 | 7,7 | 0,55 | 5,8 | 0,55 | 2,7 |
| 0,6 | 5,6 | 0,6 | 7,7 | 0,6 | 5,8 | 0,6 | 2,7 |
| 0,65 | 5,6 | 0,65 | 7,7 | 0,65 | 5,8 | 0,65 | 2,7 |
| 0,7 | 5,6 | 0,7 | 7,7 | 0,7 | 5,8 | 0,7 | 2,7 |
| 0,75 | 5,6 | 0,75 | 7,5 | 0,75 | 5,8 | 0,75 | 2,7 |
| 0,8 | 5,5 | 0,8 | 7,2 | 0,8 | 5,8 | 0,8 | 2,6 |
| 0,85 | 5,0 | 0,85 | 6,6 | 0,85 | 5,8 | 0,85 | 2,4 |
| 0,9 | 4,3 | 0,9 | 5,6 | 0,9 | 5,0 | 0,9 | 2,1 |
| 0,95 | 3,1 | 0,95 | 3,9 | 0,95 | 3,6 | 0,95 | 1,7 |
| 1 | 1,2 | 1 | 1,2 | 1 | 1,2 | 1 | 1,2 |

**HF - HFO-1234yf - HFC-245cb - HFO-1225yeZ**

| **Organics 0,34 F1234yf + 0,33 F245cb +0.33 F1225yeZ** | | **Organics 0,99 F1234yf + 0,005 F245cb + 0.005 F1225yeZ** | | **Organics 0,005 F1234yf + 0,99 F245cb + 0.005 F1225yeZ** | | **Organics 0,005 F1234yf + 0,005 F245cb + 0.99 F1225yeZ** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 5,6 | 0 | 6,8 | 0 | 4,7 | 0 | 5,2 |
| 0,05 | 6,7 | 0,05 | 7,7 | 0,05 | 5,8 | 0,05 | 6,2 |
| 0,1 | 6,7 | 0,1 | 7,8 | 0,1 | 5,8 | 0,1 | 6,2 |
| 0,15 | 6,7 | 0,15 | 7,7 | 0,15 | 5,8 | 0,15 | 6,2 |
| 0,2 | 6,7 | 0,2 | 7,7 | 0,2 | 5,8 | 0,2 | 6,2 |
| 0,25 | 6,7 | 0,25 | 7,7 | 0,25 | 5,8 | 0,25 | 6,2 |
| 0,3 | 6,7 | 0,3 | 7,7 | 0,3 | 5,8 | 0,3 | 6,2 |
| 0,35 | 6,7 | 0,35 | 7,7 | 0,35 | 5,8 | 0,35 | 6,2 |
| 0,4 | 6,7 | 0,4 | 7,7 | 0,4 | 5,8 | 0,4 | 6,2 |
| 0,45 | 6,7 | 0,45 | 7,7 | 0,45 | 5,8 | 0,45 | 6,2 |
| 0,5 | 6,7 | 0,5 | 7,7 | 0,5 | 5,8 | 0,5 | 6,2 |
| 0,55 | 6,7 | 0,55 | 7,7 | 0,55 | 5,8 | 0,55 | 6,2 |
| 0,6 | 6,7 | 0,6 | 7,7 | 0,6 | 5,8 | 0,6 | 6,2 |
| 0,65 | 6,7 | 0,65 | 7,7 | 0,65 | 5,8 | 0,65 | 6,2 |
| 0,7 | 6,6 | 0,7 | 7,7 | 0,7 | 5,8 | 0,7 | 6,1 |
| 0,75 | 6,6 | 0,75 | 7,6 | 0,75 | 5,8 | 0,75 | 5,9 |
| 0,8 | 6,4 | 0,8 | 7,3 | 0,8 | 5,8 | 0,8 | 5,6 |
| 0,85 | 5,9 | 0,85 | 6,6 | 0,85 | 5,8 | 0,85 | 5,1 |
| 0,9 | 5,0 | 0,9 | 5,6 | 0,9 | 5,0 | 0,9 | 4,2 |
| 0,95 | 3,5 | 0,95 | 3,9 | 0,95 | 3,6 | 0,95 | 3,0 |
| 1 | 1,2 | 1 | 1,2 | 1 | 1,2 | 1 | 1,2 |

**HF - HFO-1234yf - HFC-245cb - HFO-1225zc**

| **Organics 0,34 F1234yf + 0,33 F245cb + 0,33 F1225zc** | | **Organics 0,99 F1234yf + 0,005 F245cb + 0,005 F1225zc** | | **Organics 0,005 F1234yf + 0,99 F245cb +0,005 F1225zc** | | **Organics 0,005 F1234yf + 0,005 F245cb + 0.99 F1225zc** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 5,7 | 0 | 6,8 | 0 | 4.7 | 0 | 5,3 |
| 0,05 | 6,7 | 0,05 | 7,7 | 0,05 | 5,8 | 0,05 | 6,3 |
| 0,1 | 6,7 | 0,1 | 7,8 | 0,1 | 5,8 | 0,1 | 6,3 |
| 0,15 | 6,7 | 0,15 | 7,7 | 0,15 | 5,8 | 0,15 | 6,3 |
| 0,2 | 6,7 | 0,2 | 7,7 | 0,2 | 5,8 | 0,2 | 6,3 |
| 0,25 | 6,7 | 0,25 | 7,7 | 0,25 | 5,8 | 0,25 | 6,3 |
| 0,3 | 6,7 | 0,3 | 7,7 | 0,3 | 5,8 | 0,3 | 6,3 |
| 0,35 | 6,7 | 0,35 | 7,7 | 0,35 | 5,8 | 0,35 | 6,3 |
| 0,4 | 6,7 | 0,4 | 7,7 | 0,4 | 5,8 | 0,4 | 6,3 |
| 0,45 | 6,7 | 0,45 | 7,7 | 0,45 | 5,8 | 0,45 | 6,3 |
| 0,5 | 6,7 | 0,5 | 7,7 | 0,5 | 5,8 | 0,5 | 6,3 |
| 0,55 | 6,7 | 0,55 | 7,7 | 0,55 | 5,8 | 0,55 | 6,2 |
| 0,6 | 6,7 | 0,6 | 7,7 | 0,6 | 5,8 | 0,6 | 6,1 |
| 0,65 | 6,7 | 0,65 | 7,7 | 0,65 | 5,8 | 0,65 | 6,0 |
| 0,7 | 6,7 | 0,7 | 7,7 | 0,7 | 5,8 | 0,7 | 5,9 |
| 0,75 | 6,6 | 0,75 | 7,6 | 0,75 | 5,8 | 0,75 | 5,6 |
| 0,8 | 6,3 | 0,8 | 7,2 | 0,8 | 5,8 | 0,8 | 5,2 |
| 0,85 | 5,8 | 0,85 | 6,6 | 0,85 | 5,8 | 0,85 | 4,6 |
| 0,9 | 4,9 | 0,9 | 5,6 | 0,9 | 5,0 | 0,9 | 3,8 |
| 0,95 | 3,4 | 0,95 | 3,9 | 0,95 | 3,6 | 0,95 | 2,7 |
| 1 | 1,2 | 1 | 1,2 | 1 | 1,2 | 1 | 1,2 |

**HF - HFO-1234yf - HFO-1234zeE - Trifluoropropyne**

| **Organics 0,34 F1234yf + 0,33 F1234zeE + 0.33 TFP** | | **Organics 0,99 F1234yf + 0,005 F1234zeE + 0,005 TFP** | | **Organics 0,005 F1234yf + 0,99 F1234zeE + 0,005 TFP** | | **Organics 0,005 F1234yf + 0,005 F1234zeE + 0.99 TFP** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 8,0 | 0 | 6,8 | 0 | 4,9 | 0 | 11,6 |
| 0,05 | 8,8 | 0,05 | 7,8 | 0,05 | 5,9 | 0,05 | 12,4 |
| 0,1 | 8,8 | 0,1 | 7,8 | 0,1 | 5,9 | 0,1 | 12,3 |
| 0,15 | 8,8 | 0,15 | 7,8 | 0,15 | 5,9 | 0,15 | 12,2 |
| 0,2 | 8,8 | 0,2 | 7,8 | 0,2 | 5,9 | 0,2 | 12,1 |
| 0,25 | 8,7 | 0,25 | 7,8 | 0,25 | 5,9 | 0,25 | 12,0 |
| 0,3 | 8,7 | 0,3 | 7,7 | 0,3 | 5,9 | 0,3 | 11,9 |
| 0,35 | 8,7 | 0,35 | 7,8 | 0,35 | 5,9 | 0,35 | 11,8 |
| 0,4 | 8,7 | 0,4 | 7,8 | 0,4 | 5,9 | 0,4 | 11,8 |
| 0,45 | 8,7 | 0,45 | 7,8 | 0,45 | 5,9 | 0,45 | 11,8 |
| 0,5 | 8,7 | 0,5 | 7,8 | 0,5 | 5,8 | 0,5 | 11,8 |
| 0,55 | 8,7 | 0,55 | 7,8 | 0,55 | 5,8 | 0,55 | 11,8 |
| 0,6 | 8,7 | 0,6 | 7,8 | 0,6 | 5,7 | 0,6 | 11,8 |
| 0,65 | 8,7 | 0,65 | 7,8 | 0,65 | 5,6 | 0,65 | 11,8 |
| 0,7 | 8,6 | 0,7 | 7,7 | 0,7 | 5,4 | 0,7 | 11,8 |
| 0,75 | 8,5 | 0,75 | 7,6 | 0,75 | 5,2 | 0,75 | 11,8 |
| 0,8 | 8,1 | 0,8 | 7,3 | 0,8 | 4,8 | 0,8 | 11,6 |
| 0,85 | 7,4 | 0,85 | 6,7 | 0,85 | 4,3 | 0,85 | 10,8 |
| 0,9 | 6,2 | 0,9 | 5,6 | 0,9 | 3,6 | 0,9 | 9.1 |
| 0,95 | 4,2 | 0,95 | 3,9 | 0,95 | 2,6 | 0,95 | 6,1 |
| 1 | 1,2 | 1 | 1,2 | 1 | 1,2 | 1 | 1,2 |

**HF - HFO-1234yf - HFO-1234zeE - HCFC-244bb**

| **Organics 0,34 F1234yf + 0,33 F1234zeE + 0.33 F244bb** | | **Organics 0,99 F1234yf + 0,005 F1234zeE + 0,005 F244bb** | | **Organics 0,005 F1234yf + 0,99 F1234zeE +0,005 F244bb** | | **Organics 0,005 F1234yf + 0,005 F1234zeE +0,99 F244bb** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,4 | 0 | 6,8 | 0 | 4,9 | 0 | 0,7 |
| 0,05 | 5,4 | 0,05 | 7,7 | 0,05 | 5,8 | 0,05 | 1,8 |
| 0,1 | 5,4 | 0,1 | 7,7 | 0,1 | 5,8 | 0,1 | 1,8 |
| 0,15 | 5,4 | 0,15 | 7,7 | 0,15 | 5,8 | 0,15 | 1,8 |
| 0,2 | 5,4 | 0,2 | 7,7 | 0,2 | 5,8 | 0,2 | 1,8 |
| 0,25 | 5,4 | 0,25 | 7,7 | 0,25 | 5,8 | 0,25 | 1,8 |
| 0,3 | 5,4 | 0,3 | 7,7 | 0,3 | 5,8 | 0,3 | 1,8 |
| 0,35 | 5,4 | 0,35 | 7,7 | 0,35 | 5,8 | 0,35 | 1,8 |
| 0,4 | 5,4 | 0,4 | 7,7 | 0,4 | 5,8 | 0,4 | 1,8 |
| 0,45 | 5,3 | 0,45 | 7,7 | 0,45 | 5,8 | 0,45 | 1,8 |
| 0,5 | 5,3 | 0,5 | 7,7 | 0,5 | 5,8 | 0,5 | 1,8 |
| 0,55 | 5,3 | 0,55 | 7,7 | 0,55 | 5,7 | 0,55 | 1,8 |
| 0,6 | 5,2 | 0,6 | 7,7 | 0,6 | 5,6 | 0,6 | 1,8 |
| 0,65 | 5,2 | 0,65 | 7,7 | 0,65 | 5,5 | 0,65 | 1,8 |
| 0,7 | 5,1 | 0,7 | 7,7 | 0,7 | 5,4 | 0,7 | 1,8 |
| 0,75 | 5,0 | 0,75 | 7,5 | 0,75 | 5,1 | 0,75 | 1,8 |
| 0,8 | 4,8 | 0,8 | 7,2 | 0,8 | 4,8 | 0,8 | 1,8 |
| 0,85 | 4,3 | 0,85 | 6,6 | 0,85 | 4,3 | 0,85 | 1,8 |
| 0,9 | 3,7 | 0,9 | 5,6 | 0,9 | 3,6 | 0,9 | 1,7 |
| 0,95 | 2,7 | 0,95 | 3,8 | 0,95 | 2,6 | 0,95 | 1,5 |
| 1 | 1,2 | 1 | 1,2 | 1 | 1,2 | 1 | 1,2 |

**HF - HFO-1234yf - HFO-1234zeE - HFC-245fa**

| **Organics 0,34 F1234yf + 0,33 F1234zeE + 0,33 F245fa** | | **Organics 0,99 F1234yf + 0,005 F1234zeE + 0,005 F245fa** | | **Organics 0,005 F1234yf + 0,99 F1234zeE + 0,005 F245fa** | | **Organics 0,005 F1234yf + 0,005 F1234zeE + 0.99 F245fa** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,5 | 0 | 6,8 | 0 | 4,9 | 0 | 1,6 |
| 0,05 | 5,6 | 0,05 | 7,7 | 0,05 | 5,8 | 0,05 | 2,7 |
| 0,1 | 5,6 | 0,1 | 7,7 | 0,1 | 5,8 | 0,1 | 2,7 |
| 0,15 | 5,6 | 0,15 | 7,7 | 0,15 | 5,8 | 0,15 | 2,7 |
| 0,2 | 5,6 | 0,2 | 7,7 | 0,2 | 5,8 | 0,2 | 2,7 |
| 0,25 | 5,6 | 0,25 | 7,7 | 0,25 | 5,8 | 0,25 | 2,7 |
| 0,3 | 5,6 | 0,3 | 7,7 | 0,3 | 5,8 | 0,3 | 2,7 |
| 0,35 | 5,6 | 0,35 | 7,7 | 0,35 | 5,8 | 0,35 | 2,7 |
| 0,4 | 5,6 | 0,4 | 7,7 | 0,4 | 5,8 | 0,4 | 2,7 |
| 0,45 | 5,6 | 0,45 | 7,7 | 0,45 | 5,8 | 0,45 | 2,7 |
| 0,5 | 5,6 | 0,5 | 7,7 | 0,5 | 5,8 | 0,5 | 2,7 |
| 0,55 | 5,5 | 0,55 | 7,7 | 0,55 | 5,7 | 0,55 | 2,7 |
| 0,6 | 5,5 | 0,6 | 7,7 | 0,6 | 5,6 | 0,6 | 2,7 |
| 0,65 | 5,5 | 0,65 | 7,7 | 0,65 | 5,5 | 0,65 | 2,7 |
| 0,7 | 5,4 | 0,7 | 7,7 | 0,7 | 5,4 | 0,7 | 2,7 |
| 0,75 | 5,3 | 0,75 | 7,5 | 0,75 | 5,1 | 0,75 | 2,7 |
| 0,8 | 5,0 | 0,8 | 7,2 | 0,8 | 4,8 | 0,8 | 2,6 |
| 0,85 | 4,5 | 0,85 | 6,6 | 0,85 | 4,3 | 0,85 | 2,4 |
| 0,9 | 3,8 | 0,9 | 5,6 | 0,9 | 3,6 | 0,9 | 2,1 |
| 0,95 | 2,7 | 0,95 | 3,9 | 0,95 | 2,6 | 0,95 | 1,7 |
| 1 | 1,2 | 1 | 1,2 | 1 | 1,2 | 1 | 1,2 |

**HF - HFO-1234yf - HFO-1234zeE - HFO-1225yeZ**

| **Organics 0,34 F1234yf + 0,33 F1234zeE + 0.33** | | **Organics 0,99 F1234yf + 0,005 F1234zeE + 0.005** | | **Organics 0,005 F1234yf+ 0,99 F1234zeE + 0.005** | | **Organics 0,005 F1234yf + 0,005 F1234zeE + 0.99** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 5,7 | 0 | 6,8 | 0 | 4,9 | 0 | 5,2 |
| 0,05 | 6,6 | 0,05 | 7,7 | 0,05 | 5,8 | 0,05 | 6,2 |
| 0,1 | 6,7 | 0,1 | 7,8 | 0,1 | 5,9 | 0,1 | 6,2 |
| 0,15 | 6,7 | 0,15 | 7,7 | 0,15 | 5,9 | 0,15 | 6,2 |
| 0,2 | 6,7 | 0,2 | 7,7 | 0,2 | 5,9 | 0,2 | 6,2 |
| 0,25 | 6,7 | 0,25 | 7,7 | 0,25 | 5,9 | 0,25 | 6,2 |
| 0,3 | 6,7 | 0,3 | 7,7 | 0,3 | 5,9 | 0,3 | 6,2 |
| 0,35 | 6,7 | 0,35 | 7,7 | 0,35 | 5,9 | 0,35 | 6,2 |
| 0,4 | 6,7 | 0,4 | 7,7 | 0,4 | 5,8 | 0,4 | 6,2 |
| 0,45 | 6,7 | 0,45 | 7,7 | 0,45 | 5,8 | 0,45 | 6,2 |
| 0,5 | 6,7 | 0,5 | 7,7 | 0,5 | 5,8 | 0,5 | 6,2 |
| 0,55 | 6,7 | 0,55 | 7,7 | 0,55 | 5,7 | 0,55 | 6,2 |
| 0,6 | 6,6 | 0,6 | 7,7 | 0,6 | 5,7 | 0,6 | 6,2 |
| 0,65 | 6,6 | 0,65 | 7,7 | 0,65 | 5,6 | 0,65 | 6,2 |
| 0,7 | 6,5 | 0,7 | 7,7 | 0,7 | 5,4 | 0,7 | 6,1 |
| 0,75 | 6,3 | 0,75 | 7,6 | 0,75 | 5,2 | 0,75 | 5,9 |
| 0,8 | 5,9 | 0,8 | 7,2 | 0,8 | 4,8 | 0,8 | 5,6 |
| 0,85 | 5,4 | 0,85 | 6,6 | 0,85 | 4,3 | 0,85 | 5,1 |
| 0,9 | 4,5 | 0,9 | 5,6 | 0,9 | 3,6 | 0,9 | 4,2 |
| 0,95 | 3,1 | 0,95 | 3,9 | 0,95 | 2,6 | 0,95 | 3,0 |
| 1 | 1,2 | 1 | 1,2 | 1 | 1,2 | 1 | 1,2 |

**HF - HFO-1234yf - HFO-1234zeE - HFO-1225zc**

| **Organics 0,34 F1234yf + 0,33 F1234zeE + 0,33 F1225zc** | | **Organics 0,99 F1234yf + 0,005 F1234zeE + 0,005 F1225zc** | | **Organics 0,005 F1234yf + 0,99 F1234zeE + 0,005 F1225zc** | | **Organics 0,005 F1234yf + 0,005 F1234zeE + 0,99 F1225zc** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | | | | | | | |
| | bar | | bar | | bar | | bar |
| 0 | 5,7 | 0 | 6,8 | 0 | 4,9 | 0 | 5,3 |
| 0,05 | 6,7 | 0,05 | 7,7 | 0,05 | 5,8 | 0,05 | 6,3 |
| 0,1 | 6,7 | 0,1 | 7,8 | 0,1 | 5,9 | 0,1 | 6,3 |
| 0,15 | 6,7 | 0,15 | 7,7 | 0,15 | 5,9 | 0,15 | 6,3 |
| 0,2 | 6,7 | 0,2 | 7,7 | 0,2 | 5,9 | 0,2 | 6,3 |
| 0,25 | 6,7 | 0,25 | 7,7 | 0,25 | 5,9 | 0,25 | 6,3 |
| 0,3 | 6,7 | 0,3 | 7,7 | 0,3 | 5,9 | 0,3 | 6,3 |
| 0,35 | 6,7 | 0,35 | 7,7 | 0,35 | 5,9 | 0,35 | 6,3 |
| 0,4 | 6,7 | 0,4 | 7,7 | 0,4 | 5,8 | 0,4 | 6,3 |
| 0,45 | 6,7 | 0,45 | 7,7 | 0,45 | 5,8 | 0,45 | 6,3 |
| 0,5 | 6,7 | 0,5 | 7,7 | 0,5 | 5,8 | 0,5 | 6,2 |
| 0,55 | 6,7 | 0,55 | 7,7 | 0,55 | 5,7 | 0,55 | 6,2 |
| 0,6 | 6,6 | 0,6 | 7,7 | 0,6 | 5,7 | 0,6 | 6,1 |
| 0,65 | 6,5 | 0,65 | 7,7 | 0,65 | 5,6 | 0,65 | 6,0 |
| 0,7 | 6,4 | 0,7 | 7,7 | 0,7 | 5,4 | 0,7 | 5,9 |
| 0,75 | 6,2 | 0,75 | 7,6 | 0,75 | 5,2 | 0,75 | 5,6 |
| 0,8 | 5,8 | 0,8 | 7,2 | 0,8 | 4,8 | 0,8 | 5,2 |
| 0,85 | 5,3 | 0,85 | 6,6 | 0,85 | 4,3 | 0,85 | 4,6 |
| 0,9 | 4,4 | 0,9 | 5,6 | 0,9 | 3,6 | 0,9 | 3,8 |
| 0,95 | 3,1 | 0,95 | 3,9 | 0,95 | 2,6 | 0,95 | 2,7 |
| 1 | 1,2 | 1 | 1,2 | 1 | 1,2 | 1 | 1,2 |

**HF - HFO-1234yf - HFO-1225yeZ - Trifluoropropyne**

| **Organics 0,34 F1234yf + 0,33 F1225yeZ + 0,33 TFP** | | **Organics 0,99 F1234yf + 0,005 F1225yeZ + 0,005 TFP** | | **Organics 0,005 F1234yf + 0,99 F1225yeZ + 0,005 TFP** | | **Organics 0,005 F1234yf + 0,005 F1225yeZ + 0,99 TFP** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 8,2 | 0 | 6,8 | 0 | 5,2 | 0 | 11,6 |
| 0,05 | 9,0 | 0,05 | 7,8 | 0,05 | 6,3 | 0,05 | 12,4 |
| 0,1 | 9,0 | 0,1 | 7,8 | 0,1 | 6,3 | 0,1 | 12,3 |
| 0,15 | 9,0 | 0,15 | 7,8 | 0,15 | 6,3 | 0,15 | 12,2 |
| 02 | 8,9 | 0,2 | 7,8 | 0,2 | 6,3 | 0,2 | 12,1 |
| 0,25 | 8,9 | 0,25 | 7,8 | 0,25 | 6,3 | 0,25 | 12,0 |
| 0,3 | 8,9 | 0,3 | 7,7 | 0,3 | 6,3 | 0,3 | 11,9 |
| 0,35 | 8,8 | 0,35 | 7,8 | 0,35 | 6,3 | 0,35 | 11,8 |
| 0,4 | 8,8 | 0,4 | 7,8 | 0,4 | 6,3 | 0,4 | 11,7 |
| 0,45 | 8,8 | 0,45 | 7,8 | 0,45 | 6,3 | 0,45 | 11,7 |
| 0,5 | 8,9 | 0,5 | 7,8 | 0,5 | 6,3 | 0,5 | 11,7 |
| 0,55 | 8,9 | 0,55 | 7,8 | 0,55 | 6,3 | 0,55 | 11,8 |
| 0,6 | 8,9 | 0,6 | 7,8 | 0,6 | 6,3 | 0,6 | 11,8 |
| 0,65 | 8,9 | 0,65 | 7,8 | 0,65 | 6,3 | 0,65 | 11,8 |
| 0,7 | 8,9 | 0,7 | 7,7 | 0,7 | 6,2 | 0,7 | 11,8 |
| 0,75 | 8,7 | 0,75 | 7,6 | 0,75 | 6,0 | 0,75 | 11,8 |
| 0,8 | 8,3 | 0,8 | 7,3 | 0,8 | 5,6 | 0,8 | 11,6 |
| 0,85 | 7,6 | 0,85 | 6,7 | 0,85 | 5,1 | 0,85 | 10,8 |
| 0,9 | 6,4 | 0,9 | 5,6 | 0,9 | 4,3 | 0,9 | 9,1 |
| 0,95 | 4,3 | 0,95 | 3,9 | 0,95 | 3,0 | 0,95 | 6,1 |
| 1 | 1,2 | 1 | 1,2 | 1 | 1,2 | 1 | 1,2 |

**HF - HFO-1234yf - HCFC-244bb - Trifluoropropyne**

| **Organics 0,34 F1234yf + 0,33 F244bb + 0,33 TFP** | | **Organics 0,99 F1234yf + 0,005 F244bb + 0,005 TFP** | | **Organics 0,005 F1234yf + 0,99 F244bb + 0,005 TFP** | | **Organics 0,005 F1234yf + 0,005 F244bb + 0,99 TFP** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 7,0 | 0 | 6,8 | 0 | 0,8 | 0 | 11,6 |
| 0,05 | 8,0 | 0,05 | 7,8 | 0,05 | 1,9 | 0,05 | 12,3 |
| 0,1 | 7,9 | 0,1 | 7,8 | 0,1 | 1,9 | 0,1 | 12,3 |
| 0,15 | 7,9 | 0,15 | 7,8 | 0,15 | 1,9 | 0,15 | 12,2 |
| 02 | 7,9 | 0,2 | 7,7 | 0,2 | 1,9 | 0,2 | 12.0 |
| 0,25 | 7,9 | 0,25 | 7,7 | 0,25 | 1,9 | 0,25 | 11,9 |
| 0,3 | 7,9 | 0,3 | 7,7 | 0,3 | 1,9 | 0,3 | 11,8 |
| 0,35 | 7,9 | 0,35 | 7,7 | 0,35 | 1,9 | 0,35 | 11,8 |
| 0,4 | 7,8 | 0,4 | 7,7 | 0,4 | 1,9 | 0,4 | 11,7 |
| 0,45 | 7,8 | 0,45 | 7,7 | 0,45 | 1,9 | 0,45 | 11,7 |
| 0,5 | 7,8 | 0,5 | 7,7 | 0,5 | 1,9 | 0,5 | 11,7 |
| 0,55 | 7,8 | 0,55 | 7,7 | 0,55 | 1,9 | 0,55 | 11,7 |
| 0,6 | 7,7 | 0,6 | 7,7 | 0,6 | 1,9 | 0,6 | 11,7 |
| 0,65 | 7,7 | 0,65 | 7,7 | 0,65 | 1,9 | 0,65 | 11,7 |
| 0,7 | 7,6 | 0,7 | 7,7 | 0,7 | 1,9 | 0,7 | 11,7 |
| 0,75 | 7,5 | 0,75 | 7,6 | 0,75 | 1,9 | 0,75 | 11,7 |
| 0,8 | 7,2 | 0,8 | 7,3 | 0,8 | 1,9 | 0,8 | 11,5 |
| 0,85 | 6,6 | 0,85 | 6,7 | 0,85 | 1,9 | 0,85 | 10,8 |
| 0,9 | 5,6 | 0,9 | 5,6 | 0,9 | 1,8 | 0,9 | 9,1 |
| 0,95 | 3,9 | 0,95 | 3,9 | 0,95 | 1,5 | 0,95 | 6,1 |
| 1 | 1,2 | 1 | 1,2 | 1 | 1,2 | 1 | 1,2 |

**HF - HFO-1234yf - HCFC-244bb - HFC-245fa**

| **Organics 0,34 F1234yf + 0,33 F244bb + 0,33 F245fa** | | **Organics 0,99 F1234yf + 0,005 F244bb + 0,005 F245fa** | | **Organics 0,005 F1234yf + 0,99 F244bb + 0,005 F245fa** | | **Organics 0,005 F1234yf + 0,005 F244bb + 0,99 F245fa** | |
|---|---|---|---|---|---|---|---|
| MASSFRAC | TOTAL | MASSFRAC | TOTAL | MASSFRAC | TOTAL | MASSFRAC | TOTAL |
| HF | PRES | HF | PRES | HF | PRES | HF | PRES |
| | bar | | bar | | bar | | bar |
| 0 | 3,3 | 0 | 6,7 | 0 | 0,7 | 0 | 1,5 |
| 0,05 | 4,4 | 0,05 | 7,7 | 0,05 | 1,8 | 0,05 | 2,7 |
| 0,1 | 4,4 | 0,1 | 7,7 | 0,1 | 1,8 | 0,1 | 2,7 |
| 0,15 | 4,4 | 0,15 | 7,7 | 0,15 | 1,8 | 0,15 | 2,7 |
| 02 | 4,4 | 0,2 | 7,7 | 0,2 | 1,8 | 0,2 | 2,7 |
| 0,25 | 4,4 | 0,25 | 7,7 | 0,25 | 1,8 | 0,25 | 2,7 |
| 0,3 | 4,4 | 0,3 | 7,7 | 0,3 | 1,8 | 0,3 | 2,7 |
| 0,35 | 4,4 | 0,35 | 7,7 | 0,35 | 1,8 | 0,35 | 2,7 |
| 0,4 | 4,4 | 0,4 | 7,7 | 0,4 | 1,8 | 0,4 | 2,7 |
| 0,45 | 4,3 | 0,45 | 7,7 | 0,45 | 1,8 | 0,45 | 2,7 |
| 0,5 | 4,3 | 0,5 | 7,7 | 0,5 | 1,8 | 0,5 | 2,7 |
| 0,55 | 4,3 | 0,55 | 7,7 | 0,55 | 1,8 | 0,55 | 2,7 |
| 0,6 | 4,3 | 0,6 | 7,7 | 0,6 | 1,8 | 0,6 | 2,7 |
| 0,65 | 42 | 0.65 | 7,7 | 0.65 | 1,8 | 0.65 | 2,7 |
| 0,7 | 4,2 | 0,7 | 7,7 | 0,7 | 1,8 | 0,7 | 2,7 |
| 0,75 | 4,1 | 0,75 | 7,5 | 0,75 | 1,8 | 0,75 | 2,6 |
| 0,8 | 4,0 | 0,8 | 7,2 | 0,8 | 1,8 | 0,8 | 2,6 |
| 0,85 | 3,7 | 0,85 | 6,6 | 0,85 | 1,8 | 0,85 | 2,4 |
| 0,9 | 3,2 | 0,9 | 5,6 | 0,9 | 1,7 | 0,9 | 2,1 |
| 0,95 | 2,4 | 0,95 | 3,8 | 0,95 | 1,5 | 0,95 | 1,7 |
| 1 | 1,2 | 1 | 1,2 | 1 | 1,2 | 1 | 1,2 |

**HF - HF-1234yf - HF-1233xf - HCFC-244bb**

| Organics 0,9 F1233xf + 0,05 F1234yf+0,05 F244bb | | Organics 0,05 F1233xf + 0,9 F1234yf +0,05 F244bb | | Organics 0,05 F1233xf + 0,05 F1234yf +0,9 F244bb | | Organics 0,4 F1233xf + 0,3 F1234yf +0,3 F244bb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,8 | 0 | 6,3 | 0 | 1,1 | 0 | 3,1 |
| 0,05 | 2,9 | 0,05 | 7,3 | 0,05 | 2,2 | 0,05 | 4,2 |
| 0,1 | 2,9 | 0,1 | 7,3 | 0,1 | 2,2 | 0,1 | 4,2 |
| 0,15 | 2,9 | 0,15 | 7,3 | 0,15 | 2,2 | 0,15 | 4,2 |
| 0,2 | 2,9 | 0,2 | 7,3 | 0,2 | 2,2 | 0,2 | 4,2 |
| 0,25 | 2,9 | 0,25 | 7,3 | 0,25 | 2,2 | 0,25 | 4,2 |
| 0,3 | 2,9 | 0,3 | 7,3 | 0,3 | 2,2 | 0,3 | 4,2 |
| 0,35 | 2,9 | 0,35 | 7,3 | 0,35 | 2,2 | 0,35 | 4,2 |
| 0,4 | 2,9 | 0,4 | 7,3 | 0,4 | 2,2 | 0,4 | 4,2 |
| 0,45 | 2,9 | 0,45 | 7,3 | 0,45 | 2,2 | 0,45 | 4,2 |
| 0,5 | 2,9 | 0,5 | 7,3 | 0,5 | 2,2 | 0,5 | 4,1 |
| 0,55 | 2,9 | 0,55 | 7,3 | 0,55 | 2,2 | 0,55 | 4,1 |
| 0,6 | 2,9 | 0,6 | 7,3 | 0,6 | 2,2 | 0,6 | 4,1 |
| 0,65 | 2,9 | 0,65 | 7,3 | 0,65 | 2,2 | 0,65 | 4,1 |
| 0,7 | 2,9 | 0,7 | 7,3 | 0,7 | 2,1 | 0,7 | 4,0 |
| 0,75 | 2,9 | 0,75 | 7.1 | 0,75 | 2,1 | 0,75 | 4.0 |
| 0,8 | 2,8 | 0,8 | 6,8 | 0,8 | 2,1 | 0,8 | 3,9 |
| 0,85 | 2,6 | 0,85 | 6,2 | 0,85 | 2,1 | 0,85 | 3,5 |
| 0,9 | 2,2 | 0,9 | 5,2 | 0,9 | 1,9 | 0,9 | 3,1 |
| 0,95 | 1,8 | 0,95 | 3,6 | 0,95 | 1,6 | 0,95 | 2,3 |
| 1 | 1,2 | 1 | 1,2 | 1 | 1,2 | 1 | 1,2 |

### Exemple 3 : mélanges pentanaires

**HF - HCFO-1233xf - HFC-245cb - HFO-1234yf - HFO-1243zf**

| Organics 0,9 F1233xf + 0,033 F1234yf +0,033 F245cb +0,034 F1243zf | | Organics 0,033 F1233of + 0,9 F1234yf +0,033 F245cb +0,034 F1243zf | | Organics 0,033 F1233xf + 0,033 F1234yf +0,9 F245cb +0,034 F1243zf | | Organics 0,034 F1233xf + 0,033 F1234yf +0,033 F245cb +0,9 F1243zf | | Organics 0,25 F1233xf + 0,25 F1234yf+0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,0 | 0 | 6,5 | 0 | 4,7 | 0 | 5,7 | 0 | 4,8 |
| 0,05 | 3,2 | 0,05 | 7,5 | 0,05 | 5,8 | 0,05 | 6,7 | 0,05 | 5,9 |
| 0,1 | 3,2 | 0,1 | 7,5 | 0,1 | 5,9 | 0,1 | 6,7 | 0,1 | 5,9 |
| 0,15 | 3,2 | 0,15 | 7,5 | 0,15 | 5,9 | 0,15 | 6,7 | 0,15 | 5,9 |
| 0,2 | 3,2 | 0,2 | 7,5 | 0,2 | 5,9 | 0,2 | 6,7 | 0,2 | 5,9 |
| 0,25 | 3,2 | 0,25 | 7,5 | 0,25 | 5,9 | 0,25 | 6,7 | 0,25 | 5,9 |
| 0,3 | 3,2 | 0,3 | 7,5 | 0,3 | 5,9 | 0,3 | 6,7 | 0,3 | 5,9 |
| 0,35 | 3,2 | 0,35 | 7,5 | 0,35 | 5,9 | 0,35 | 6,7 | 0,35 | 5,9 |
| 0,4 | 3,2 | 0,4 | 7,5 | 0,4 | 5,9 | 0,4 | 6,7 | 0,4 | 5,9 |
| 0,45 | 3,2 | 0,45 | 7,5 | 0,45 | 5,9 | 0,45 | 6,7 | 0,45 | 5,9 |
| 0,5 | 3,1 | 0,5 | 7,5 | 0,5 | 5,9 | 0,5 | 6,7 | 0,5 | 5,9 |
| 0,55 | 3,1 | 0,55 | 7,5 | 0,55 | 5,9 | 0,55 | 6,7 | 0,55 | 5,9 |
| 0,6 | 3,1 | 0,6 | 7,5 | 0,6 | 5,9 | 0,6 | 6,6 | 0,6 | 5,9 |
| 0,65 | 3,1 | 0,65 | 7,5 | 0,65 | 5,9 | 0,65 | 6,6 | 0,65 | 5,9 |
| 0,7 | 3,1 | 0,7 | 7,5 | 0,7 | 5,9 | 0,7 | 6,5 | 0,7 | 5,9 |
| 0,75 | 3,1 | 0,75 | 7,4 | 0,75 | 5,9 | 0,75 | 6,3 | 0,75 | 5,9 |
| 0,8 | 3,0 | 0,8 | 7,0 | 0,8 | 5,9 | 0,8 | 6,1 | 0,8 | 5,6 |
| 0,85 | 2,7 | 0,85 | 6,5 | 0,85 | 5,8 | 0,85 | 5,6 | 0,85 | 5,2 |
| 0,9 | 2,4 | 0,9 | 5,4 | 0,9 | 5,0 | 0,9 | 4,7 | 0,9 | 4,4 |
| 0,95 | 1,9 | 0,95 | 3,8 | 0,95 | 3,5 | 0,95 | 3,4 | 0,95 | 31 |
| 1 | 1,2 | 1 | 1,2 | 1 | 1,2 | 1 | 1,2 | 1 | 1,2 |

**HF - HCFO-1233xf -HFO-1234yf - HFO-1234zeE - HFO-1234zeZ**

| Organics 0,9 F1233xf + 0,033 F1234zeE +0,033 F1234zeZ +0,034 F1234yf | | Organics 0,033 F1233xf + 0,9 F1234zeE +0,033 F1234zeZ +0,034 F1234yf | | Organics 0,033 F1233xf + 0,033 F1234zeE +0,9 F1234zeZ +0,034 F1234yf | | Organics 0,034 F1233xf + 0,033 F1234zeE +0,033 F1234zeZ +0,9 F1234yf | | Organics 0,25 F1233xf + 0,25 F1234zeE +0,25 F1234zeZ +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 2,1 | 0 | 6,4 | 0 | 3,8 |
| 0,05 | 3,0 | 0,05 | 5,7 | 0,05 | 3,2 | 0,05 | 7,4 | 0,05 | 4,9 |
| 0,1 | 3,0 | 0,1 | 5,7 | 0,1 | 3,2 | 0,1 | 7,4 | 0,1 | 4,9 |
| 0,15 | 3,0 | 0,15 | 5,7 | 0,15 | 3,2 | 0,15 | 7,4 | 0,15 | 4,9 |
| 0,2 | 3,0 | 0,2 | 5,7 | 0,2 | 3,2 | 0,2 | 7,4 | 0,2 | 4,9 |
| 0,25 | 3,0 | 0,25 | 5,7 | 0,25 | 3,2 | 0,25 | 7,4 | 0,25 | 4,9 |
| 0,3 | 3,0 | 0,3 | 5,7 | 0,3 | 3,2 | 0,3 | 7,4 | 0,3 | 4,9 |
| 0,35 | 3,0 | 0,35 | 5,7 | 0,35 | 3,2 | 0,35 | 7,4 | 0,35 | 4,9 |
| 0,4 | 3,0 | 0,4 | 5,7 | 0,4 | 3,2 | 0,4 | 7,4 | 0,4 | 4,9 |
| 0,45 | 3,0 | 0,45 | 5,7 | 0,45 | 3,2 | 0,45 | 7,4 | 0,45 | 4,9 |
| 0,5 | 3,0 | 0,5 | 5,7 | 0,5 | 3,2 | 0,5 | 7,4 | 0,5 | 4,9 |
| 0,55 | 3,0 | 0,55 | 5,6 | 0,55 | 3,2 | 0,55 | 7,4 | 0,55 | 4,9 |
| 0,6 | 3,0 | 0,6 | 5,6 | 0,6 | 3,2 | 0,6 | 7,4 | 0,6 | 4,8 |
| 0,65 | 3,0 | 0,65 | 5,5 | 0,65 | 3,2 | 0,65 | 7,4 | 0,65 | 4,8 |
| 0,7 | 3,0 | 0,7 | 5,3 | 0,7 | 3,2 | 0,7 | 7,4 | 0,7 | 4,8 |
| 0,75 | 2,9 | 0,75 | 5,1 | 0,75 | 3,1 | 0,75 | 7,2 | 0,75 | 4,6 |
| 0,8 | 2,8 | 0,8 | 4,7 | 0,8 | 3,0 | 0,8 | 6,9 | 0,8 | 4,4 |
| 0,85 | 2,6 | 0,85 | 4,3 | 0,85 | 2,8 | 0,85 | 6,3 | 0,85 | 4,0 |
| 0,9 | 2,3 | 0,9 | 3,6 | 0,9 | 2,4 | 0,9 | 5,3 | 0,9 | 3,4 |
| 0,95 | 1,8 | 0,95 | 2,6 | 0,95 | 1,9 | 0,95 | 3,7 | 0,95 | 2,5 |
| 1 | 1,2 | 1 | 1,2 | 1 | 1,2 | 1 | 1,2 | 1 | 1,2 |

**HF - HCFO-1233xf - HFO-1234yf - HFO-1234zeE - HFO-1243zf**

| Organics 0,9 F1233xf + 0,033 F1234zeE +0,033 F1243zf +0,034 F1234yf | | Organics 0,033 F1233xf + 0,9 F1234zeE +0,033 F1243zf +0,034 F1234yf | | Organics 0,033 F1233xf + 0,033 F1234zeE +0,9 F1243zf +0,034 F1234yf | | Organics 0,034 F1233xf + 0,033 F1234zeE+ 0,033 F1243zf +0,9 F1234yf | | Organics 0,25 F1233xf + 0,25 F1234zeE +0,25 F1243zf +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,1 | 0 | 4,9 | 0 | 5,7 | 0 | 6,5 | 0 | 4,9 |
| 0,05 | 3,2 | 0,05 | 5,8 | 0,05 | 6,7 | 0,05 | 7,5 | 0,05 | 5,9 |
| 0,1 | 3,2 | 0,1 | 5,9 | 0,1 | 6,7 | 0,1 | 7,5 | 0,1 | 5,9 |
| 0,15 | 3,2 | 0,15 | 5,9 | 0,15 | 6,7 | 0,15 | 7,5 | 0,15 | 5,9 |
| 0,2 | 3,2 | 0,2 | 5,9 | 0,2 | 6,7 | 0,2 | 7,5 | 0,2 | 5,9 |
| 0,25 | 3,2 | 0,25 | 5,9 | 0,25 | 6,7 | 0,25 | 7,5 | 0,25 | 5,9 |
| 0,3 | 3,2 | 0,3 | 5,9 | 0,3 | 6,7 | 0,3 | 7,5 | 0,3 | 5,9 |
| 0,35 | 3,2 | 0,35 | 5,9 | 0,35 | 6,7 | 0,35 | 7,5 | 0,35 | 5,9 |
| 0,4 | 3,1 | 0,4 | 5,8 | 0,4 | 6,7 | 0,4 | 7,5 | 0,4 | 5,9 |
| 0,45 | 3,1 | 0,45 | 5,8 | 0,45 | 6,7 | 0,45 | 7,5 | 0,45 | 5,9 |
| 0,5 | 3,1 | 0,5 | 5,8 | 0,5 | 6,7 | 0,5 | 7,5 | 0,5 | 5,9 |
| 0,55 | 3,1 | 0,55 | 58 | 0,55 | 6,7 | 0,55 | 7,5 | 0,55 | 5,9 |
| 0,6 | 3,1 | 0,6 | 5,7 | 0,6 | 6,6 | 0,6 | 7,5 | 0,6 | 5,9 |
| 0,65 | 3,1 | 0,65 | 5,6 | 0,65 | 6,6 | 0,65 | 7,5 | 0,65 | 5,8 |
| 0,7 | 3,1 | 0,7 | 5,4 | 0,7 | 6,5 | 0,7 | 7,5 | 0,7 | 5,7 |
| 0,75 | 3,1 | 0,75 | 5,2 | 0,75 | 6,3 | 0,75 | 7,3 | 0,75 | 5,6 |
| 0,8 | 2,9 | 0,8 | 4.9 | 0,8 | 6,0 | 0,8 | 7,0 | 0,8 | 5,3 |
| 0,85 | 2,7 | 0,85 | 4,4 | 0,85 | 5,5 | 0,85 | 6,4 | 0,85 | 4,8 |
| 0,9 | 2,4 | 0,9 | 3,6 | 0,9 | 4,7 | 0,9 | 5,4 | 0,9 | 4,0 |
| 0,95 | 1,9 | 0,95 | 2,6 | 0,95 | 3,3 | 0,95 | 3,7 | 0,95 | 2,9 |
| 1 | 1,2 | 1 | 1,2 | 1 | 1,2 | 1 | 1,2 | 1 | 1,2 |

**HF - HCFO-1233xf - HFO-1234yf - HFO-1234zeE - HCFO-1233zdE**

| Organics 0,9 F1233xf + 0,033 F1234zeE +0,033 F1233zdE +0,034 F1234yf | | Organics 0,033 F1233xf + 0,9 F1234zeE +0,033 F1233zdE +0,034 F1234yf | | Organics 0,033 F1233xf + 0,033 F1234zeE +0,9 F1233zdE +0,034 F1234yf | | Organics 0,034 F1233xf + 0,033 F1234zeE +0,033 F1233zdE +0,9 F1234yf | | Organics 0,25 F1233xf + 0,25 F1234zeE +0,25 F1233zdE +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 1,7 | 0 | 6,4 | 0 | 3,8 |
| 0,05 | 3,0 | 0,05 | 5,7 | 0,05 | 2,8 | 0,05 | 7,4 | 0,05 | 4,8 |
| 0,1 | 3,0 | 0,1 | 5,7 | 0,1 | 2,8 | 0,1 | 7,4 | 0,1 | 4,8 |
| 0,15 | 3,0 | 0,15 | 5,7 | 0,15 | 2,8 | 0,15 | 7,4 | 0,15 | 4,8 |
| 0,2 | 3,0 | 0,2 | 5,7 | 0,2 | 2,8 | 0,2 | 7,4 | 0,2 | 4,8 |
| 0,25 | 3,0 | 0,25 | 5,7 | 0,25 | 2,8 | 0,25 | 7,4 | 0,25 | 4,8 |
| 0,3 | 3,0 | 0,3 | 5,7 | 0,3 | 2,8 | 0,3 | 7,4 | 0,3 | 4,8 |
| 0,35 | 3,0 | 0,35 | 5,7 | 0,35 | 2,8 | 0,35 | 7,4 | 0,35 | 4,8 |
| 0,4 | 3,0 | 0,4 | 5,7 | 0,4 | 2,8 | 0,4 | 7,4 | 0,4 | 4,8 |
| 0,45 | 3,0 | 0,45 | 5,7 | 0,45 | 2,8 | 0,45 | 7,4 | 0,45 | 4,8 |
| 0,5 | 3,0 | 0,5 | 5,7 | 0,5 | 2,8 | 0,5 | 7,4 | 0,5 | 4,8 |
| 0,55 | 3,0 | 0,55 | 5,6 | 0,55 | 2,8 | 0,55 | 7,4 | 0,55 | 4,8 |
| 0,6 | 3,0 | 0,6 | 5,5 | 0,6 | 2,8 | 0,6 | 7,4 | 0,6 | 4,8 |
| 0,65 | 2,9 | 0,65 | 5,4 | 0,65 | 2,7 | 0,65 | 7,4 | 0,65 | 4,8 |
| 0,7 | 2,9 | 0,7 | 5,3 | 0,7 | 2,7 | 0,7 | 7,4 | 0,7 | 4,7 |
| 0,75 | 2,9 | 0,75 | 5,1 | 0,75 | 2,7 | 0,75 | 7,2 | 0,75 | 4,6 |
| 0,8 | 2,8 | 0,8 | 4,7 | 0,8 | 2,6 | 0,8 | 6,9 | 0,8 | 4,3 |
| 0,85 | 2,6 | 0,85 | 4,2 | 0,85 | 2,4 | 0,85 | 6,3 | 0,85 | 3,9 |
| 0,9 | 2,3 | 0,9 | 3,6 | 0,9 | 2,1 | 0,9 | 5,3 | 0,9 | 3,3 |
| 0,95 | 1,8 | 0,95 | 2,6 | 0,95 | 1,7 | 0,95 | 3,7 | 0,95 | 2,4 |
| 1 | 1,2 | 1 | 1,2 | 1 | 1,2 | 1 | 1,2 | 1 | 1,2 |

**HF - HCFO-1233xf - HFO-1234yf - HFO-1234zeZ - HFO-1243zf**

| Organics 0,9 F1233xf + 0,033 F1234zeZ +0,033 F1243zf +0,034 F1234yf | | Organics 0,033 F1233xf + 0,9 F1234zeZ + 0,033 F1243zf +0,034 F1234yf | | Organics 0,033 F1233xf + 0,033 F1234zeZ +0,9 F1243zf +0,034 F1234yf | | Organics 0,034 F1233xf + 0,033 F1234zeZ +0,033 F1243zf +0,9 F1234yf | | Organics 0,25 F1233xf + 0,25 F1234zeZ +0,25 F1243zf +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 2,1 | 0 | 5,6 | 0 | 6,4 | 0 | 4,1 |
| 0,05 | 3,1 | 0,05 | 3,2 | 0,05 | 6,6 | 0,05 | 7,4 | 0,05 | 5,2 |
| 0,1 | 3,1 | 0,1 | 3,2 | 0,1 | 6,6 | 0,1 | 7,4 | 0,1 | 5,2 |
| 0,15 | 3,1 | 0,15 | 3,2 | 0,15 | 6,6 | 0,15 | 7,4 | 0,15 | 5,2 |
| 0,2 | 3,1 | 0,2 | 3,2 | 0,2 | 6,6 | 0,2 | 7,4 | 0,2 | 5,2 |
| 0,25 | 3,1 | 0,25 | 3,2 | 0,25 | 6,6 | 0,25 | 7,4 | 0,25 | 5,2 |
| 0,3 | 3,1 | 0,3 | 3,2 | 0,3 | 6,6 | 0,3 | 7,4 | 0,3 | 5,2 |
| 0,35 | 3,0 | 0,35 | 3,2 | 0,35 | 6,6 | 0,35 | 7,4 | 0,35 | 5,2 |
| 0,4 | 3,0 | 0,4 | 3,2 | 0,4 | 6,6 | 0,4 | 7,4 | 0,4 | 5,2 |
| 0,45 | 3,0 | 0,45 | 3,2 | 0,45 | 6,6 | 0,45 | 7,4 | 0,45 | 5,2 |
| 0,5 | 3,0 | 0,5 | 3,2 | 0,5 | 6,6 | 0,5 | 7,4 | 0,5 | 5,2 |
| 0,55 | 3,0 | 0,55 | 3,2 | 0,55 | 6,6 | 0,55 | 7,4 | 0,55 | 5,2 |
| 0,6 | 3,0 | 0,6 | 3,2 | 0,6 | 6,5 | 0,6 | 7,4 | 0,6 | 5,2 |
| 0,65 | 3,0 | 0,65 | 3,2 | 0,65 | 6,5 | 0,65 | 7,4 | 0,65 | 5,2 |
| 0,7 | 3,0 | 0,7 | 3,2 | 0,7 | 6,4 | 0,7 | 7,4 | 0,7 | 5,1 |
| 0,75 | 3,0 | 0,75 | 3,2 | 0,75 | 6,2 | 0,75 | 7.2 | 0,75 | 5,0 |
| 0,8 | 2,9 | 0,8 | 3,1 | 0,8 | 6,0 | 0,8 | 6,9 | 0,8 | 4,8 |
| 0,85 | 2,6 | 0,85 | 2,8 | 0,85 | 5,5 | 0,85 | 6,3 | 0,85 | 4,4 |
| 0,9 | 2,3 | 0,9 | 2,5 | 0,9 | 4,6 | 0,9 | 5,3 | 0,9 | 3,7 |
| 0,95 | 1,8 | 0,95 | 2,0 | 0,95 | 3,3 | 0,95 | 3,7 | 0,95 | 2,7 |
| 1 | 1,2 | 1 | 1,2 | 1 | 1,2 | 1 | 1,2 | 1 | 1,2 |

**HF - HCFO-1233xf - HFO-1234yf - HFO-1234zeZ - HCFO-1233zdE**

| Organics 0,9 F1233xf + 0,033 F1234zeZ +0,033 F1233zdE +0,034 F1234yf | | Organics 0,033 F1233xf + 0,9 F1234zeZ +0,033 F1233zdE +0,034 F1234yf | | Organics 0,033 F1233xf + 0,033 F1234zeZ +0,9 F1233zdE +0,034 F1234yf | | Organics 0,034 F1233xf + 0,033 F1234zeZ +0,033 F1233zdE +0,9 F1234yf | | Organics 0,25 F1233xf + 0,25 F1234zeZ +0,25 F1233zdE +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,8 | 0 | 2,0 | 0 | 1,6 | 0 | 6,3 | 0 | 3,0 |
| 0,05 | 2,9 | 0,05 | 3,1 | 0,05 | 2,7 | 0,05 | 7,3 | 0,05 | 4,1 |
| 0,1 | 2,9 | 0,1 | 3,1 | 0,1 | 2,7 | 0,1 | 7,3 | 0,1 | 4,1 |
| 0,15 | 2,9 | 0,15 | 3,1 | 0,15 | 2,7 | 0,15 | 7,3 | 0,15 | 4,1 |
| 0,2 | 2,9 | 0,2 | 3,1 | 0,2 | 2,7 | 0,2 | 7,3 | 0,2 | 4,1 |
| 0,25 | 2,9 | 0,25 | 3,1 | 0,25 | 2,7 | 0,25 | 7,3 | 0,25 | 4,1 |
| 0,3 | 2,9 | 0,3 | 3,1 | 0,3 | 2,7 | 0,3 | 7,3 | 0,3 | 4,1 |
| 0,35 | 2,9 | 0,35 | 3,1 | 0,35 | 2,7 | 0,35 | 7,3 | 0,35 | 4,1 |
| 0,4 | 2,9 | 0,4 | 3,1 | 0,4 | 2,7 | 0,4 | 7,3 | 0,4 | 4,1 |
| 0,45 | 2,9 | 0,45 | 3,1 | 0,45 | 2,7 | 0,45 | 7,3 | 0,45 | 4,1 |
| 0,5 | 2,9 | 0,5 | 3,1 | 0,5 | 2,7 | 0,5 | 7,3 | 0,5 | 4,1 |
| 0,55 | 2,9 | 0,55 | 3,1 | 0,55 | 2,7 | 0,55 | 7,3 | 0,55 | 4,1 |
| 0,6 | 2,9 | 0,6 | 3,1 | 0,6 | 2,7 | 0,6 | 7,3 | 0,6 | 4,1 |
| 0,65 | 2,9 | 0,65 | 3,1 | 0,65 | 2,7 | 0,65 | 7,3 | 0,65 | 4,1 |
| 0,7 | 2,9 | 0,7 | 3,1 | 0,7 | 2,7 | 0,7 | 7,3 | 0,7 | 4,0 |
| 0,75 | 2,8 | 0,75 | 3,1 | 0,75 | 2,6 | 0,75 | 7,1 | 0,75 | 4,0 |
| 0,8 | 2,7 | 0,8 | 2,9 | 0,8 | 2,5 | 0,8 | 6,8 | 0,8 | 3,8 |
| 0,85 | 2,5 | 0,85 | 2,7 | 0,85 | 2,4 | 0,85 | 6,2 | 0,85 | 3,5 |
| 0,9 | 2,2 | 0,9 | 2,4 | 0,9 | 2,1 | 0,9 | 5,3 | 0,9 | 3,0 |
| 0,95 | 1,8 | 0,95 | 1,9 | 0,95 | 1,7 | 0,95 | 3,7 | 0,95 | 2,3 |
| 1 | 1,2 | 1 | 1,2 | 1 | 1,2 | 1 | 1,2 | 1 | 1,2 |

**HF - HCFO-1233xf - HFO-1234yf - HCFO-1233zdE - HFO-1243zf**

| Organics 0,9 F1233xf + 0,033 F1243zf +0,033 F1233zdE +0,034 F1234yf | | Organics 0,033 F1233xf + 0,9 F1243zf +0,033 F1233zdE +0,034 F1234yf | | Organics 0,033 F1233xf + 0,033 F1243zf +0,9 F1233zdE +0,034 F1234yf | | Organics 0,034 F1233xf + 0,033 F1243zf +0,033 F1233zdE +0,9 F1234yf | | Organics 0,25 F1233xf + 0,25 F1243zf +0,25 F1233zdE +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 5,6 | 0 | 1,7 | 0 | 6,4 | 0 | 4,1 |
| 0,05 | 3,0 | 0,05 | 6,6 | 0,05 | 2,8 | 0,05 | 7,4 | 0,05 | 5,2 |
| 0,1 | 3,0 | 0,1 | 6,6 | 0,1 | 2,8 | 0,1 | 7,4 | 0,1 | 5,2 |
| 0,15 | 3,0 | 0,15 | 6,6 | 0,15 | 2,8 | 0,15 | 7,4 | 0,15 | 5,2 |
| 0,2 | 3,0 | 0,2 | 6,6 | 0,2 | 2,8 | 0,2 | 7,4 | 0,2 | 5,2 |
| 0,25 | 3,0 | 0,25 | 6,6 | 0,25 | 2,8 | 0,25 | 7,4 | 0,25 | 5,2 |
| 0,3 | 3,0 | 0,3 | 6,6 | 0,3 | 2,8 | 0,3 | 7,4 | 0,3 | 5,2 |
| 0,35 | 3,0 | 0,35 | 6,6 | 0,35 | 2,8 | 0,35 | 7,4 | 0,35 | 5,2 |
| 0,4 | 3,0 | 0,4 | 6,6 | 0,4 | 2,8 | 0,4 | 7,4 | 0,4 | 5,2 |
| 0,45 | 3,0 | 0,45 | 6,6 | 0,45 | 2,8 | 0,45 | 7,4 | 0,45 | 5,2 |
| 0,5 | 3,0 | 0,5 | 6,6 | 0,5 | 2,8 | 0,5 | 7,4 | 0,5 | 5,1 |
| 0,55 | 3,0 | 0,55 | 6,6 | 0,55 | 2,8 | 0,55 | 7,4 | 0,55 | 5,1 |
| 0,6 | 3,0 | 0,6 | 6,5 | 0,6 | 2,8 | 0,6 | 7,4 | 0,6 | 5,1 |
| 0,65 | 3,0 | 0,65 | 6,5 | 0,65 | 2,8 | 0,65 | 7,4 | 0,65 | 5,1 |
| 0,7 | 3,0 | 0,7 | 6,4 | 0,7 | 2,8 | 0,7 | 7,4 | 0,7 | 5,1 |
| 0,75 | 3,0 | 0,75 | 6,2 | 0,75 | 2,8 | 0,75 | 7,2 | 0,75 | 4,9 |
| 0,8 | 2,8 | 0,8 | 6,0 | 0,8 | 2,7 | 0,8 | 6,9 | 0,8 | 4,7 |
| 0,85 | 2,6 | 0,85 | 5,5 | 0,85 | 2,5 | 0,85 | 6,3 | 0,85 | 4,3 |
| 0,9 | 2,3 | 0,9 | 4,6 | 0,9 | 2,2 | 0,9 | 5,3 | 0,9 | 3,7 |
| 0,95 | 1,8 | 0,95 | 3,3 | 0,95 | 1,8 | 0,95 | 3,7 | 0,95 | 2,7 |
| 1 | 1,2 | 1 | 1,2 | 1 | 1,2 | 1 | 1,2 | 1 | 1,2 |

**HF - HFO-1234yf - HFO-1234zeE - HFO-1234zeZ - HCFO-1233zdE**

| Organics 0,9 F1234yf + 0,033 F1233zdE +0,033 F1234zeE +0,034 F1234zeZ | | Organics 0,033 F1234yf + 0,9 F1233zdE +0,033 F1234zeE +0,034 F1234zeZ | | Organics 0,033 F1234yf + 0,033 F1233zdE +0,9 F1234zeE +0,034 F1234zeZ | | Organics 0,034 F1234yf + 0,033 F1233zdE +0,033 F1234zeE +0,9 F1234zeZ | | Organics 0,25 F1234yf + 0,25 F1233zdE +0,25 F1234zeE +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 1,7 | 0 | 4,7 | 0 | 2,1 | 0 | 3,8 |
| 0,05 | 7,4 | 0,05 | 2,8 | 0,05 | 5,7 | 0,05 | 3,2 | 0,05 | 4,8 |
| 0,1 | 7,4 | 0,1 | 2,8 | 0,1 | 5,7 | 0,1 | 3,2 | 0,1 | 4,8 |
| 0,15 | 7,4 | 0,15 | 2,8 | 0,15 | 5,7 | 0,15 | 3,2 | 0,15 | 4,8 |
| 0,2 | 7,4 | 0,2 | 2,8 | 0,2 | 5,7 | 0,2 | 3,2 | 0,2 | 4,8 |
| 0,25 | 7,4 | 0,25 | 2,8 | 0,25 | 5,7 | 0,25 | 3,2 | 0,25 | 4,8 |
| 0,3 | 7,4 | 0,3 | 2,8 | 0,3 | 5,7 | 0,3 | 3,2 | 0,3 | 4,8 |
| 0,35 | 7,4 | 0,35 | 2,8 | 0,35 | 5,7 | 0,35 | 3,2 | 0,35 | 4,8 |
| 0,4 | 7,4 | 0,4 | 2,8 | 0,4 | 5,7 | 0,4 | 3,2 | 0,4 | 4,8 |
| 0,45 | 7,4 | 0,45 | 2,8 | 0,45 | 5,7 | 0,45 | 3,2 | 0,45 | 4,8 |
| 0,5 | 7,4 | 0,5 | 2,8 | 0,5 | 5,7 | 0,5 | 3,2 | 0,5 | 4,8 |
| 0,55 | 7,4 | 0,55 | 2,8 | 0,55 | 5,6 | 0,55 | 3,2 | 0,55 | 4,8 |
| 0,6 | 7,4 | 0,6 | 2,8 | 0,6 | 5,5 | 0,6 | 3,2 | 0,6 | 4,8 |
| 0,65 | 7,4 | 0,65 | 2,7 | 0,65 | 5,4 | 0,65 | 3,2 | 0,65 | 4,8 |
| 0,7 | 7,4 | 0,7 | 2,7 | 0,7 | 5,3 | 0,7 | 3,2 | 0,7 | 4,7 |
| 0,75 | 7,2 | 0,75 | 2,7 | 0,75 | 5,1 | 0,75 | 3,1 | 0,75 | 4,6 |
| 0,8 | 6,9 | 0,8 | 2,6 | 0,8 | 4,7 | 0,8 | 3,0 | 0,8 | 4,3 |
| 0,85 | 6,3 | 0,85 | 2,4 | 0,85 | 4,2 | 0,85 | 2,8 | 0,85 | 4,0 |
| 0,9 | 5,3 | 0,9 | 2,1 | 0,9 | 3,6 | 0,9 | 2,4 | 0,9 | 3,4 |
| 0,95 | 3,7 | 0,95 | 1,7 | 0,95 | 2,6 | 0,95 | 1,9 | 0,95 | 2,5 |
| 1 | 1,2 | 1 | 1,2 | 1 | 1,2 | 1 | 1,2 | 1 | 1,2 |

**HF - HFO-1234yf - HFO-1234zeE - HFO-1234zeZ - HFO-1243zf**

| Organics 0,9 F1234yf + 0,033 F1234zeZ +0,033 F1234zeE +0,034 F1243zf | | Organics 0,033 F1234yf + 0,9 F1234zeZ +0,033 F1234zeE +0,034 F1243zf | | Organics 0,033 F1234yf + 0,033 F1234zeZ +0,9 F1234zeE +0,034 F1243zf | | Organics 0,034 F1234yf+ 0,033 F1234zeZ +0,033 F1234zeE +0,9 F1243zf | | Organics 0,25 F1234yf + 0,25 F1234zeZ +0,25 F1234zeE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 2,2 | 0 | 4,9 | 0 | 5,7 | 0 | 4,9 |
| 0,05 | 7,5 | 0,05 | 3,3 | 0,05 | 5,8 | 0,05 | 6,7 | 0,05 | 5,9 |
| 0,1 | 7,5 | 0,1 | 3,3 | 0,1 | 5,9 | 0,1 | 6,7 | 0,1 | 5,9 |
| 0,15 | 7,5 | 0,15 | 3,3 | 0,15 | 5,8 | 0,15 | 6,7 | 0,15 | 5,9 |
| 0,2 | 7,5 | 0,2 | 3,3 | 0,2 | 5,8 | 0,2 | 6,7 | 0,2 | 5,9 |
| 0,25 | 7,5 | 0,25 | 3,3 | 0,25 | 5,8 | 0,25 | 6,7 | 0,25 | 5,9 |
| 0,3 | 7,5 | 0,3 | 3,3 | 0,3 | 5,8 | 0,3 | 6,7 | 0,3 | 5,9 |
| 0,35 | 7,5 | 0,35 | 3,3 | 0,35 | 5,8 | 0,35 | 6,7 | 0,35 | 5,9 |
| 0,4 | 7,5 | 0,4 | 3,3 | 0,4 | 5,8 | 0,4 | 6,7 | 0,4 | 5,9 |
| 0,45 | 7,5 | 0,45 | 3,3 | 0,45 | 5,8 | 0,45 | 6,7 | 0,45 | 5,9 |
| 0,5 | 7,5 | 0,5 | 3,3 | 0,5 | 5,8 | 0,5 | 6,7 | 0,5 | 5,9 |
| 0,55 | 7,5 | 0,55 | 3,3 | 0,55 | 5,8 | 0,55 | 6,7 | 0,55 | 5,9 |
| 0,6 | 7,5 | 0,6 | 3,3 | 0,6 | 5,7 | 0,6 | 6,6 | 0,6 | 5,9 |
| 0,65 | 7,5 | 0,65 | 3,3 | 0,65 | 5,6 | 0,65 | 6,6 | 0,65 | 5,8 |
| 0,7 | 7,5 | 0,7 | 3,3 | 0,7 | 5,4 | 0,7 | 6,5 | 0,7 | 5,7 |
| 0,75 | 7,3 | 0,75 | 3,3 | 0,75 | 5,2 | 0,75 | 6,3 | 0,75 | 5,6 |
| 0,8 | 7,0 | 0,8 | 3,1 | 0,8 | 4,9 | 0,8 | 6,0 | 0,8 | 5,3 |
| 0,85 | 6,4 | 0,85 | 2,9 | 0,85 | 4,4 | 0,85 | 5,5 | 0,85 | 4,8 |
| 0,9 | 5,4 | 0,9 | 2,5 | 0,9 | 3,7 | 0,9 | 4,7 | 0,9 | 4,1 |
| 0,95 | 3,7 | 0,95 | 2,0 | 0,95 | 2,6 | 0,95 | 3,3 | 0,95 | 2,9 |
| 1 | 1,2 | 1 | 1,2 | 1 | 1,2 | 1 | 1,2 | 1 | 1,2 |

**HF - HFO-1234yf - HFO-1234zeE - HCFO-1233zdE - HFO-1243zf**

| Organics 0,9 F1234yf + 0,033 F1233zdE +0,033 F1234zeE +0,034 F1243zf | | Organics 0,033 F1234yf + 0,9 F1233zdE +0,033 F1234zeE +0,034 F1243zf | | Organics 0,033 F1234yf + 0,033 F1233zdE +0,9 F1234zeE +0,034 F1243zf | | Organics 0,034 F1234yf + 0,033 F1233zdE +0,033 F1234zeE +0,9 F1243zf | | Organics 0,25 F1234yf + 0,25 F1233zdE +0,25 F1234zeE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 1,9 | 0 | 4,9 | 0 | 5,7 | 0 | 4,8 |
| 0,05 | 7,5 | 0,05 | 3,0 | 0,05 | 5,8 | 0,05 | 6,7 | 0,05 | 5,8 |
| 0,1 | 7,5 | 0,1 | 3,0 | 0,1 | 5,9 | 0,1 | 6,7 | 0,1 | 5,9 |
| 0,15 | 7,5 | 0,15 | 3,0 | 0,15 | 5,8 | 0,15 | 6,7 | 0,15 | 5,9 |
| 0,2 | 7,5 | 0,2 | 3,0 | 0,2 | 5,8 | 0,2 | 6,7 | 0,2 | 5,9 |
| 0,25 | 7,5 | 0,25 | 3,0 | 0,25 | 5,8 | 0,25 | 6,7 | 0,25 | 5,9 |
| 0,3 | 7,5 | 0,3 | 3,0 | 0,3 | 5,8 | 0,3 | 6,7 | 0,3 | 5,9 |
| 0,35 | 7,5 | 0,35 | 2,9 | 0,35 | 5,8 | 0,35 | 6,7 | 0,35 | 5,9 |
| 0,4 | 7,5 | 0,4 | 2,9 | 0,4 | 5,8 | 0,4 | 6,7 | 0,4 | 5,9 |
| 0,45 | 7,5 | 0,45 | 2,9 | 0,45 | 5,8 | 0,45 | 6,7 | 0,45 | 5,9 |
| 0,5 | 7,5 | 0,5 | 2,9 | 0,5 | 5,8 | 0,5 | 6,7 | 0,5 | 5,8 |
| 0,55 | 7,5 | 0,55 | 2,9 | 0,55 | 5,8 | 0,55 | 6,6 | 0,55 | 5,8 |
| 0,6 | 7,5 | 0,6 | 2,9 | 0,6 | 5,7 | 0,6 | 6,6 | 0,6 | 5,8 |
| 0,65 | 7,5 | 0,65 | 2,9 | 0,65 | 5,6 | 0,65 | 6,6 | 0,65 | 5,8 |
| 0,7 | 7,5 | 0,7 | 2,9 | 0,7 | 5,4 | 0,7 | 6,5 | 0,7 | 5,7 |
| 0,75 | 7,3 | 0,75 | 2,9 | 0,75 | 5,2 | 0,75 | 6,3 | 0,75 | 5,5 |
| 0,8 | 7,0 | 0,8 | 2,7 | 0,8 | 4,9 | 0,8 | 6,0 | 0,8 | 5,2 |
| 0,85 | 6,4 | 0,85 | 2,5 | 0,85 | 4,4 | 0,85 | 5,5 | 0,85 | 4,8 |
| 0,9 | 5,4 | 0,9 | 2,2 | 0,9 | 3,6 | 0,9 | 4,7 | 0,9 | 4,0 |
| 0,95 | 3,7 | 0,95 | 1,8 | 0,95 | 2,6 | 0,95 | 3,3 | 0,95 | 2,9 |
| 1 | 1,2 | 1 | 1,2 | 1 | 1,2 | 1 | 1,2 | 1 | 1,2 |

**HF - HFO-1234yf - HFO-1234zeZ - HCFO-1233zdE - HFO-1243zf**

| Organics 0,9 F1234yf + 0,033 F1233zdE +0,033 F1234zeZ +0,034 F1243zf | | Organics 0,033 F1234yf + 0,9 F1233zdE+0,033 F1234zeZ +0,034 F1243zf | | Organics 0,033 F1234yf + 0,033 F1233zdE +0,9 F1234zeZ +0,034 F1243zf | | Organics 0,034 F1234yf + 0,033 F1233zdE +0,033 F1234zeZ +0,9 F1243zf | | Organics 0,25 F1234yf + 0,25 F1233zdE +0,25 F1234zeZ +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 1,7 | 0 | 2,1 | 0 | 5,6 | 0 | 4,1 |
| 0,05 | 7,4 | 0,05 | 2,9 | 0,05 | 3,2 | 0,05 | 6,6 | 0,05 | 5,2 |
| 0,1 | 7,4 | 0,1 | 2,9 | 0,1 | 3,2 | 0,1 | 6,6 | 0,1 | 5,2 |
| 0,15 | 7,4 | 0,15 | 2,9 | 0,15 | 3,2 | 0,15 | 6,6 | 0,15 | 5,2 |
| 0,2 | 7,4 | 0,2 | 2,9 | 0,2 | 3,2 | 0,2 | 6,6 | 0,2 | 5,2 |
| 0,25 | 7,4 | 0,25 | 2,8 | 0,25 | 3,2 | 0,25 | 6,6 | 0,25 | 5,2 |
| 0,3 | 7,4 | 0,3 | 2,8 | 0,3 | 3,2 | 0,3 | 6,6 | 0,3 | 5,2 |
| 0,35 | 7,4 | 0,35 | 2,8 | 0,35 | 3,2 | 0,35 | 6,6 | 0,35 | 5,1 |
| 0,4 | 7,4 | 0,4 | 2,8 | 0,4 | 3,2 | 0,4 | 6,6 | 0,4 | 5,1 |
| 0,45 | 7,4 | 0,45 | 2,8 | 0,45 | 3,2 | 0,45 | 6,6 | 0,45 | 5,1 |
| 0,5 | 7,4 | 0,5 | 2,8 | 0,5 | 3,2 | 0,5 | 6,6 | 0,5 | 5,1 |
| 0,55 | 7,4 | 0,55 | 2,8 | 0,55 | 3,2 | 0,55 | 6,6 | 0,55 | 5,1 |
| 0,6 | 7,4 | 0,6 | 2,8 | 0,6 | 3,2 | 0,6 | 6,5 | 0,6 | 5,1 |
| 0,65 | 7,4 | 0,65 | 2,8 | 0,65 | 3,2 | 0,65 | 6,5 | 0,65 | 5,1 |
| 0,7 | 7,4 | 0,7 | 2,8 | 0,7 | 3,2 | 0,7 | 6,4 | 0,7 | 5,1 |
| 0,75 | 7,2 | 0,75 | 2,8 | 0,75 | 3,2 | 0,75 | 6,2 | 0,75 | 5,0 |
| 0,8 | 6,9 | 0,8 | 2,7 | 0,8 | 3,0 | 0,8 | 5,9 | 0,8 | 4,7 |
| 0,85 | 6,3 | 0,85 | 2,5 | 0,85 | 2,8 | 0,85 | 5,5 | 0,85 | 4,3 |
| 0,9 | 5,3 | 0,9 | 2,2 | 0,9 | 2,5 | 0,9 | 4,6 | 0,9 | 3,7 |
| 0,95 | 3,7 | 0,95 | 1,8 | 0,95 | 2,0 | 0,95 | 3,3 | 0,95 | 2,7 |
| 1 | 1,2 | 1 | 1,2 | 1 | 1,2 | 1 | 1,2 | 1 | 1,2 |

**HF - HFO-1234yf - HFC-245cb - HFO-1234zeE - HCFO-1233zdE**

| Organics 0,9 F1234yf + 0,033 F1234zeE +0,033 F245cb +0,034 F1233zdE | | Organics 0,033 F1234yf + 0,9 F1234zeE +0,033 F245cb +0,034 F1233zdE | | Organics 0,033 F1234yf + 0,033 F1234zeE +0,9 F245cb +0,034 F1233zdE | | Organics 0,034 F1234yf + 0,033 F1234zeE +0,033 F245cb +0,9 F1233zdE | | Organics 0,25 F1234yf + 0,25 F1234zeE +0,25 F245cb +0,25 F1233zdE | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 4,8 | 0 | 4,6 | 0 | 1,8 | 0 | 4,5 |
| 0,05 | 7,5 | 0,05 | 5,8 | 0,05 | 5,8 | 0,05 | 2,9 | 0,05 | 5,6 |
| 0,1 | 7,5 | 0,1 | 5,8 | 0,1 | 5,8 | 0,1 | 2,9 | 0,1 | 5,6 |
| 0,15 | 7,5 | 0,15 | 5,8 | 0,15 | 5,8 | 0,15 | 2,9 | 0,15 | 5,6 |
| 0,2 | 7,5 | 0,2 | 5,8 | 0,2 | 5,8 | 0,2 | 2,9 | 0,2 | 5,6 |
| 0,25 | 7,5 | 0,25 | 5,8 | 0,25 | 5,8 | 0,25 | 2,9 | 0,25 | 5,6 |
| 0,3 | 7,5 | 0,3 | 5,8 | 0,3 | 5,8 | 0,3 | 2,9 | 0,3 | 5,6 |
| 0,35 | 7,5 | 0,35 | 5,8 | 0,35 | 5,8 | 0,35 | 2,9 | 0,35 | 5,6 |
| 0,4 | 7,5 | 0,4 | 5,8 | 0,4 | 5,8 | 0,4 | 2,9 | 0,4 | 5,6 |
| 0,45 | 7,5 | 0,45 | 5,8 | 0,45 | 5,8 | 0,45 | 2,9 | 0,45 | 5,6 |
| 0,5 | 7,5 | 0,5 | 5,8 | 0,5 | 5,8 | 0,5 | 2,9 | 0,5 | 5,6 |
| 0,55 | 7,5 | 0,55 | 5,7 | 0,55 | 5,8 | 0,55 | 2,9 | 0,55 | 5,6 |
| 0,6 | 7,5 | 0,6 | 5,7 | 0,6 | 5,8 | 0,6 | 2,9 | 0,6 | 5,6 |
| 0,65 | 7,5 | 0,65 | 5,6 | 0,65 | 5,8 | 0,65 | 2,9 | 0,65 | 5,6 |
| 0,7 | 7,5 | 0,7 | 5,4 | 0,7 | 5,8 | 0,7 | 2,9 | 0,7 | 5,6 |
| 0,75 | 7,3 | 0,75 | 5,2 | 0,75 | 5,8 | 0,75 | 2,9 | 0,75 | 5,5 |
| 0,8 | 7,0 | 0,8 | 4,9 | 0,8 | 5,8 | 0,8 | 2,7 | 0,8 | 5,2 |
| 0,85 | 6,4 | 0,85 | 4,4 | 0,85 | 5,7 | 0,85 | 2,5 | 0,85 | 4,8 |
| 0,9 | 5,4 | 0,9 | 3,6 | 0,9 | 4,9 | 0,9 | 2,2 | 0,9 | 4,1 |
| 0,95 | 3,7 | 0,95 | 2,6 | 0,95 | 3,5 | 0,95 | 1,8 | 0,95 | 2,9 |
| 1 | 1,2 | 1 | 1,2 | 1 | 1,2 | 1 | 1,2 | 1 | 1,2 |

**HF - HFO-1234yf - HFC-245cb - HFO-1234zeE - HFO-1234zeZ**

| Organics 0,9 F1234yf + 0,033 F1234zeE +0,033 F245cb +0,034 F1234zeZ | | Organics 0,033 F1234yf + 0,9 F1234zeE +0,033 F245cb +0,034 F1234zeZ | | Organics 0,033 F1234yf + 0,033 F1234zeE +0,9 F245cb +0,034 F1234zeZ | | Organics 0,034 F1234yf + 0,033 F1234zeE +0,033 F245cb +0,9 F1234zeZ | | Organics 0,25 F1234yf + 0,25 F1234zeE +0,25 F245cb +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 4,8 | 0 | 4,6 | 0 | 2,2 | 0 | 4,5 |
| 0,05 | 7,5 | 0,05 | 5,8 | 0,05 | 5,8 | 0,05 | 3,3 | 0,05 | 5,6 |
| 0,1 | 7,5 | 0,1 | 5,8 | 0,1 | 5,8 | 0,1 | 3,3 | 0,1 | 5,6 |
| 0,15 | 7,5 | 0,15 | 5,8 | 0,15 | 5,8 | 0,15 | 3,3 | 0,15 | 5,6 |
| 0,2 | 7,5 | 0,2 | 5,8 | 0,2 | 5,8 | 0,2 | 3,3 | 0,2 | 5,6 |
| 0,25 | 7,5 | 0,25 | 5,8 | 0,25 | 5,8 | 0,25 | 3,3 | 0,25 | 5,6 |
| 0,3 | 7,5 | 0,3 | 5,8 | 0,3 | 5,8 | 0,3 | 3,3 | 0,3 | 5,6 |
| 0,35 | 7,5 | 0,35 | 5,8 | 0,35 | 5,8 | 0,35 | 3,3 | 0,35 | 5,6 |
| 0,4 | 75 | 0,4 | 5,8 | 0,4 | 5,8 | 0,4 | 3,3 | 0,4 | 5,6 |
| 0,45 | 7,5 | 0,45 | 5,8 | 0,45 | 5,8 | 0,45 | 3,3 | 0,45 | 5,6 |
| 0,5 | 7,5 | 0,5 | 5,8 | 0,5 | 5,8 | 0,5 | 3,3 | 0,5 | 5,6 |
| 0,55 | 7,5 | 0,55 | 5,7 | 0,55 | 5,8 | 0,55 | 3,3 | 0,55 | 5,6 |
| 0,6 | 7,5 | 0,6 | 5,7 | 0,6 | 5,8 | 0,6 | 3,3 | 0,6 | 5,6 |
| 0,65 | 7,5 | 0,65 | 5,6 | 0,65 | 5,8 | 0,65 | 3,3 | 0,65 | 5,6 |
| 0,7 | 7,5 | 0,7 | 5,4 | 0,7 | 5,8 | 0,7 | 3,3 | 0,7 | 5,6 |
| 0,75 | 7,3 | 0,75 | 5,2 | 0,75 | 5,8 | 0,75 | 3,3 | 0,75 | 5,5 |
| 0,8 | 7,0 | 0,8 | 4,9 | 0,8 | 5,8 | 0,8 | 3,1 | 0,8 | 5,3 |
| 0,85 | 6,4 | 0,85 | 4,4 | 0,85 | 5,7 | 0,85 | 2,9 | 0,85 | 4,9 |
| 0,9 | 5,4 | 0,9 | 3,7 | 0,9 | 4,9 | 0,9 | 2,5 | 0,9 | 4,1 |
| 0,95 | 3,7 | 0,95 | 2,6 | 0,95 | 3,5 | 0,95 | 2,0 | 0,95 | 3,0 |
| 1 | 1,2 | 1 | 1,2 | 1 | 1,2 | 1 | 1,2 | 1 | 1,2 |

**HF - HFO-1234yf - HFC-245cb - HFO-1234zeE - HFO-1243zf**

| Organics 0,9 F1234yf + 0,033 F1234zeE +0,033 F245cb +0,034 F1243zf | | Organics 0,033 F1234yf + 0,9 F1234zeE +0,033 F245cb +0,034 F1243zf | | Organics 0,033 F1234yf + 0,033 F1234zeE +0,9 F245cb +0,034 F1243zf | | Organics 0,034 F1234yf + 0,033 F1234zeE +0,033 F245cb +0,9 F1243zf | | Organics 0,25 F1234yf + 0,25 F1234zeE +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,6 | 0 | 5,0 | 0 | 4,8 | 0 | 5,8 | 0 | 5,6 |
| 0,05 | 7,6 | 0,05 | 5,9 | 0,05 | 6,0 | 0,05 | 6,8 | 0,05 | 6,6 |
| 0,1 | 7,6 | 0,1 | 6,0 | 0,1 | 6,0 | 0,1 | 6,8 | 0,1 | 6,6 |
| 0,15 | 7,6 | 0,15 | 6,0 | 0,15 | 6,0 | 0,15 | 6,8 | 0,15 | 6,6 |
| 0,2 | 7,6 | 0,2 | 6,0 | 0,2 | 6,0 | 0,2 | 6,8 | 0,2 | 6,6 |
| 0,25 | 7,6 | 0,25 | 6,0 | 0,25 | 6,0 | 0,25 | 6,8 | 0,25 | 6,6 |
| 0,3 | 7,6 | 0,3 | 6,0 | 0,3 | 6,0 | 0,3 | 6,8 | 0,3 | 6,6 |
| 0,35 | 7,6 | 0,35 | 6,0 | 0,35 | 6,0 | 0,35 | 6,8 | 0,35 | 6,6 |
| 0,4 | 7,6 | 0,4 | 6,0 | 0,4 | 6,0 | 0,4 | 6,8 | 0,4 | 6,6 |
| 0,45 | 7,6 | 0,45 | 5,9 | 0,45 | 6,0 | 0,45 | 6,8 | 0,45 | 6,6 |
| 0,5 | 7,6 | 0,5 | 5,9 | 0,5 | 6,0 | 0,5 | 6,8 | 0,5 | 6,6 |
| 0,55 | 7,6 | 0,55 | 5,9 | 0,55 | 6,0 | 0,55 | 6,7 | 0,55 | 6,6 |
| 0,6 | 7,6 | 0,6 | 5,8 | 0,6 | 6,0 | 0,6 | 6,7 | 0,6 | 6,6 |
| 0,65 | 7,6 | 0,65 | 5,7 | 0,65 | 5,9 | 0,65 | 6,7 | 0,65 | 6,6 |
| 0,7 | 7,6 | 0,7 | 5,6 | 0,7 | 5,9 | 0,7 | 6,6 | 0,7 | 6,6 |
| 0,75 | 7,4 | 0,75 | 5,3 | 0,75 | 5,9 | 0,75 | 6,4 | 0,75 | 6,4 |
| 0,8 | 7,1 | 0,8 | 5,0 | 0,8 | 5,9 | 0,8 | 6,1 | 0,8 | 6,1 |
| 0,85 | 6,5 | 0,85 | 4,5 | 0,85 | 5,8 | 0,85 | 5,6 | 0,85 | 5,6 |
| 0,9 | 5,5 | 0,9 | 3,7 | 0,9 | 5,0 | 0,9 | 4,8 | 0,9 | 4,8 |
| 0,95 | 3,8 | 0,95 | 2,7 | 0,95 | 3,5 | 0,95 | 3,4 | 0,95 | 3,4 |
| 1 | 1,2 | 1 | 1,2 | 1 | 1,2 | 1 | 1,2 | 1 | 1,2 |

**HF - HFO-1234yf - HFC-245cb - HFO-1234zeZ - HCFO-1233zdE**

| Organics 0,9 F1234yf + 0,033 F1234zeZ +0,033 F245cb +0,034 F1233zdE | | Organics 0,033 F1234yf + 0,9 F1234zeZ +0,033 F245cb +0,034 F1233zdE | | Organics 0,033 F1234yf + 0,033 F1234zeZ +0,9 F245cb +0,034 F1233zdE | | Organics 0,034 F1234yf + 0,033 F1234zeZ +0,033 F245cb +0,9 F1233zdE | | Organics 0,25 F1234yf + 0,25 F1234zeZ +0,25 F245cb +0,25 F1233zdE | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 2,1 | 0 | 4,5 | 0 | 1,7 | 0 | 3,7 |
| 0,05 | 7,4 | 0,05 | 3,2 | 0,05 | 5,7 | 0,05 | 2,8 | 0,05 | 4,8 |
| 0,1 | 7,4 | 0,1 | 3,2 | 0,1 | 5,7 | 0,1 | 2,8 | 0,1 | 4,8 |
| 0,15 | 7,4 | 0,15 | 3,2 | 0,15 | 5,7 | 0,15 | 2,8 | 0,15 | 4,8 |
| 0,2 | 7,4 | 0,2 | 3,2 | 0,2 | 5,7 | 0,2 | 2,8 | 0,2 | 4,8 |
| 025 | 74 | 0,25 | 3,2 | 0,25 | 5,7 | 0,25 | 2,8 | 0,25 | 4,8 |
| 0,3 | 7,4 | 0,3 | 3,2 | 0,3 | 5,7 | 0,3 | 2,8 | 0,3 | 4,8 |
| 0,35 | 7,4 | 0,35 | 3,2 | 0,35 | 5,7 | 0,35 | 2,8 | 0,35 | 4,8 |
| 0,4 | 7,4 | 0,4 | 3,2 | 0,4 | 5,7 | 0,4 | 2,8 | 0,4 | 4,8 |
| 0,45 | 7,4 | 0,45 | 3,2 | 0,45 | 5,7 | 0,45 | 2,8 | 0,45 | 4,8 |
| 0,5 | 7,4 | 0,5 | 3,2 | 0,5 | 5,7 | 0,5 | 2,8 | 0,5 | 4,8 |
| 0,55 | 7,4 | 0,55 | 3,2 | 0,55 | 5,7 | 0,55 | 2,8 | 0,55 | 4,8 |
| 0,6 | 7,4 | 0,6 | 3,2 | 0,6 | 5,7 | 0,6 | 2,8 | 0,6 | 4,9 |
| 0,65 | 7,4 | 0,65 | 3,2 | 0,65 | 5,7 | 0,65 | 2,8 | 0,65 | 4,9 |
| 0,7 | 7,4 | 0,7 | 3,2 | 0,7 | 5,7 | 0,7 | 2,8 | 0,7 | 4,9 |
| 0,75 | 7,2 | 0,75 | 3,2 | 0,75 | 5,7 | 0,75 | 2,8 | 0,75 | 4,9 |
| 0,8 | 6,9 | 0,8 | 3,0 | 0,8 | 5,7 | 0,8 | 2,7 | 0,8 | 4,7 |
| 0,85 | 6,4 | 0,85 | 2,8 | 0,85 | 5,7 | 0,85 | 2,5 | 0,85 | 4,3 |
| 0,9 | 5,4 | 0,9 | 2,5 | 0,9 | 4,9 | 0,9 | 2,2 | 0,9 | 3,7 |
| 0,95 | 3,7 | 0,95 | 2,0 | 0,95 | 3,5 | 0,95 | 1,8 | 0,95 | 2,7 |
| 1 | 1,2 | 1 | 1,2 | 1 | 1,2 | 1 | 1,2 | 1 | 1,2 |

**HF - HFO-1234yf - HFC-245cb - HFO-1234zeZ - HFO-1243zf**

| Organics 0,9 F1234yf + 0,033 F1234zeZ +0,033 F245cb +0,034 F1243zf | | Organics 0,033 F1234yf + 0,9 F1234zeZ +0,033 F245cb +0,034 F1243zf | | Organics 0,033 F1234yf + 0,033 F1234zeZ +0,9 F245cb +0,034 F1243zf | | Organics 0,034 F1234yf + 0,033 F1234zeZ +0,033 F245cb +0,9 F1243zf | | Organics 0,25 F1234yf + 0,25 F1234zeZ +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 2,2 | 0 | 4,7 | 0 | 5,7 | 0 | 4,8 |
| 0,05 | 7,5 | 0,05 | 3,3 | 0,05 | 5,8 | 0,05 | 6,7 | 0,05 | 5,9 |
| 0,1 | 7,5 | 0,1 | 3,3 | 0,1 | 5,8 | 0,1 | 6,7 | 0,1 | 5,9 |
| 0,15 | 7,5 | 0,15 | 3,3 | 0,15 | 5,8 | 0,15 | 6,7 | 0,15 | 5,9 |
| 0,2 | 7,5 | 0,2 | 3,3 | 0,2 | 5,8 | 0,2 | 6,7 | 0,2 | 5,9 |
| 0,25 | 7,5 | 0,25 | 3,3 | 0,25 | 5,8 | 0,25 | 6,7 | 0,25 | 5,9 |
| 0,3 | 7,5 | 0,3 | 3,3 | 0,3 | 5,8 | 0,3 | 6,7 | 0,3 | 5,9 |
| 0,35 | 7,5 | 0,35 | 3,3 | 0,35 | 5,8 | 0,35 | 6,7 | 0,35 | 5,9 |
| 0,4 | 7,5 | 0,4 | 3,3 | 0,4 | 5,8 | 0,4 | 6,7 | 0,4 | 5,9 |
| 0,45 | 7,5 | 0,45 | 3,3 | 0,45 | 5,8 | 0,45 | 6,7 | 0,45 | 5,9 |
| 0,5 | 7,5 | 0,5 | 3,3 | 0,5 | 5,8 | 0,5 | 6,7 | 0,5 | 5,9 |
| 0,55 | 7,5 | 0,55 | 3,3 | 0,55 | 5,8 | 0,55 | 6,7 | 0,55 | 5,9 |
| 0,6 | 7,5 | 0,6 | 3,3 | 0,6 | 5,8 | 0,6 | 6,6 | 0,6 | 5,9 |
| 0,65 | 7,5 | 0,65 | 3,3 | 0,65 | 5,8 | 0,65 | 6,6 | 0,65 | 5,9 |
| 0,7 | 75 | 0,7 | 3,3 | 0,7 | 5,9 | 0,7 | 6,5 | 0,7 | 5,9 |
| 0,75 | 7,3 | 0,75 | 3,3 | 0,75 | 5,9 | 0,75 | 6,3 | 0,75 | 5,9 |
| 0,8 | 7,0 | 0,8 | 3,2 | 0,8 | 5,9 | 0,8 | 6,1 | 0,8 | 5,6 |
| 0,85 | 6,5 | 0,85 | 2,9 | 0,85 | 5,8 | 0,85 | 5,6 | 0,85 | 5,2 |
| 0,9 | 5,4 | 0,9 | 2,6 | 0,9 | 5,0 | 0,9 | 4,7 | 0,9 | 4,4 |
| 0,95 | 3,8 | 0,95 | 2,0 | 0,95 | 3,5 | 0,95 | 3,4 | 0,95 | 3,2 |
| 1 | 1,2 | 1 | 1,2 | 1 | 1,2 | 1 | 1,2 | 1 | 1,2 |

**HF - HFO-1234yf - HFC-245cb - HCFO-1233zdE - HFO-1243zf**

| Organics 0,9 F1234yf + 0,033 F1233zdE +0,033 F245cb +0,034 F1243zf | | Organics 0,033 F1234yf + 0,9 F1233zdE +0,033 F245cb +0,034 F1243zf | | Organics 0,033 F1234yf + 0,033 F1233zdE +0,9 F245cb +0,034 F1243zf | | Organics 0,034 F1234yf + 0,033 F1233zdE +0,033 F245cb +0,9 F1243zf | | Organics 0,25 F1234yf + 0,25 F1233zdE +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 1,8 | 0 | 4,7 | 0 | 5,7 | 0 | 4,8 |
| 0,05 | 7,5 | 0,05 | 2,9 | 0,05 | 5,8 | 0,05 | 6,7 | 0,05 | 5,9 |
| 0,1 | 7,5 | 0,1 | 2,9 | 0,1 | 5,8 | 0,1 | 6,7 | 0,1 | 5,9 |
| 0,15 | 7,5 | 0,15 | 2,9 | 0,15 | 5,8 | 0,15 | 6,7 | 0,15 | 5,9 |
| 0,2 | 7,5 | 0,2 | 2,9 | 0,2 | 5,8 | 0,2 | 6,7 | 0,2 | 5,9 |
| 0,25 | 7,5 | 0,25 | 2,9 | 0,25 | 5,8 | 0,25 | 6,7 | 0,25 | 5,9 |
| 0,3 | 7,5 | 0,3 | 2,9 | 0,3 | 5,8 | 0,3 | 6,7 | 0,3 | 5,9 |
| 0,35 | 7,5 | 0,35 | 2,9 | 0,35 | 5,8 | 0,35 | 6,7 | 0,35 | 5,9 |
| 0,4 | 7,5 | 0,4 | 2,9 | 0,4 | 5,8 | 0,4 | 6,7 | 0,4 | 5,9 |
| 0,45 | 7,5 | 0,45 | 2,9 | 0,45 | 5,8 | 0,45 | 6,7 | 0,45 | 5,9 |
| 0,5 | 7,5 | 0,5 | 2,9 | 0,5 | 5,8 | 0,5 | 6,7 | 0,5 | 5,9 |
| 0,55 | 7,5 | 0,55 | 2,9 | 0,55 | 5,8 | 0,55 | 6,7 | 0,55 | 5,9 |
| 0,6 | 7,5 | 0,6 | 2,9 | 0,6 | 5,8 | 0,6 | 6,6 | 0,6 | 5,9 |
| 0,65 | 7,5 | 0,65 | 2,9 | 0,65 | 5,8 | 0,65 | 6,6 | 0,65 | 5,9 |
| 0,7 | 7,5 | 0,7 | 2,9 | 0,7 | 5,8 | 0,7 | 6,5 | 0,7 | 5,9 |
| 0,75 | 7,3 | 0,75 | 2,9 | 0,75 | 5,8 | 0,75 | 6,3 | 0,75 | 5,8 |
| 0,8 | 7,0 | 0,8 | 2,8 | 0,8 | 5,8 | 0,8 | 6,1 | 0,8 | 5,6 |
| 0,85 | 6,5 | 0,85 | 2,6 | 0,85 | 5,8 | 0,85 | 5,6 | 0,85 | 5,1 |
| 0,9 | 5,4 | 0,9 | 2,3 | 0,9 | 5,0 | 0,9 | 4,7 | 0,9 | 4,4 |
| 0,95 | 3,8 | 0,95 | 1,8 | 0,95 | 3,5 | 0,95 | 3,4 | 0,95 | 3,1 |
| 1 | 1,2 | 1 | 1,2 | 1 | 1,2 | 1 | 1,2 | 1 | 1,2 |

**HF - HCFO-1233xf - HFC-245cb - HCFO-1233zdE - HFO-1234yf**

| Organics 0,9 F1233xf + 0,033 F1233zdE +0,033 F245cb +0,034 F1234yf | | Organics 0,033 F1233xf + 0,9 F1233zdE +0,033 F245cb +0,034 F1234yf | | Organics 0,033 F1233xf + 0,033 F1233zdE +0,9 F245cb +0,034 F1234yf | | Organics 0,034 F1233xf + 0,033 F1233zdE +0,033 F245cb +0,9 F1234yf | | Organics 0,25 F1233xf + 0,25 F1233zdE +0,25 F245cb +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 1,7 | 0 | 4,5 | 0 | 6,4 | 0 | 3,7 |
| 0,05 | 3,0 | 0,05 | 2,8 | 0,05 | 5,7 | 0,05 | 7,4 | 0,05 | 4,8 |
| 0,1 | 3,0 | 0,1 | 2,8 | 0,1 | 5,7 | 0,1 | 7,4 | 0,1 | 4,8 |
| 0,15 | 3,0 | 0,15 | 2,8 | 0,15 | 5,7 | 0,15 | 7,4 | 0,15 | 4,8 |
| 0,2 | 3,0 | 0,2 | 2,8 | 0,2 | 5,7 | 0,2 | 7,4 | 0,2 | 4,8 |
| 0,25 | 3,0 | 0,25 | 2,8 | 0,25 | 5,7 | 0,25 | 7,4 | 0,25 | 4,8 |
| 0,3 | 3,0 | 0,3 | 2,8 | 0,3 | 5,7 | 0,3 | 7,4 | 0,3 | 4,8 |
| 0,35 | 3,0 | 0,35 | 2,8 | 0,35 | 5,7 | 0,35 | 7,4 | 0,35 | 4,8 |
| 0,4 | 3,0 | 0,4 | 2,8 | 0,4 | 5,7 | 0,4 | 7,4 | 0,4 | 4,8 |
| 0,45 | 3,0 | 0,45 | 2,8 | 0,45 | 5,7 | 0,45 | 7,4 | 0,45 | 4,8 |
| 0,5 | 3,0 | 0,5 | 2,8 | 0,5 | 5,7 | 0,5 | 7,4 | 0,5 | 4,8 |
| 0,55 | 3,0 | 0,55 | 2,8 | 0,55 | 5,7 | 0,55 | 7,4 | 0,55 | 4,8 |
| 0,6 | 3,0 | 0,6 | 2,8 | 0,6 | 5,7 | 0,6 | 7,4 | 0,6 | 4,8 |
| 0,65 | 3,0 | 0,65 | 2,8 | 0,65 | 5,7 | 0,65 | 7,4 | 0,65 | 4,9 |
| 0,7 | 3,0 | 0,7 | 2,8 | 0,7 | 5,7 | 0,7 | 7,4 | 0,7 | 4,9 |
| 0,75 | 3,0 | 0,75 | 2,8 | 0,75 | 5,7 | 0,75 | 7,2 | 0,75 | 4,9 |
| 0,8 | 2,8 | 0,8 | 2,7 | 0,8 | 5,7 | 0,8 | 6,9 | 0,8 | 4,7 |
| 0,85 | 2,6 | 0,85 | 2,5 | 0,85 | 5,7 | 0,85 | 6,4 | 0,85 | 4,3 |
| 0,9 | 2,3 | 0,9 | 2,2 | 0,9 | 4,9 | 0,9 | 5,4 | 0,9 | 3,7 |
| 0,95 | 1,8 | 0,95 | 1,8 | 0,95 | 3,5 | 0,95 | 3,7 | 0,95 | 2,7 |
| 1 | 1,2 | 1 | 1,2 | 1 | 1,2 | 1 | 1,2 | 1 | 1,2 |

**HF - HCFO-1233xf - HFC-245cb - HFO-1234zeE - HFO-1234yf**

| Organics 0,9 F1233xf + 0,033 F1234zeE +0,033 F245cb +0,034 F1234yf | | Organics 0,033 F1233xf + 0,9 F1234zeE +0,033 F245cb +0,034 F1234yf | | Organics 0,033 F1233xf + 0,033 F1234zeE +0,9 F245cb +0,034 F1234yf | | Organics 0,034 F1233xf + 0,033 F1234zeE +0,033 F245cb +0,9 F1234yf | | Organics 0,25 F1233xf + 0,25 F1234zeE +0,25 F245cb +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,0 | 0 | 4,8 | 0 | 4,6 | 0 | 6,5 | 0 | 4,6 |
| 0,05 | 3,1 | 0,05 | 5,8 | 0,05 | 5,8 | 0,05 | 7,5 | 0,05 | 5,6 |
| 0,1 | 3,1 | 0,1 | 5,8 | 0,1 | 5,8 | 0,1 | 7,5 | 0,1 | 5,6 |
| 0,15 | 3,1 | 0,15 | 5,8 | 0,15 | 5,8 | 0,15 | 7,5 | 0,15 | 5,6 |
| 0,2 | 3,1 | 0,2 | 5,8 | 0,2 | 5,8 | 0,2 | 7,5 | 0,2 | 5,6 |
| 0,25 | 3,1 | 0,25 | 5,8 | 0,25 | 5,8 | 0,25 | 7,5 | 0,25 | 5,6 |
| 0,3 | 3,1 | 0,3 | 5,8 | 0,3 | 5,8 | 0,3 | 7,5 | 0,3 | 5,6 |
| 0,35 | 3,1 | 0,35 | 5,8 | 0,35 | 5,8 | 0,35 | 7,5 | 0,35 | 5,6 |
| 0,4 | 3,1 | 0,4 | 5,8 | 0,4 | 5,8 | 0,4 | 7,5 | 0,4 | 5,6 |
| 0,45 | 3,1 | 0,45 | 5,8 | 0,45 | 5,8 | 0,45 | 7,5 | 0,45 | 5,6 |
| 0,5 | 3,1 | 0,5 | 5,8 | 0,5 | 5,8 | 0,5 | 7,5 | 0,5 | 5,6 |
| 0,55 | 3,1 | 0,55 | 5,7 | 0,55 | 5,8 | 0,55 | 7,5 | 0,55 | 5,6 |
| 0,6 | 3,1 | 0,6 | 5,7 | 0,6 | 5,8 | 0,6 | 7,5 | 0,6 | 5,6 |
| 0,65 | 3,1 | 0,65 | 5,6 | 0,65 | 5,8 | 0,65 | 7,5 | 0,65 | 5,7 |
| 0,7 | 3,1 | 0,7 | 5,4 | 0,7 | 5,8 | 0,7 | 7,5 | 0,7 | 5,7 |
| 0,75 | 3,1 | 0,75 | 5,2 | 0,75 | 5,8 | 0,75 | 7,3 | 0,75 | 5,5 |
| 0,8 | 2,9 | 0,8 | 4,9 | 0,8 | 5,8 | 0,8 | 7,0 | 0,8 | 5,3 |
| 0,85 | 2,7 | 0,85 | 4,4 | 0,85 | 5,7 | 0,85 | 6,4 | 0,85 | 4,8 |
| 0,9 | 2,4 | 0,9 | 3,7 | 0,9 | 4,9 | 0,9 | 5,4 | 0,9 | 4,1 |
| 0,95 | 1,9 | 0,95 | 2,6 | 0,95 | 3,5 | 0,95 | 3,7 | 0,95 | 2,9 |
| 1 | 1,2 | 1 | 1,2 | 1 | 1,2 | 1 | 1,2 | 1 | 1,2 |

**HF - HCFO-1233xf - HFC-245cb - HFO-1234zeZ - HFO-1234yf**

| Organics 0,9 F1233xf + 0,033 F1234zeZ +0,033 F245cb +0,034 F1234yf | | Organics 0,033 F1233xf + 0,9 F1234zeZ +0,033 F245cb +0,034 F1234yf | | Organics 0,033 F1233xf + 0,033 F1234zeZ +0,9 F245cb +0,034 F1234yf | | Organics 0,034 F1233xf + 0,033 F1234zeZ +0,033 F245cb +0,9 F1234yf | | Organics 0,25 F1233xf + 0,25 F1234zeZ +0,25 F245cb +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 2,1 | 0 | 4,5 | 0 | 6,4 | 0 | 3,7 |
| 0,05 | 3,0 | 0,05 | 3,2 | 0,05 | 5,7 | 0,05 | 7,4 | 0,05 | 4,9 |
| 0,1 | 3,0 | 0,1 | 3,2 | 0,1 | 5,7 | 0,1 | 7,4 | 0,1 | 4,9 |
| 0,15 | 3,0 | 0,15 | 3,2 | 0,15 | 5,7 | 0,15 | 7,4 | 0,15 | 4,9 |
| 0,2 | 3,0 | 0,2 | 3,2 | 0,2 | 5,7 | 0,2 | 7,4 | 0,2 | 4,9 |
| 0,25 | 3,0 | 0,25 | 3,2 | 0,25 | 5,7 | 0,25 | 7,4 | 0,25 | 4,9 |
| 0,3 | 3,0 | 0,3 | 3,2 | 0,3 | 5,7 | 0,3 | 7,4 | 0,3 | 4,9 |
| 0,35 | 3,0 | 0,35 | 3,2 | 0,35 | 5,7 | 0,35 | 7,4 | 0,35 | 4,9 |
| 0,4 | 3,0 | 0,4 | 3,2 | 0,4 | 5,7 | 0,4 | 7,4 | 0,4 | 4,9 |
| 0,45 | 3,0 | 0,45 | 3,2 | 0,45 | 5,7 | 0,45 | 7,4 | 0,45 | 4,9 |
| 0,5 | 3,0 | 0,5 | 3,2 | 0,5 | 5,7 | 0,5 | 7,4 | 0,5 | 4,9 |
| 0,55 | 3,0 | 0,55 | 3,2 | 0,55 | 5,7 | 0,55 | 7,4 | 0,55 | 4,9 |
| 0,6 | 3,0 | 0,6 | 3,2 | 0,6 | 5,7 | 0,6 | 7,4 | 0,6 | 4,9 |
| 0,65 | 3,0 | 0,65 | 3,2 | 0,65 | 5,7 | 0,65 | 7,4 | 0,65 | 4,9 |
| 0,7 | 3,0 | 0,7 | 3,2 | 0,7 | 5,7 | 0,7 | 7,4 | 0,7 | 4,9 |
| 0,75 | 3,0 | 0,75 | 3,2 | 0,75 | 5,7 | 0,75 | 7,2 | 0,75 | 5,0 |
| 0,8 | 2,8 | 0,8 | 3,0 | 0,8 | 5,7 | 0,8 | 6,9 | 0,8 | 4,8 |
| 0,85 | 2,6 | 0,85 | 2,8 | 0,85 | 5,7 | 0,85 | 6,4 | 0,85 | 4,4 |
| 0,9 | 2,3 | 0,9 | 2,5 | 0,9 | 4,9 | 0,9 | 5,4 | 0,9 | 3,8 |
| 0,95 | 1,8 | 0,95 | 2,0 | 0,95 | 3,5 | 0,95 | 3,7 | 0,95 | 2,7 |
| 1 | 1,2 | 1 | 1,2 | 1 | 1,2 | 1 | 1,2 | 1 | 1,2 |

**HF - HCFO-1233xf - HFO-1234yf - HFC-245cb - HCFC-244bb**

| | Organics 0,25 F1233xf + 0,25 F1234yf + +0,25 F245cb + 0,25 F244bb | Organics 0,97 F1233xf + 0,01 F1234yf + 0,01 F245cb + 0,01 F244bb | Organics 0,01 F1233xf + 0,97 F1234yf + 0,01 F245cb + 0,01 F244bb | Organics 0,01 F1233xf + 0,01 F1234yf + 0,97 F245cb + 0,01 F244bb | Organics 0,01 F1233xf + 0,01 F1234yf + 0,01 F245cb + 0,97 F244bb |
|---|---|---|---|---|---|
| MASSFRAC | TOTAL | TOTAL | TOTAL | TOTAL | TOTAL |
| HF | PRESSURE | HF | PRESSURE | HF | PRESSURE |
| | bar | bar | bar | bar | bar |
| 0 | 3,6 | 1,6 | 6,7 | 4,6 | 0,8 |
| 0,05 | 4,7 | 2,7 | 7,6 | 5,8 | 1,9 |
| 0,1 | 4,7 | 2,7 | 7,7 | 5,8 | 1,9 |
| 0,15 | 4,7 | 2,7 | 7,6 | 5,8 | 1,9 |
| 0,2 | 4,7 | 2,7 | 7,6 | 5,8 | 1,9 |
| 0,25 | 4,7 | 2,7 | 7,6 | 5,8 | 1,9 |
| 0,3 | 4,7 | 2,7 | 7,6 | 5,8 | 1,9 |
| 0,35 | 4,7 | 2,7 | 7,6 | 5,8 | 1,9 |
| 0,4 | 4,7 | 2,7 | 7,6 | 5,8 | 1,9 |
| 0,45 | 4,7 | 2,7 | 7,6 | 5,8 | 1,9 |
| 0,5 | 4,7 | 2,7 | 7,6 | 5,8 | 1,9 |
| 0,55 | 4,7 | 2,7 | 7,6 | 5,8 | 1,9 |
| 0,6 | 4,7 | 2,7 | 7,6 | 5,8 | 1,9 |
| 0,65 | 4,7 | 2,7 | 7,6 | 5,8 | 1,9 |
| 0,7 | 4,6 | 2,7 | 7,6 | 5,8 | 1,9 |
| 0,75 | 4,6 | 2,7 | 7,5 | 5,8 | 1,9 |
| 0,8 | 4,6 | 2,6 | 7,2 | 5,8 | 1,9 |
| 0,85 | 4,2 | 2,4 | 6,6 | 5,8 | 1,9 |
| 0,9 | 3,6 | 2,1 | 5,5 | 5,0 | 1,8 |
| 0,95 | 2,7 | 1,7 | 3,8 | 3,5 | 1,5 |
| 1 | 1,2 | 1,2 | 1,2 | 1,2 | 1,2 |

### Exemple 4 : Mélanges à six composés

**HF - HCFO-1233xf - HFO-1234yf - HFC-245cb - HFO-1234zeE- HFO-1234zeZ**

| **Organics 0,2 F1233xf + 0,2 F1234yf + 0,2 F245cb 0,2 F1234zeE + 0,2 F1234zeZ** | | **Organics 0,96 F1233xf + 0,01 F1234yf + 0,01 F245cb + 0,01 F1234zeE + 0,01 F1234zeZ** | | **Organics 0,01 F1233xf + 0,96 F1234yf + 0,01 F245cb + 0,01 F1234zeE + 0,01 F1234zeZ** | | **Organics 0,01 F1233xf + 0,01 F1234yf + 0,96 F245cb + 0,01 F1234zeE + 0,01 F1234zeZ** | | **Organics 0,01 F1233xf + 0,01 F1234yf + 0,01 F245cb + 0,96 F1234zeE + 0,01 F1234zeZ** | | **Organics 0,01 F1233xf + 0,01 F1234yf + 0,01 F245cb + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,0 | 0 | 1,7 | 0 | 6,7 | 0 | 4,6 | 0 | 4,8 | 0 | 1,9 |
| 0,05 | 5,1 | 0,05 | 2,8 | 0,05 | 7,6 | 0,05 | 5,8 | 0,05 | 5,8 | 0,05 | 3,0 |
| 0,1 | 5,1 | 0,1 | 2,8 | 0,1 | 7,6 | 0,1 | 5,8 | 0,1 | 5,8 | 0,1 | 3,0 |
| 0,15 | 5,1 | 0,15 | 2,8 | 0,15 | 7,6 | 0,15 | 5,8 | 0,15 | 5,8 | 0,15 | 3,0 |
| 0,2 | 5,1 | 0,2 | 2,8 | 0,2 | 7,6 | 0,2 | 5,8 | 0,2 | 5,8 | 0,2 | 3,0 |
| 0,25 | 5,1 | 0,25 | 2,8 | 0,25 | 7,6 | 0,25 | 5,8 | 0,25 | 5,8 | 0,25 | 3,0 |
| 0,3 | 5,1 | 0,3 | 2,8 | 0,3 | 7,6 | 0,3 | 5,8 | 0,3 | 5,8 | 0,3 | 3,0 |
| 0,35 | 5,1 | 0,35 | 2,8 | 0,35 | 7,6 | 0,35 | 5,8 | 0,35 | 5,8 | 0,35 | 3,0 |
| 0,4 | 5,1 | 0,4 | 2,8 | 0,4 | 7,6 | 0,4 | 5,8 | 0,4 | 5,8 | 0,4 | 3,0 |
| 0,45 | 5,1 | 0,45 | 2,8 | 0,45 | 7,6 | 0,45 | 5,8 | 0,45 | 5,8 | 0,45 | 3,0 |
| 0,5 | 5,1 | 0,5 | 2,8 | 0,5 | 7,6 | 0,5 | 5,8 | 0,5 | 5,7 | 0,5 | 3,0 |
| 0,55 | 5,1 | 0,55 | 2,8 | 0,55 | 7,6 | 0,55 | 5,8 | 0,55 | 5,7 | 0,55 | 3,0 |
| 0,6 | 5,1 | 0,6 | 2,8 | 0,6 | 7,6 | 0,6 | 5,8 | 0,6 | 5,6 | 0,6 | 3,0 |
| 0,65 | 5,1 | 0,65 | 2,8 | 0,65 | 7,6 | 0,65 | 5,8 | 0,65 | 5,5 | 0,65 | 3,0 |
| 0,7 | 5,1 | 0,7 | 2,8 | 0,7 | 7,6 | 0,7 | 5,8 | 0,7 | 5,4 | 0,7 | 3,0 |
| 0,75 | 5,0 | 0,75 | 2,8 | 0,75 | 7,5 | 0,75 | 5,8 | 0,75 | 5,1 | 0,75 | 3,0 |
| 0,8 | 4,8 | 0,8 | 2,6 | 0,8 | 7,1 | 0,8 | 5,8 | 0,8 | 4,8 | 0,8 | 2,9 |
| 0,85 | 4,4 | 0,85 | 2,5 | 0,85 | 6,5 | 0,85 | 5,8 | 0,85 | 4,3 | 0,85 | 2,7 |
| 0,9 | 3,7 | 0,9 | 2,2 | 0,9 | 5,5 | 0,9 | 5,0 | 0,9 | 3,6 | 0,9 | 2,4 |
| 0,95 | 2,7 | 0,95 | 1,8 | 0,95 | 3,8 | 0,95 | 3,5 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeE- HCFO-1233zdE**

| **Organics 0,2 F1233xf + 0,2 F1234yf + 0,2 F245cb 0,2 F1234zeE + 0,2 F1233zdE** | | **Organics 0,96 F1233xf + 0,01 F1234yf + 0,01 F245cb + 0,01 F1234zeE + 0,01 F1233zdE** | | **Organics 0,01 F1233xf** + **0,96 F1234yf + 0,01 F245cb + 0,01 F1234zeE + 0,01 F1233zdE** | | **Organics 0,01 F1233xf** + **0,01 F1234yf + 0,96 F245cb + 0,01 F1234zeE + 0,01 F1233zdE** | | **Organics 0,01 F1233xf + 0,01 F1234yf + 0,01 F245cb + 0,96 F1234zeE + 0,01 F1233zdE** | | **Organics 0,01 F1233xf +0,01 F1234yf + 0,01 F245cb + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,9 | 0 | 1,7 | 0 | 6,7 | 0 | 4,6 | 0 | 4,8 | 0 | 1,5 |
| 0,05 | 5,0 | 0,05 | 2,8 | 0,05 | 7,6 | 0,05 | 5,8 | 0,05 | 5,8 | 0,05 | 2,6 |
| 0,1 | 5,0 | 0,1 | 2,8 | 0,1 | 7,6 | 0,1 | 5,8 | 0,1 | 5,8 | 0,1 | 2,6 |
| 0,15 | 5,0 | 0,15 | 2,8 | 0,15 | 7,6 | 0,15 | 5,8 | 0,15 | 5,8 | 0,15 | 2,6 |
| 0,2 | 5,0 | 0,2 | 2,8 | 0,2 | 7,6 | 0,2 | 5,8 | 0,2 | 5,8 | 0,2 | 2,6 |
| 0,25 | 5,0 | 0,25 | 2,8 | 0,25 | 7,6 | 0,25 | 5,8 | 0,25 | 5,8 | 0,25 | 2,6 |
| 0,3 | 5,0 | 0,3 | 2,8 | 0,3 | 7,6 | 0,3 | 5,8 | 0,3 | 5,8 | 0,3 | 2,6 |
| 0,35 | 5,0 | 0,35 | 2,8 | 0,35 | 7,6 | 0,35 | 5,8 | 0,35 | 5,8 | 0,35 | 2,6 |
| 0,4 | 5,0 | 0,4 | 2,8 | 0,4 | 7,6 | 0,4 | 5,8 | 0,4 | 5,8 | 0,4 | 2,6 |
| 0,45 | 5,0 | 0,45 | 2,8 | 0,45 | 7,6 | 0,45 | 5,8 | 0,45 | 5,8 | 0,45 | 2,6 |
| 0,5 | 5,0 | 0,5 | 2,8 | 0,5 | 7,6 | 0,5 | 5,8 | 0,5 | 5,7 | 0,5 | 2,6 |
| 0,55 | 5,0 | 0,55 | 2,8 | 0,55 | 7,6 | 0,55 | 5,8 | 0,55 | 5,7 | 0,55 | 2,6 |
| 0,6 | 5,0 | 0,6 | 2,8 | 0,6 | 7,6 | 0,6 | 5,8 | 0,6 | 5,6 | 0,6 | 2,6 |
| 0,65 | 5,0 | 0,65 | 2,8 | 0,65 | 7,6 | 0,65 | 5,8 | 0,65 | 5,5 | 0,65 | 2,6 |
| 0,7 | 5,1 | 0,7 | 2,8 | 0,7 | 7,6 | 0,7 | 5,8 | 0,7 | 5,4 | 0,7 | 2,6 |
| 0,75 | 5,0 | 0,75 | 2,8 | 0,75 | 7,5 | 0,75 | 5,8 | 0,75 | 5,1 | 0,75 | 2,6 |
| 0,8 | 4,7 | 0,8 | 2,6 | 0,8 | 7,1 | 0,8 | 5,8 | 0,8 | 4,8 | 0,8 | 2,4 |
| 0,85 | 4,3 | 0,85 | 2,5 | 0,85 | 6,5 | 0,85 | 5,8 | 0,85 | 4,3 | 0,85 | 2,3 |
| 0,9 | 3,7 | 0,9 | 2,2 | 0,9 | 5,5 | 0,9 | 5,0 | 0,9 | 3,6 | 0,9 | 2,0 |
| 0,95 | 2,7 | 0,95 | 1,8 | 0,95 | 3,8 | 0,95 | 3,5 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeE- HFO-1243zf**

| **Organics 0,2 F1233xf + 0,2 F1234yf + 0,2 F245cb 0,2 F1234zeE + 0,2 F1243zf** | | **Organics 0,96 F1233xf +0,01 F1234yf + 0,01 F245cb + 0,01 F1234zeE+0,01 F1243zf** | | **Organics 0,01 F1233xf + 0,96 F1234yf + 0,01 F245cb + 0,01 F1234zeE + 0,01 F1243zf** | | **Organics 0,01 F1233xf + 0,01 F1234yf + 0,96 F245cb + 0,01 F1234zeE + 0,01 F1243zf** | | **Organics 0,01 F1233xf + 0,01 F1234yf + 0,01 F245cb + 0,96 F1234zeE + 0,01 F1243zf** | | **Organics 0,01 F1233xf+0,01 F1234yf + 0,01 F245cb + 0,01 F1234zeE + 0,96 F1243zf** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,9 | 0 | 1,7 | 0 | 6,7 | 0 | 4,7 | 0 | 4,9 | 0 | 5,8 |
| 0,05 | 5,9 | 0,05 | 2,8 | 0,05 | 7,7 | 0,05 | 5,8 | 0,05 | 5,8 | 0,05 | 6,7 |
| 0,1 | 5,9 | 0,1 | 2,8 | 0,1 | 7,7 | 0,1 | 5,8 | 0,1 | 5,9 | 0,1 | 6,8 |
| 0,15 | 5,9 | 0,15 | 2,8 | 0,15 | 7,7 | 0,15 | 5,8 | 0,15 | 5,8 | 0,15 | 6,8 |
| 0,2 | 5,9 | 0,2 | 2,8 | 0,2 | 7,7 | 0,2 | 5,8 | 0,2 | 5,8 | 0,2 | 6,8 |
| 0,25 | 5,9 | 0,25 | 2,8 | 0,25 | 7,7 | 0,25 | 5,8 | 0,25 | 5,8 | 0,25 | 6,8 |
| 0,3 | 5,9 | 0,3 | 2,8 | 0,3 | 7,7 | 0,3 | 5,8 | 0,3 | 5,8 | 0,3 | 6,8 |
| 0,35 | 5,9 | 0,35 | 2,8 | 0,35 | 7,7 | 0,35 | 5,8 | 0,35 | 5,8 | 0,35 | 6,8 |
| 0,4 | 5,9 | 0,4 | 2,8 | 0,4 | 7,7 | 0,4 | 5,8 | 0,4 | 5,8 | 0,4 | 6,8 |
| 0,45 | 5,9 | 0,45 | 2,8 | 0,45 | 7,7 | 0,45 | 5,8 | 0,45 | 5,8 | 0,45 | 6,7 |
| 0,5 | 5,9 | 0,5 | 2,8 | 0,5 | 7,7 | 0,5 | 5,8 | 0,5 | 5,8 | 0,5 | 6,7 |
| 0,55 | 5,9 | 0,55 | 2,8 | 0,55 | 7,7 | 0,55 | 5,8 | 0,55 | 5,7 | 0,55 | 6,7 |
| 0,6 | 5,9 | 0,6 | 2,8 | 0,6 | 7,7 | 0,6 | 5,8 | 0,6 | 5,7 | 0,6 | 6,7 |
| 0,65 | 5,9 | 0,65 | 2,8 | 0,65 | 7,7 | 0,65 | 5,8 | 0,65 | 5,6 | 0,65 | 6,6 |
| 0,7 | 5,9 | 0,7 | 2,8 | 0,7 | 7,6 | 0,7 | 5,8 | 0,7 | 5,4 | 0,7 | 6,5 |
| 0,75 | 5,7 | 0,75 | 2,8 | 0,75 | 7,5 | 0,75 | 5,8 | 0,75 | 5,2 | 0,75 | 6,4 |
| 0,8 | 5,5 | 0,8 | 2,7 | 0,8 | 7,2 | 0,8 | 5,8 | 0,8 | 4,8 | 0,8 | 6,1 |
| 0,85 | 5,0 | 0,85 | 2,5 | 0,85 | 6,6 | 0,85 | 5,8 | 0,85 | 4,3 | 0,85 | 5,6 |
| 0,9 | 4,2 | 0,9 | 2,2 | 0,9 | 5,5 | 0,9 | 5,0 | 0,9 | 3,6 | 0,9 | 4,8 |
| 0,95 | 3,0 | 0,95 | 1,8 | 0,95 | 3,8 | 0,95 | 3,5 | 0,95 | 2,6 | 0,95 | 3,4 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeZ- HCFO-1233zdE**

| **Organics 0,2 F1233xf + 0,2 F1234yf + 0,2 F245cb 0,2 F1234zeZ + 0,2 F1233zdE** | | **Organics 0,96 F1233xf + 0,01 F1234yf + 0,01 F245cb + 0,01 F1234zeZ + 0,01 F1233zdE** | | **Organics 0,01 F1233xf + 0,96 F1234yf + 0,01 F245cb + 0,01 F1234zeZ + 0,01 F1233zdE** | | **Organics 0,01 F1233xf + 0,01 F1234yf + 0,96 F245cb + 0,01 F1234zeZ + 0,01 F1233zdE** | | **Organics 0,01 F1233xf + 0,01 F1234yf + 0,01 F245cb + 0,96 F1234zeZ + 0,01 F1233zdE** | | **Organics 0,01 F1233xf + 0,01 F1234yf + 0,01 F245cb + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,3 | 0 | 1,6 | 0 | 6,6 | 0 | 4,6 | 0 | 1,9 | 0 | 1,4 |
| 0,05 | 4,4 | 0,05 | 2,8 | 0,05 | 7,6 | 0,05 | 5,8 | 0,05 | 3,0 | 0,05 | 2,5 |
| 0,1 | 4,4 | 0,1 | 2,8 | 0,1 | 7,6 | 0,1 | 5,8 | 0,1 | 3,0 | 0,1 | 2,5 |
| 0,15 | 4,4 | 0,15 | 2,8 | 0,15 | 7,6 | 0,15 | 5,8 | 0,15 | 3,0 | 0,15 | 2,5 |
| 0,2 | 4,4 | 0,2 | 2,8 | 0,2 | 7,6 | 0,2 | 5,8 | 0,2 | 3,0 | 0,2 | 2,5 |
| 0,25 | 4,4 | 0,25 | 2,8 | 0,25 | 7,6 | 0,25 | 5,8 | 0,25 | 3,0 | 0,25 | 2,5 |
| 0,3 | 4,4 | 0,3 | 2,8 | 0,3 | 7,6 | 0,3 | 5,8 | 0,3 | 3,0 | 0,3 | 2,5 |
| 0,35 | 4,4 | 0,35 | 2,8 | 0,35 | 7,6 | 0,35 | 5,8 | 0,35 | 3,0 | 0,35 | 2,5 |
| 0,4 | 4,4 | 0,4 | 2,8 | 0,4 | 7,6 | 0,4 | 5,8 | 0,4 | 3,0 | 0,4 | 2,5 |
| 0,45 | 4,4 | 0,45 | 2,8 | 0,45 | 7,6 | 0,45 | 5,8 | 0,45 | 3,0 | 0,45 | 2,5 |
| 0,5 | 4,4 | 0,5 | 2,8 | 0,5 | 7,6 | 0,5 | 5,8 | 0,5 | 3,0 | 0,5 | 2,5 |
| 0,55 | 4,4 | 0,55 | 2,8 | 0,55 | 7,6 | 0,55 | 5,8 | 0,55 | 3,0 | 0,55 | 2,5 |
| 0,6 | 4,4 | 0,6 | 2,8 | 0,6 | 7,6 | 0,6 | 5,8 | 0,6 | 3,0 | 0,6 | 2,5 |
| 0,65 | 4,5 | 0,65 | 2,8 | 0,65 | 7,6 | 0,65 | 5,8 | 0,65 | 3,0 | 0,65 | 2,5 |
| 0,7 | 4,5 | 0,7 | 2,8 | 0,7 | 7,6 | 0,7 | 5,8 | 0,7 | 3,0 | 0,7 | 2,5 |
| 0,75 | 4,5 | 0,75 | 2,8 | 0,75 | 7,4 | 0,75 | 5,8 | 0,75 | 3,0 | 0,75 | 2,5 |
| 0,8 | 4,3 | 0,8 | 2,6 | 0,8 | 7,1 | 0,8 | 5,8 | 0,8 | 2,9 | 0,8 | 2,4 |
| 0,85 | 4,0 | 0,85 | 2,4 | 0,85 | 6,5 | 0,85 | 5,8 | 0,85 | 2,7 | 0,85 | 2,3 |
| 0,9 | 3,4 | 0,9 | 2,2 | 0,9 | 5,5 | 0,9 | 5,0 | 0,9 | 2,3 | 0,9 | 2,0 |
| 0,95 | 2,5 | 0,95 | 1,7 | 0,95 | 3,8 | 0,95 | 3,5 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeZ- HFO-1243zf**

| **Organics 0,2 F1233xf + 0,2 F1234yf + 0,2 F245cb 0,2 F1234zeZ + 0,2 F1243zf** | | **Organics 0,96 F1233xf + 0,01 F1234yf + 0,01 F245cb + 0,01 F1234zeZ + 0,01 F1243zf** | | **Organics 0,01 F1233xf + 0,96 F1234yf + 0,01 F245cb + 0,01 F1234zeZ + 0,01 F1243zf** | | **Organics 0,01 F1233xf + 0,01 F1234yf + 0,96 F245cb + 0,01 F1234zeZ + 0,01 F1243zf** | | **Organics 0,01 F1233xf + 0,01 F1234yf + 0,01 F245cb + 0,96 F1234zeZ + 0,01 F1243zf** | | **Organics 0,01 F1233xf+0,01 F1234yf + 0,01 F245cb + 0,01 F1234zeZ + 0,96 F1243zf** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 1,7 | 0 | 6,7 | 0 | 4,6 | 0 | 1,9 | 0 | 5,8 |
| 0,05 | 5,3 | 0,05 | 2,8 | 0,05 | 7,6 | 0,05 | 5,8 | 0,05 | 3,0 | 0,05 | 6,7 |
| 0,1 | 5,3 | 0,1 | 2,8 | 0,1 | 7,7 | 0,1 | 5,8 | 0,1 | 3,0 | 0,1 | 6,8 |
| 0,15 | 5,3 | 0,15 | 2,8 | 0,15 | 7,6 | 0,15 | 5,8 | 0,15 | 3,0 | 0,15 | 6,7 |
| 0,2 | 5,3 | 0,2 | 2,8 | 0,2 | 7,6 | 0,2 | 5,8 | 0,2 | 3,0 | 0,2 | 6,7 |
| 0,25 | 5,3 | 0,25 | 2,8 | 0,25 | 7,6 | 0,25 | 5,8 | 0,25 | 3,0 | 0,25 | 6,7 |
| 0,3 | 5,3 | 0,3 | 2,8 | 0,3 | 7,6 | 0,3 | 5,8 | 0,3 | 3,0 | 0,3 | 6,7 |
| 0,35 | 5,3 | 0,35 | 2,8 | 0,35 | 7,6 | 0,35 | 5,8 | 0,35 | 3,0 | 0,35 | 6,7 |
| 0,4 | 5,3 | 0,4 | 2,8 | 0,4 | 7,6 | 0,4 | 5,8 | 0,4 | 3,0 | 0,4 | 6,7 |
| 0,45 | 5,3 | 0,45 | 2,8 | 0,45 | 7,6 | 0,45 | 5,8 | 0,45 | 3,0 | 0,45 | 6,7 |
| 0,5 | 5,3 | 0,5 | 2,8 | 0,5 | 7,6 | 0,5 | 5,8 | 0,5 | 3,0 | 0,5 | 6,7 |
| 0,55 | 5,3 | 0,55 | 2,8 | 0,55 | 7,6 | 0,55 | 5,8 | 0,55 | 3,0 | 0,55 | 6,7 |
| 0,6 | 5,3 | 0,6 | 2,8 | 0,6 | 7,6 | 0,6 | 5,8 | 0,6 | 3,0 | 0,6 | 6,7 |
| 0,65 | 5,3 | 0,65 | 2,8 | 0,65 | 7,6 | 0,65 | 5,8 | 0,65 | 3,0 | 0,65 | 6,6 |
| 0,7 | 5,3 | 0,7 | 2,8 | 0,7 | 7,6 | 0,7 | 5,8 | 0,7 | 3,0 | 0,7 | 6,5 |
| 0,75 | 5,3 | 0,75 | 2,8 | 0,75 | 7,5 | 0,75 | 5,8 | 0,75 | 3,0 | 0,75 | 6,4 |
| 0,8 | 5,1 | 0,8 | 2,7 | 0,8 | 7,2 | 0,8 | 5,8 | 0,8 | 2,9 | 0,8 | 6,1 |
| 0,85 | 4,7 | 0,85 | 2,5 | 0,85 | 6,6 | 0,85 | 5,8 | 0,85 | 2,7 | 0,85 | 5,6 |
| 0,9 | 4,0 | 0,9 | 2,2 | 0,9 | 5,5 | 0,9 | 5,0 | 0,9 | 2,4 | 0,9 | 4,7 |
| 0,95 | 2,9 | 0,95 | 1,8 | 0,95 | 3,8 | 0,95 | 3,5 | 0,95 | 1,9 | 0,95 | 3,4 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HCFO-1233zdE- HFO-1243zf**

| **Organics 0,2 F1233xf + 0,2 F1234yf + 0,2 F245cb 0,2 F1233zdE + 0,2 F1243zf** | | **Organics 0,96 F1233xf + 0,01 F1234yf + 0,01 F245cb + 0,01 F1233zdE + 0,01 F1243zf** | | **Organics 0,01 F1233xf + 0,96 F1234yf + 0,01 F245cb + 0,01 F1233zdE + 0,01 F1243zf** | | **Organics 0,01 F1233xf + 0,01 F1234yf + 0,96 F245cb + 0,01 F1233zdE + 0,01 F1243zf** | | **Organics 0,01 F1233xf + 0,01 F1234yf + 0,01 F245cb + 0,96 F1233zdE + 0,01 F1243zf** | | **Organics 0,01 F1233xf+0,01 F1234yf + 0,01 F245cb + 0,01 F1233zdE + 0,96 F1243zf** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 1,7 | 0 | 6,7 | 0 | 4,6 | 0 | 1,5 | 0 | 5,8 |
| 0,05 | 5,3 | 0,05 | 2,8 | 0,05 | 7,6 | 0,05 | 5,8 | 0,05 | 2,6 | 0,05 | 6,7 |
| 0,1 | 5,3 | 0,1 | 2,8 | 0,1 | 7,7 | 0,1 | 5,8 | 0,1 | 2,6 | 0,1 | 6,8 |
| 0,15 | 5,3 | 0,15 | 2,8 | 0,15 | 7,6 | 0,15 | 5,8 | 0,15 | 2,6 | 0,15 | 6,7 |
| 0,2 | 5,3 | 0,2 | 2,8 | 0,2 | 7,6 | 0,2 | 5,8 | 0,2 | 2,6 | 0,2 | 6,7 |
| 0,25 | 5,3 | 0,25 | 2,8 | 0,25 | 7,6 | 0,25 | 5,8 | 0,25 | 2,6 | 0,25 | 6,7 |
| 0,3 | 5,3 | 0,3 | 2,8 | 0,3 | 7,6 | 0,3 | 5,8 | 0,3 | 2,6 | 0,3 | 6,7 |
| 0,35 | 5,3 | 0,35 | 2,8 | 0,35 | 7,6 | 0,35 | 5,8 | 0,35 | 2,6 | 0,35 | 6,7 |
| 0,4 | 5,3 | 0,4 | 2,8 | 0,4 | 7,6 | 0,4 | 5,8 | 0,4 | 2,6 | 0,4 | 6,7 |
| 0,45 | 5,3 | 0,45 | 2,8 | 0,45 | 7,6 | 0,45 | 5,8 | 0,45 | 2,6 | 0,45 | 6,7 |
| 0,5 | 5,3 | 0,5 | 2,8 | 0,5 | 7,6 | 0,5 | 5,8 | 0,5 | 2,6 | 0,5 | 6,7 |
| 0,55 | 5,3 | 0,55 | 2,8 | 0,55 | 7,6 | 0,55 | 5,8 | 0,55 | 2,6 | 0,55 | 6,7 |
| 0,6 | 5,3 | 0,6 | 2,8 | 0,6 | 7,6 | 0,6 | 5,8 | 0,6 | 2,6 | 0,6 | 6,7 |
| 0,65 | 5,3 | 0,65 | 2,8 | 0,65 | 7,6 | 0,65 | 5,8 | 0,65 | 2,6 | 0,65 | 6,6 |
| 0,7 | 5,3 | 0,7 | 2,8 | 0,7 | 7,6 | 0,7 | 5,8 | 0,7 | 2,6 | 0,7 | 6,5 |
| 0,75 | 5,2 | 0,75 | 2,8 | 0,75 | 7,5 | 0,75 | 5,8 | 0,75 | 2,6 | 0,75 | 6,4 |
| 0,8 | 5,0 | 0,8 | 2,7 | 0,8 | 7,2 | 0,8 | 5,8 | 0,8 | 2,5 | 0,8 | 6,1 |
| 0,85 | 4,6 | 0,85 | 2,5 | 0,85 | 6,6 | 0,85 | 5,8 | 0,85 | 2,3 | 0,85 | 5,6 |
| 0,9 | 3,9 | 0,9 | 2,2 | 0,9 | 5,5 | 0,9 | 5,0 | 0,9 | 2,0 | 0,9 | 4,7 |
| 0,95 | 2,8 | 0,95 | 1,8 | 0,95 | 3,8 | 0,95 | 3,5 | 0,95 | 1,7 | 0,95 | 3,4 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFO-1234zeE - HFO-1234zeZ - HCFO-1233zdE**

| **Organics 0,2 F1233xf + 0,2 F1234yf + 0,2 F1233zdE 0,2 F1234zeE + 0,2 F1234zeZ** | | **Organics 0,96 F1233xf + 0,01 F1234yf + 0,01 F1233zdE + 0,01 F1234zeE + 0,01 F1234zeZ** | | **Organics 0,01 F1233xf+ 0,96 F1234yf + 0,01 F1233zdE + 0,01 F1234zeE + 0,01 F1234zeZ** | | **Organics 0,01 F1233xf + 0,01 F1234yf + 0,96 F1233zdE + 0,01 F1234zeE +0,01 F1234zeZ** | | **Organics 0,01 F1233xf + 0,01 F1234yf + 0,01 F1233zdE + 0,96 F1234zeE + 0,01 F1234zeZ** | | **Organics 0,01 F1233xf+0,01 F1234yf + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,4 | 0 | 1,6 | 0 | 6,6 | 0 | 1,4 | 0 | 4,8 | 0 | 1,9 |
| 0,05 | 4,4 | 0,05 | 2,8 | 0,05 | 7,6 | 0,05 | 2,5 | 0,05 | 5,8 | 0,05 | 3,0 |
| 0,1 | 4,4 | 0,1 | 2,8 | 0,1 | 7,6 | 0,1 | 2,5 | 0,1 | 5,8 | 0,1 | 3,0 |
| 0,15 | 4,4 | 0,15 | 2,8 | 0,15 | 7,6 | 0,15 | 2,5 | 0,15 | 5,8 | 0,15 | 3,0 |
| 0,2 | 4,4 | 0,2 | 2,8 | 0,2 | 7,6 | 0,2 | 2,5 | 0,2 | 5,8 | 0,2 | 3,0 |
| 0,25 | 4,4 | 0,25 | 2,8 | 0,25 | 7,6 | 0,25 | 2,5 | 0,25 | 5,8 | 0,25 | 3,0 |
| 0,3 | 4,4 | 0,3 | 2,8 | 0,3 | 7,6 | 0,3 | 2,5 | 0,3 | 5,8 | 0,3 | 3,0 |
| 0,35 | 4,4 | 0,35 | 2,8 | 0,35 | 7,6 | 0,35 | 2,5 | 0,35 | 5,8 | 0,35 | 3,0 |
| 0,4 | 4,4 | 0,4 | 2,8 | 0,4 | 7,6 | 0,4 | 2,5 | 0,4 | 5,8 | 0,4 | 3,0 |
| 0,45 | 4,4 | 0,45 | 2,7 | 0,45 | 7,6 | 0,45 | 2,5 | 0,45 | 5,7 | 0,45 | 3,0 |
| 0,5 | 4,4 | 0,5 | 2,7 | 0,5 | 7,6 | 0,5 | 2,5 | 0,5 | 5,7 | 0,5 | 3,0 |
| 0,55 | 4,4 | 0,55 | 2,7 | 0,55 | 7,6 | 0,55 | 2,5 | 0,55 | 5,7 | 0,55 | 3,0 |
| 0,6 | 4,4 | 0,6 | 2,7 | 0,6 | 7,6 | 0,6 | 2,5 | 0,6 | 5,6 | 0,6 | 3,0 |
| 0,65 | 4,4 | 0,65 | 2,7 | 0,65 | 7,6 | 0,65 | 2,5 | 0,65 | 5,5 | 0,65 | 3,0 |
| 0,7 | 4,4 | 0,7 | 2,7 | 0,7 | 7,6 | 0,7 | 2,5 | 0,7 | 5,3 | 0,7 | 3,0 |
| 0,75 | 4,2 | 0,75 | 2,7 | 0,75 | 7,4 | 0,75 | 2,5 | 0,75 | 5,1 | 0,75 | 3,0 |
| 0,8 | 4,0 | 0,8 | 2,6 | 0,8 | 7.1 | 0,8 | 2,4 | 0,8 | 4,8 | 0,8 | 2,8 |
| 0,85 | 3,6 | 0,85 | 2,4 | 0,85 | 6,5 | 0,85 | 2,2 | 0,85 | 4,3 | 0,85 | 2,6 |
| 0,9 | 3,1 | 0,9 | 2,1 | 0,9 | 5,5 | 0,9 | 2,0 | 0,9 | 3,6 | 0,9 | 2,3 |
| 0,95 | 2,3 | 0,95 | 1,7 | 0,95 | 3,8 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFO-1234zeE - HFO-1234zeZ - HFO-1243zf**

| **Organics 0,2 F1233xf + 0,2 F1234yf + 0,2 F1243zf 0,2 F1234zeE + 0,2 F1234zeZ** | | **Organics 0,96 F1233xf + 0,01 F1234yf + 0,01 F1243zf + 0,01 F1234zeE + 0,01 F1234zeZ** | | **Organics 0,01 F1233xf + 0,96 F1234yf + 0,01 F1243zf + 0,01 F1234zeE + 0,01 F1234zeZ** | | **Organics 0,01 F1233xf + 0,01 F1234yf + 0,96 F1243zf + 0,01 F1234zeE + 0,01 F1234zeZ** | | **Organics 0,01 F1233xf + 0,01 F1234yf + 0,01 F1243zf + 0,96 F1234zeE + 0,01 F1234zeZ** | | **Organics 0,01 F1233xf + 0,01 F1234yf + 0,01 F1243zf + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,3 | 0 | 1,7 | 0 | 6,7 | 0 | 5,8 | 0 | 4,8 | 0 | 1,9 |
| 0,05 | 5,3 | 0,05 | 2,8 | 0,05 | 7,6 | 0,05 | 6,7 | 0,05 | 5,8 | 0,05 | 3,0 |
| 0,1 | 5,3 | 0,1 | 2,8 | 0,1 | 7,6 | 0,1 | 6,7 | 0,1 | 5,8 | 0,1 | 3,0 |
| 0,15 | 5,3 | 0,15 | 2,8 | 0,15 | 7,6 | 0,15 | 6,7 | 0,15 | 5,8 | 0,15 | 3,0 |
| 0,2 | 5,3 | 0,2 | 2,8 | 0,2 | 7,6 | 0,2 | 6,7 | 0,2 | 5,8 | 0,2 | 3,0 |
| 0,25 | 5,3 | 0,25 | 2,8 | 0,25 | 7,6 | 0,25 | 6,7 | 0,25 | 5,8 | 0,25 | 3,0 |
| 0,3 | 5,3 | 0,3 | 2,8 | 0,3 | 7,6 | 0,3 | 6,7 | 0,3 | 5,8 | 0,3 | 3,0 |
| 0,35 | 5,3 | 0,35 | 2,8 | 0,35 | 7,6 | 0,35 | 6,7 | 0,35 | 5,8 | 0,35 | 3,0 |
| 0,4 | 5,3 | 0,4 | 2,8 | 0,4 | 7,6 | 0,4 | 6,7 | 0,4 | 5,8 | 0,4 | 3,0 |
| 0,45 | 5,3 | 0,45 | 2,8 | 0,45 | 7,6 | 0,45 | 6,7 | 0,45 | 5,8 | 0,45 | 3,0 |
| 0,5 | 5,3 | 0,5 | 2,8 | 0,5 | 7,6 | 0,5 | 6,7 | 0,5 | 5,8 | 0,5 | 3,0 |
| 0,55 | 5,3 | 0,55 | 2,8 | 0,55 | 7,6 | 0,55 | 6,7 | 0,55 | 5,7 | 0,55 | 3,0 |
| 0,6 | 5,3 | 0,6 | 2,8 | 0,6 | 7,6 | 0,6 | 6,7 | 0,6 | 5,6 | 0,6 | 3,0 |
| 0,65 | 5,3 | 0,65 | 2,8 | 0,65 | 7,6 | 0,65 | 6,6 | 0,65 | 5,5 | 0,65 | 3,0 |
| 0,7 | 5,2 | 0,7 | 2,8 | 0,7 | 7,6 | 0,7 | 6,5 | 0,7 | 5,4 | 0,7 | 3,0 |
| 0,75 | 5,0 | 0,75 | 2,8 | 0,75 | 7,5 | 0,75 | 6,3 | 0,75 | 5,1 | 0,75 | 3,0 |
| 0,8 | 4,8 | 0,8 | 2,7 | 0,8 | 7.1 | 0,8 | 6,1 | 0,8 | 4,8 | 0,8 | 2,9 |
| 0,85 | 4,3 | 0,85 | 2,5 | 0,85 | 6,5 | 0,85 | 5,6 | 0,85 | 4,3 | 0,85 | 2,7 |
| 0,9 | 3,7 | 0,9 | 2,2 | 0,9 | 5,5 | 0,9 | 4,7 | 0,9 | 3,6 | 0,9 | 2,4 |
| 0,95 | 2,7 | 0,95 | 1,8 | 0,95 | 3,8 | 0,95 | 3,4 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFO-1234zeE - HCFO-1233zdE - HFO-1243zf**

| **Organics 0,2 F1233xf + 0,2 F1234yf + 0,2 F1243zf + 0,2 F1234zeE + 0,2 F1233zdE** | | **Organics 0,96 F1233xf +0,01 F1234yf + 0,01 F1243zf + 0,01 F1234zeE+0,01 F1233zdE** | | **Organics 0,01 F1233xf + 0,96 F1234yf + 0,01 F1243zf + 0,01 F1234zeE + 0,01 F1233zdE** | | **Organics 0,01 F1233xf + 0,01 F1234yf + 0,96 F1243zf + 0,01 F1234zeE + 0,01 F1233zdE** | | **Organics 0,01 F1233xf+0,01 F1234yf+0,01 F1243zf + 0,96 F1234zeE + 0,01 F1233zdE** | | **Organics 0,01 F1233xf +0,01 F1234yf+0,01 F1243zf + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,3 | 0 | 1,7 | 0 | 6,7 | 0 | 5,8 | 0 | 4,8 | 0 | 1,5 |
| 0,05 | 5,3 | 0,05 | 2,8 | 0,05 | 7,6 | 0,05 | 6,7 | 0,05 | 5,8 | 0,05 | 2,6 |
| 0,1 | 5,3 | 0,1 | 2,8 | 0,1 | 7,6 | 0,1 | 6,7 | 0,1 | 5,8 | 0,1 | 2,6 |
| 0,15 | 5,3 | 0,15 | 2,8 | 0,15 | 7,6 | 0,15 | 6,7 | 0,15 | 5,8 | 0,15 | 2,6 |
| 0,2 | 5,3 | 0,2 | 2,8 | 0,2 | 7,6 | 0,2 | 6,7 | 0,2 | 5,8 | 0,2 | 2,6 |
| 0,25 | 5,3 | 0,25 | 2,8 | 0,25 | 7,6 | 0,25 | 6,7 | 0,25 | 5,8 | 0,25 | 2,6 |
| 0,3 | 5,3 | 0,3 | 2,8 | 0,3 | 7,6 | 0,3 | 6,7 | 0,3 | 5,8 | 0,3 | 2,6 |
| 0,35 | 5,3 | 0,35 | 2,8 | 0,35 | 7,6 | 0,35 | 6,7 | 0,35 | 5,8 | 0,35 | 2,6 |
| 0,4 | 5,3 | 0,4 | 2,8 | 0,4 | 7,6 | 0,4 | 6,7 | 0,4 | 5,8 | 0,4 | 2,6 |
| 0,45 | 5,3 | 0,45 | 2,8 | 0,45 | 7,6 | 0,45 | 6,7 | 0,45 | 5,8 | 0,45 | 2,6 |
| 0,5 | 5,3 | 0,5 | 2,8 | 0,5 | 7,6 | 0,5 | 6,7 | 0,5 | 5,8 | 0,5 | 2,6 |
| 0,55 | 5,3 | 0,55 | 2,8 | 0,55 | 7,6 | 0,55 | 6,7 | 0,55 | 5,7 | 0,55 | 2,6 |
| 0,6 | 5,3 | 0,6 | 2,8 | 0,6 | 7,6 | 0,6 | 6,7 | 0,6 | 5,6 | 0,6 | 2,6 |
| 0,65 | 5,2 | 0,65 | 2,8 | 0,65 | 7,6 | 0,65 | 6,6 | 0,65 | 5,5 | 0,65 | 2,6 |
| 0,7 | 5,1 | 0,7 | 2,8 | 0,7 | 7,6 | 0,7 | 6,5 | 0,7 | 5,4 | 0,7 | 2,6 |
| 0,75 | 5,0 | 0,75 | 2,8 | 0,75 | 7,5 | 0,75 | 6,3 | 0,75 | 5,1 | 0,75 | 2,6 |
| 0,8 | 4,7 | 0,8 | 2,6 | 0,8 | 7.1 | 0,8 | 6,1 | 0,8 | 4,8 | 0,8 | 2,4 |
| 0,85 | 4,3 | 0,85 | 2,5 | 0,85 | 6,5 | 0,85 | 5,6 | 0,85 | 4,3 | 0,85 | 2,3 |
| 0,9 | 3,6 | 0,9 | 2,2 | 0,9 | 5,5 | 0,9 | 4,7 | 0,9 | 3,6 | 0,9 | 2,0 |
| 0,95 | 2,6 | 0,95 | 1,8 | 0,95 | 3,8 | 0,95 | 3,4 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFO-1234zeZ - HCFO-1233zdE - HFO-1243zf**

| **Organics 0,2 F1233xf + 0,2 F1234yf + 0,2 F1243zf + 0,2 F1234zeZ + 0,2 F1233zdE** | | **Organics 0,96 F1233xf + 0,01 F1234yf + 0,01 F1243zf + 0,01 F1234zeZ + 0,01 F1233zdE** | | **Organics 0,01 F1233xf + 0,96 F1234yf + 0,01 F1243zf + 0,01 F1234zeZ + 0,01 F1233zdE** | | **Organics 0,01 F1233xf + 0,01 F1234yf + 0,96 F1243zf + 0,01 F1234zeZ + 0,01 F1233zdE** | | **Organics 0,01 F1233xf + 0,01 F1234yf + 0,01 F1243zf + 0,96 F1234zeZ + 0,01 F1233zdE** | | **Organics 0,01 F1233xf +0,01 F1234yf + 0,01 F1243zf + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,6 | 0 | 1,7 | 0 | 6,6 | 0 | 5,7 | 0 | 1,9 | 0 | 1,4 |
| 0,05 | 4,7 | 0,05 | 2,8 | 0,05 | 7,6 | 0,05 | 6,7 | 0,05 | 3,0 | 0,05 | 2,6 |
| 0,1 | 4,7 | 0,1 | 2,8 | 0,1 | 7,6 | 0,1 | 6,7 | 0,1 | 3,0 | 0,1 | 2,6 |
| 0,15 | 4,7 | 0,15 | 2,8 | 0,15 | 7,6 | 0,15 | 6,7 | 0,15 | 3,0 | 0,15 | 2,6 |
| 0,2 | 4,7 | 0,2 | 2,8 | 0,2 | 7,6 | 0,2 | 6,7 | 0,2 | 3,0 | 0,2 | 2,6 |
| 0,25 | 4,7 | 0,25 | 2,8 | 0,25 | 7,6 | 0,25 | 6,7 | 0,25 | 3,0 | 0,25 | 2,6 |
| 0,3 | 4,7 | 0,3 | 2,8 | 0,3 | 7,6 | 0,3 | 6,7 | 0,3 | 3,0 | 0,3 | 2,6 |
| 0,35 | 4,7 | 0,35 | 2,8 | 0,35 | 7,6 | 0,35 | 6,7 | 0,35 | 3,0 | 0,35 | 2,5 |
| 0,4 | 4,7 | 0,4 | 2,8 | 0,4 | 7,6 | 0,4 | 6,7 | 0,4 | 3,0 | 0,4 | 2,5 |
| 0,45 | 4,7 | 0,45 | 2,8 | 0,45 | 7,6 | 0,45 | 6,7 | 0,45 | 3,0 | 0,45 | 2,5 |
| 0,5 | 4,7 | 0,5 | 2,8 | 0,5 | 7,6 | 0,5 | 6,7 | 0,5 | 3,0 | 0,5 | 2,5 |
| 0,55 | 4,7 | 0,55 | 2,8 | 0,55 | 7,6 | 0,55 | 6,7 | 0,55 | 3,0 | 0,55 | 2,5 |
| 0,6 | 4,7 | 0,6 | 2,8 | 0,6 | 7,6 | 0,6 | 6,6 | 0,6 | 3,0 | 0,6 | 2,5 |
| 0,65 | 4,7 | 0,65 | 2,8 | 0,65 | 7,6 | 0,65 | 6,6 | 0,65 | 3,0 | 0,65 | 2,5 |
| 0,7 | 4,7 | 0,7 | 2,8 | 0,7 | 7,6 | 0,7 | 6,5 | 0,7 | 3,0 | 0,7 | 2,5 |
| 0,75 | 4,5 | 0,75 | 2,8 | 0,75 | 7,4 | 0,75 | 6,3 | 0,75 | 3,0 | 0,75 | 2,5 |
| 0,8 | 4,3 | 0,8 | 2,6 | 0,8 | 7,1 | 0,8 | 6,0 | 0,8 | 2,9 | 0,8 | 2,4 |
| 0,85 | 3,9 | 0,85 | 2,4 | 0,85 | 6,5 | 0,85 | 5,5 | 0,85 | 2,7 | 0,85 | 2,3 |
| 0,9 | 3,4 | 0,9 | 2,2 | 0,9 | 5,5 | 0,9 | 4,7 | 0,9 | 2,3 | 0,9 | 2,0 |
| 0,95 | 2,5 | 0,95 | 1,7 | 0,95 | 3,8 | 0,95 | 3,3 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HFO-1234zeZ - HCFO-1233zdE**

| **Organics 0,2 F1234yf + 0,2 F245cb + 0,2F1233zdE + 0,2 F1234zeE + 0,2 F1234zeZ** | | **Organics 0,96 F1234yf +0,01 F245cb +0,01 F1233zdE + 0,01 F1234zeE + 0,01 F1234zeZ** | | **Organics 0,01 F1234yf + 0,96 F245cb +0,01 F1233zdE + 0,01 F1234zeE +0,01 F1234zeZ** | | **Organics 0,01 F1234yf + 0,01 F245cb + 0,96 F1233zdE + 0,01 F1234zeE + 0,01 F1234zeZ** | | **Organics 0,01 F1234yf+0,01 F245cb + 0,01 F1233zdE + 0,96 F1234zeE + 0,01 F1234zeZ** | | **Organics 0,01 F1234yf +0,01 F245cb + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,9 | 0 | 6,7 | 0 | 4,6 | 0 | 1,5 | 0 | 4,8 | 0 | 1,9 |
| 0,05 | 5,0 | 0,05 | 7,6 | 0,05 | 5,8 | 0,05 | 2,6 | 0,05 | 5,8 | 0,05 | 3,0 |
| 0,1 | 5,0 | 0,1 | 7,6 | 0,1 | 5,8 | 0,1 | 2,6 | 0,1 | 5,8 | 0,1 | 3,0 |
| 0,15 | 5,0 | 0,15 | 7,6 | 0,15 | 5,8 | 0,15 | 2,6 | 0,15 | 5,8 | 0,15 | 3,0 |
| 0,2 | 5,0 | 0,2 | 7,6 | 0,2 | 5,8 | 0,2 | 2,6 | 0,2 | 5,8 | 0,2 | 3,0 |
| 0,25 | 5,0 | 0,25 | 7,6 | 0,25 | 5,8 | 0,25 | 2,6 | 0,25 | 5,8 | 0,25 | 3,0 |
| 0,3 | 5,0 | 0,3 | 7,6 | 0,3 | 5,8 | 0,3 | 2,6 | 0,3 | 5,8 | 0,3 | 3,0 |
| 0,35 | 5,0 | 0,35 | 7,6 | 0,35 | 5,8 | 0,35 | 2,6 | 0,35 | 5,8 | 0,35 | 3,0 |
| 0,4 | 5,0 | 0,4 | 7,6 | 0,4 | 5,8 | 0,4 | 2,6 | 0,4 | 5,8 | 0,4 | 3,0 |
| 0,45 | 5,0 | 0,45 | 7,6 | 0,45 | 5,8 | 0,45 | 2,6 | 0,45 | 5,8 | 0,45 | 3,0 |
| 0,5 | 5,0 | 0,5 | 7,6 | 0,5 | 5,8 | 0,5 | 2,6 | 0,5 | 5,7 | 0,5 | 3,0 |
| 0,55 | 5,0 | 0,55 | 7,6 | 0,55 | 5,8 | 0,55 | 2,6 | 0,55 | 5,7 | 0,55 | 3,0 |
| 0,6 | 5,0 | 0,6 | 7,6 | 0,6 | 5,8 | 0,6 | 2,6 | 0,6 | 5,6 | 0,6 | 3,0 |
| 0,65 | 5,0 | 0,65 | 7,6 | 0,65 | 5,8 | 0,65 | 2,6 | 0,65 | 5,5 | 0,65 | 3,0 |
| 0,7 | 5,1 | 0,7 | 7,6 | 0,7 | 5,8 | 0,7 | 2,6 | 0,7 | 5,4 | 0,7 | 3,0 |
| 0,75 | 5,0 | 0,75 | 7,5 | 0,75 | 5,8 | 0,75 | 2,6 | 0,75 | 5,1 | 0,75 | 3,0 |
| 0,8 | 4,7 | 0,8 | 7,1 | 0,8 | 5,8 | 0,8 | 2,5 | 0,8 | 4,8 | 0,8 | 2,9 |
| 0,85 | 4,3 | 0,85 | 6,5 | 0,85 | 5,8 | 0,85 | 2,3 | 0,85 | 4,3 | 0,85 | 2,7 |
| 0,9 | 3,7 | 0,9 | 5,5 | 0,9 | 5,0 | 0,9 | 2,0 | 0,9 | 3,6 | 0,9 | 2,4 |
| 0,95 | 2,7 | 0,95 | 3,8 | 0,95 | 3,5 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HFO-1234zeZ - HFO-1243zf**

| **Organics 0,2 F1234yf + 0,2 F245cb + 0,2 F1243zf + 0,2 F1234zeE + 0,2 F1234zeZ** | | **Organics 0,96 F1234yf +0,01 F245cb +0,01 F1243zf + 0,01 F1234zeE + 0,01 F1234zeZ** | | **Organics 0,01 F1234yf + 0,96 F245cb + 0,01 F1243zf + 0,01 F1234zeE + 0,01 F1234zeZ** | | **Organics 0,01 F1234yf + 0,01 F245cb + 0,96 F1243zf + 0,01 F1234zeE + 0,01 F1234zeZ** | | **Organics 0,01 F1234yf + 0,01 F245cb + 0,01 F1243zf + 0,96 F1234zeE + 0,01 F1234zeZ** | | **Organics 0,01 F1234yf +0,01 F245cb + 0,01 F1243zf + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | | | | | | | | | | | |
| | bar | | bar | | bar | | bar | | bar | | bar |
| 0 | 4,8 | 0 | 6,7 | 0 | 4,7 | 0 | 5,8 | 0 | 4,9 | 0 | 2,0 |
| 0,05 | 5,9 | 0,05 | 7,7 | 0,05 | 5,8 | 0,05 | 6,7 | 0,05 | 5,8 | 0,05 | 3,1 |
| 0,1 | 5,9 | 0,1 | 7,7 | 0,1 | 5,8 | 0,1 | 6,8 | 0,1 | 5,9 | 0,1 | 3,1 |
| 0,15 | 5,9 | 0,15 | 7,7 | 0,15 | 5,8 | 0,15 | 6,8 | 0,15 | 5,8 | 0,15 | 3,1 |
| 0,2 | 5,9 | 0,2 | 7,7 | 0,2 | 5,8 | 0,2 | 6,8 | 0,2 | 5,8 | 0,2 | 3,1 |
| 0,25 | 5,9 | 0,25 | 7,6 | 0,25 | 5,8 | 0,25 | 6,8 | 0,25 | 5,8 | 0,25 | 3,1 |
| 0,3 | 5,9 | 0,3 | 7,6 | 0,3 | 5,8 | 0,3 | 6,8 | 0,3 | 5,8 | 0,3 | 3,1 |
| 0,35 | 5,9 | 0,35 | 7,6 | 0,35 | 5,8 | 0,35 | 6,8 | 0,35 | 5,8 | 0,35 | 3,1 |
| 0,4 | 5,9 | 0,4 | 7,6 | 0,4 | 5,8 | 0,4 | 6,7 | 0,4 | 5,8 | 0,4 | 3,1 |
| 0,45 | 5,9 | 0,45 | 7,6 | 0,45 | 5,8 | 0,45 | 6,7 | 0,45 | 5,8 | 0,45 | 3,1 |
| 0,5 | 5,9 | 0,5 | 7,7 | 0,5 | 5,8 | 0,5 | 6,7 | 0,5 | 5,8 | 0,5 | 3,1 |
| 0,55 | 5,9 | 0,55 | 7,7 | 0,55 | 5,8 | 0,55 | 6,7 | 0,55 | 5,7 | 0,55 | 3,1 |
| 0,6 | 5,9 | 0,6 | 7,7 | 0,6 | 5,8 | 0,6 | 6,7 | 0,6 | 5,7 | 0,6 | 3,1 |
| 0,65 | 5,9 | 0,65 | 7,7 | 0,65 | 5,8 | 0,65 | 6,6 | 0,65 | 5,6 | 0,65 | 3,1 |
| 0,7 | 5,9 | 0,7 | 7,6 | 0,7 | 5,8 | 0,7 | 6,5 | 0,7 | 5,4 | 0,7 | 3,1 |
| 0,75 | 5,7 | 0,75 | 7,5 | 0,75 | 5,8 | 0,75 | 6,4 | 0,75 | 5,2 | 0,75 | 3,1 |
| 0,8 | 5,5 | 0,8 | 7,2 | 0,8 | 5,8 | 0,8 | 6,1 | 0,8 | 4,8 | 0,8 | 2,9 |
| 0,85 | 5,0 | 0,85 | 6,6 | 0,85 | 5,8 | 0,85 | 5,6 | 0,85 | 4,3 | 0,85 | 2,7 |
| 0,9 | 4,3 | 0,9 | 5,5 | 0,9 | 5,0 | 0,9 | 4,8 | 0,9 | 3,6 | 0,9 | 2,4 |
| 0,95 | 3,0 | 0,95 | 3,8 | 0,95 | 3,5 | 0,95 | 3,4 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HCFO-1233zdE- HFO-1243zf**

| **Organics 0,2 F1234yf + 0,2 F245cb + 0,2 F1243zf + 0,2 F1234zeE + 0,2 F1233zdE** | | **Organics 0,96 F1234yf +0,01 F245cb +0,01 F1243zf + 0,01 F1234zeE + 0,01 F1233zdE** | | **Organics 0,01 F1234yf + 0,96 F245cb + 0,01 F1243zf + 0,01 F1234zeE + 0,01 F1233zdE** | | **Organics 0,01 F1234yf + 0,01 F245cb + 0,96 F1243zf + 0,01 F1234zeE + 0,01 F1233zdE** | | **Organics 0,01 F1234yf + 0,01 F245cb + 0,01 F1243zf + 0,96 F1234zeE + 0,01 F1233zdE** | | **Organics 0,01 F1234yf + 0,01 F245cb + 0,01 F1243zf + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,8 | 0 | 6,7 | 0 | 4,7 | 0 | 5,8 | 0 | 4,9 | 0 | 1,5 |
| 0,05 | 5,9 | 0,05 | 7,7 | 0,05 | 5,8 | 0,05 | 6,7 | 0,05 | 5,8 | 0,05 | 2,6 |
| 0,1 | 5,9 | 0,1 | 7,7 | 0,1 | 5,8 | 0,1 | 6,8 | 0,1 | 5,8 | 0,1 | 2,6 |
| 0,15 | 5,9 | 0,15 | 7,7 | 0,15 | 5,8 | 0,15 | 6,8 | 0,15 | 5,8 | 0,15 | 2,6 |
| 0,2 | 5,9 | 0,2 | 7,7 | 0,2 | 5,8 | 0,2 | 6,8 | 0,2 | 5,8 | 0,2 | 2,6 |
| 0,25 | 5,9 | 0,25 | 7,6 | 0,25 | 5,8 | 0,25 | 6,8 | 0,25 | 5,8 | 0,25 | 2,6 |
| 0,3 | 5,9 | 0,3 | 7,6 | 0,3 | 5,8 | 0,3 | 6,8 | 0,3 | 5,8 | 0,3 | 2,6 |
| 0,35 | 5,9 | 0,35 | 7,6 | 0,35 | 5,8 | 0,35 | 6,8 | 0,35 | 5,8 | 0,35 | 2,6 |
| 0,4 | 5,9 | 0,4 | 7,7 | 0,4 | 5,8 | 0,4 | 6,7 | 0,4 | 5,8 | 0,4 | 2,6 |
| 0,45 | 5,9 | 0,45 | 7,7 | 0,45 | 5,8 | 0,45 | 6,7 | 0,45 | 5,8 | 0,45 | 2,6 |
| 0,5 | 5,9 | 0,5 | 7,7 | 0,5 | 5,8 | 0,5 | 6,7 | 0,5 | 5,8 | 0,5 | 2,6 |
| 0,55 | 5,9 | 0,55 | 7,7 | 0,55 | 5,8 | 0,55 | 6,7 | 0,55 | 5,7 | 0,55 | 2,6 |
| 0,6 | 5,9 | 0,6 | 7,7 | 0,6 | 5,8 | 0,6 | 6,7 | 0,6 | 5,7 | 0,6 | 2,6 |
| 0,65 | 5,9 | 0,65 | 7,7 | 0,65 | 5,8 | 0,65 | 6,6 | 0,65 | 5,6 | 0,65 | 2,6 |
| 0,7 | 5,9 | 0,7 | 7,6 | 0,7 | 5,8 | 0,7 | 6,5 | 0,7 | 5,4 | 0,7 | 2,6 |
| 0,75 | 5,7 | 0,75 | 7,5 | 0,75 | 5,8 | 0,75 | 6,4 | 0,75 | 5,2 | 0,75 | 2,6 |
| 0,8 | 5,4 | 0,8 | 7,2 | 0,8 | 5,8 | 0,8 | 6,1 | 0,8 | 4,8 | 0,8 | 2,5 |
| 0,85 | 5,0 | 0,85 | 6,6 | 0,85 | 5,8 | 0,85 | 5,6 | 0,85 | 4,3 | 0,85 | 2,3 |
| 0,9 | 4,2 | 0,9 | 5,5 | 0,9 | 5,0 | 0,9 | 4,8 | 0,9 | 3,6 | 0,9 | 2,1 |
| 0,95 | 3,0 | 0,95 | 3,8 | 0,95 | 3,5 | 0,95 | 3,4 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeZ - HCFO-1233zdE- HFO-1243zf**

| **Organics 0,2 F1234yf + 0,2 F245cb + 0,2 F1243zf + 0,2 F1234zeZ + 0,2 F1233zdE** | | **Organics 0,96 F1234yf + 0,01 F245cb + 0,01 F1243zf + 0,01 F1234zeZ + 0,01 F1233zdE** | | **Organics 0,01 F1234yf + 0,96 F245cb + 0,01 F1243zf + 0,01 F1234zeZ + 0,01 F1233zdE** | | **Organics 0,01 F1234yf + 0,01 F245cb + 0,96 F1243zf + 0,01 F1234zeZ + 0,01 F1233zdE** | | **Organics 0,01 F1234yf + 0,01 F245cb + 0,01 F1243zf + 0,96 F1234zeZ + 0,01 F1233zdE** | | **Organics 0,01 F1234yf + 0,01 F245cb + 0,01 F1243zf + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 6,7 | 0 | 4,6 | 0 | 5,8 | 0 | 1,9 | 0 | 1,5 |
| 0,05 | 5,3 | 0,05 | 7,6 | 0,05 | 5,8 | 0,05 | 6,7 | 0,05 | 3,0 | 0,05 | 2,6 |
| 0,1 | 5,3 | 0,1 | 7,6 | 0,1 | 5,8 | 0,1 | 6,7 | 0,1 | 3,0 | 0,1 | 2,6 |
| 0,15 | 5,3 | 0,15 | 7,6 | 0,15 | 5,8 | 0,15 | 6,7 | 0,15 | 3,0 | 0,15 | 2,6 |
| 0,2 | 5,3 | 0,2 | 7,6 | 0,2 | 5,8 | 0,2 | 6,7 | 0,2 | 3,0 | 0,2 | 2,6 |
| 0,25 | 5,3 | 0,25 | 7,6 | 0,25 | 5,8 | 0,25 | 6,7 | 0,25 | 3,0 | 0,25 | 2,6 |
| 0,3 | 5,3 | 0,3 | 7,6 | 0,3 | 5,8 | 0,3 | 6,7 | 0,3 | 3,0 | 0,3 | 2,6 |
| 0,35 | 5,3 | 0,35 | 7,6 | 0,35 | 5,8 | 0,35 | 6,7 | 0,35 | 3,0 | 0,35 | 2,6 |
| 0,4 | 5,3 | 0,4 | 7,6 | 0,4 | 5,8 | 0,4 | 6,7 | 0,4 | 3,0 | 0,4 | 2,6 |
| 0,45 | 5,3 | 0,45 | 7,6 | 0,45 | 5,8 | 0,45 | 6,7 | 0,45 | 3,0 | 0,45 | 2,6 |
| 0,5 | 5,3 | 0,5 | 7,6 | 0,5 | 5,8 | 0,5 | 6,7 | 0,5 | 3,0 | 0,5 | 2,6 |
| 0,55 | 5,3 | 0,55 | 7,6 | 0,55 | 5,8 | 0,55 | 6,7 | 0,55 | 3,0 | 0,55 | 2,6 |
| 0,6 | 5,3 | 0,6 | 7,6 | 0,6 | 5,8 | 0,6 | 6,7 | 0,6 | 3,0 | 0,6 | 2,6 |
| 0,65 | 5,3 | 0,65 | 7,6 | 0,65 | 5,8 | 0,65 | 6,6 | 0,65 | 3,0 | 0,65 | 2,6 |
| 0,7 | 5,3 | 0,7 | 7,6 | 0,7 | 5,8 | 0,7 | 6,5 | 0,7 | 3,0 | 0,7 | 2,6 |
| 0,75 | 5,2 | 0,75 | 7,5 | 0,75 | 5,8 | 0,75 | 6,4 | 0,75 | 3,0 | 0,75 | 2,6 |
| 0,8 | 5,0 | 0,8 | 7,2 | 0,8 | 5,8 | 0,8 | 6,1 | 0,8 | 2,9 | 0,8 | 2,5 |
| 0,85 | 4,6 | 0,85 | 6,6 | 0,85 | 5,8 | 0,85 | 5,6 | 0,85 | 2,7 | 0,85 | 2,3 |
| 0,9 | 3,9 | 0,9 | 5,5 | 0,9 | 5,0 | 0,9 | 4,7 | 0,9 | 2,4 | 0,9 | 2,0 |
| 0,95 | 2,9 | 0,95 | 3,8 | 0,95 | 3,5 | 0,95 | 3,4 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HFO-1234yf- HFO-1234zeE - HFO-1234zeZ - HCFO-1233zdE- HFO-1243zf**

| **Organics 0,2 F1234yf + 0,2 F1243zf + 0,2 F1233zdE 0,2 F1234zeE +0,2 F1234zeZ** | | **Organics 0,96 F1234yf + 0,01 F1243zf + 0,01 F1233zdE + 0,01 F1234zeE + 0,01 F1234zeZ** | | **Organics 0,01 F1234yf + 0,96 F1243zf + 0,01 F1233zdE + 0,01 F1234zeE + 0,01 F1234zeZ** | | **Organics 0,01 F1234yf + 0,01 F1243zf + 0,96 F1233zdE + 0,01 F1234zeE + 0,01 F1234zeZ** | | **Organics 0,01 F1234yf + 0,01 F1243zf + 0,01 F1233zdE + 0,96 F1234zeE + 0,01 F1234zeZ** | | **Organics 0,01 F1234yf + 0,01 F1243zf + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 6,7 | 0 | 5,8 | 0 | 1,5 | 0 | 4,8 | 0 | 1,9 |
| 0,05 | 5,3 | 0,05 | 7,6 | 0,05 | 6,7 | 0,05 | 2,6 | 0,05 | 5,8 | 0,05 | 3,0 |
| 0,1 | 5,3 | 0,1 | 7,6 | 0,1 | 6,7 | 0,1 | 2,6 | 0,1 | 5,8 | 0,1 | 3,0 |
| 0,15 | 5,3 | 0,15 | 7,6 | 0,15 | 6,7 | 0,15 | 2,6 | 0,15 | 5,8 | 0,15 | 3,0 |
| 0,2 | 5,3 | 0,2 | 7,6 | 0,2 | 6,7 | 0,2 | 2,6 | 0,2 | 5,8 | 0,2 | 3,0 |
| 0,25 | 5,3 | 0,25 | 7,6 | 0,25 | 6,7 | 0,25 | 2,6 | 0,25 | 5,8 | 0,25 | 3,0 |
| 0,3 | 5,3 | 0,3 | 7,6 | 0,3 | 6,7 | 0,3 | 2,6 | 0,3 | 5,8 | 0,3 | 3,0 |
| 0,35 | 5,3 | 0,35 | 7,6 | 0,35 | 6,7 | 0,35 | 2,6 | 0,35 | 5,8 | 0,35 | 3,0 |
| 0,4 | 5,3 | 0,4 | 7,6 | 0,4 | 6,7 | 0,4 | 2,6 | 0,4 | 5,8 | 0,4 | 3,0 |
| 0,45 | 5,3 | 0,45 | 7,6 | 0,45 | 6,7 | 0,45 | 2,6 | 0,45 | 5,8 | 0,45 | 3,0 |
| 0,5 | 5,3 | 0,5 | 7,6 | 0,5 | 6,7 | 0,5 | 2,6 | 0,5 | 5,8 | 0,5 | 3,0 |
| 0,55 | 5,3 | 0,55 | 7,6 | 0,55 | 6,7 | 0,55 | 2,6 | 0,55 | 5,7 | 0,55 | 3,0 |
| 0,6 | 5,2 | 0,6 | 7,6 | 0,6 | 6,7 | 0,6 | 2,6 | 0,6 | 5,6 | 0,6 | 3,0 |
| 0,65 | 5,2 | 0,65 | 7,6 | 0,65 | 6,6 | 0,65 | 2,6 | 0,65 | 5,5 | 0,65 | 3,0 |
| 0,7 | 5,2 | 0,7 | 7,6 | 0,7 | 6,5 | 0,7 | 2,6 | 0,7 | 5,4 | 0,7 | 3,0 |
| 0,75 | 5,0 | 0,75 | 7,5 | 0,75 | 6,3 | 0,75 | 2,6 | 0,75 | 5,1 | 0,75 | 3,0 |
| 0,8 | 4,7 | 0,8 | 7,1 | 0,8 | 6,1 | 0,8 | 2,5 | 0,8 | 4,8 | 0,8 | 2,9 |
| 0,85 | 4,3 | 0,85 | 6,5 | 0,85 | 5,6 | 0,85 | 2,3 | 0,85 | 4,3 | 0,85 | 2,7 |
| 0,9 | 3,7 | 0,9 | 5,5 | 0,9 | 4,7 | 0,9 | 2,0 | 0,9 | 3,6 | 0,9 | 2,4 |
| 0,95 | 2,7 | 0,95 | 3,8 | 0,95 | 3,4 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFC-244bb- HFC-245fa - Trifluoropropyne- HFO-1225yeZ- HFO-1225zc**

| | Organics 0,2 F244bb + 0,2 F245fa + 0,2 TFP + 0,2 F1225yeZ + 0,2 F1225zc | Organics 0,96 F244bb + 0,01 F245fa + 0,01 TFP + 0,01 F1225yeZ + 0,01 F1225zc | Organics 0,01 F244bb + 0,96 F245fa + 0,01 TFP + 0,01 F1225yeZ + 0,01 F1225zc | Organics 0,01 F244bb + 0,01 F245fa + 0,96 TFP + 0,01 F1225yeZ + 0,01 F1225zc | Organics 0,01 F244bb + 0,01 F245fa + 0,01 TFP + 0,96 F1225yeZ + 0,01 F1225zc | Organics 0,01 F244bb + 0,01 F245fa + 0,01 TFP + 0,01 F1225yeZ + 0,96 F1225zc |
|---|---|---|---|---|---|---|
| MASSFRAC | TOTAL | TOTAL | TOTAL | TOTAL | TOTAL | TOTAL |
| HF | PRESSURE | PRES | PRES | PRES | PRES | PRES |
| | bar | bar | bar | bar | bar | bar |
| 0 | 5,4 | 0,9 | 1,7 | 11,4 | 5,2 | 5,3 |
| 0,05 | 6,4 | 2,1 | 2,8 | 12,1 | 6,2 | 6,3 |
| 0,1 | 6,4 | 2,1 | 2,8 | 12,1 | 6,2 | 6,3 |
| 0,15 | 6,4 | 2,1 | 2,8 | 12,0 | 6,2 | 6,3 |
| 0,2 | 6,4 | 2,1 | 2,8 | 11,8 | 6,2 | 6,3 |
| 0,25 | 6,4 | 2,1 | 2,8 | 11,7 | 6,2 | 6,3 |
| 0,3 | 6,4 | 2,1 | 2,8 | 11,7 | 6,2 | 6,3 |
| 0,35 | 6,3 | 2,0 | 2,8 | 11,6 | 6,2 | 6,3 |
| 0,4 | 6,3 | 2,0 | 2,8 | 11,5 | 6,2 | 6,3 |
| 0,45 | 6,3 | 2,0 | 2,8 | 11,5 | 6,2 | 6,3 |
| 0,5 | 6,3 | 2,0 | 2,8 | 11,6 | 6,2 | 6,2 |
| 0,55 | 6,2 | 2,0 | 2,8 | 11,6 | 6,2 | 6,2 |
| 0,6 | 6,2 | 2,0 | 2,8 | 11,6 | 6,2 | 6,1 |
| 0,65 | 6,1 | 2,0 | 2,8 | 11,6 | 6,2 | 6,0 |
| 0,7 | 6,1 | 2,0 | 2,8 | 11,6 | 6,1 | 5,9 |
| 0,75 | 5,9 | 2,0 | 2,8 | 11,6 | 5,9 | 5,6 |
| 0,8 | 5,6 | 2,0 | 2,7 | 11,4 | 5,6 | 5,2 |
| 0,85 | 5,1 | 1,9 | 2,5 | 10,6 | 5,1 | 4,7 |
| 0,9 | 4,3 | 1,8 | 2,2 | 8,9 | 4,2 | 3,8 |
| 0,95 | 3,0 | 1,6 | 1,8 | 6,0 | 3,0 | 2,7 |
| 1 | 1,2 | 1,2 | 1,2 | 1,2 | 1,2 | 1,2 |

### Exemple 5 : Mélanges à sept composés

**HF - HCFO-1233xf - HFO-1234yf - HFC-245cb - HCFO-1233zdE - HFO-1234zeE - HFO-1234zeZ**

| **Organics 0,15 F1233xf + 0,17 F1234yf + 0,17 F245cb + 0,17 F1233zdE + 0,17 F1234zeE + 0,17 F1234zeZ** | | **Organics 0,01 F1233xf + 0,95 F1234yf + 0,01 F245cb + 0,01 F1233zdE + 0,01 F1234zeE + 0,01 F1234zeZ** | | **Organics 0,01 F1233xf + 0,01 F1234yf + 0,95 F245cb + 0,01 F1233zdE + 0,01 F1234zeE + 0,01 F1234zeZ** | | **Organics 0,01 F1233xf + 0,01 F1234yf + 0,01 F245cb + 0,95 F1233zdE + 0,01 F1234zeE + 0,01 F1234zeZ** | | **Organics 0,01 F1233xf + 0,01 F1234yf + 0,01 F245cb + 0,01 F1233zdE + 0,95 F1234zeE + 0,01 F1234zeZ** | | **Organics 0,01 F1233xf + 0,01 F1234yf + 0,01 F245cb + 0,01 F1233zdE + 0,01 F1234zeE + 0,95 F1234zeZ** | | **Organics 0,95 F1233xf + 0,01 F1234yf + 0,01 F245cb + 0,01 F1233zdE + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | | | | | | | | | | | | | |
| | bar | | bar | | bar | | bar | | bar | | bar | | bar |
| 0 | 3,6 | 0 | 6,6 | 0 | 4,6 | 0 | 1,5 | 0 | 4,8 | 0 | 1,9 | 0 | 1,7 |
| 0,05 | 4,7 | 0,05 | 7,6 | 0,05 | 5,8 | 0,05 | 2,6 | 0,05 | 5,8 | 0,05 | 3,0 | 0,05 | 2,8 |
| 0,1 | 4,7 | 0,1 | 7,6 | 0,1 | 5,8 | 0,1 | 2,6 | 0,1 | 5,8 | 0,1 | 3,0 | 0,1 | 2,8 |
| 0,15 | 4,7 | 0,15 | 7,6 | 0,15 | 5,8 | 0,15 | 2,6 | 0,15 | 5,8 | 0,15 | 3,0 | 0,15 | 2,8 |
| 0,2 | 4,7 | 0,2 | 7,6 | 0,2 | 5,8 | 0,2 | 2,6 | 0,2 | 5,8 | 0,2 | 3,0 | 0,2 | 2,8 |
| 0,25 | 4,7 | 0,25 | 7,6 | 0,25 | 5,8 | 0,25 | 2,6 | 0,25 | 5,8 | 0,25 | 3,0 | 0,25 | 2,8 |
| 0,3 | 4,7 | 0,3 | 7,6 | 0,3 | 5,8 | 0,3 | 2,6 | 0,3 | 5,8 | 0,3 | 3,0 | 0,3 | 2,8 |
| 0,35 | 4,7 | 0,35 | 7,6 | 0,35 | 5,8 | 0,35 | 2,6 | 0,35 | 5,8 | 0,35 | 3,0 | 0,35 | 2,8 |
| 0,4 | 4,7 | 0,4 | 7,6 | 0,4 | 5,8 | 0,4 | 2,6 | 0,4 | 5,8 | 0,4 | 3,0 | 0,4 | 2,8 |
| 0,45 | 4,7 | 0,45 | 7,6 | 0,45 | 5,8 | 0,45 | 2,6 | 0,45 | 5,7 | 0,45 | 3,0 | 0,45 | 2,8 |
| 0,5 | 4,7 | 0,5 | 7,6 | 0,5 | 5,8 | 0,5 | 2,6 | 0,5 | 5,7 | 0,5 | 3,0 | 0,5 | 2,8 |
| 0,55 | 4,7 | 0,55 | 7,6 | 0,55 | 5,8 | 0,55 | 2,6 | 0,55 | 5,7 | 0,55 | 3,0 | 0,55 | 2,8 |
| 0,6 | 4,7 | 0,6 | 7,6 | 0,6 | 5,8 | 0,6 | 2,6 | 0,6 | 5,6 | 0,6 | 3,0 | 0,6 | 2,8 |
| 0,65 | 4,7 | 0,65 | 7,6 | 0,65 | 5,8 | 0,65 | 2,6 | 0,65 | 5,5 | 0,65 | 3,0 | 0,65 | 2,8 |
| 0,7 | 4,7 | 0,7 | 7,6 | 0,7 | 5,8 | 0,7 | 2,6 | 0,7 | 5,3 | 0,7 | 3,0 | 0,7 | 2,8 |
| 0,75 | 4,6 | 0,75 | 7,4 | 0,75 | 5,8 | 0,75 | 2,6 | 0,75 | 5,1 | 0,75 | 3,0 | 0,75 | 2,8 |
| 0,8 | 4,4 | 0,8 | 7,1 | 0,8 | 5,8 | 0,8 | 2,5 | 0,8 | 4,8 | 0,8 | 2,9 | 0,8 | 2,6 |
| 0,85 | 4,1 | 0,85 | 6,5 | 0,85 | 5,8 | 0,85 | 2,3 | 0,85 | 4,3 | 0,85 | 2,7 | 0,85 | 2,5 |
| 0,9 | 3,5 | 0,9 | 5,5 | 0,9 | 5,0 | 0,9 | 2,0 | 0,9 | 3,6 | 0,9 | 2,4 | 0,9 | 2,2 |
| 0,95 | 2,5 | 0,95 | 3,8 | 0,95 | 3,5 | 0,95 | 1,7 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HCFO-1233zdE - HFO-1234zeE - HFO-1243zf**

| **Organics 0,15 F1233xf + 0,17 F1234yf + 0,17 F245cb + 0,17 F1233zdE + 0,17 F1234zeE + 0,17 F1243zf** | | **Organics 0,01 F1233xf + 0,95 F1234yf + 0,01 F245cb + 0,01 F1233zdE + 0,01 F1234zeE + 0,01 F1243zf** | | **Organics 0,01 F1233xf + 0,01 F1234yf + 0,95 F245cb + 0,01 F1233zdE + 0,01 F1234zeE + 0,01 F1243zf** | | **Organics 0,01 F1233xf + 0,01 F1234yf + 0,01 F245cb + 0,95 F1233zdE + 0,01 F1234zeE + 0,01 F1243zf** | | **Organics 0,01 F1233xf + 0,01 F1234yf + 0,01 F245cb + 0,01 F1233zdE + 0,95 F1234zeE + 0,01 F1243zf** | | **Organics 0,01 F1233xf + 0,01 F1234yf + 0,01 F245cb + 0,01 F1233zdE + 0,01 F1234zeE + 0,95 F1243zf** | | **Organics 0,95 F1233xf + 0,01 F1234yf + 0,01 F245cb + 0,01 F1233zdE + 0,01 F1234zeE + 0,01 F1243zf** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,4 | 0 | 6,6 | 0 | 4,6 | 0 | 1,5 | 0 | 4,8 | 0 | 5,8 | 0 | 1,7 |
| 0,05 | 5,4 | 0,05 | 7,6 | 0,05 | 5,8 | 0,05 | 2,6 | 0,05 | 5,8 | 0,05 | 6,7 | 0,05 | 2,8 |
| 0,1 | 5,4 | 0,1 | 7,6 | 0,1 | 5,8 | 0,1 | 2,6 | 0,1 | 5,8 | 0,1 | 6,7 | 0,1 | 2,8 |
| 0,15 | 5,4 | 0,15 | 7,6 | 0,15 | 5,8 | 0,15 | 2,6 | 0,15 | 5,8 | 0,15 | 6,7 | 0,15 | 2,8 |
| 0,2 | 5,4 | 0,2 | 7,6 | 0,2 | 5,8 | 0,2 | 2,6 | 0,2 | 5,8 | 0,2 | 6,7 | 0,2 | 2,8 |
| 0,25 | 5,4 | 0,25 | 7,6 | 0,25 | 5,8 | 0,25 | 2,6 | 0,25 | 5,8 | 0,25 | 6,7 | 0,25 | 2,8 |
| 0,3 | 5,4 | 0,3 | 7,6 | 0,3 | 5,8 | 0,3 | 2,6 | 0,3 | 5,8 | 0,3 | 6,7 | 0,3 | 2,8 |
| 0,35 | 5,4 | 0,35 | 7,6 | 0,35 | 5,8 | 0,35 | 2,6 | 0,35 | 5,8 | 0,35 | 6,7 | 0,35 | 2,8 |
| 0,4 | 5,4 | 0,4 | 7,6 | 0,4 | 5,8 | 0,4 | 2,6 | 0,4 | 5,8 | 0,4 | 6,7 | 0,4 | 2,8 |
| 0,45 | 5,4 | 0,45 | 7,6 | 0,45 | 5,8 | 0,45 | 2,6 | 0,45 | 5,8 | 0,45 | 6,7 | 0,45 | 2,8 |
| 0,5 | 5,4 | 0,5 | 7,6 | 0,5 | 5,8 | 0,5 | 2,6 | 0,5 | 5,8 | 0,5 | 6,7 | 0,5 | 2,8 |
| 0,55 | 5,4 | 0,55 | 7,6 | 0,55 | 5,8 | 0,55 | 2,6 | 0,55 | 5,7 | 0,55 | 6,7 | 0,55 | 2,8 |
| 0,6 | 5,4 | 0,6 | 7,6 | 0,6 | 5,8 | 0,6 | 2,6 | 0,6 | 5,6 | 0,6 | 6,7 | 0,6 | 2,8 |
| 0,65 | 5,4 | 0,65 | 7,6 | 0,65 | 5,8 | 0,65 | 2,6 | 0,65 | 5,5 | 0,65 | 6,6 | 0,65 | 2,8 |
| 0,7 | 5,4 | 0,7 | 7,6 | 0,7 | 5,8 | 0,7 | 2,6 | 0,7 | 5,4 | 0,7 | 6,5 | 0,7 | 2,8 |
| 0,75 | 5,3 | 0,75 | 7,4 | 0,75 | 5,8 | 0,75 | 2,6 | 0,75 | 5,1 | 0,75 | 6,3 | 0,75 | 2,8 |
| 0,8 | 5,0 | 0,8 | 7,1 | 0,8 | 5,8 | 0,8 | 2,5 | 0,8 | 4,8 | 0,8 | 6,1 | 0,8 | 2,7 |
| 0,85 | 4,6 | 0,85 | 6,5 | 0,85 | 5,8 | 0,85 | 2,3 | 0,85 | 4,3 | 0,85 | 5,6 | 0,85 | 2,5 |
| 0,9 | 3,9 | 0,9 | 5,5 | 0,9 | 5,0 | 0,9 | 2,1 | 0,9 | 3,6 | 0,9 | 4,7 | 0,9 | 2,2 |
| 0,95 | 2,8 | 0,95 | 3,8 | 0,95 | 3,5 | 0,95 | 1,7 | 0,95 | 2,6 | 0,95 | 3,4 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HCFO-1233zdE - HFO-1234zeZ - HFO-1243zf**

| **Organics 0,15 F1233xf + 0,17 F1234yf+ 0,17 F245cb + 0,17 F1243zf + 0,17 F1233zdE + 0,17 F1234zeZ** | | **Organics 0,01 F1233xf + 0,95 F1234yf + 0,01 F245cb + 0,01 F1243zf + 0,01 F1233zdE + 0,01 F1234zeZ** | | **Organics 0,01 F1233xf + 0,01 F1234yf + 0,95 F245cb + 0,01 F1243zf + 0,01 F1233zdE + 0,01 F1234zeZ** | | **Organics 0,01 F1233xf + 0,01 F1234yf + 0,01 F245cb + 0,95 F1243zf + 0,01 F1233zdE + 0,01 F1234zeZ** | | **Organics 0,01 F1233xf + 0,01 F1234yf + 0,01 F245cb + 0,01 F1243zf + 0,95 F1233zdE + 0,01 F1234zeZ** | | **Organics 0,01 F1233xf + 0,01 F1234yf + 0,01 F245cb + 0,01 F1243zf + 0,01 F1233zdE + 0,95 F1234zeZ** | | **Organics 0,95 F1233xf + 0,01 F1234yf + 0,01 F245cb + 0,01 F1243zf + 0,01 F1233zdE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 3,8 | 0 | 6,6 | 0 | 4,6 | 0 | 5,7 | 0 | 1,5 | 0 | 1,9 | 0 | 1,7 |
| 0,05 | 4,9 | 0,05 | 7,6 | 0,05 | 5,8 | 0,05 | 6,7 | 0,05 | 2,6 | 0,05 | 3,0 | 0,05 | 2,8 |
| 0,1 | 4,9 | 0,1 | 7,6 | 0,1 | 5,8 | 0,1 | 6,7 | 0,1 | 2,6 | 0,1 | 3,0 | 0,1 | 2,8 |
| 0,15 | 4,9 | 0,15 | 7,6 | 0,15 | 5,8 | 0,15 | 6,7 | 0,15 | 2,6 | 0,15 | 3,0 | 0,15 | 2,8 |
| 0,2 | 4,9 | 0,2 | 7,6 | 0,2 | 5,8 | 0,2 | 6,7 | 0,2 | 2,6 | 0,2 | 3,0 | 0,2 | 2,8 |
| 0,25 | 4,9 | 0,25 | 7,6 | 0,25 | 5,8 | 0,25 | 6,7 | 0,25 | 2,6 | 0,25 | 3,0 | 0,25 | 2,8 |
| 0,3 | 4,9 | 0,3 | 7,6 | 0,3 | 5,8 | 0,3 | 6,7 | 0,3 | 2,6 | 0,3 | 3,0 | 0,3 | 2,8 |
| 0,35 | 4,9 | 0,35 | 7,6 | 0,35 | 5,8 | 0,35 | 6,7 | 0,35 | 2,6 | 0,35 | 3,0 | 0,35 | 2,8 |
| 0,4 | 4,9 | 0,4 | 7,6 | 0,4 | 5,8 | 0,4 | 6,7 | 0,4 | 2,6 | 0,4 | 3,0 | 0,4 | 2,8 |
| 0,45 | 4,9 | 0,45 | 7,6 | 0,45 | 5,8 | 0,45 | 6,7 | 0,45 | 2,6 | 0,45 | 3,0 | 0,45 | 2,8 |
| 0,5 | 4,9 | 0,5 | 7,6 | 0,5 | 5,8 | 0,5 | 6,7 | 0,5 | 2,6 | 0,5 | 3,0 | 0,5 | 2,8 |
| 0,55 | 4,9 | 0,55 | 7,6 | 0,55 | 5,8 | 0,55 | 6,7 | 0,55 | 2,6 | 0,55 | 3,0 | 0,55 | 2,8 |
| 0,6 | 4,9 | 0,6 | 7,6 | 0,6 | 5,8 | 0,6 | 6,6 | 0,6 | 2,6 | 0,6 | 3,0 | 0,6 | 2,8 |
| 0,65 | 4,9 | 0,65 | 7,6 | 0,65 | 5,8 | 0,65 | 6,6 | 0,65 | 2,6 | 0,65 | 3,0 | 0,65 | 2,8 |
| 0,7 | 4,9 | 0,7 | 7,6 | 0,7 | 5,8 | 0,7 | 6,5 | 0,7 | 2,6 | 0,7 | 3,0 | 0,7 | 2,8 |
| 0,75 | 4,9 | 0,75 | 7,4 | 0,75 | 5,8 | 0,75 | 6,3 | 0,75 | 2,6 | 0,75 | 3,0 | 0,75 | 2,8 |
| 0,8 | 4,7 | 0,8 | 7,1 | 0,8 | 5,8 | 0,8 | 6,0 | 0,8 | 2,5 | 0,8 | 2,9 | 0,8 | 2,7 |
| 0,85 | 4,3 | 0,85 | 6,5 | 0,85 | 5,8 | 0,85 | 5,5 | 0,85 | 2,3 | 0,85 | 2,7 | 0,85 | 2,5 |
| 0,9 | 3,7 | 0,9 | 5,5 | 0,9 | 5,0 | 0,9 | 4,7 | 0,9 | 2,0 | 0,9 | 2,4 | 0,9 | 2,2 |
| 0,95 | 2,7 | 0,95 | 3,8 | 0,95 | 3,5 | 0,95 | 3,3 | 0,95 | 1,7 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb -HFO-1234zeE - HFO-1234zeZ - HFO-1243zf**

| **Organics 0,15 F1233xf + 0,17 F1234yf + 0,17 F245cb + 0,17 F1243zf + 0,17 F1234zeE + 0,17 F1234zeZ** | | **Organics 0,01 F1233xf + 0,95 F1234yf + 0,01 F245cb + 0,01 F1243zf + 0,01 F1234zeE + 0,01 F1234zeZ** | | **Organics 0,01 F1233xf + 0,01 F1234yf + 0,95 F245cb + 0,01 F1243zf + 0,01 F1234zeE + 0,01 F1234zeZ** | | **Organics 0,01 F1233xf + 0,01 F1234yf + 0,01 F245cb + 0,95 F1243zf + 0,01 F1234zeE + 0,01 F1234zeZ** | | **Organics 0,01 F1233xf + 0,01 F1234yf + 0,01 F245cb + 0,01 F1243zf + 0,95 F1234zeE + 0,01 F1234zeZ** | | **Organics 0,01 F1233xf + 0,01 F1234yf + 0,01 F245cb + 0,01 F1243zf + 0,01 F1234zeE + 0,95 F1234zeZ** | | **Organics 0,95 F1233xf + 0,01 F1234yf + 0,01 F245cb + 0,01 F1243zf + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,4 | 0 | 6,6 | 0 | 4,6 | 0 | 5,8 | 0 | 4,8 | 0 | 2,0 | 0 | 1,7 |
| 0,05 | 5,5 | 0,05 | 7,6 | 0,05 | 5,8 | 0,05 | 6,7 | 0,05 | 5,8 | 0,05 | 31 | 0,05 | 2,8 |
| 0,1 | 5,5 | 0,1 | 7,6 | 0,1 | 5,8 | 0,1 | 6,7 | 0,1 | 5,8 | 0,1 | 3,1 | 0,1 | 2,8 |
| 0,15 | 5,5 | 0,15 | 7,6 | 0,15 | 5,8 | 0,15 | 6,7 | 0,15 | 5,8 | 0,15 | 3,1 | 0,15 | 2,8 |
| 0,2 | 5,5 | 0,2 | 7,6 | 0,2 | 5,8 | 0,2 | 6,7 | 0,2 | 5,8 | 0,2 | 3,1 | 0,2 | 2,8 |
| 0,25 | 5,5 | 0,25 | 7,6 | 0,25 | 5,8 | 0,25 | 6,7 | 0,25 | 5,8 | 0,25 | 3,1 | 0,25 | 2,8 |
| 0,3 | 5,5 | 0,3 | 7,6 | 0,3 | 5,8 | 0,3 | 6,7 | 0,3 | 5,8 | 0,3 | 3,1 | 0,3 | 2,8 |
| 0,35 | 5,5 | 0,35 | 7,6 | 0,35 | 5,8 | 0,35 | 6,7 | 0,35 | 5,8 | 0,35 | 3,1 | 0,35 | 2,8 |
| 0,4 | 5,5 | 0,4 | 7,6 | 0,4 | 5,8 | 0,4 | 6,7 | 0,4 | 5,8 | 0,4 | 3,1 | 0,4 | 2,8 |
| 0,45 | 5,5 | 0,45 | 7,6 | 0,45 | 5,8 | 0,45 | 6,7 | 0,45 | 5,8 | 0,45 | 3,1 | 0,45 | 2,8 |
| 0,5 | 5,5 | 0,5 | 7,6 | 0,5 | 5,8 | 0,5 | 6,7 | 0,5 | 5,8 | 0,5 | 3,1 | 0,5 | 2,8 |
| 0,55 | 5,5 | 0,55 | 7,6 | 0,55 | 5,8 | 0,55 | 6,7 | 0,55 | 5,7 | 0,55 | 3,1 | 0,55 | 2,8 |
| 0,6 | 5,5 | 0,6 | 7,6 | 0,6 | 5,8 | 0,6 | 6,7 | 0,6 | 5,6 | 0,6 | 3,1 | 0,6 | 2,8 |
| 0,65 | 5,5 | 0,65 | 7,6 | 0,65 | 5,8 | 0,65 | 6,6 | 0,65 | 5,5 | 0,65 | 3,1 | 0,65 | 2,8 |
| 0,7 | 5,5 | 0,7 | 7,6 | 0,7 | 5,8 | 0,7 | 6,5 | 0,7 | 5,4 | 0,7 | 3,1 | 0,7 | 2,8 |
| 0,75 | 5,3 | 0,75 | 7,4 | 0,75 | 5,8 | 0,75 | 6,3 | 0,75 | 5,1 | 0,75 | 3,1 | 0,75 | 2,8 |
| 0,8 | 5,1 | 0,8 | 7,1 | 0,8 | 5,8 | 0,8 | 6,1 | 0,8 | 4,8 | 0,8 | 2,9 | 0,8 | 2,7 |
| 0,85 | 4,6 | 0,85 | 6,5 | 0,85 | 5,8 | 0,85 | 5,6 | 0,85 | 4,3 | 0,85 | 2,7 | 0,85 | 2,5 |
| 0,9 | 3,9 | 0,9 | 5,5 | 0,9 | 5,0 | 0,9 | 4,7 | 0,9 | 36 | 0,9 | 2,4 | 0,9 | 2,2 |
| 0,95 | 2,8 | 0,95 | 3,8 | 0,95 | 3,5 | 0,95 | 3,4 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234yf - HCFO-1233zdE -HFO-1234zeE - HFO-1234zeZ - HFO-1243zf**

| **Organics 0,15 F1233xf + 0,17 F1234yf + 0,17 F1233zdE + 0,17 F1243zf + 0,17 F1234zeE + 0,17 F1234zeZ** | | **Organics 0,01 F1233xf + 0,95 F1234yf + 0,01 F1233zdE + 0,01 F1243zf + 0,01 F1234zeE + 0,01 F1234zeZ** | | **Organics 0,01 F1233xf + 0,01 F1234yf + 0,95 F1233zdE + 0,01 F1243zf + 0,01 F1234zeE + 0,01 F1234zeZ** | | **Organics 0,01 F1233xf + 0,01 F1234yf + 0,01 F1233zdE + 0,95 F1243zf + 0,01 F1234zeE + 0,01 F1234zeZ** | | **Organics 0,01 F1233xf + 0,01 F1234yf + 0,01 F1233zdE + 0,01 F1243zf + 0,95 F1234zeE + 0,01 F1234zeZ** | | **Organics 0,01 F1233xf + 0,01 F1234yf + 0,01 F1233zdE + 0,01 F1243zf + 0,01 F1234zeE + 0,95 F1234zeZ** | | **Organics 0,95 F1233xf + 0,01 F1234yf + 0,01 F1233zdE + 0,01 F1243zf + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 3,9 | 0 | 6,6 | 0 | 1,5 | 0 | 5,7 | 0 | 4,8 | 0 | 1,9 | 0 | 1,7 |
| 0,05 | 5,0 | 0,05 | 7,6 | 0,05 | 2,6 | 0,05 | 6,7 | 0,05 | 5,8 | 0,05 | 3,0 | 0,05 | 2,8 |
| 0,1 | 4,9 | 0,1 | 7,6 | 0,1 | 2,6 | 0,1 | 6,7 | 0,1 | 5,8 | 0,1 | 3,0 | 0,1 | 2,8 |
| 0,15 | 4,9 | 0,15 | 7,6 | 0,15 | 2,6 | 0,15 | 6,7 | 0,15 | 5,8 | 0,15 | 3,0 | 0,15 | 2,8 |
| 0,2 | 4,9 | 0,2 | 7,6 | 0,2 | 2,6 | 0,2 | 6,7 | 0,2 | 5,8 | 0,2 | 3,0 | 0,2 | 2,8 |
| 0,25 | 4,9 | 0,25 | 7,6 | 0,25 | 2,6 | 0,25 | 6,7 | 0,25 | 5,8 | 0,25 | 3,0 | 0,25 | 2,8 |
| 0,3 | 4,9 | 0,3 | 7,6 | 0,3 | 2,6 | 0,3 | 6,7 | 0,3 | 5,8 | 0,3 | 3,0 | 0,3 | 2,8 |
| 0,35 | 4,9 | 0,35 | 7,6 | 0,35 | 2,6 | 0,35 | 6,7 | 0,35 | 5,8 | 0,35 | 3,0 | 0,35 | 2,8 |
| 0,4 | 4,9 | 0,4 | 7,6 | 0,4 | 2,6 | 0,4 | 6,7 | 0,4 | 5,8 | 0,4 | 3,0 | 0,4 | 2,8 |
| 0,45 | 4,9 | 0,45 | 7,6 | 0,45 | 2,6 | 0,45 | 6,7 | 0,45 | 5,8 | 0,45 | 3,0 | 0,45 | 2,8 |
| 0,5 | 4,9 | 0,5 | 7,6 | 0,5 | 2,6 | 0,5 | 6,7 | 0,5 | 5,7 | 0,5 | 3,0 | 0,5 | 2,8 |
| 0,55 | 4,9 | 0,55 | 7,6 | 0,55 | 2,6 | 0,55 | 6,7 | 0,55 | 5,7 | 0,55 | 3,0 | 0,55 | 2,8 |
| 0,6 | 4,9 | 0,6 | 7,6 | 0,6 | 2,6 | 0,6 | 6,6 | 0,6 | 5,6 | 0,6 | 3,0 | 0,6 | 2,8 |
| 0,65 | 4,9 | 0,65 | 7,6 | 0,65 | 2,6 | 0,65 | 6,6 | 0,65 | 5,5 | 0,65 | 3,0 | 0,65 | 2,8 |
| 0,7 | 4,8 | 0,7 | 7,6 | 0,7 | 2,6 | 0,7 | 6,5 | 0,7 | 5,3 | 0,7 | 3,0 | 0,7 | 2,8 |
| 0,75 | 4,7 | 0,75 | 7,4 | 0,75 | 2,6 | 0,75 | 6,3 | 0,75 | 5,1 | 0,75 | 3,0 | 0,75 | 2,8 |
| 0,8 | 4,4 | 0,8 | 7,1 | 0,8 | 2,5 | 0,8 | 6,0 | 0,8 | 4,8 | 0,8 | 2,9 | 0,8 | 2,6 |
| 0,85 | 4,0 | 0,85 | 6,5 | 0,85 | 2,3 | 0,85 | 5,5 | 0,85 | 4,3 | 0,85 | 2,7 | 0,85 | 2,5 |
| 0,9 | 3,4 | 0,9 | 5,5 | 0,9 | 2,0 | 0,9 | 4,7 | 0,9 | 3,6 | 0,9 | 2,4 | 0,9 | 2,2 |
| 0,95 | 2,5 | 0,95 | 3,8 | 0,95 | 1,7 | 0,95 | 3,3 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HFO-1234yf - HFC-245cb - HCFO-1233zdE -HFO-1234zeE - HFO-1234zeZ - HFO-1243zf**

| **Organics 0,15 F1234yf + 0,17 F245cb + 0,17 F1233zdE + 0,17 F1234zeE + 0,17 F1234zeZ + 0,17 F1243zf** | | **Organics 0,95 F1234yf + 0,01 F245cb + 0,01 F1233zdE + 0,01 F1234zeE + 0,01 F1234zeZ + 0,01 F1243zf** | | **Organics 0,01 F1234yf + 0,95 F245cb + 0,01 F1233zdE + 0,01 F1234zeE + 0,01 F1234zeZ + 0,01 F1243zf** | | **Organics 0,01 F1234yf + 0,01 F245cb + 0,95 F1233zdE + 0,01 F1234zeE + 0,01 F1234zeZ + 0,01 F1243zf** | | **Organics 0,01 F1234yf + 0,01 F245cb + 0,01 F1233zdE + 0,95 F1234zeE + 0,01 F1234zeZ + 0,01 F1243zf** | | **Organics 0,01 F1234yf + 0,01 F245cb + 0,01 F1233zdE + 0,01 F1234zeE + 0,01 F1234zeZ + 0,95 F1243zf** | | **Organics 0,01 F1234yf + 0,01 F245cb + 0,01 F1233zdE + 0,01 F1234zeE + 0,95 F1234zeZ + 0,01 F1243zf** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,3 | 0 | 6,6 | 0 | 4,6 | 0 | 1,5 | 0 | 4,8 | 0 | 5,8 | 0 | 2,0 |
| 0,05 | 5,3 | 0,05 | 7,6 | 0,05 | 5,8 | 0,05 | 2,6 | 0,05 | 5,8 | 0,05 | 6,7 | 0,05 | 3,1 |
| 0,1 | 5,3 | 0,1 | 7,6 | 0,1 | 5,8 | 0,1 | 2,6 | 0,1 | 5,8 | 0,1 | 6,7 | 0,1 | 3,1 |
| 0,15 | 5,3 | 0,15 | 7,6 | 0,15 | 5,8 | 0,15 | 2,6 | 0,15 | 5,8 | 0,15 | 6,7 | 0,15 | 3,1 |
| 0,2 | 5,3 | 0,2 | 7,6 | 0,2 | 5,8 | 0,2 | 2,6 | 0,2 | 5,8 | 0,2 | 6,7 | 0,2 | 3,1 |
| 0,25 | 5,3 | 0,25 | 7,6 | 0,25 | 5,8 | 0,25 | 2,6 | 0,25 | 5,8 | 0,25 | 6,7 | 0,25 | 3,1 |
| 0,3 | 5,3 | 0,3 | 7,6 | 0,3 | 5,8 | 0,3 | 2,6 | 0,3 | 5,8 | 0,3 | 6,7 | 0,3 | 3,1 |
| 0,35 | 5,3 | 0,35 | 7,6 | 0,35 | 5,8 | 0,35 | 2,6 | 0,35 | 5,8 | 0,35 | 6,7 | 0,35 | 3,1 |
| 0,4 | 5,3 | 0,4 | 7,6 | 0,4 | 5,8 | 0,4 | 2,6 | 0,4 | 5,8 | 0,4 | 6,7 | 0,4 | 3,1 |
| 0,45 | 5,3 | 0,45 | 7,6 | 0,45 | 5,8 | 0,45 | 2,6 | 0,45 | 5,8 | 0,45 | 6,7 | 0,45 | 3,1 |
| 0,5 | 5,3 | 0,5 | 7,6 | 0,5 | 5,8 | 0,5 | 2,6 | 0,5 | 5,8 | 0,5 | 6,7 | 0,5 | 3,1 |
| 0,55 | 5,3 | 0,55 | 7,6 | 0,55 | 5,8 | 0,55 | 2,6 | 0,55 | 5,7 | 0,55 | 6,7 | 0,55 | 3,1 |
| 0,6 | 5,3 | 0,6 | 7,6 | 0,6 | 5,8 | 0,6 | 2,6 | 0,6 | 5,6 | 0,6 | 6,7 | 0,6 | 3,1 |
| 0,65 | 5,3 | 0,65 | 7,6 | 0,65 | 5,8 | 0,65 | 2,6 | 0,65 | 5,5 | 0,65 | 6,6 | 0,65 | 3,1 |
| 0,7 | 5,3 | 0,7 | 7,6 | 0,7 | 5,8 | 0,7 | 2,6 | 0,7 | 5,4 | 0,7 | 6,5 | 0,7 | 3,1 |
| 0,75 | 5,2 | 0,75 | 7,4 | 0,75 | 5,8 | 0,75 | 2,6 | 0,75 | 5,1 | 0,75 | 6,3 | 0,75 | 3,1 |
| 0,8 | 4,9 | 0,8 | 7,1 | 0,8 | 5,8 | 0,8 | 2,5 | 0,8 | 4,8 | 0,8 | 6,1 | 0,8 | 2,9 |
| 0,85 | 4,5 | 0,85 | 6,5 | 0,85 | 5,8 | 0,85 | 2,3 | 0,85 | 4,3 | 0,85 | 5,6 | 0,85 | 2,7 |
| 0,9 | 3,9 | 0,9 | 5,5 | 0,9 | 5,0 | 0,9 | 2,1 | 0,9 | 3,6 | 0,9 | 4,7 | 0,9 | 2,4 |
| 0,95 | 2,8 | 0,95 | 3,8 | 0,95 | 3,5 | 0,95 | 1,7 | 0,95 | 2,6 | 0,95 | 3,4 | 0,95 | 1,9 |
| 1 | 1,2 | 1 | 1,2 | 1 | 1,2 | 1 | 1,2 | 1 | 1,2 | 1 | 1,2 | 1 | 1,2 |

**HF - HFO-1234yf - HCFC-244bb- HFC-245fa - Trifluoropropyne- HFO-1225yeZ-HFO-1225zc**

| | **Organics 0,15 F1234yf + 0,17 F244bb + 0,17 F245fa + 0,17TFP + 0,17 F1225yeZ + 0,17 F1225zc** | **Organics 0,95 F1234yf + 0,01 F244bb + 0,01 F245fa + 0,01 TFP + 0,01 F1225yeZ + 0,01 F1225zc** | **Organics 0,01 F1234yf + 0,95 F244bb + 0,01 F245fa + 0,01 TFP + 0,01 F1225yeZ + 0,01 F1225zc** | **Organics 0,01 F1234yf + 0,01 F244bb+0,95 F245fa + 0,01 TFP + 0,01 F1225yeZ + 0,01 F1225zc** | **Organics 0,01 F1234yf + 0,01 F244bb + 0,01 F245fa + 0,95 TFP + 0,01 F1225yeZ + 0,01 F1225zc** | **Organics 0,01 F1234yf + 0,01 F244bb + 0,01 F245fa + 0,01 TFP + 0,95 F1225yeZ + 0,01 F1225zc** | **Organics 0,01 F1234yf + 0,01 F244bb + 0,01 F245fa + 0,01 TFP + 0,01 F1225yeZ + 0,95 F1225zc** |
|---|---|---|---|---|---|---|---|
| MASSFRAC | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE |
| | bar | bar | bar | bar | bar | bar | bar |
| 0 | 5,6 | 6,7 | 1,0 | 1,8 | 11,3 | 5,2 | 5,3 |
| 0,05 | 66 | 7,7 | 2,1 | 2,9 | 12,1 | 6,2 | 6,3 |
| 0,1 | 6,6 | 7,7 | 2,1 | 2,9 | 12,0 | 6,2 | 6,3 |
| 0,15 | 6,6 | 7,7 | 2,1 | 2,9 | 11,9 | 6,2 | 6,3 |
| 0,2 | 6,6 | 7,7 | 2,1 | 2,9 | 11,8 | 6,2 | 6,3 |
| 0,25 | 6,6 | 7,7 | 2,1 | 2,9 | 11,7 | 6,2 | 6,3 |
| 0,3 | 6,6 | 7,7 | 2,1 | 2,9 | 11,6 | 6,2 | 6,3 |
| 0,35 | 6,5 | 7,7 | 2,1 | 2,9 | 11,5 | 6,2 | 6,3 |
| 0,4 | 6,5 | 7,7 | 2,1 | 2,9 | 11,5 | 6,2 | 6,3 |
| 0,45 | 6,5 | 7,7 | 2,1 | 2,9 | 11,5 | 6,2 | 6,3 |
| 0,5 | 6,5 | 7,7 | 2,1 | 2,9 | 11,5 | 6,2 | 6,3 |
| 0,55 | 6,5 | 7,7 | 2,1 | 2,9 | 11,5 | 6,2 | 6,2 |
| 0,6 | 6,4 | 7,7 | 2,1 | 2,9 | 11,5 | 6,2 | 6,2 |
| 0,65 | 6,4 | 7,7 | 2,1 | 2,9 | 11,5 | 6,2 | 6,0 |
| 0,7 | 6,3 | 7,7 | 2,1 | 2,9 | 11,5 | 6,1 | 5,9 |
| 0,75 | 6,2 | 7,5 | 2,1 | 2,9 | 11,6 | 5,9 | 5,6 |
| 0,8 | 5,9 | 7,2 | 2,0 | 2,8 | 11,3 | 5,6 | 5,2 |
| 0,85 | 5,3 | 6,6 | 2,0 | 2,6 | 10,5 | 5,1 | 4,7 |
| 0,9 | 4,5 | 5,5 | 1,9 | 2,3 | 8,9 | 4,2 | 3,9 |
| 0,95 | 3,2 | 3,8 | 1,6 | 1,8 | 6,0 | 3,0 | 2,7 |
| 1 | 1,2 | 1,2 | 1,2 | 1,2 | 1,2 | 1,2 | 1,2 |

**HF - HFC-245cb - HCFC-244bb- HFC-245fa - Trifluoropropyne- HFO-1225yeZ-HFO-1225zc**

| | **Organics 0,15 F245cb + 0,17 F244bb + 0,17 F245fa + 0,17TFP + 0,17 F1225yeZ + 0,17 F1225zc** | **Organics 0,95 F245cb + 0,01 F244bb + 0,01 F245fa + 0,01 TFP + 0,01 F1225yeZ + 0,01 F1225zc** | **Organics 0,01 F245cb + 0,95 F244bb + 0,01 F245fa + 0,01 TFP + 0,01 F1225yeZ + 0,01 F1225zc** | **Organics 0,01 F245cb + 0,01 F244bb + 0,95 F245fa + 0,01 TFP + 0,01 F1225yeZ + 0,01 F1225zc** | **Organics 0,01 F245cb + 0,01 F244bb + 0,01 F245fa + 0,95 TFP + 0,01 F1225yeZ + 0,01 F1225zc** | **Organics 0,01 F245cb + 0,01 F244bb + 0,01 F245fa + 0,01 TFP + 0,95 F1225yeZ + 0,01 F1225zc** | **Organics 0,01 F245cb + 0,01 F244bb + 0,01 F245fa + 0,01 TFP + 0,01 F1225yeZ + 0,95 F1225zc** |
|---|---|---|---|---|---|---|---|
| MASSFRAC | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE |
| | bar | bar | bar | bar | bar | bar | bar |
| 0 | 5,3 | 4,7 | 1,0 | 1,8 | 11,3 | 5,2 | 5,3 |
| 0,05 | 6,3 | 5,9 | 2,1 | 2,9 | 12,1 | 6,2 | 6,3 |
| 0,1 | 6,3 | 5,9 | 2,1 | 2,9 | 12,0 | 6,2 | 6,3 |
| 0,15 | 6,3 | 5,9 | 2,1 | 2,9 | 11,9 | 6,2 | 6,3 |
| 0,2 | 6,3 | 5,9 | 2,1 | 2,9 | 11,8 | 6,2 | 6,3 |
| 0,25 | 6,3 | 5,9 | 2,1 | 2,9 | 11,7 | 6,2 | 6,3 |
| 0,3 | 6,3 | 5,9 | 2,1 | 2,9 | 11,6 | 6,2 | 6,3 |
| 0,35 | 6,3 | 5,9 | 2,1 | 2,9 | 11,5 | 6,2 | 6,3 |
| 0,4 | 6,2 | 5,9 | 2,1 | 2,9 | 11,5 | 6,2 | 6,3 |
| 0,45 | 6,2 | 5,9 | 2,1 | 2,9 | 11,5 | 6,2 | 6,3 |
| 0,5 | 6,2 | 5,9 | 2,1 | 2,9 | 11,5 | 6,2 | 6,2 |
| 0,55 | 6,2 | 5,9 | 2,1 | 2,9 | 11,5 | 6,2 | 6,2 |
| 0,6 | 6,2 | 5,9 | 2,1 | 2,9 | 11,5 | 6,2 | 6,1 |
| 0,65 | 6,1 | 5,9 | 2,1 | 2,9 | 11,5 | 6,2 | 6,0 |
| 0,7 | 6,1 | 5,9 | 2,0 | 2,8 | 11,5 | 6,1 | 5,9 |
| 0,75 | 6,0 | 5,8 | 2,0 | 2,8 | 11,5 | 5,9 | 5,6 |
| 0,8 | 5,7 | 5,8 | 2,0 | 2,8 | 11,3 | 5,6 | 5,2 |
| 0,85 | 5,2 | 5,8 | 2,0 | 2,6 | 10,5 | 5,1 | 4,7 |
| 0,9 | 4,4 | 5,0 | 1,9 | 2,3 | 8,9 | 4,2 | 3,9 |
| 0,95 | 3,1 | 3,5 | 1,6 | 1,8 | 6,0 | 3,0 | 2,7 |
| 1 | 1,2 | 1,2 | 1,2 | 1,2 | 1,2 | 1,2 | 1,2 |

### Exemple 6 : Systèmes à 8 composés

**HF - HCFO-1233xf - HFO-1234yf - HFC-245cb - HCFO-1233zdE - HFO-1234zeE - HFO-1234zeZ - HFO-1243zf**

| Organics 0,01 F1233xf **+ 0,94 F1234yf** + 0,01 F245cb +0,01 F1233zdE + 0,01 F1234zeE + 0,01 F1234zeZ + 0,01 F1243zf | | Organics 0,01 F1233xf + 0,01 F1234yf + **0,94 F245cb** +0,01 F1233zdE + 0,01 F1234zeE + 0,01 F1234zeZ + 0,01 F1243zf | | Organics 0,01 F1233xf + 0,01 F1234yf + 0,01 F245cb **+0,94 F1233zdE** + 0,01 F1234zeE + 0,01 F1234zeZ + 0,01 F1243zf | | Organics 0,01 F1233xf + 0,01 F1234yf + 0,01 F245cb +0,01 **F1233zdE** + **0,94 F1234zeE** + 0,01 F1234zeZ + 0,01 F1243zf | | Organics 0,01 F1233xf + 0,01 F1234yf + 0,01F 245cb +0,01 F1233zdE + 0,01 F1234zeE + **0,94 F1234zeZ** + 0,01 F1243zf | | Organics 0,01 F1233xf + 0,01 F1234yf + 0,01 F245cb +0,01 F1233zdE + 0,01 F1234zeE + 0,01 F1234zeZ + **0,94 F1243zf** | | Organics 0,16 F1233xf + 0,14 F1234yf + 0,14 F245cb +0,14 F1233zdE + 0,14 F1234zeE + 0,14 F1234zeZ + 0,14 F1243zf | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 6,6 | 0 | 4,6 | 0 | 1,5 | 0 | 4,8 | 0 | 2,0 | 0 | 5,7 | 0 | 3,9 |
| 0,05 | 7,6 | 0,05 | 5,8 | 0,05 | 2,6 | 0,05 | 5,8 | 0,05 | 3,1 | 0,05 | 6,7 | 0,05 | 5,0 |
| 0,1 | 7,6 | 0,1 | 5,8 | 0,1 | 2,6 | 0,1 | 5,8 | 0,1 | 3,1 | 0,1 | 6,7 | 0,1 | 5,0 |
| 0,15 | 7,6 | 0,15 | 5,8 | 0,15 | 2,6 | 0,15 | 5,8 | 0,15 | 3,1 | 0,15 | 6,7 | 0,15 | 5,0 |
| 0,2 | 7,6 | 0,2 | 5,8 | 0,2 | 2,6 | 0,2 | 5,8 | 0,2 | 3,1 | 0,2 | 6,7 | 0,2 | 5,0 |
| 0,25 | 7,6 | 0,25 | 5,8 | 0,25 | 2,6 | 0,25 | 5,8 | 0,25 | 3,1 | 0,25 | 6,7 | 0,25 | 5,0 |
| 0,3 | 7,6 | 0,3 | 5,8 | 0,3 | 2,6 | 0,3 | 5,8 | 0,3 | 3,1 | 0,3 | 6,7 | 0,3 | 5,0 |
| 0,35 | 7,6 | 0,35 | 5,8 | 0,35 | 2,6 | 0,35 | 5,8 | 0,35 | 3,1 | 0,35 | 6,7 | 0,35 | 5,0 |
| 0,4 | 7,6 | 0,4 | 5,8 | 0,4 | 2,6 | 0,4 | 5,8 | 0,4 | 3,1 | 0,4 | 6,7 | 0,4 | 5,0 |
| 0,45 | 7,6 | 0,45 | 5,8 | 0,45 | 2,6 | 0,45 | 5,8 | 0,45 | 3,1 | 0,45 | 6,7 | 0,45 | 5,0 |
| 0,5 | 7,6 | 0,5 | 5,8 | 0,5 | 2,6 | 0,5 | 5,7 | 0,5 | 3,1 | 0,5 | 6,7 | 0,5 | 5,0 |
| 0,55 | 7,6 | 0,55 | 5,8 | 0,55 | 2,6 | 0,55 | 5,7 | 0,55 | 3,1 | 0,55 | 6,7 | 0,55 | 5,0 |
| 0,6 | 7,6 | 0,6 | 5,8 | 0,6 | 2,6 | 0,6 | 5,6 | 0,6 | 3,1 | 0,6 | 6,6 | 0,6 | 5,0 |
| 0,65 | 76 | 0,65 | 5,8 | 0,65 | 2,6 | 0,65 | 5,5 | 0,65 | 3,1 | 0,65 | 6,6 | 0,65 | 5,0 |
| 0,7 | 7,5 | 0,7 | 5,8 | 0,7 | 2,6 | 0,7 | 5,4 | 0,7 | 3,1 | 0,7 | 6,5 | 0,7 | 5,0 |
| 0,75 | 7,4 | 0,75 | 5,8 | 0,75 | 2,6 | 0,75 | 5,1 | 0,75 | 3,1 | 0,75 | 6,3 | 0,75 | 4,9 |
| 0,8 | 71 | 0,8 | 5,8 | 0,8 | 2,5 | 0,8 | 4,8 | 0,8 | 2,9 | 0,8 | 6,0 | 0,8 | 4,6 |
| 0,85 | 6,5 | 0,85 | 5,8 | 0,85 | 2,3 | 0,85 | 4,3 | 0,85 | 2,7 | 0,85 | 5,5 | 0,85 | 4,2 |
| 0,9 | 5,5 | 0,9 | 5,0 | 0,9 | 2,1 | 0,9 | 3,6 | 0,9 | 2,4 | 0,9 | 4,7 | 0,9 | 3,6 |
| 0,95 | 3,8 | 0,95 | 3,5 | 0,95 | 1,7 | 0,95 | 2,6 | 0,95 | 1,9 | 0,95 | 3,3 | 0,95 | 2,6 |
| 1 | 1,2 | 1 | 1,2 | 1 | 1,2 | 1 | 1,2 | 1 | 1,2 | 1 | 1,2 | 1 | 1,2 |

**HF-HFO-1234yf-HFC-245cb-HCFO-1233xf-HFO-1234zeE-HFO-1234zeZ-HCFO-1233zdE-HCFC-244bb**

| | **Organics 0,94 F1233xf + 0,01 F1234yf + 0,01 F245cb + 0,01 F1233zdE + 0,01 F1234zeE + 0,01 F1234zeZ + 0,01 F244bb** | **Organics 0,01 F1233xf + 0,94 F1234yf + 0,01 F245cb +0,01 F1233zdE + 0,01 F1234zeE + 0,01 F1234zeZ + 0,01 F244bb** | **Organics 0,01 F1233xf + 0,01 F1234yf + 0,94 F245cb +0,01 F1233zdE + 0,01 F1234zeE + 0,01 F1234zeZ + 0,01 F244bb** | **Organics 0,01 F1233xf + 0,01 F1234yf + 0,01 F245cb +0,94 F1233zdE + 0,01 F1234zeE + 0,01 F1234zeZ + 0,01 F244bb** | **Organics 0,01 F1233xf + 0,01 F1234yf + 0,01 F245cb +0,01 F1233zdE + 0,94 F1234zeE + 0,01 F1234zeZ + 0,01 F244bb** | **Organics 0,01 F1233xf + 0,01 F1234yf + 0,01 F245cb +0,01 F1233zdE + 0,01 F1234zeE + 0,94 F1234zeZ + 0,01 F244bb** | **Organics 0,01 F1233xf + 0,01 F1234yf + 0,01 F245cb +0,01 F1233zdE + 0,01 F1234zeE + 0,01 F1234zeZ +0,94 F244bb** | **Organics 0,16 F1233xf + 0,14 F1234yf + 0,14 F245cb +0,14 F1233zdE + 0,14 F1234zeE + 0,14 F1234zeZ +0,14 F244bb** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 1,7 | 6,6 | 4,5 | 1,5 | 4,8 | 1,9 | 0,8 | 3,2 |
| 0,05 | 2,8 | 7,5 | 5,7 | 2,6 | 5,7 | 3,0 | 2,0 | 4,3 |
| 0,1 | 2,8 | 7,5 | 5,7 | 2,6 | 5,7 | 3,0 | 2,0 | 4,3 |
| 0,15 | 2,8 | 7,5 | 5,7 | 2,6 | 5,7 | 3,0 | 2,0 | 4,3 |
| 0,2 | 2,8 | 7,5 | 5,7 | 2,6 | 5,7 | 3,0 | 2,0 | 4,3 |
| 0,25 | 2,8 | 7,5 | 5,7 | 2,6 | 5,7 | 3,0 | 2,0 | 4,3 |
| 0,3 | 2,8 | 7,5 | 5,7 | 2,6 | 5,7 | 3,0 | 2,0 | 4,3 |
| 0,35 | 2,8 | 7,5 | 5,7 | 2,6 | 5,7 | 3,0 | 2,0 | 4,3 |
| 0,4 | 2,8 | 7,5 | 5,7 | 2,6 | 5,7 | 3,0 | 2,0 | 4,3 |
| 0,45 | 2,8 | 7,5 | 5,7 | 2,6 | 5,7 | 3,0 | 2,0 | 4,3 |
| 0,5 | 2,8 | 7,5 | 5,7 | 2,6 | 5,7 | 3,0 | 2,0 | 4,3 |
| 0,55 | 2,8 | 7,5 | 5,7 | 2,6 | 5,6 | 3,0 | 2,0 | 4,2 |
| 0,6 | 2,8 | 7,5 | 5,7 | 2,6 | 5,6 | 3,0 | 2,0 | 4,2 |
| 0,65 | 2,8 | 7,5 | 5,7 | 2,6 | 5,5 | 3,0 | 2,0 | 4,2 |
| 0,7 | 2,8 | 7,5 | 5,7 | 2,6 | 5,3 | 3,0 | 2,0 | 4,2 |
| 0,75 | 2,8 | 7,4 | 5,7 | 2,6 | 5,1 | 3,0 | 1,9 | 4,2 |
| 0,8 | 2,6 | 7,0 | 5,7 | 2,4 | 4,7 | 2,9 | 1,9 | 4,0 |
| 0,85 | 2,4 | 6,5 | 5,7 | 2,3 | 4,3 | 2,7 | 1,9 | 3,7 |
| 0,9 | 2,2 | 5,4 | 4,9 | 2,0 | 3,6 | 2,4 | 1,8 | 3,2 |
| 0,95 | 1,8 | 3,8 | 3,5 | 1,7 | 2,6 | 1,9 | 1,6 | 2,4 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-Trifluoropropyne**

| | **Organics 0,16 F1234yf + 0,14 F245cb + 0,14 F1233xf + 0,14 F1234zeE + 0,14 F1234zeZ + 0,14 F1233zdE + 0,14 TPF** | **Organics 0,94 F1234yf + 0,01 F245cb + 0,01 F1233xf+ 0,01 F1234zeE + 0,01 F1234zeZ + 0,01 F1233zdE + 0,01 TPF** | **Organics 0,01 F1234yf+ 0,94 F245cb + 0,01 F1233xf + 0,01 F1234zeE + 0,01 F1234zeZ+ 0,01 F1233zdE + 0,01 TPF** | **Organics 0,01 F1234yf + 0,01 F245cb + 0,94 F1233xf + 0,01 F1234zeE + 0,01 F1234zeZ + 0,01 F1233zdE + 0,01 TPF** | **Organics 0,01 F1234yf+ 0,01 F245cb + 0,01 F1233xf+ 0,94 F1234zeE + 0,01 F1234zeZ + 0,01 F1233zdE + 0,01 TPF** | **Organics 0,01 F1234yf + 0,01 F245cb + 0,01 F1233xf + 0,01 F1234zeE + 0,94 F1234zeZ + 0,01 F1233zdE + 0,01 TPF** | **Organics 0,01 F1234yf + 0,01 F245cb + 0,01 F1233xf + 0,01 F1234zeE + 0,01 F1234zeZ + 0,94 F1233zdE + 0,01 TPF** | **Organics 0,01 F1234yf + 0,01 F245cb + 0,01 F1233xf + 0,01 F1234zeE + 0,01 F1234zeZ + 0,01 F1233zdE + 0,94 TPF** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 5,0 | 6,7 | 4,7 | 1,8 | 4,9 | 2,0 | 1,6 | 11,2 |
| 0,05 | 6,0 | 7,6 | 5,8 | 2,9 | 5,8 | 3,1 | 2,7 | 12,0 |
| 0,1 | 6,0 | 7,6 | 5,8 | 2,9 | 5,9 | 3,1 | 2,7 | 11,9 |
| 0,15 | 6,0 | 7,6 | 5,8 | 2,9 | 5,9 | 3,1 | 2,7 | 11,8 |
| 0,2 | 6,0 | 7,6 | 5,8 | 2,9 | 5,9 | 3,1 | 2,7 | 11,7 |
| 0,25 | 6,0 | 7,6 | 5,8 | 2,9 | 5,8 | 3,1 | 2,7 | 11,6 |
| 0,3 | 6,0 | 7,6 | 5,8 | 2,9 | 5,8 | 3,1 | 2,7 | 11,5 |
| 0,35 | 6,0 | 7,6 | 5,8 | 2,9 | 5,8 | 3,1 | 2,7 | 11,5 |
| 0,4 | 6,0 | 7,6 | 5,8 | 2,9 | 5,8 | 3,1 | 2,7 | 11,4 |
| 0,45 | 6,0 | 7,6 | 5,8 | 2,9 | 5,8 | 3,1 | 2,7 | 11,4 |
| 0,5 | 6,0 | 7,6 | 5,8 | 2,9 | 5,8 | 3,1 | 2,7 | 11,4 |
| 0,55 | 6,0 | 7,6 | 5,8 | 2,9 | 5,8 | 3,1 | 2,7 | 11,5 |
| 0,6 | 6,0 | 7,6 | 5,8 | 2,9 | 5,7 | 3,1 | 2,7 | 11,5 |
| 0,65 | 6,0 | 7,6 | 5,8 | 2,9 | 5,6 | 3,1 | 2,7 | 11,5 |
| 0,7 | 6,0 | 7,6 | 5,8 | 2,9 | 5,4 | 3,1 | 2,7 | 11,5 |
| 0,75 | 5,9 | 7,5 | 5,8 | 2,9 | 5,2 | 3,1 | 2,7 | 11,5 |
| 0,8 | 5,6 | 7,1 | 5,8 | 2,8 | 4,9 | 3,0 | 2,6 | 11,2 |
| 0,85 | 5,1 | 6,6 | 5,8 | 2,6 | 4,4 | 2,8 | 2,4 | 10,4 |
| 0,9 | 4,3 | 5,5 | 5,0 | 2,2 | 3,6 | 2,4 | 2,1 | 8,8 |
| 0,95 | 3,1 | 3,8 | 3,5 | 1,8 | 2,6 | 1,9 | 1,7 | 5,9 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-HFC-245fa**

| | **Organics 0,16 F1234yf + 0,14 F245cb + 0,14 F1233xf + 0,14 F1234zeE + 0,14 F1234zeZ + 0,14 F1233zdE + 0,14 F245fa** | **Organics 0,94 F1234yf + 0,01 F245cb + 0,01 F1233xf+ 0,01 F1234zeE + 0,01 F1234zeZ + 0,01 F1233zdE + 0,01 F245fa** | **Organics 0,01 F1234yf+ 0,94 F245cb + 0,01 F1233xf+ 0,01 F1234zeE + 0,01 F1234zeZ + 0,01 F1233zdE + 0,01 F245fa** | **Organics 0,01 F1234yf+ 0,01 F245cb + 0,94 F1233xf + 0,01 F1234zeE + 0,01 F1234zeZ + 0,01 F1233zdE + 0,01 F245fa** | **Organics 0,01 F1234yf+ 0,01 F245cb + 0,01 F1233xf+ 0,94 F1234zeE + 0,01 F1234zeZ + 0,01 F1233zdE + 0,01 F245fa** | **Organics 0,01 F1234yf+ 0,01 F245cb + 0,01 F1233xf+ 0,01 F1234zeE + 0,94 F1234zeZ + 0,01 F1233zdE + 0,01 F245fa** | **Organics 0,01 F1234yf + 0,01 F245cb + 0,01 F1233xf + 0,01 F1234zeE + 0,01 F1234zeZ + 0,94 F1233zdE + 0,01 F245fa** | **Organics 0,01 F1234yf + 0,01 F245cb + 0,01 F1233xf + 0,01 F1234zeE + 0,01 F1234zeZ + 0,01 F1233zdE + 0,94 F245fa** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,4 | 6,6 | 4,5 | 1,7 | 4,8 | 1,9 | 1,5 | 1,6 |
| 0,05 | 4,5 | 7,5 | 5,7 | 2,8 | 5,7 | 3,0 | 2,6 | 2,8 |
| 0,1 | 4,5 | 7,5 | 5,7 | 2,8 | 5,7 | 3,0 | 2,6 | 2,8 |
| 0,15 | 4,5 | 7,5 | 5,7 | 2,8 | 5,7 | 3,0 | 2,6 | 2,8 |
| 0,2 | 4,5 | 7,5 | 5,7 | 2,8 | 5,7 | 3,0 | 2,6 | 2,8 |
| 0,25 | 4,5 | 7,5 | 5,7 | 2,8 | 5,7 | 3,0 | 2,6 | 2,8 |
| 0,3 | 4,5 | 7,5 | 5,7 | 2,8 | 5,7 | 3,0 | 2,6 | 2,8 |
| 0,35 | 4,5 | 7,5 | 5,7 | 2,8 | 5,7 | 3,0 | 2,6 | 2,8 |
| 0,4 | 4,5 | 7,5 | 5,7 | 2,8 | 5,7 | 3,0 | 2,6 | 2,8 |
| 0,45 | 4,5 | 7,5 | 5,7 | 2,8 | 5,7 | 3,0 | 2,6 | 2,8 |
| 0,5 | 4,5 | 7,5 | 5,7 | 2,8 | 5,7 | 3,0 | 2,6 | 2,8 |
| 0,55 | 4,5 | 7,5 | 5,7 | 2,8 | 5,6 | 3,0 | 2,6 | 2,8 |
| 0,6 | 4,5 | 7,5 | 5,7 | 2,8 | 5,6 | 3,0 | 2,6 | 2,8 |
| 0,65 | 4,5 | 7,5 | 5,7 | 2,8 | 5,5 | 3,0 | 2,6 | 2,8 |
| 0,7 | 4,5 | 7,5 | 5,7 | 2,8 | 5,3 | 3,0 | 2,6 | 2,8 |
| 0,75 | 4,4 | 7,4 | 5,7 | 2,8 | 5,1 | 3,0 | 2,6 | 2,8 |
| 0,8 | 4,2 | 7,1 | 5,8 | 2,6 | 4,7 | 2,9 | 2,5 | 2,7 |
| 0,85 | 3,9 | 6,5 | 5,7 | 2,5 | 4,3 | 2,7 | 2,3 | 2,5 |
| 0,9 | 3,3 | 5,4 | 4,9 | 2,2 | 3,6 | 2,4 | 2,0 | 2,2 |
| 0,95 | 2,4 | 3,8 | 3,5 | 1,8 | 2,6 | 1,9 | 1,7 | 1,8 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-HFO-1225yeZ**

| | **Organics 0,16 F1234yf + 0,14 F245cb + 0,14 F1233xf + 0,14 F1234zeE + 0,14 F1234zeZ + 0,14 F1233zdE + 0,14 F1225yeZ** | **Organics 0,94 F1234yf + 0,01 F245cb + 0,01 F1233xf+ 0,01 F1234zeE + 0,01 F1234zeZ + 0,01 F1233zdE + 0,01 F1225yeZ** | **Organics 0,01 F1234yf+ 0,94 F245cb + 0,01 F1233xf+ 0,01 F1234zeE + 0,01 F1234zeZ + 0,01 F1233zdE + 0,01 F1225yeZ** | **Organics 0,01 F1234yf+ 0,01 F245cb + 0,94 F1233xf + 0,01 F1234zeE + 0,01 F1234zeZ + 0,01 F1233zdE + 0,01 F1225yeZ** | **Organics 0,01 F1234yf + 0,01 F245cb + 0,01 F1233xf+ 0,94 F1234zeE + 0,01 F1234zeZ + 0,01 F1233zdE + 0,01 F1225yeZ** | **Organics 0,01 F1234yf+ 0,01 F245cb + 0,01 F1233xf+ 0,01 F1234zeE + 0,94 F1234zeZ + 0,01 F1233zdE + 0,01 F1225yeZ** | **Organics 0,01 F1234yf+ 0,01 F245cb + 0,01 F1233xf+ 0,01 F1234zeE + 0,01 F1234zeZ + 0,94 F1233zdE + 0,01 F1225yeZ** | **Organics 0,01 F1234yf + 0,01 F245cb + 0,01 F1233xf + 0,01 F1234zeE + 0,01 F1234zeZ + 0,01 F1233zdE + 0,94 F1225yeZ** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,8 | 6,6 | 4,6 | 1,7 | 4,8 | 1,9 | 1,5 | 5,1 |
| 0,05 | 4,9 | 7,6 | 5,8 | 2,8 | 5,8 | 3,0 | 2,6 | 6,1 |
| 0,1 | 4,9 | 7,6 | 5,8 | 2,8 | 5,8 | 3,0 | 2,6 | 6,1 |
| 0,15 | 4,9 | 7,6 | 5,8 | 2,8 | 5,8 | 3,0 | 2,6 | 6,1 |
| 0,2 | 4,9 | 7,5 | 5,8 | 2,8 | 5,8 | 3,0 | 2,6 | 6,1 |
| 0,25 | 4,9 | 7,6 | 5,8 | 2,8 | 5,8 | 3,0 | 2,6 | 6,1 |
| 0,3 | 4,9 | 7,6 | 5,8 | 2,8 | 5,8 | 3,0 | 2,6 | 6,1 |
| 0,35 | 4,9 | 7,6 | 5,8 | 2,8 | 5,8 | 3,0 | 2,6 | 6,1 |
| 0,4 | 4,9 | 7,6 | 5,8 | 2,8 | 5,8 | 3,0 | 2,6 | 6,1 |
| 0,45 | 4,9 | 7,6 | 5,8 | 2,8 | 5,8 | 3,0 | 2,6 | 6,1 |
| 0,5 | 4,9 | 7,6 | 5,8 | 2,8 | 5,7 | 3,0 | 2,6 | 6,1 |
| 0,55 | 4,9 | 7,6 | 5,8 | 2,8 | 5,7 | 3,0 | 2,6 | 6,1 |
| 0,6 | 4,9 | 7,6 | 5,8 | 2,8 | 5,6 | 3,0 | 2,6 | 6,1 |
| 0,65 | 4,9 | 7,6 | 5,8 | 2,8 | 5,5 | 3,0 | 2,6 | 6,1 |
| 0,7 | 5,0 | 7,5 | 5,8 | 2,8 | 5,3 | 3,0 | 2,6 | 6,0 |
| 0,75 | 4,8 | 7,4 | 5,8 | 2,8 | 5,1 | 3,0 | 2,6 | 5,8 |
| 0,8 | 4,6 | 7,1 | 5,8 | 2,7 | 4,8 | 2,9 | 2,5 | 5,5 |
| 0,85 | 4,2 | 6,5 | 5,8 | 2,5 | 4,3 | 2,7 | 2,3 | 5,0 |
| 0,9 | 3,6 | 5,5 | 4,9 | 2,2 | 3,6 | 2,4 | 2,1 | 4,2 |
| 0,95 | 2,6 | 3,8 | 3,5 | 1,8 | 2,6 | 1,9 | 1,7 | 2,9 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFC-1233zdE-HFO-1225zc**

| | **Organics 0,16 F1234yf + 0,14 F245cb + 0,14 F1233xf + 0,14 F1234zeE + 0,14 F1234zeZ + 0,14 F1233zdE + 0,14 F1225zc** | **Organics 0,94 F1234yf + 0,01 F245cb + 0,01 F1233xf+ 0,01 F1234zeE + 0,01 F1234zeZ + 0,01 F1233zdE + 0,01 F1225zc** | **Organics 0,01 F1234yf+ 0,94 F245cb + 0,01 F1233xf+ 0,01 F1234zeE + 0,01 F1234zeZ + 0,01 F1233zdE + 0,01 F1225zc** | **Organics 0,01 F1234yf + 0,01 F245cb + 0,94 F1233xf + 0,01 F1234zeE + 0,01 F1234zeZ + 0,01 F1233zdE + 0,01 F1225zc** | **Organics 0,01 F1234yf + 0,01 F245cb + 0,01 F1233xf+ 0,94 F1234zeE + 0,01 F1234zeZ + 0,01 F1233zdE + 0,01 F1225zc** | **Organics 0,01 F1234yf + 0,01 F245cb + 0,01 F1233xf + 0,01 F1234zeE + 0,94 F1234zeZ + 0,01 F1233zdE + 0,01 F1225zc** | **Organics 0,01 F1234yf+ 0,01 F245cb + 0,01 F1233xf+ 0,01 F1234zeE + 0,01 F1234zeZ + 0,94 F1233zdE + 0,01 F1225zc** | **Organics 0,01 F1234yf + 0,01 F245cb + 0,01 F1233xf + 0,01 F1234zeE + 0,01 F1234zeZ + 0,01 F1233zdE + 0,94 F1225zc** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,9 | 6,6 | 4,6 | 1,7 | 4,8 | 1,9 | 1,5 | 5,2 |
| 0,05 | 4,9 | 7,6 | 5,8 | 2,8 | 5,8 | 3,0 | 2,6 | 6,2 |
| 0,1 | 4,9 | 7,6 | 5,8 | 2,8 | 5,8 | 3,0 | 2,6 | 6,2 |
| 0,15 | 4,9 | 7,6 | 5,8 | 2,8 | 5,8 | 3,0 | 2,6 | 6,2 |
| 0,2 | 4,9 | 7,6 | 5,8 | 2,8 | 5,8 | 3,0 | 2,6 | 6,2 |
| 0,25 | 4,9 | 7,6 | 5,8 | 2,8 | 5,8 | 3,0 | 2,6 | 6,2 |
| 0,3 | 4,9 | 7,6 | 5,8 | 2,8 | 5,8 | 3,0 | 2,6 | 6,2 |
| 0,35 | 4,9 | 7,6 | 5,8 | 2,8 | 5,8 | 3,0 | 2,6 | 6,2 |
| 0,4 | 4,9 | 7,6 | 5,8 | 2,8 | 5,8 | 3,0 | 2,6 | 6,2 |
| 0,45 | 4,9 | 7,6 | 5,8 | 2,8 | 5,8 | 3,0 | 2,6 | 6,2 |
| 0,5 | 4,9 | 7,6 | 5,8 | 2,8 | 5,7 | 3,0 | 2,6 | 6,1 |
| 0,55 | 4,9 | 7,6 | 5,8 | 2,8 | 5,7 | 3,0 | 2,6 | 6,1 |
| 0,6 | 4,9 | 7,6 | 5,8 | 2,8 | 5,6 | 3,0 | 2,6 | 6,0 |
| 0,65 | 4,9 | 7,6 | 5,8 | 2,8 | 5,5 | 3,0 | 2,6 | 5,9 |
| 0,7 | 4,9 | 7,5 | 5,8 | 2,8 | 5,3 | 3,0 | 2,6 | 5,8 |
| 0,75 | 4,8 | 7,4 | 5,8 | 2,8 | 5,1 | 3,0 | 2,6 | 5,5 |
| 0,8 | 4,6 | 7,1 | 5,8 | 2,7 | 4,8 | 2,9 | 2,5 | 5,1 |
| 0,85 | 4,2 | 6,5 | 5,8 | 2,5 | 4,3 | 2,7 | 2,3 | 4,6 |
| 0,9 | 3,5 | 5,5 | 4,9 | 2,2 | 3,6 | 2,4 | 2,1 | 3,8 |
| 0,95 | 2,6 | 3,8 | 3,5 | 1,8 | 2,6 | 1,9 | 1,7 | 2,7 |
| 1 | 1,2 | 1,2 | 1,2 | 1,2 | 1,2 | 1,2 | 1,2 | 1,2 |

### Exemple 7 : Systèmes à 13 composés

**HF - HFO-1234yf - HFC-245cb - HCFO-1233xf - HCFO-1233zdE - HFO-1234zeE - HFO-1234zeZ - HFC-1243zf - HCFC-244bb - TFP - HFC-245fa - HFO-1225yeZ - HFO-1225zc**

| | **Organics 0,087 F1234yf +0,083 F245cb + 0,083 F1233xf + 0,083 F1233zdE + 0,083 F1234zeE + 0,083 F1234zeZ + 0,083 F1243zf + 0,083 F244bb + 0,083 F245fa + 0,083 TFP + 0,083 F1225yeZ + 0,83 F1225zc** | **Organics 0,89 F1234yf +0,01 F245cb + 0,01 F1233xf+ 0,01 F1233zdE + 0,01 F1234zeE + 0,01 F1234zeZ+ 0,01 F1243zf + 0,01 F244bb + 0,01 F245fa + 0,01 TFP+ 0,01 F1225yeZ + 0,1 F1225zc** | **Organics 0,01 F1234yf +0,01 F245cb + 0,89 F1233xf + 0,01 F1233zdE + 0,01 F1234zeE + 0,01 F1234zeZ + 0,01 F1243zf + 0,01 F244bb + 0,01 F245fa + 0,01 TFP + 0,01 F1225yeZ + 0,1 F1225zc** | **Organics 0,01 F1234yf +0,01 F245cb + 0,01 F1233xf+ 0,01 F1233zdE + 0,89 F1234zeE + 0,01 F1234zeZ + 0,01 F1243zf + 0,01 F244bb + 0,01 F245fa + 0,01 TFP + 0,01 F1225yeZ+ 0,1 F1225zc** | **Organics 0,01 F1234yf + 0,01 F245cb + 0,01 F1233xf+ 0,89 F1233zdE + 0,01 F1234zeE + 0,01 F1234zeZ + 0,01 F1243zf + 0,01 F244bb + 0,01 F245fa + 0,01 TFP + 0,01 F1225yeZ + 0,1 F1225zc** |
|---|---|---|---|---|---|
| MASSFRAC | TOTAL | TOTAL | TOTAL | TOTAL | TOTAL |
| HF | PRESSURE | PRESSURE | PRESSURE | PRESSURE | PRESSURE |
| | bar | bar | bar | bar | bar |
| 0 | 4,5 | 6,5 | 1,9 | 4,8 | 1,7 |
| 0,05 | 5,6 | 7,5 | 3,0 | 5,8 | 2,9 |
| 01 | 5,6 | 7,5 | 3,0 | 5,8 | 2,9 |
| 0,15 | 5,6 | 7,5 | 3,0 | 5,8 | 2,8 |
| 0,2 | 5,6 | 7,5 | 3,0 | 5,8 | 2,8 |
| 0,25 | 5,6 | 7,5 | 3,0 | 5,8 | 2,8 |
| 0,3 | 5,6 | 7,5 | 3,0 | 5,8 | 2,8 |
| 0,35 | 5,6 | 7,5 | 3,0 | 5,8 | 2,8 |
| 0,4 | 5,6 | 7,5 | 3,0 | 5,8 | 2,8 |
| 0,45 | 5,5 | 7,5 | 3,0 | 5,8 | 2,8 |
| 0,5 | 5,5 | 7,5 | 3,0 | 5,8 | 2,8 |
| 0,55 | 5,5 | 7,5 | 3,0 | 5,7 | 2,8 |
| 0,6 | 5,5 | 7,5 | 3,0 | 5,6 | 2,8 |
| 0,65 | 5,5 | 7,5 | 3,0 | 5,5 | 2,8 |
| 0,7 | 5,4 | 7,5 | 3,0 | 5,4 | 2,8 |
| 0,75 | 5,3 | 7,3 | 3,0 | 5,2 | 2,8 |
| 0,8 | 5,1 | 7,0 | 2,8 | 4,8 | 2.7 |
| 0,85 | 4,6 | 6,4 | 2,6 | 4,3 | 2,5 |
| 0,9 | 3,9 | 5,4 | 2,3 | 3,6 | 2,2 |
| 0,95 | 2,8 | 3,7 | 1,8 | 2,6 | 1,8 |
| 1 | 1,2 | 1,2 | 1,2 | 1,2 | 1,2 |

### Exemple 8 : Plage de température et de pression de mélange ternaire

| | Enveloppe de points d'ébullition | |
|---|---|---|
| Ternaire | Température °C | Pression bar abs |
| HF-HFO-1234yf-HFC-245cb | 0 à 40 | ∼2.4 à ∼11.8 |
| HF-HFO-1234yf-HFO-1234zeE | 0 à 40 | ∼2.6 à ∼11.6 |
| HF-HFO-1234yf-HFO-1234zeZ | 0 à 40 | ∼1.3 à ∼11.4 |
| HF-HFO-1234yf-HFO-1243zf | 0 à 40 | ∼3.0 à ∼11.7 |
| HF-HFO-1234yf-HCFO-1233xf | 0 à 40 | ∼1.1 à ∼11.4 |
| HF-HFO-1234yf-HCFO-1233zdE | 0 à 40 | ∼1.0 à ∼11.4 |
| HF-HFO-1234yf-Trifluoropropyne | 0 à 40 | ∼8.0 à ∼12.2 |
| HF-HFO-1234yf-HFC-244bb | 0 à 40 | ∼2.1 à ∼7.5 |
| HF-HFO-1234yf-HFC-245fa | 0 à 40 | ∼2.9 à ∼7.5 |
| HF-HFO-1234yf-HFO-1225yeZ | 0 à 40 | ∼6.3 à ∼7.7 |
| HF-HFO-1234yf-HFO-1225zc | 0 à 40 | ∼6.4 à ∼7.7 |

### Exemple 9 : Plage de température et de pression de mélange quaternaire

| | Enveloppe de points d'ébullition | |
|---|---|---|
| Quaternaire | Température°C | Pression bar abs |
| HF - HCFO-1233xf - HFC-245cb - HFO-1234yf | 0 à 40 | ∼1.2 à ∼10.0 |
| HF - HFO-1234yf - HFC-245cb - HCFO-1233zdE | 0 à 40 | ∼1.1 à ∼11.3 |
| HF - HFO-1234yf - HFC-245cb - HFO-1234zeE | 0 à 40 | ∼2.5 à ∼11.5 |
| HF - HFO-1234yf - HFC-245cb - HFO-1234zeZ | 0 à 40 | ∼1.3 à ∼11.3 |
| HF - HFO-1234yf - HFC-245cb - HFO-1243zf | 0 à 40 | ∼2.5 à ∼11.6 |
| HF - HCFO-1233xf - HFO-1234yf - HCFO-1233zdE | 0 à 40 | ∼1.0 à ∼11.1 |
| HF - HCFO-1233xf - HFO-1234yf - HFO-1234zeE | 0 à 40 | ∼1.2 à ∼11.3 |
| HF - HCFO-1233xf - HFO-1234yf - HFO-1234zeZ | 0 à 40 | ∼1.0 à ∼11.0 |
| HF - HCFO-1233xf - HFO-1234yf - HFO-1243zf | 0 à 40 | ∼1.3 à ∼11.3 |
| HF - HFO-1234yf - HFO-1234zeE - HFO-1234zeZ | 0 à 40 | ∼1.3 à ∼11.3 |
| HF - HFO-1234yf - HFO-1234zeE - HCFO-1233zdE | 0 à 40 | ∼1.1 à ∼11.3 |
| HF - HFO-1234yf - HFO-1234zeE - HFO-1243zf | 0 à 40 | ∼3.0 à ∼11.6 |
| HF - HFO-1234yf - HFO-1234zeZ - HCFO-1233zdE | 0 à 40 | ∼1.0 à ∼11.0 |
| HF - HFO-1234yf - HFO-1234zeZ - HFO-1243zf | 0 à 40 | ∼1.3 à ∼11.3 |
| HF - HFO-1234yf - HCFO-1233zdE - HFO-1243zf | 0 à 40 | ∼1.2 à ∼11.3 |
| HF - HFO-1234yf - HFC-245cb - Trifluoropropyne | 0 à 40 | ∼5.9 à ∼12.4 |
| HF - HFO-1234yf - HFC-245cb - HCFC-244bb | 0 à 40 | ∼1.8 à ∼7.7 |
| HF - HFO-1234yf - HFC-245cb - HFC-245fa | 0 à 40 | ∼2.7 à ∼7.7 |
| HF - HFO-1234yf - HFC-245cb - HFO-1225yeZ | 0 à 40 | ∼5.8 à ∼7.7 |
| HF - HFO-1234yf - HFC-245cb - HFO-1225zc | 0 à 40 | ∼5.8 à ∼7.7 |
| HF - HFO-1234yf - HFO-1234zeE - Trifluoropropyne | 0 à 40 | ∼5.9 à ∼12.4 |
| HF - HFO-1234yf - HFO-1234zeE - HCFC-244bb | 0 à 40 | ∼1.8 à ∼7.7 |
| HF - HFO-1234yf - HFO-1234zeE - HFC-245fa | 0 à 40 | ∼2.7 à ∼7.7 |
| HF - HFO-1234yf - HFO-1234zeE - HFO-1225yeZ | 0 à 40 | ∼5.2 à ∼7.7 |
| HF - HFO-1234yf - HFO-1234zeE - HFO-1225zc | 0 à 40 | ∼5.2à ∼7.7 |
| HF - HFO-1234yf - HFO-1225yeZ - Trifluoropropyne | 0 à 40 | ∼6.3 à ∼12.4 |
| HF - HFO-1234yf - HCFC-244bb - Trifluoropropyne | 0 à 40 | ∼1.9 à ∼12.4 |
| HF - HFO-1234yf - HCFC-244bb - HFC-245fa | 0 à 40 | ∼1.8 à ∼7.7 |
| HF-HFO-1234yf - HCFO-1233xf - HCFC-244bb | 0 à 40 | ∼0.8 à ∼11.0 |

### Exemple10: Plage de température et de pression de mélange pentanaire

| | Enveloppe de points d'ébullition | |
|---|---|---|
| Système à 5 composés | Température °C | Pression bar abs |
| HF - HCFO-1233xf - HFC-245cb - HCFO-1233zdE - HFO-1234yf | 0 à 40 | ∼1.0 à ∼11.2 |
| HF - HCFO-1233xf - HFC-245cb - HFO-1234zeE - HFO-1234yf | 0 à 40 | ∼1.0 à ∼11.4 |
| HF - HCFO-1233xf - HFC-245cb - HFO-1234zeZ - HFO-1234yf | 0 à 40 | ∼1.1 à ∼11.2 |
| HF - HCFO-1233xf - HFC-245cb - HFO-1234yf - HFO-1243zf | 0 à 40 | ∼1.2 à ∼11.4 |
| HF - HCFO-1233xf - HFO-1234yf - HFO-1234zeE - HFO-1234zeZ | 0 à 40 | ∼1.1 à ∼11.2 |
| HF - HCFO-1233xf - HFO-1234yf - HFO-1234zeE - HFO-1243zf | 0 à 40 | ∼1.2 à ∼11.4 |
| HF - HCFO-1233xf - HFO-1234yf - HFO-1234zeE - HCFO-1233zdE | 0 à 40 | ∼1.0 à ∼11.2 |
| HF - HCFO-1233xf - HFO-1234yf - HFO-1234zeZ - HFO-1243zf | 0 à 40 | ∼1.2 à ∼11.2 |
| HF - HCFO-1233xf - HFO-1234yf - HFO-1234zeZ - HCFO-1233zdE | 0 à 40 | ∼1.0∼11.1 |
| HF - HCFO-1233xf - HFO-1234yf - HCFO-1233zdE - HFO-1243zf | 0 à 40 | ∼1.0 ∼11.2 |
| HF - HFO-1234yf - HFO-1234zeE - HFO-1234zeZ - HCFO-1233zdE | 0 à 40 | ∼1.0 à ∼11.2 |
| HF - HFO-1234yf - HFO-1234zeE - HFO-1234zeZ - HFO-1243zf | 0 à 40 | ∼1.3 à ∼11.4 |
| HF - HFO-1234yf - HFO-1234zeE - HCFO-1233zdE - HFO-1243zf | 0 à 40 | ∼1.0 à ∼11.4 |
| HF - HFO-1234yf - HFO-1234zeZ - HCFO-1233zdE - HFO-1243zf | 0 à 40 | ∼1.1 à ∼11.2 |
| HF - HFO-1234yf - HFC-245cb - HFO-1234zeE - HCFO-1233zdE | 0 à 40 | ∼1.1 à ∼11.4 |
| HF - HFO-1234yf - HFC-245cb - HFO-1234zeE - HFO-1234zeZ | 0 à 40 | ∼1.3 à ∼9.1 |
| HF - HFO-1234yf - HFC-245cb - HFO-1234zeE - HFO-1243zf | 0 à 40 | ∼2.5 à ∼11.6 |
| HF - HFO-1234yf - HFC-245cb - HFO-1234zeZ - HCFO-1233zdE | 0 à 40 | ∼1.0 à ∼11.2 |
| HF - HFO-1234yf - HFC-245cb - HFO-1234zeZ- HFO-1243zf | 0 à 40 | ∼1.3 à ∼10.3 |
| HF - HFO-1234yf - HFC-245cb - HCFO-1233zdE - HFO-1243zf | 0 à 40 | ∼1.1 à ∼11.4 |
| HF - HCFO-1233xf - HFO-1234yf - HFC-245cb - HCFC-244bb | 0 à 40 | ∼0.7 à ∼11.6 |

### Exemple 11 : Plage de température et de pression de système à 6 composés

| | Enveloppe de points d'ébullition | |
|---|---|---|
| Système à 6 composés | Température °C | Pression bar abs |
| HF-HCFO-1233xf-HFO-1234yf-HFC-245cb-HFO-1234zeE-HFO-1234zeZ | 0 à 40 | ∼1.0 ∼11.6 |
| HF-HCFO-1233xf-HFO-1234yf-HFC-245cb-HFO-1234zeE-HCFO-1233zdE | 0 à 40 | ∼0.9 à ∼11.6 |
| HF-HCFO-1233xf-HFO-1234yf-HFC-245cb-HFO-1234zeE-HFO-1243zf | 0 à 40 | ∼1.0 à ∼11.6 |
| HF-HCFO-1233xf-HFO-1234yf-HFC-245cb-HFO-1234zeZ-HCFO-1233zdE | 0 à 40 | ∼0.9 à ∼11.5 |
| HF-HCFO-1233xf-HFO-1234yf-HFC-245cb-HFO-1234zeZ-HFO-1243zf | 0 à 40 | ∼1.0 à ∼11.6 |
| HF-HCFO-1233xf-HFO-1234yf-HFC-245cb-HCFO-1233zdE-HFO-1243zf | 0 à 40 | ∼0.9 à ∼11.6 |
| HF-HCFO-1233xf-HFO-1234yf-HFO-1234zeE-HFO-1234zeZ-HCFO-1233zdE | 0 à 40 | ∼0.9 à ∼11.5 |
| HF-HCFO-1233xf-HFO-1234yf-HFO-1234zeE-HFO-1234zeZ-HFO-1243zf | 0 à 40 | ∼1.0 à ∼11.6 |
| HF-HCFO-1233xf-HFO-1234yf-HFO-1234zeE-HCFO-1233zdE-HFO-1243zf | 0 à 40 | ∼0.9 à ∼11.6 |
| HF-HCFO-1233xf-HFO-1234yf-HFO-1234zeZ-HCFO-1233zdE-HFO-1243zf | 0 à 40 | ∼0.9 à ∼11.6 |
| HF-HFO-1234yf-HFC-245cb-HFO-1234zeE-HFO-1234zeZ-HCFO-1233zdE | 0 à 40 | ∼0.9 ∼11.6 |
| HF-HFO-1234yf-HFC-245cb-HFO-1234zeE-HFO-1234zeZ-HFO-1243zf | 0 à 40 | ∼1.2 à ∼11.6 |
| HF-HFO-1234yf-HFC-245cb-HFO-1234zeE-HCFO-1233zdE-HFO-1243zf | 0 à 40 | ∼1.0 à ∼11.7 |
| HF-HFO-1234yf-HFC-245cb-HFO-1234zeZ-HCFO-1233zdE-HFO-1243zf | 0 à 40 | ∼0.9 à -11.6 |
| HF-HFO-1234yf-HFO-1234zeE-HFO-1234zeZ-HCFO-1233zdE-HFO-1243zf | 0 à 40 | ∼0.9 à ∼11.6 |
| HF - HCFC-244bb- HFC-245fa - Trifluoropropyne-HFO-1225yeZ-HFO-1225zc | 0 à 40 | ∼0.7 à ∼18.0 |

### Exemple 12 : Plage de température et de pression de système à 7 composés

| | Enveloppe de points d'ébullition | |
|---|---|---|
| Système à 7 composés | Température °C | Pression bar abs |
| HF-HCFO-1233xf-HFO-1234yf-HFC-245cb-HCFO-1233zdE-HFO-1234zeE-HFO-1234zeZ | 0 à 40 | ∼0.9 ∼11.5 |
| HF-HCFO-1233xf-HFO-1234yf-HFC-245cb-HCFO-1233zdE-HFO-1234zeE-HFO-1243zf | 0 à 40 | ∼1.0 à ∼11.6 |
| HF-HCFO-1233xf-HFO-1234yf-HFC-245cb-HCFO-1233zdE-HFO-1234zeZ-HFO-1243zf | 0 à 40 | ∼0.9 à ∼11.5 |
| HF-HCFO-1233xf-HFO-1234yf-HFC-245cb-HFO-1234zeE-HFO-1234zeZ-HFO-1243zf | 0 à 40 | ∼1.1 à ∼11.6 |
| HF-HCFO-1233xf-HFO-1234yf-HCFO-1233zdE-HFO-1234zeE-HFO-1234zeZ-HFO-1243zf | 0 à 40 | ∼0.9 à ∼11.5 |
| HF-HFO-1234yf-HFC-245cb-HCFO-1233zdE-HFO-1234zeE-HFO-1234zeZ-HFO-1243zf | 0 à 40 | ∼1.0 à ∼11.6 |
| HF - HFO-1234yf - HCFC-244bb- HFC-245fa - Trifluoropropyne-HFO-1225yeZ- HFO-1225zc | 0 à 40 | ∼0.7 à ∼17.5 |
| HF - HFC-245cb - HCFC-244bb- HFC-245fa - Trifluoropropyne-HFO-1225yeZ- HFO-1225zc | 0 à 40 | ∼0.7 à ∼17.5 |

### Exemple 13 : Plage de température et de pression de système à 8 composés

| | Enveloppe de points d'ébullition | |
|---|---|---|
| Système à 8 composés | Température °C | Pression bar abs |
| HF-HCFO-1233xf-HFO-1234yf-HFC-245cb-HCFO-1233zdE-HFO-1234zeE-HFO-1234zeZ-HFO-1243zf | 0 à 40 | ∼1.0 ∼11.5 |
| HF-HFO-1234yf-HFC-245cb-HCFO-1233xf-HFO-1234zeE-HFO-1234zeZ-HCFO-1233zdE-HCFC-244bb | 0 à 40 | ∼0.7 à ∼11.5 |
| HF-HFO-1234yf-HFC-245cb-HCFO-1233xf-HFO-1234zeE-HFO-1234zeZ-HCFO-1233zdE-Trifluoropropyne | 0 à 40 | ∼1.0 à ∼17.4 |
| HF-HFO-1234yf-HFC-245cb-HCFO-1233xf-HFO-1234zeE-HFO-1234zeZ-HCFO-1233zdE-HFC-245fa | 0 à 40 | ∼0.9 à ∼11.5 |
| HF-HFO-1234yf-HFC-245cb-HCFO-1233xf-HFO-1234zeE-HFO-1234zeZ-HCFO-1233zdE-HFO-1225yeZ | 0 à 40 | ∼1.0 à ∼11.5 |
| HF-HFO-1234yf-HFC-245cb-HCFO-1233xf-HFO-1234zeE-HFO-1234zeZ-HCFC-1233zdE-HFO-1225zc | 0 à 40 | ∼1.0 à ∼11.5 |

### Exemple 14 : Plage de température et de pression de système à 13 composés

| | Enveloppe de points d'ébullition | |
|---|---|---|
| Système à 13 composés | Température °C | Pression bar abs |
| HF - HFO-1234yf - HFC-245cb - HCFO-1233xf - HCFO-1233zdE - HFO-1234zeE - HFO-1234zeZ - HFC-1243zf - HCFC-244bb - TFP - HFC-245fa - HFO-1225yeZ - HFO-1225zc | 0 à 40 | ∼0,7 ∼18.0 |

### Exemple 15 : Plages de décantation de mélanges ternaires

| | **Plages de décantation** | | |
|---|---|---|---|
| | **Pourcentage massique d'HF** | | |
| **Ternaires** | **Isotherme 0°C** | **Isotherme 25°C** | **Isotherme 40°C** |
| **HF-HFO-1234yf-HFC-245cb** | **5-80** | **10-80** | **30-80** |
| **HF-HFO-1234yf-HFO-1234zeE** | **5-65** | ***** | ***** |
| **HF-HFO-1234yf-HFO-1234zeZ** | **5-75** | **10-70** | **15-30** |
| **HF-HFO-1234yf-HFO-1243zf** | **5-70** | ***** | ***** |
| **HF-HFO-1234yf-HCFO-1233xf** | **5-75** | **5-75** | **15-50** |
| **HF-HFO-1234yf-HCFO-1233zdE** | **5-75** | **5-70** | **15-55** |
| **HF-HFO-1234yf- Trifluoropropyne** | **10-70** | **40-70** | **40-70** |
| **HF-HFO-1234yf-HFC-244bb** | **5-80** | **5-80** | **5-75** |
| **HF-HFO-1234yf-HFC-245fa** | **5-75** | **5-70** | **5-55** |
| **HF-HFO-1234yf-HFO-1225yeZ** | **5-70** | **15-65** | **60-65** |
| **HF-HFO-1234yf-HFO-1225zc** | **5-65** | ***** | ***** |

### Exemple 16 : Plages de décantation de mélanges quaternaires

| | **Plages de décantation** | | |
|---|---|---|---|
| | **Pourcentage massique d'HF** | | |
| **Quaternaire** | **Isotherme 0°C** | **Isotherme 25°C** | **Isotherme 40°C** |
| **HF - HCFO-1233xf - HFC-245cb - HFO-1234yf** | **5-75** | **5-75** | **15-70** |
| **HF - HFO-1234yf - HFC-245cb - HCFO-1233zdE** | **5-75** | **5-75** | **15-70** |
| **HF - HFO-1234yf - HFC-245cb - HFO-1234zeE** | **5-75** | **10-70** | ***** |
| **HF - HFO-1234yf - HFC-245cb - HFO-1234zeZ** | **5-75** | **10-75** | **20-70** |
| **HF - HFO-1234yf - HFC-245cb - HFO-1243zf** | **5-75** | **15-70** | **45-70** |
| **HF - HCFO-1233xf - HFO-1234yf - HCFO-1233zdE** | **5-75** | **5-70** | **10-60** |
| **HF - HCFO-1233xf - HFO-1234yf - HFO-1234zeE** | **5-70** | **10-60** | ***** |
| **HF - HCFO-1233xf - HFO-1234yf - HFO-1234zeZ** | **5-75** | **5-70** | **10-60** |
| **HF - HCFO-1233xf - HFO-1234yf - HFO-1243zf** | **5-75** | **10-65** | ***** |
| **HF - HFO-1234yf - HFO-1234zeE - HFO-1234zeZ** | **5-70** | **10-60** | ***** |
| **HF - HFO-1234yf - HFO-1234zeE - HCFO-1233zdE** | **5-70** | **10-60** | ***** |
| **HF - HFO-1234yf - HFO-1234zeE - HFO-1243zf** | **5-65** | ***** | ***** |
| **HF - HFO-1234yf - HFO-1234zeZ - HCFO-1233zdE** | **5-75** | **5-70** | **10-60** |
| **HF - HFO-1234yf - HFO-1234zeZ - HFO-1243zf** | **5-75** | **10-65** | ***** |
| **HF - HFO-1234yf - HCFO-1233zdE - HFO-1243zf** | **5-75** | **10-65** | ***** |
| **HF - HFO-1234yf - HFC-245cb - Trifluoropropyne** | **5-75** | **30-75** | **40-75** |
| **HF - HFO-1234yf - HFC-245cb - HCFC-244bb** | **5-80** | **5-80** | **10-70** |
| **HF - HFO-1234yf - HFC-245cb - HFC-245fa** | **5-80** | **5-75** | **15-70** |
| **HF - HFO-1234yf - HFC-245cb - HFO-1225yeZ** | **5-75** | **10-75** | **30-75** |
| **HF - HFO-1234yf - HFC-245cb - HFO-1225zc** | **5-75** | **10-70** | **40-70** |
| **HF - HFO-1234yf - HFO-1234zeE - Trifluoropropyne** | **10-65** | ***** | ***** |
| **HF - HFO-1234yf - HFO-1234zeE - HCFC-244bb** | **5-80** | **5-75** | **10-65** |
| **HF - HFO-1234yf - HFO-1234zeE - HFC-245fa** | **5-70** | **10-65** | ***** |
| **HF - HFO-1234yf - HFO-1234zeE - HFO-1225yeZ** | **5-70** | **20-45** | ***** |
| **HF - HFO-1234yf - HFO-1234zeE - HFO-1225zc** | **5-65** | ***** | ***** |
| **HF - HFO-1234yf - HFO-1225yeZ - Trifluoropropyne** | **5-70** | ***** | ***** |
| **HF - HFO-1234yf - HCFC-244bb - Trifluoropropyne** | **5-80** | **10-75** | **30-70** |
| **HF - HFO-1234yf - HCFC-244bb - HFC-245fa** | **5-80** | **5-80** | **5-75** |
| **HF - HCFO-1233xf - HFO-1234yf -HCFC-244bb** | **5-80** | **5-75** | **5-75** |

### Exemple 17 : Plages de décantation de mélanges pentanaires

| | **Plages de décantation** | | |
|---|---|---|---|
| | **Pourcentage massique d'HF** | | |
| **Système à 5 composés** | **Isotherme 0°C** | **Isotherme 25°C** | **Isotherme 40°C** |
| **HF - HCFO-1233xf - HFC-245cb - HCFO-1233zdE - HFO-1234yf** | **5-75** | **5-75** | **10-65** |
| **HF - HCFO-1233xf - HFC-245cb - HFO-1234zeE - HFO-1234yf** | **5-75** | **10-70** | ***** |
| **HF - HCFO-1233xf - HFC-245cb - HFO-1234zeZ - HFO-1234yf** | **5-75** | **5-75** | **10-65** |
| **HF - HCFO-1233xf - HFC-245cb - HFO-1234yf - HFO-1243zf** | **5-75** | **10-70** | ***** |
| **HF - HCFO-1233xf - HFO-1234yf - HFO-1234zeE - HFO-1234zeZ** | **5-75** | **5-65** | **15-45** |
| **HF - HCFO-1233xf - HFO-1234yf - HFO-1234zeE - HFO-1243zf** | **5-70** | **10-60** | ***** |
| **HF - HCFO-1233xf - HFO-1234yf - HFO-1234zeE - HCFO-1233zdE** | **5-75** | **5-65** | **10-50** |
| **HF - HCFO-1233xf - HFO-1234yf - HFO-1234zeZ - HFO-1243zf** | **5-75** | **10-65** | **20-40** |
| **HF - HCFO-1233xf - HFO-1234yf - HFO-1234zeZ - HCFO-1233zdE** | **5-75** | **5-70** | **10-60** |
| **HF - HCFO-1233xf - HFO-1234yf - HCFO-1233zdE - HFO-1243zf** | **5-75** | **5-65** | **15-45** |
| **HF - HFO-1234yf - HFO-1234zeE - HFO-1234zeZ - HCFO-1233zdE** | **5-75** | **5-65** | **15-45** |
| **HF - HFO-1234yf - HFO-1234zeE - HFO-1234zeZ - HFO-1243zf** | **5-70** | **10-60** | ***** |
| **HF - HFO-1234yf - HFO-1234zeE - HCFO-1233zdE - HFO-1243zf** | **5-70** | **10-60** | ***** |
| **HF - HFO-1234yf - HFO-1234zeZ - HCFO-1233zdE - HFO-1243zf** | **5-75** | **10-65** | **15-45** |
| **HF - HFO-1234yf - HFC-245cb - HFO-1234zeE - HCFO-1233zdE** | **5-75** | **10-70** | ***** |
| **HF - HFO-1234yf - HFC-245cb - HFO-1234zeE - HFO-1234zeZ** | **5-75** | **10-70** | ***** |
| **HF - HFO-1234yf - HFC-245cb - HFO-1234zeE - HFO-1243zf** | **5-75** | **15-65** | ***** |
| **HF - HFO-1234yf - HFC-245cb - HFO-1234zeZ - HCFO-1233zdE** | **5-75** | **5-75** | **10-65** |
| **HF - HFO-1234yf - HFC-245cb - HFO-1234zeZ-HFO-1243zf** | **5-75** | **10-70** | ***** |
| **HF - HFO-1234yf - HFC-245cb - HCFO-1233zdE - HFO-1243zf** | **5-75** | **10-70** | ***** |
| **HF - HCFO-1233xf - HFO-1234yf - HFC-245cb - HCFC-244bb** | **5-80** | **5-80** | **5-75** |

### Exemple 18 : Plages de décantation de système à 6 composés

| | **Plages de décantation** | | |
|---|---|---|---|
| | **Pourcentage massic ue d'HF** | | |
| **Système à 6 composés** | **Isotherme 0°C** | **Isotherme 25°C** | **Isotherme 40°C** |
| **HF-HCFO-1233xf-HFO-1234yf-HFC-245cb-HFO-1234zeE-HFO-1234zeZ** | **5-75** | **5-70** | **15-55** |
| **HF-HCFO-1233xf-HFO-1234yf-HFC-245cb-HFO-1234zeE-HCFO-1233zdE** | **5-75** | **5-70** | **15-55** |
| **HF-HCFO-1233xf-HFO-1234yf-HFC-245cb-HFO-1234zeE-HFO-1243zf** | **5-75** | **10-65** | ***** |
| **HF-HCFO-1233xf-HFO-1234yf-HFC-245cb-HFO-1234zeZ-HCFO-1233zdE** | **5-75** | **5-75** | **10-65** |
| **HF-HCFO-1233xf-HFO-1234yf-HFC-245cb-HFO-1234zeZ-HFO-1243zf** | **5-75** | **10-70** | **15-50** |
| **HF-HCFO-1233xf-HFO-1234yf-HFC-245cb-HCFO-1233zdE-HFO-1243zf** | **5-75** | **5-70** | **15-55** |
| **HF-HCFO-1233xf-HFO-1234yf-HFO-1234zeE-HFO-1234zeZ-HCFO-1233zdE** | **5-75** | **5-65** | **10-55** |
| **HF-HCFO-1233xf-HFO-1234yf-HFO-1234zeE-HFO-1234zeZ-HFO-1243zf** | **5-70** | **10-65** | ***** |
| **HF-HCFO-1233xf-HFO-1234yf-HFO-1234zeE-HCFO-1233zdE-HFO-1243zf** | **5-70** | **10-75** | ***** |
| **HF-HCFO-1233xf-HFO-1234yf-HFO-1234zeZ-HCFO-1233zdE-HFO-1243zf** | **5-75** | **5-70** | **10-55** |
| **HF-HFO-1234yf-HFC-245cb-HFO-1234zeE-HFO-1234zeZ-HCFO-1233zdE** | **5-75** | **5-70** | **15-55** |
| **HF-HFO-1234yf-HFC-245cb-HFO-1234zeE-HFO-1234zeZ-HFO-1243zf** | **5-75** | **10-65** | ***** |
| **HF-HFO-1234yf-HFC-245cb-HFO-1234zeE-HCFO-1233zdE-HFO-1243zf** | **5-75** | **10-65** | ***** |
| **HF-HFO-1234yf-HFC-245cb-HFO-1234zeZ-HCFO-1233zdE-HFO-1243zf** | **5-75** | **10-70** | **15-55** |
| **HF-HFO-1234yf-HFO-1234zeE-HFO-1234zeZ-HCFO-1233zdE-HFO-1243zf** | **5-70** | **10-65** | ***** |
| **HF - HCFC-244bb- HFC-245fa - Trifluoropropyne-HFO-1225yeZ-HFO-1225zc** | **5-75** | **10-70** | **15-60** |

### Exemple 19 : Plages de décantation de système à 7 composés

| | **Plages de décantation** | | |
|---|---|---|---|
| | **Pourcentage massique d'HF** | | |
| **Système à 7 composés** | **Température** | | |
| | **0 °C** | **25 °C** | **40 °C** |
| **HF-HCFO-1233xf-HFO-1234yf-HFC-245cb-HCFO-1233zdE-HFO-1234zeE-HFO-1234zeZ** | **5-75** | **5-70** | **10-60** |
| **HF-HCFO-1233xf-HFO-1234yf-HFC-245cb-HCFO-1233zdE-HFO-1234zeE-HFO-1243zf** | **5-75** | **10-65** | **20** |
| **HF-HCFO-1233xf-HFO-1234yf-HFC-245cb-HCFO-1233zdE-HFO-1234zeZ-HFO-1243zf** | **5-70** | **5-70** | **10-60** |
| **HF-HCFO-1233xf-HFO-1234yf-HFC-245cb-HFO-1234zeE-HFO-1234zeZ-HFO-1243zf** | **5-75** | **10-65** | ***** |
| **HF-HCFO-1233xf-HFO-1234yf-HCFO-1233zdE-HFO-1234zeE-HFO-1234zeZ-HFO-1243zf** | **5-75** | **10-65** | **15-45** |
| **HF-HFO-1234yf-HFC-245cb-HCFO-1233zdE-HFO-1234zeE-HFO-1234zeZ-HFO-1243zf** | **5-75** | **10-65** | ***** |
| **HF - HFO-1234yf - HCFC-244bb- HFC-245fa - Trifluoropropyne-HFO-1225yeZ- HFO-1225zc** | **5-75** | **5-70** | ***** |
| **HF - HFC-245cb - HCFC-244bb- HFC-245fa - Trifluoropropyne-HFO-1225yeZ-HFO-1225zc** | **5-75** | **5-75** | **15-65** |

### Exemple 20 : Plages de décantation de système à 8 composés

| | **Plages de décantation** | | |
|---|---|---|---|
| | **Pourcentage massique d'HF** | | |
| **Système à 8 composés** | **Température** | | |
| | **0 °C** | **25 °C** | **40 °C** |
| **HF-HCFO-1233xf-HFO-1234yf-HFC-245cb-HCFO-1233zdE-HFO-1234zeE-HFO-1234zeZ-HFO-1243zf** | **5-75%** | **5-70%** | **15-50%** |
| **HF-HFO-1234yf-HFC-245cb-HCFO-1233xf-HFO-1234zeE-HFO-1234zeZ-HCFO-1233zdE-HCFC-244bb** | **5-80** | **5-75** | **5-70** |
| **HF-HFO-1234yf-HFC-245cb-HCFO-1233xf-HFO-1234zeE-HFO-1234zeZ-HCFO-1233zdE- Trifluoropropyne** | **5-75** | **10-65** | ***** |
| **HF-HFO-1234yf-HFC-245cb-HCFO-1233xf-HFO-1234zeE-HFO-1234zeZ-HCFO-1233zdE-HFC-245fa** | **5-75** | **5-70** | **10-60** |
| **HF-HFO-1234yf-HFC-245cb-HCFO-1233xf-HFO-1234zeE-HFO-1234zeZ-HCFO-1233zdE-HFO-1225yeZ** | **5-75** | **5-70** | **15-55** |
| **HF-HFO-1234yf-HFC-245cb-HCFO-1233xf-HFO-1234zeE-HFO-1234zeZ-HCFC-1233zdE-HFO-1225zc** | **5-75** | **5-65** | **15-50** |

### Exemple 21 : Plages de décantation de système à 13 composés

| | **Plages de décantation** | | |
|---|---|---|---|
| | **Pourcentage massique d'HF** | | |
| **Système à 13 composés** | **Température** | | |
| | **0 °C** | **25 °C** | **40 °C** |
| **HF - HFO-1234yf - HFC-245cb - HCFO-1233xf - HCFO-1233zdE - HFO-1234zeE - HFO-1234zeZ - HFC-1243zf - HCFC-244bb - TFP - HFC-245fa - HFO-1225yeZ - HFO-1225zc** | **5-75%** | **10-70%** | **15-60%** |

### Exemple 22 : Mélanges binaires

**HF - Trifluoropropyne**

| | **Binary HF - Trifluoropropyne** | | |
|---|---|---|---|
| | Isotherm 0°C | Isotherm 25°C | Isotherm 40°C |
| HF Massfrac | Pressure | Pressure | Pressure |
| | bar | bar | bar |
| 0 | 5,9 | 11,7 | 16,7 |
| 0,05 | 6,2 | 12,4 | 18,0 |
| 0,1 | 6,1 | 12,3 | 17,8 |
| 0,15 | 6,1 | 12,2 | 17,6 |
| 0,2 | 6,1 | 12,1 | 17,3 |
| 0,25 | 6,1 | 12,0 | 17,1 |
| 0,3 | 6,1 | 11,9 | 16,8 |
| 0,35 | 6,1 | 11,8 | 16,6 |
| 0,4 | 6,1 | 11,8 | 16,6 |
| 0,45 | 6,1 | 11,8 | 16,6 |
| 0,5 | 6,1 | 11,8 | 16,6 |
| 0,55 | 6,1 | 11,8 | 16,6 |
| 0,6 | 6,1 | 11,8 | 16,6 |
| 0,65 | 6,1 | 11,8 | 16,6 |
| 0,7 | 6,1 | 11,8 | 16,6 |
| 0,75 | 6,0 | 11,8 | 16,6 |
| 0,8 | 5,7 | 11,6 | 16,6 |
| 0,85 | 5,3 | 10,8 | 15,7 |
| 0,9 | 4,4 | 9,2 | 13,4 |
| 0,95 | 2,9 | 6,1 | 9,0 |
| 1 | 0,5 | 1,2 | 1,9 |

**HF-F244bb**

| | **Binary HF** - **F244bb** | | |
|---|---|---|---|
| | Isotherm 0°C | Isotherm 25°C | Isotherm 40°C |
| HF Massfrac | Pressure | Pressure | Pressure |
| | bar | bar | bar |
| 0 | 0,2 | 0,6 | 1,1 |
| 0,05 | 0,7 | 1,8 | 3,0 |
| 0,1 | 0,7 | 1,8 | 3,0 |
| 0,15 | 0,7 | 1,8 | 3,0 |
| 0,2 | 0,7 | 1,8 | 3,0 |
| 0,25 | 0,7 | 1,8 | 3,0 |
| 0,3 | 0,7 | 1,8 | 3,0 |
| 0,35 | 0,7 | 1,8 | 3,0 |
| 0,4 | 0,7 | 1,8 | 3,0 |
| 0,45 | 0,7 | 1,8 | 3,0 |
| 0,5 | 0,7 | 1,8 | 3,0 |
| 0,55 | 0,7 | 1,8 | 3,0 |
| 0,6 | 0,7 | 1,8 | 3,0 |
| 0,65 | 0,7 | 1,8 | 3,0 |
| 0,7 | 0,7 | 1,8 | 3,0 |
| 0,75 | 0,7 | 1,8 | 3,0 |
| 0,8 | 0,7 | 1,8 | 3,0 |
| 0,85 | 0,7 | 1,8 | 3,0 |
| 0,9 | 0,7 | 1,7 | 2,9 |
| 0,95 | 0,6 | 1,5 | 2,5 |
| 1 | 0,5 | 1,2 | 1,9 |

**HF - F245fa**

| | **Binary HF - F245fa** | | |
|---|---|---|---|
| | Isotherm 0°C | Isotherm 25°C | Isotherm 40°C |
| HFMassfrac | Pressure | Pressure | Pressure |
| | bar | bar | bar |
| 0 | 0,5 | 1,5 | 2,5 |
| 0,05 | 1,0 | 2,6 | 4,4 |
| 0,1 | 1,0 | 2,6 | 4,4 |
| 0,15 | 1,0 | 2,6 | 4,4 |
| 0,2 | 1,0 | 2,6 | 4,4 |
| 0,25 | 1,0 | 2,6 | 4,4 |
| 0,3 | 1,0 | 2,6 | 4,4 |
| 0,35 | 1,0 | 2,6 | 4,4 |
| 0,4 | 1,0 | 2,6 | 4,4 |
| 0,45 | 1,0 | 2,6 | 4,4 |
| 0,5 | 1,0 | 2,6 | 4,4 |
| 0,55 | 1,0 | 2,6 | 4,4 |
| 0,6 | 1,0 | 2,6 | 4,4 |
| 0,65 | 1,0 | 2,6 | 4,4 |
| 0,7 | 1,0 | 2,6 | 4,4 |
| 0,75 | 1,0 | 2,6 | 4,4 |
| 0,8 | 1,0 | 2,6 | 4,2 |
| 0,85 | 0,9 | 2,4 | 3,9 |
| 0,9 | 0,8 | 2,1 | 3,5 |
| 0,95 | 0,7 | 1,7 | 2,9 |
| 1 | 0,5 | 1,2 | 1,9 |

### Exemple 23 : Plage de température et de pression de mélanges binaires

| | Enveloppe de points d'ébullition | |
|---|---|---|
| Binaire | Température °C | Pression bar abs |
| HF-Trifluoropropyne | 0 à 40 | ∼6.0 à ∼18.0 |
| HF - F244bb | 0 à 40 | ∼0.7 à ∼3.0 |
| HF - F245fa | 0 à 40 | ∼1.0 à -4.4 |

### Exemple 24 : Plages de décantation de mélanges binaires

| | **Plages de décantation Pourcentage massique d'HF** | | |
|---|---|---|---|
| **Binaire** | **Température** | | |
| | 0°C | 25°C | **40°C** |
| **HF- Trifluoropropyne** | **20-65** | **40-75** | **40-80** |
| **HF - F244bb** | **5-85** | **5-85** | **5-85** |
| **HF - F245fa** | **5-80** | **5-75** | **5-70** |

## Revendications

1. Composition hétéro-azéotropique ou quasi-hétéro-azéotropique comprenant du fluorure d'hydrogène, du 2,3,3,3-tetrafluoropropene , et un ou plusieurs composé(s) (hydro)halogénocarboné(s) comprenant entre 1 et 3 atome(s) de carbone choisi(s) parmi le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le 1,1,1,3,3-pentafluoropropane, le 2-chloro, 1,1,1,2-tetrafluoropropane, le trifluoropropyne, le Z-1,1,1,2,3-pentafluoropropène et le 1,1,1,3,3-pentafluoropropène.

2. Composition selon la revendication 1 **caractérisée en ce qu'**elle comprend du fluorure d'hydrogène, du 2,3,3,3-tetrafluoropropene , et un ou plusieurs composé(s) organique(s) choisi(s) parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le 1,1,1,3,3-pentafluoropropane, le 2-chloro, 1,1,1,2-tetrafluoropropane, le trifluoropropyne, le Z-1,1,1,2,3-pentafluoropropène et le 1,1,1,3,3-pentafluoropropène.

3. Composition selon l'une quelconque des revendications précédentes **caractérisée en ce qu'**elle comprend du fluorure d'hydrogène, du 2,3,3,3-tetrafluoropropene , et un ou plusieurs composé(s) organique(s) choisi(s) parmi le 1,1,1,2,2-pentafluoropropane, le E-1,3,3,3-tetrafluoropropene, le Z-1,3,3,3-tetrafluoropropene, le 3,3,3-trifluoropropene, le 3,3,3-trifluoro-2-chloropropene et le E-3, 3, 3- trifluoro-1-chloropropene.

4. Composition selon l'une quelconque des revendications précédentes **caractérisée en ce qu'**elle comprend du fluorure d'hydrogène, du 2,3,3,3-tetrafluoropropene, du 1,1,1,2,2-pentafluoropropane et au moins un composé choisi parmi le E-1,3,3,3-tetrafluoropropene, le Z-1,3,3,3-tetrafluoropropene, le 3,3,3-trifluoropropene, le 3,3,3-trifluoro-2-chloropropene, le E-3,3,3-trifluoro-1-chloropropene, le 1,1,1,3,3-pentafluoropropane, le 2-chloro,1,1,1,2-tetrafluoropropane, le trifluoropropyne, le Z-1,1,1,2,3-pentafluoropropène et le 1,1,1,3,3-pentafluoropropène.

5. Composition selon l'une quelconque des revendications précédentes **caractérisée en ce qu'**elle comprend du fluorure d'hydrogène, du 2,3,3,3-tetrafluoropropene, du E-1,3,3,3-tetrafluoropropene et éventuellement au moins un composé choisi parmi le Z-1,3,3,3-tetrafluoropropene, le 3,3,3-trifluoropropene, le 3,3,3-trifluoro-2-chloropropene, le E-3,3,3-trifluoro-1-chloropropene, le 1,1,1,3,3-pentafluoropropane, le 2-chloro,1,1,1,2-tetrafluoropropane, le trifluoropropyne, le Z-1,1,1,2,3-pentafluoropropène et le 1,1,1,3,3-pentafluoropropène.

6. Composition selon l'une quelconque des revendications précédentes **caractérisée en ce qu'**elle comprend du fluorure d'hydrogène, du 2,3,3,3-tetrafluoropropene, du Z-1,3,3,3-tetrafluoropropene et éventuellement au moins un composé choisi parmi le 3,3,3-trifluoropropene, le 3,3,3-trifluoro-2-chloropropene, 1,1,1,3,3-pentafluoropropane et le E-3,3,3-trifluoro-1-chloropropene.

7. Composition selon l'une quelconque des revendications précédentes **caractérisée en ce qu'**elle comprend du fluorure d'hydrogène, du 2,3,3,3-tetrafluoropropene, du 3,3,3-trifluoropropene et éventuellement au moins un composé choisi parmi le 3,3,3-trifluoro-2-chloropropene et le E-3,3,3-trifluoro-1-chloropropene.

8. Composition selon l'une quelconque des revendications précédentes **caractérisée en ce qu'**elle comprend du fluorure d'hydrogène, du 2,3,3,3-tetrafluoropropene, du 3,3,3-trifluoro-2-chloropropene et éventuellement le E-3,3,3-trifluoro-1-chloropropene.

9. Composition selon l'une quelconque des revendications précédentes **caractérisée en ce qu'**elle comprend du fluorure d'hydrogène, du 2,3,3,3-tetrafluoropropene et du E-3,3,3-trifluoro-1-chloropropene.

10. Composition selon l'une quelconque des revendications précédentes dans laquelle la composition comprend de 1 à 85 % en poids de fluorure d'hydrogène et de 99 à 15 % en poids de la somme des composés organiques.

11. Composition selon l'une quelconque des revendications précédentes dans laquelle la composition comprend de 5 à 80 % en poids de fluorure d'hydrogène et de 95 à 20 % en poids de la somme des composés organiques.

12. Composition selon l'une quelconque des revendications précédentes dans laquelle le point d'ébullition de la dite composition est compris entre -20 °C et 80 °C, et à une pression comprise entre 0,1 et 44 bars absolue, préférentiellement compris entre 0°C et 40 °C et préférentiellement à une pression comprise entre 0,7 et 18 bars absolue, avantageusement entre 0,9 et 12,5 bars absolue.

## Patentansprüche

1. Heteroazeotrope oder quasiheteroazeotrope Zusammensetzung, umfassend Fluorwasserstoff, 2,3,3,3-Tetrafluorpropen und eine oder mehrere (Hydro)-halogenkohlenstoffverbindungen mit zwischen 1 und 3 Kohlenstoffatomen, die aus E-1,3,3,3-Tetrafluorpropen, Z-1,3,3,3-Tetrafluorpropen, 3,3,3-Trifluorpropen, 3,3,3-Trifluor-2-chlorpropen, E-3, 3,3-Trifluor-1-chlorpropen, 1, 1, 1, 3, 3-Pentafluorpropan, 2-Chlor-1,1,1,2-tetrafluorpropan, Trifluorpropin, Z-1,1,1,2,3-Pentafluorpropen und 1,1,1,3,3-Pentafluorpropen ausgewählt sind.

2. Zusammensetzung nach Anspruch 1, **dadurch gekennzeichnet, dass** sie Fluorwasserstoff, 2,3,3,3-Tetrafluorpropen und eine oder mehrere organische Verbindungen, die aus 1,1,1,2,2-Pentafluorpropan, E-1,3,3,3-Tetrafluorpropen, Z-1,3,3,3-Tetrafluorpropen, 3,3,3-Trifluorpropen, 3,3,3-Trifluor-2-chlorpropen, E-3,3,3-Trifluor-1-chlorpropen, 1,1,1,3,3-Pentafluorpropan, 2-Chlor-1,1,1,2-tetra-fluorpropan, Trifluorpropin, Z-1,1,1,2,3-Pentafluorpropen und 1,1,1,3,3-Pentafluorpropen ausgewählt sind, umfasst.

3. Zusammensetzung nach einem der vorhergehenden Ansprüche, **dadurch gekennzeichnet, dass** sie Fluorwasserstoff, 2,3,3,3-Tetrafluorpropen und eine oder mehrere organische Verbindungen, die aus 1,1,1,2,2-Pentafluorpropan, E-1,3,3,3-Tetrafluorpropen, Z-1,3,3,3-Tetrafluorpropen, 3,3,3-Trifluorpropen, 3,3,3-Trifluor-2-chlorpropen und E-3,3,3-Trifluor-1-chlorpropen ausgewählt sind, umfasst.

4. Zusammensetzung nach einem der vorhergehenden Ansprüche, **dadurch gekennzeichnet, dass** sie Fluorwasserstoff, 2,3,3,3-Tetrafluorpropen, 1,1,1,2,2-Pentafluorpropan und mindestens eine Verbindung, die aus E-1,3,3,3-Tetrafluorpropen, Z-1,3,3,3-Tetrafluorpropen, 3,3,3-Trifluorpropen, 3,3,3-Trifluor-2-chlorpropen, E-3,3,3-Trifluor-1-chlorpropen, 1,1,1,3,3-Pentafluorpropan, 2-Chlor-1,1,1,2-tetrafluorpropan, Trifluorpropin, Z-1,1,1,2,3-Pentafluorpropen und 1,1,1,3,3-Pentafluorpropen ausgewählt ist, umfasst.

5. Zusammensetzung nach einem der vorhergehenden Ansprüche, **dadurch gekennzeichnet, dass** sie Fluorwasserstoff, 2,3,3,3-Tetrafluorpropen, E-1,3,3,3-Tetrafluorpropen und gegebenenfalls mindestens eine Verbindung, die aus Z-1,3,3,3-Tetrafluorpropen, 3,3,3-Trifluorpropen, 3,3,3-Trifluor-2-chlorpropen, E-3,3,3-Trifluor-1-chlorpropen, 1,1,1,3,3-Pentafluorpropan, 2-Chlor-1,1,1,2-tetrafluorpropan, Trifluorpropin, Z-1,1,1,2,3-Pentafluorpropen und 1,1,1,3,3-Pentafluorpropen ausgewählt ist, umfasst.

6. Zusammensetzung nach einem der vorhergehenden Ansprüche, **dadurch gekennzeichnet, dass** sie Fluorwasserstoff, 2,3,3,3-Tetrafluorpropen, Z-1,3,3,3-Tetrafluorpropen und gegebenenfalls mindestens eine Verbindung, die aus 3,3,3-Trifluorpropen, 3,3,3-Trifluor-2-chlorpropen, 1,1,1,3,3-Pentafluorpropan und E-3,3,3-Trifluor-1-chlorpropen ausgewählt ist, umfasst.

7. Zusammensetzung nach einem der vorhergehenden Ansprüche, **dadurch gekennzeichnet, dass** sie Fluorwasserstoff, 2,3,3,3-Tetrafluorpropen, 3,3,3-Trifluorpropen und gegebenenfalls mindestens eine Verbindung, die aus 3,3,3-Trifluor-2-chlorpropen und E-3,3,3-Trifluor-1-chlorpropen ausgewählt ist, umfasst.

8. Zusammensetzung nach einem der vorhergehenden Ansprüche, **dadurch gekennzeichnet, dass** sie Fluorwasserstoff, 2,3,3,3-Tetrafluorpropen, 3,3,3-Trifluorpropen, 3,3,3-Trifluor-2-chlorpropen und gegebenenfalls E-3,3,3-Trifluor-1-chlorpropen umfasst.

9. Zusammensetzung nach einem der vorhergehenden Ansprüche, **dadurch gekennzeichnet, dass** sie Fluorwasserstoff, 2,3,3,3-Tetrafluorpropen und E-3,3,3-Trifluor-1-chlorpropen umfasst.

10. Zusammensetzung nach einem der vorhergehenden Ansprüche, wobei die Zusammensetzung 1 bis 85 Gew.-% Fluorwasserstoff und 99 bis 15 Gew.-% der Summe der organischen Verbindungen umfasst.

11. Zusammensetzung nach einem der vorhergehenden Ansprüche, wobei die Zusammensetzung 5 bis 80 Gew.-% Fluorwasserstoff und 95 bis 20 Gew.-% der Summe der organischen Verbindungen umfasst.

12. Zusammensetzung nach einem der vorhergehenden Ansprüche, wobei der Siedepunkt der Zusammensetzung zwischen -20 °C und 80 °C und bei einem Druck zwischen 0,1 und 44 bar absolut vorzugsweise zwischen 0°C und 40°C und vorzugsweise bei einem Druck zwischen 0,7 und 18 bar absolut, vorteilhafterweise zwischen 0,9 und 12,5 bar absolut, liegt.

## Claims

1. Heteroazeotropic or quasi-heteroazeotropic composition comprising hydrogen fluoride, 2,3,3,3-tetrafluoropropene, and one or more (hydro)halocarbon compounds comprising between 1 and 3 carbon atoms, chosen from E-1,3,3,3-tetrafluoropropene, Z-1,3,3,3-tetrafluoropropene, 3,3,3-trifluoropropene, 3,3,3-trifluoro-2-chloropropene, E-3,3,3-trifluoro-1-chloropropene, 1,1,1,3,3-pentafluoropropane, 2-chloro,1,1,1,2-tetrafluoropropane, trifluoropropyne, Z-1,1,1,2,3-pentafluoropropene and 1,1,1,3,3-pentafluoropropene.

2. Composition according to Claim 1,**characterized in that** it comprises hydrogen fluoride, 2,3,3,3-tetrafluoropropene and one or more organic compounds chosen from 1,1,1,2,2-pentafluoropropane, E-1,3,3,3-tetrafluoropropene, Z-1,3,3,3-tetrafluoropropene, 3,3,3-trifluoropropene, 3,3,3-trifluoro-2-chloropropene, E-3,3,3-trifluoro-1-chloropropene, 1,1,1,3,3-pentafluoropropane, 2-chloro,1,1,1,2-tetrafluoropropane, trifluoropropyne, Z-1,1,1,2,3-pentafluoropropene and 1,1,1,3,3-pentafluoropropene.

3. Composition according to any one of the preceding claims, **characterized in that** it comprises hydrogen fluoride, 2,3,3,3-tetrafluoropropene, and one or more organic compounds chosen from 1,1,1,2,2-pentafluoropropane, E-1,3,3,3-tetrafluoropropene, Z-1, 3, 3,3-tetrafluoropropene, 3,3,3-trifluoropropene, 3,3,3-trifluoro-2-chloropropene and E-3,3,3-trifluoro-1-chloropropene.

4. Composition according to any one of the preceding claims, **characterized in that** it comprises hydrogen fluoride, 2,3,3,3-tetrafluoropropene, 1,1,1,2,2-pentafluoropropane and at least one compound chosen from E-1,3,3,3-tetrafluoropropene, Z-1,3,3,3-tetrafluoropropene, 3,3,3-trifluoropropene, 3,3,3-trifluoro-2-chloropropene, E-3,3,3-trifluoro-1-chloropropene, 1,1,1,3,3-pentafluoropropane, 2-chloro,1,1,1,2-tetrafluoropropane, trifluoropropyne, Z-1,1,1,2,3-pentafluoropropene and 1,1,1,3,3-pentafluoropropene.

5. Composition according any one of the preceding claims, **characterized in that** it comprises hydrogen fluoride, 2,3,3,3-tetrafluoropropene, E-1,3,3,3-tetrafluoropropene and optionally at least one compound chosen from Z-1,3,3,3-tetrafluoropropene, 3,3,3-trifluoropropene, 3,3,3-trifluoro-2-chloropropene, E-3,3,3-trifluoro-1-chloropropene, 1,1,1,3,3-pentafluoropropane, 2-chloro,1,1,1,2-tetrafluoropropane, trifluoropropyne, Z-1,1,1,2,3-pentafluoropropene and 1,1,1,3,3-pentafluoropropene.

6. Composition according to any one of the preceding claims, **characterized in that** it comprises hydrogen fluoride, 2,3,3,3-tetrafluoropropene, Z-1,3,3,3-tetrafluoropropene and optionally at least one compound chosen from 3,3,3-trifluoropropene, 3,3,3-trifluoro-2-chloropropene, 1,1,1,3,3-pentafluoropropane and E-3,3,3-trifluoro-1-chloropropene.

7. Composition according to any one of the preceding claims, **characterized in that** it comprises hydrogen fluoride, 2,3,3,3-tetrafluoropropene, 3,3,3-trifluoropropene and optionally at least one compound chosen from 3,3,3-trifluoro-2-chloropropene and E-3,3,3-trifluoro-1-chloropropene.

8. Composition according to any one of the preceding claims, **characterized in that** it comprises hydrogen fluoride, 2,3,3,3-tetrafluoropropene, 3,3,3-trifluoro-2-chloropropene and optionally E-3,3,3-trifluoro-1-chloropropene.

9. Composition according to any one of the preceding claims, **characterized in that** it comprises hydrogen fluoride, 2,3,3,3-tetrafluoropropene and E-3,3,3-trifluoro-1-chloropropene.

10. Composition according to any one of the preceding claims, in which the composition comprises from 1% to 85% by weight of hydrogen fluoride and from 99% to 15% by weight of the sum of the organic compounds.

11. Composition according to any one of the preceding claims, in which the composition comprises from 5% to 80% by weight of hydrogen fluoride and from 95% to 20% by weight of the sum of the organic compounds.

12. Composition according to any one of the preceding claims, in which the boiling point of said composition is between -20°C and 80°C, and at a pressure of between 0.1 and 44 bar absolute, preferentially between 0°C and 40°C and preferentially at a pressure of between 0.7 and 18 bar absolute, advantageously between 0.9 and 12.5 bar absolute.
